# EUROPEAN PATENT APPLICATION

(11) **EP 4 520 345 A1**
(43) Date of publication of application: **12.03.2025**
(21) Application number: 23195733.3
(22) Date of filing: 06.09.2023
(51) Int. Cl.: A61K 39/00

(54) **PRODUCT**

(71) Applicant: MYNEO NV, 9000 Gent (BE)
(72) Inventor: The designation of the inventor has not yet been filed
(74) Representative: J A Kemp LLP

(57) **Abstract**

The present invention relates to novel polypeptides, polynucleotides encoding said polypeptides, and compositions comprising said polypeptides or polynucleotides. The invention also concerns uses of said polypeptides, polynucleotides and compositions for the treatment, prevention, and diagnosis of cancer, particularly colorectal cancer.

## Description

### Field of the Invention

The present invention relates to novel polypeptides, polynucleotides encoding said polypeptides, and compositions comprising said polypeptides or polynucleotides. The invention also concerns uses of said polypeptides, polynucleotides and compositions for the treatment, prevention, and diagnosis of cancer, particularly colorectal cancer.

### Background of the Invention

Tumour-associated antigens (TAAs) have been proposed as a possible source of neoantigens for cancer therapies. However, clinical trials have shown significant adverse events when administering TAA-based neoantigens, such as neurological and respiratory toxicity associated with *anti-MAGE-A3* TCR gene and *CEACAM5*-specific CAR T-cell therapy, causing TAA-based cancer therapeutics to be abandoned. The present invention reveals a potential new class of molecules to further expand the number of public neoantigens and to enlarge the pool of potential off-the-shelf antigen-targeting therapeutics for colorectal cancer. These novel molecules are expressed from large non-coding regions of the genome, such as from Long non-coding RNAs (IncRNAs).

### Summary of the Invention

The inventors have established the existence of a cohort of IncRNAs (which may be referred to herein as camyoRNAs) that are specifically expressed in cancer tissue, in particular colorectal cancer tissue. The inventors have also identified smORFs (small open reading frames) comprised within said lncRNAs that are translated into proteins (which may be referred to herein as 'camyopeptides') in cancer tissue, in particular colorectal cancer tissue. Said translated proteins are also described for the first time herein as being processed in cells and presented by the major histocompatibility complex (MHC) in the form of immunogenic peptide fragments. These peptides may be referred to herein as `camyotopes'. The cancer-specific lncRNAs, and polypeptides derived therefrom, represent powerful candidates for the treatment and diagnosis of colorectal cancer, with no or very limited off-target effects.

In contrast to tumour-specific antigens (TSA), camyopeptides and camyotopes are not restricted to the occurrence of recurrent mutations within a host gene. Due to the tumour-specific expression profiles of the camyoRNAs from which they derive, any immunogenic peptide derived from the translated smORFs can be targeted as a TAA (tumour-associated antigen) in colorectal cancer therapies, thus significantly enlarging the pool of potential neoantigens for targeting via immunotherapy. They can also be used as biomarkers for the diagnosis of colorectal cancer. In addition, the workflow to verify the presence of camyoRNA-derived TAAs is faster and less complex as it involves a straightforward RNA sequencing-based differential expression analysis of the camyoRNA in normal and colorectal cancer cohorts, making variant calling obsolete. Using camyoRNA-based TAAs could increase the efficiency of "off-the-shelf' therapeutics (i.e. immunotherapeutics generally suitable for cancer patients, as opposed to immunotherapeutics tailored to a specific patient having a specific antigen variant) by increasing both the population coverage and the number of targetable antigens per patient. The camyoRNAs, camyopeptides and camyotopes described herein are specific to colorectal cancer with expression profiles similar to well-known protein-coding TAAs in the art, and are therefore expected to have similar therapeutic vaccine target potential.

Off-the-shelf vaccines targeting camyoRNAs, camyopeptides and camyotopes such as those described herein may offer several advantages. The per-patient development cost is significantly lower in comparison to personalized vaccines since they only need to be produced once, and patient selection can be performed in a fast and cost-efficient way by means of for example straightforward multiplex quantitative PCR (qPCR) analyses. In addition, the tumorspecific nature of camyoRNAs could be correlated to a role in tumor initiation and progression. This, combined with the multiplicity of camyoRNAs present in any given patient should reduce the chance of immune evasion when targeting them as part of a vaccine.

In summary, the inventors have discovered that a cohort of lncRNAs are overexpressed in colorectal cancer cells relative to healthy cells. Polypeptides encoded by said lncRNAs represent tumour neoantigens that are determined to be presented by MHC and are immunogenic. Accordingly, the inventors have advantageously determined that said polypeptides can be utilized in a therapeutic setting for the purposes of inducing an anti-tumour immune response in colorectal cancer, for example via formulation as, and administration of, a cancer vaccine. The present invention therefore relates to novel polypeptides which are capable of eliciting an anti-tumour immune response in a subject. The same cohort of lncRNAs, and polypeptides encoded by them, may also be useful as markers for the diagnosis of colorectal cancer in a subject.

The present invention provides a polypeptide encoded by an open reading frame in a long non-coding RNA gene (lncRNA), wherein the lncRNA is overexpressed in colorectal cancer cells relative to healthy cells, preferably wherein the lncRNA is defined according to any one of SEQ ID NOs 1 to 18 (which sequences are also provided by the invention and may be referred to herein as exemplary 'camyoRNAs' associated with colorectal cancer). The said polypeptide may comprise a 6-amino acid sequence, 7-amino acid sequence, or preferably an 8-amino acid sequence, or variant thereof, comprised within any one of the polypeptide sequences defined according to SEQ ID NOs 19 to 97 (which sequences may be referred to herein as exemplary 'camyopeptides' associated with colorectal cancer). The said polypeptide may comprise or consist of any one of the polypeptide sequences defined according to SEQ ID NOs 98 to 611 (which sequences may be referred to herein as exemplary 'camyotopes' associated with colorectal cancer - this term indicating that they are immunogenic epitopes of the camyopeptides).

The present invention also provides:
- a polynucleotide encoding one or more said polypeptides, and a vector and/or a cell comprising said polynucleotide;
- a composition comprising any said polypeptide, polynucleotide, cell or vector, and optionally any one or more of a pharmaceutically acceptable carrier, preservative or diluent;
- a method of treating colorectal cancer in an individual, comprising administering to the individual said polypeptide, polynucleotide, cell, vector or composition;

### Brief Description of the Figures

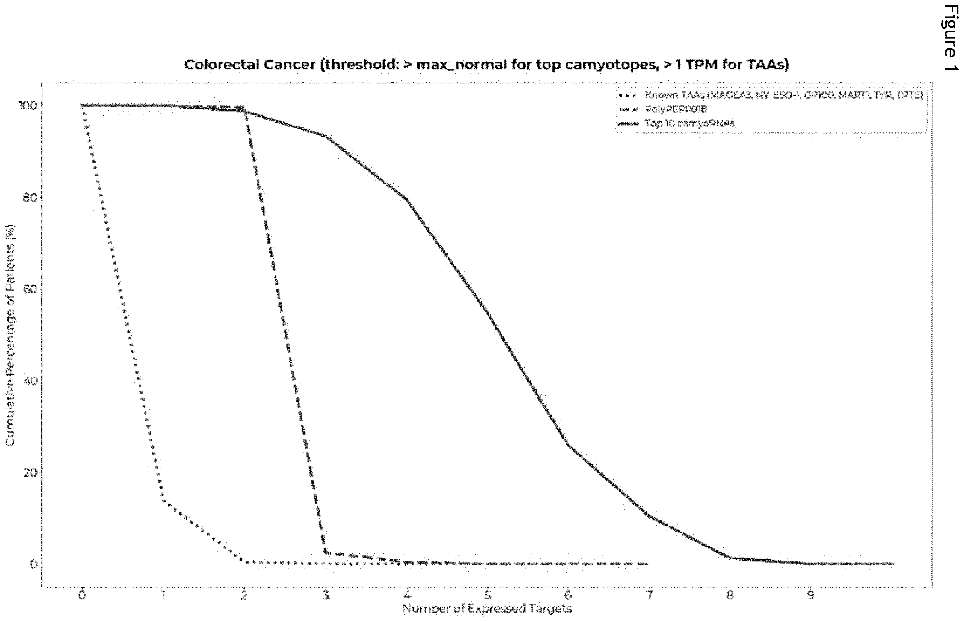

**FIGURE 1** Cumulative distribution of the 10 highest expressed camyoRNAs in CRC (bold line), of 6 known TAAs (dotted line), and of TAAs included in the known-in-the-art PolyPEPI1018 multi-target vaccine (dashed line). The plots visualise the percentage of the cancer samples that expresses a number of targets. For example, in 50% of the cancer samples, 5 or more camyoRNAs are expressed.

### Brief Description of the Sequences

SEQ ID NOs: 1-18 are polynucleotide sequences defining 18 lncRNAs, which sequences may be referred to herein as exemplary 'camyoRNAs' associated with colorectal cancer.

SEQ ID NOs: 19-97 are polypeptide sequences which are translated from open reading frames within the 18 lncRNAs of SEQ ID NOs 1-18, which polypeptide sequences may be referred to herein as exemplary 'camyopeptides' associated with colorectal cancer.

SEQ ID NOs: 98-611 are polypeptide sequences derived from the larger polypeptide sequences defined according to SEQ ID NOs 19-97, which sequences may be referred to herein as exemplary `camyotopes' associated with colorectal cancer.

### Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting.

In addition, as used in this specification and the appended claims, the singular forms "a", "an", and "the" include plural referents unless the content clearly dictates otherwise. Thus, for example, reference to "a polypeptide" includes "polypeptides", and the like.

The terms "polypeptide", and "peptide" are interchangeably used herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers. Polypeptides can also undergo maturation or post-translational modification processes that may include, but are not limited to: glycosylation, proteolytic cleavage, lipidization, signal peptide cleavage, propeptide cleavage, phosphorylation, and such like. A peptide can be made using recombinant techniques, e.g., through the expression of a recombinant or synthetic polynucleotide. A recombinantly produced peptide is typically substantially free of culture medium, e.g., culture medium represents less than about 20 %, more preferably less than about 10%, and most preferably less than about 5 % of the volume of the protein preparation.

The term "amino acid" in the context of the present disclosure is used in its broadest sense and is meant to include organic compounds containing amine (NH₂) and carboxyl (COOH) functional groups, along with a side chain (e.g., an R group) specific to each amino acid. In some embodiments, the amino acids refer to naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term "amino acid" further includes D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the definition of amino acid. Such analogues and mimetics are referred to herein as "functional equivalents" of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983, which is incorporated herein by reference.

The term "protein" is used to describe a folded polypeptide having a secondary or tertiary structure. The protein may be composed of a single polypeptide, or may comprise multiple polypeptides that are assembled to form a multimer. The multimer may be a homo-oligomer, or a hetero-oligomer. The protein may be a naturally occurring, or wild type protein, or a modified, or non-naturally occurring protein. The protein may, for example, differ from a wild type protein by the addition, substitution or deletion of one or more amino acids.

A "variant" of a protein encompasses peptides, oligopeptides, polypeptides, proteins and enzymes having amino acid substitutions, deletions and/or insertions relative to the unmodified or wild-type protein in question and having similar biological and functional activity as the unmodified protein from which they are derived. The term "amino acid identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

For all aspects and embodiments of the present invention, a polypeptide "variant" has at least 50%, 60%, 70%, 80%, 90%, 95% or 99% complete sequence identity to the specified corresponding amino acid sequence. Sequence identity can also be to a fragment or portion of the full length polynucleotide or polypeptide. Hence, a sequence may have only 50 % overall sequence identity with a full length reference sequence, but a sequence of a particular region, domain or subunit could share 80 %, 90 %, or as much as 99 % sequence identity with the reference sequence.

The term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified", "mutant" or "variant" refers to a gene or gene product that displays modifications in sequence (e.g., substitutions, truncations, or insertions), post-translational modifications and/or functional properties (e.g., altered characteristics) when compared to the wild-type gene or gene product.

Methods for introducing or substituting naturally-occurring amino acids are well known in the art. For instance, methionine (M) may be substituted with arginine (R) by replacing the codon for methionine (ATG) with a codon for arginine (CGT) at the relevant position in a polynucleotide encoding the mutant monomer. Methods for introducing or substituting non-naturally-occurring amino acids are also well known in the art. For instance, non-naturally-occurring amino acids may be introduced by including synthetic aminoacyl-tRNAs in the IVTT system used to express the mutant monomer. Alternatively, they may be introduced by expressing the mutant monomer in *E. coli* that are auxotrophic for specific amino acids in the presence of synthetic (i.e., non-naturally occurring) analogues of those specific amino acids. They may also be produced by naked ligation if the mutant monomer is produced using partial peptide synthesis. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2 - Hydropathy scale**

| Side Chain | | Hydropathy | |
|---|---|---|---|
| | Ile | | 4.5 |
| | Val | | 4.2 |
| | Leu | | 3.8 |
| | Phe | | 2.8 |
| | Cys | | 2.5 |
| | Met | | 1.9 |
| | Ala | | 1.8 |
| | Gly | | -0.4 |
| | Thr | | -0.7 |
| | Ser | | -0.8 |
| | Trp | | -0.9 |
| | Tyr | | -1.3 |
| | Pro | | -1.6 |
| | His | | -3.2 |
| | Glu | | -3.5 |
| | Gln | | -3.5 |
| | Asp | | -3.5 |
| | Asn | | -3.5 |
| | Lys | | -3.9 |
| | Arg | | -4.5 |

Unless otherwise indicated, nucleic acid sequences herein are written in the 5'-to-3' direction from left to right.

"Polynucleotide", "nucleotide sequence", "DNA sequence", or "nucleic acid molecule(s)" as used herein refers to a polymeric form of nucleotides of any length, which comprises ribonucleotides or deoxyribonucleotides. This term refers only to the primary structure of the molecule. Thus, this term includes double- and single-stranded DNA, and RNA. The term "polynucleotide" as used herein, may be a single or double stranded covalently linked sequence of nucleotides in which the 3' and 5' ends on each nucleotide are joined by phosphodiester bonds. The polynucleotide may be made up of deoxyribonucleotide bases or ribonucleotide bases. Polynucleotides may be manufactured synthetically in vitro or isolated from natural sources. Polynucleotides may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Polynucleotides may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA).

The terms "patient" and "subject" are used interchangeably and typically refer to a mammal, preferably a human.

All publications, patents and patent applications cited herein, whether supra or infra, are hereby incorporated by reference in their entirety.

### Polypeptide

The invention provides a polypeptide encoded by an open reading frame in a long non-coding RNA gene (lncRNA), wherein the lncRNA is overexpressed in colorectal cancer cells relative to healthy cells, preferably wherein the lncRNA is defined according to any one of SEQ ID NOs 1 to 18.

lncRNAs are known in the art. For the first time, the present inventors have surprisingly discovered that some lncRNAs are overexpressed in colorectal cancer cells relative to healthy cells, and that polypeptides may be encoded by an open reading frame (ORF) in said cancer cells. An open reading frame known in the art as being a sequence of polynucleotides comprised within the lncRNA that begins with a start codon and ends with a stop codon. Proteins are not generally considered as being translated from lncRNAs. However, the present inventors have determined that polypeptides encoded by an ORF in a lncRNA that is overexpressed in colorectal cancer cells may be presented via a major histocompatibility complex (MHC) and consequently elicit an immune response.

Overexpression of a lncRNA in colorectal cancer cells relative to healthy cells can be determined by any suitable method in the art. Preferably, expression of the lncRNA is observed in colorectal cancer cells, and wherein said expression is significantly greater than in healthy cells. More preferably, substantially no expression is observed in healthy cells. The expression of the lncRNA in colorectal cancer cells is preferably determined via the same means as the expression of the lncRNA in healthy cells. The colorectal cancer cells and healthy cells may be derived from the same subject.

The polypeptide may comprise at least 6 to 32 amino acids, preferably about 7 to 30 amino acids, and most preferably about 8 to 11 or about 12-28 amino acids.

The polypeptide is preferably capable of presentation by MHC, wherein the MHC is expressed on the surface of antigen presenting cells (APCs). The polypeptide may be presented by MHC-1 or MHC-II, wherein most preferably the polypeptide may be presented by MHC-I. The polypeptide may comprise or consist of a human leukocyte antigen (HLA) class I restricted epitope. The polypeptide may be determined to be presented by MHC, preferably MHC-1, by any suitable method in the art. Presentation of the polypeptide may be determined by an algorithm that leverages mass spectrometry-derived MHC ligandomic data. Most preferably, the polypeptide presentation can be determined by methods incorporating utilization of mass spectrometry-derived MHC ligandomic data to reliably predict presentation. An exemplary method is disclosed in WO2022/013154, which is incorporated by reference in its entirety. This method uses the algorithm neoMS to reliably predict MHC presentation.

The polypeptide is preferably immunogenic, i.e., capable of eliciting an immune response. The immune response may be detected in at least one individual (or in a sample taken from the individual) after administration of the polypeptide to said individual (or said sample). The immune response is preferably a T cell response. The polypeptide is preferably capable of stimulating CD4⁺ and/or CD8⁺ T cells, including CD8+ T cells capable of lysing cancer cells. The polypeptide may be determined to be immunogenic by any suitable method in the art. Preferably, immunogenicity of the polypeptide may be determined by an algorithm that is trained using a positive dataset of non-self immunogenic peptide sequences, and a negative dataset consisting of MHCI-presented peptides originating from housekeeping genes. Most preferably, the polypeptide immunogenicity can be determined by methods incorporating utilization of an algorithm that is trained using a positive dataset of non-self immunogenic peptide sequences, and a negative dataset consisting of MHCI-presented peptides originating from housekeeping genes. An exemplary method is disclosed in WO2022/079255, which is incorporated by reference in its entirety. This method uses the algorithm neoIM to reliably predict immunogenicity.

Preferably, the algorithms for presentation and immunogenicity take into consideration a set of alleles that are common in the populations of Europe and the USA: HLA-I alleles (A^{∗}02:10, A^{∗}01:01, A^{∗}03:01, A^{∗}24:02, A^{∗}11:01, B^{∗}07:02, B^{∗}08:01, B^{∗}44:02, B^{∗}35:01, B^{∗}51:01, B^{∗}44:03, C^{∗}07:01, C^{∗}07:02, C^{∗}05:01, C^{∗}04:01, C^{∗}06:02, C^{∗}03:04).

A polypeptide may be further determined to be immunogenic using any suitable method, including *in vitro* methods. For example, a polypeptide may be identified as immunogenic if it has at least one of the following characteristics:
(i) It is capable of eliciting a polyfunctional T cell response (IFN-γ, IL-2, and/or TNFα -producing cells) in a PBL population of a healthy subject and/or a cancer patient as determined by an ELISPOT assay, and/or
(ii) It is capable of *in situ* detection in a sample of tumor tissue of cytotoxic T lymphocytes (CTLs) that are reactive with the polypeptide; and/or
(iii) It is capable of inducing the *in vitro* growth of specific T-cells.

Methods suitable for determining whether a polypeptide is immunogenic are also described in the Examples section below.

The polypeptide may comprise a 6-amino acid sequence, 7-amino acid sequence, an 8-amino acid sequence, a 9 amino-acid sequence, a 10 amino-acid sequence, or an 11-amino acid sequence, or variant thereof, comprised within any one of the polypeptide sequences defined according to SEQ ID NOs 19 to 97. Most preferably, the polypeptide may comprise an 8-amino acid sequence, or variant thereof, comprised within any one of the polypeptide sequences defined according to SEQ ID NOs 19 to 97.

The polypeptide may have at least 50%, at least 60%, at least 70%, at least 80%, at least 90% or at least 95% sequence identity with an amino acid sequence of the same length comprised within any one of the polypeptide sequences defined according to SEQ ID NOs 19 to 97. The polypeptide may preferably be an 8-amino acid sequence and have at least 50%, at least 60%, at least 80%, at least 90% or at least 95% sequence identity with an 8-amino acid sequence comprised within any one of the polypeptide sequences defined according to SEQ ID NOs 19 to 97. Preferably, when the polypeptide is a variant, the polypeptide is capable of presentation by MHC class I and is immunogenic.

The polypeptide may comprise, or consist of, or be a variant of, any one of the polypeptide sequences defined according to SEQ ID NOs 98 to 611. The variant may have at least 50%, at least 60%, at least 80%, at least 90% or at least 95% sequence identity with an amino acid sequence comprised within any one of the polypeptide sequences defined according to SEQ ID NOs 98 to 611.

The polypeptide of the invention may be in a substantially isolated form. It may be mixed with carriers, preservatives, or diluents (discussed in more detail below) which will not interfere with the intended use, and/or with an adjuvant (also discussed in more detail below) and still be regarded as substantially isolated. It may also be in a substantially purified form, in which case it will generally comprise at least 90%, e.g. at least 95%, 98% or 99%, of the protein in the preparation

### Polynucleotides, vectors and cells

The invention provides a polynucleotide which encodes one or more polypeptides according to the invention.

The polynucleotide may encode a single polypeptide of the invention. Other polynucleotides of the invention may encode a plurality of polypeptides of the invention, optionally wherein the coding sequences for the plurality of polypeptides are in frame relative to one another and thus the polynucleotide molecule encodes a polypeptide comprising the plurality of polypeptides of the invention in continuous sequence.

The generation and delivery of immunogenic polypeptides so that they are presented effectively on MHC molecules in order to elicit a desired immune response in an individual can be challenging. The polynucleotide of the invention may mitigate this problem by providing the coding sequences for the plurality of polypeptides that are in frame relative to one another, and thus the polynucleotide molecule encodes a polypeptide comprising the plurality of polypeptides of the invention in continuous sequence. In such a polynucleotide, it may be to intersperse the polypeptides of the invention with cleavage sites recognised by proteases that are abundant in Antigen Presenting Cells (APCs). These methods mimic antigen processing and may potentially lead to a more effective antigen presentation than can be achieved with administration of polypeptide antigens.

The terms "nucleic acid molecule" and "polynucleotide" are used interchangeably herein and refer to a polymeric form of nucleotides of any length, either deoxyribonucleotides or ribonucleotides, or analogs thereof. Non-limiting examples of polynucleotides include a gene, a gene fragment, messenger RNA (mRNA), cDNA, recombinant polynucleotides, plasmids, vectors, isolated DNA of any sequence, isolated RNA of any sequence, nucleic acid probes, and primers. Most preferably, the polynucleotide is a DNA molecule, such as a cDNA molecule, or an mRNA molecule.

Where the polynucleotide of the invention is an mRNA, the polypeptide of the invention is encoded by a nucleotide sequence comprised in an open reading frame (ORF). The mRNA may comprise a 5' terminal cap at the 5' end, a 5' untranslated region (UTR) which is included 5' of the ORF, a 3' UTR which is included 3' of the ORF, and/or a 3' tailing sequence at the 3' end. A "5' untranslated region (UTR)" is a region of an mRNA that is directly upstream (i.e., 5') from the start codon (i.e., the first codon of an mRNA transcript translated by a ribosome) that does not encode a protein or peptide.

A "3' untranslated region (UTR)" is a region of an mRNA that is directly downstream (i.e., 3') from the stop codon (i.e., the codon of an mRNA transcript that signals a termination of translation) that does not encode a protein or peptide.

An "open reading frame" (ORF) is a continuous stretch of DNA that begins with a start codon, ends with a stop codon, and encodes a protein or peptide. The start codon is typically translated as methionine and is most commonly ATG, although alternatives such as TTG, GTG and CTG are possible. Alternative start codons are still translated as methionine when they are at the start of the ORF even if the codon encodes a different amino acid otherwise. The stop codon is typically selected from TAA, TAG or TGA.

A 5' terminal cap is a specially altered nucleotide on the 5' end of some primary transcripts such as messenger RNA, which promotes stability and translation. It usually consists of a guanine nucleotide connected to mRNA via an unusual 5' to 5' triphosphate linkage. This guanosine is methylated on the 7 position directly after capping in vivo by a methyltransferase. It may thus be referred to as a 7-methylguanylate cap, abbreviated as m7G. A preferred 5' terminal cap is m7G(5')ppp(5')NlmpNp.

The 3' tailing sequence is a polyA tail, a polyA-G quartet and/or a stem loop sequence. The 3' tailing sequence is typically between 40 and 200 nucleotides in length. In some embodiments, the 3' tailing sequence is a polyA tail. A "polyA tail" is a region of mRNA that is downstream, e.g., directly downstream (i.e., 3'), from the 3' UTR that contains multiple, consecutive adenosine monophosphates. A polyA tail may contain 10 to 300 adenosine monophosphates. For example, a polyA tail may contain 10, 20, 30, 40, 50, 60, 70, 80, 90, 100, 110, 120, 130, 140, 150, 160, 170, 180, 190, 200, 210, 220, 230, 240, 250, 260, 270, 280, 290 or 300 adenosine monophosphates. In some embodiments, a polyA tail contains 50 to 250 adenosine monophosphates. In a relevant biological setting (e.g., in cells, in vivo, etc.) the poly(A) tail functions to protect mRNA from enzymatic degradation, e.g., in the cytoplasm, and aids in transcription termination, export of the mRNA from the nucleus, and translation.

Where the polynucleotide of the invention is an mRNA it may be provided as an mRNA vaccine. Said mRNA vaccine may be formulated as a lipid nanoparticle composition, for example a lipid nanoparticle composition comprising an mRNA encoding a polypeptide of the invention and an ionizable lipid.

A polynucleotide of the invention may be provided in isolated or substantially isolated form. By substantially isolated, it is meant that there may be substantial, but not total, isolation of the polypeptide from any surrounding medium. The polynucleotides may be mixed with carriers or diluents which will not interfere with their intended use and still be regarded as substantially isolated. A nucleic acid sequence which "encodes" a selected polypeptide is a nucleic acid molecule which is transcribed (in the case of DNA) and translated (in the case of mRNA) into a polypeptide *in vivo* when placed under the control of appropriate regulatory sequences, for example in an expression vector. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence.

The polynucleotides of the present invention may function as mRNA but are distinguished from wild-type mRNA in functional and/or structural features. mRNA cancer vaccines of the present invention may be encoded by *in vitro* translated (IVT) polynucleotides. An *"in vitro* transcription template (IVT)," as used herein, refers to deoxyribonucleic acid (DNA) suitable for use in an IVT reaction for the production of messenger RNA (mRNA). In some embodiments, an IVT template encodes a 5' untranslated region, contains an open reading frame, and encodes a 3' untranslated region and a polyA tail. The particular nucleotide sequence composition and length of an IVT template will depend on the mRNA of interest encoded by the template.

Polynucleotides can be synthesised according to methods well known in the art, as described by way of example in Sambrook et al (1989, Molecular Cloning - a laboratory manual; Cold Spring Harbor Press). The nucleic acid molecules of the present invention may be provided in the form of an expression cassette which includes control sequences operably linked to the inserted sequence, thus allowing for expression of the polypeptide of the invention *in vivo.* These expression cassettes, in turn, are typically provided within vectors (e.g., plasmids or recombinant viral vectors). Such an expression cassette may be administered directly to a host subject. Alternatively, a vector comprising a polynucleotide of the invention may be administered to a host subject. Preferably the polynucleotide is prepared and/or administered using a genetic vector. A suitable vector may be any vector which is capable of carrying a sufficient amount of genetic information, and allowing expression of a polypeptide of the invention.

mRNA may be prepared by any suitable technique known in the art, via any appropriate synthesis route. IVT methods are preferred. *In vitro* transcription (IVT) methods permit template-directed synthesis of RNA molecules of almost any sequence. The size of the RNA molecules that can be synthesized using IVT methods range from short oligonucleotides to long nucleic acid polymers of several thousand bases. IVT methods permit synthesis of large quantities of RNA transcript (e.g., from microgram to milligram quantities) (Beckert et ah, Synthesis of RNA by in vitro transcription, Methods Mol Biol. 703:29-41(2011); Rio et al. RNA: A Laboratory Manual. Cold Spring Harbor: Cold Spring Harbor Laboratory Press, 2011, 205-220.; Cooper, Geoffery M. The Cell: A Molecular Approach. 4th ed. Washington D.C.: ASM Press, 2007. 262-299). Generally, IVT utilizes a DNA template featuring a promoter sequence upstream of a sequence of interest. The promoter sequence is most commonly of bacteriophage origin (ex. the T7, T3 or SP6 promoter sequence) but many other promotor sequences can be tolerated including those designed de novo. Transcription of the DNA template is typically best achieved by using the RNA polymerase corresponding to the specific bacteriophage promoter sequence. Exemplary RNA polymerases include, but are not limited to T7 RNA polymerase, T3 RNA polymerase, or SP6 RNA polymerase, among others. IVT is generally initiated at a dsDNA but can proceed on a single strand. Suitable methods include, for example, those listed in WO2017/020026 (which is herein incorporated by reference).

The present invention thus includes expression vectors that comprise such polynucleotide sequences. The vector may thus encode a single polypeptide of the invention. Other vectors of the invention may thus encode a plurality of polypeptides of the invention, optionally wherein the coding sequences for the plurality of polypeptides are in frame relative to one another and thus the vector encodes a polypeptide comprising the plurality of polypeptides of the invention in continuous sequence. The vector is preferably a viral vector.

Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for expression of a peptide of the invention. Other suitable vectors would be apparent to persons skilled in the art. By way of further example in this regard we refer to Sambrook et al. (1989, Molecular Cloning - a laboratory manual; Cold Spring Harbor Press).

The invention also provides a cell, wherein the cell comprises a polynucleotide or viral vector according to the invention. The cell may be a dendritic cell (DC), wherein the DC has been matured by ex vivo, wherein the maturation comprises transfection with a polynucleotide according to the invention, or by infection with a viral vector of the invention. Methods of DC maturation are otherwise well known in the art. When it is intended to administer such a cell to a patient in any one of the methods of treatment provided herein, it is preferable for the cell to be syngeneic with respect to the patient.

Other cells of the invention include prokaryotic cells such as bacterial cells, for example *E. coli.* Such prokaryotic cells may be engineered and cultured using routine methods to produce a polypeptide of the invention.

The invention also provides a cell, wherein the cell is a CAR-T cell, and wherein the cell comprises cell surface receptors raised against one or more polynucleotides of the invention. Methods of engineering T cells derived from a patient to comprise cell surface receptors raised against an antigen such as a polypeptide of the invention are known in the art. The CAR-T cell may therefore comprise receptors capable of binding one or more polypeptides of the invention. When it is intended to administer such a cell to a patient in any one of the methods of treatment provided herein, it is preferable for the cell to be syngeneic with respect to the patient.

The present invention also provides a T cell which is raised against one or more polypeptides of the invention.

### Compositions comprising polypeptides, polynucleotides or cells

The invention provides a composition comprising the polypeptide of the invention, the polynucleotide of the invention, the vector of the invention, or the cell of the invention, and a pharmaceutically acceptable carrier.

The composition may for example comprise at least two, at least three, at least four, at least five, at least six, at least seven, at least eight different polypeptides of the invention and optionally at least one adjuvant, pharmaceutically acceptable carrier, preservative and/or excipient.

The composition may for example comprise at least two, at least three, at least four, at least five, at least six, at least seven, at least eight different polynucleotides of the invention and optionally at least one adjuvant, pharmaceutically acceptable carrier, preservative and/or excipient.

The carrier, preservative and excipient must be 'acceptable' in the sense of being compatible with the other ingredients of the composition and not deleterious to a subject to which the composition is administered. Typically, all components and the final composition are sterile and pyrogen free. The composition may be a pharmaceutical composition. The composition may preferably comprise an adjuvant.

Adjuvants are any substance whose admixture into the composition increases or otherwise modifies the immune response elicited by the composition. Adjuvants, broadly defined, are substances which promote immune responses. Adjuvants may also preferably have a depot effect, in that they also result in a slow and sustained release of an active agent from the administration site. A general discussion of adjuvants is provided in Goding, Monoclonal Antibodies: Principles & Practice (2nd edition, 1986) at pages 61-63.

Adjuvants may be selected from the group consisting of: AlK(SO₄)₂, AlNa(SO₄)₂, AlNH₄ (SO₄), silica, alum, Al(OH)₃, Ca₃ (PO₄)₂, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1'2'-dipalmitoyl-sn -glycero-3-hydroxphosphoryloxy)-ethylamine (CGP 19835A, also referred to as MTP-PE), RIBI (MPL+TDM+CWS) in a 2% squalene/Tween-80.RTM. emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in Corynebacterium parvum, Bordetella pertussis, and members of the genus Brucella, Titermax, ISCOMS, Quil A, ALLTN (see US 58767 and 5,554,372), Lipid A derivatives, choleratoxin derivatives, HSP derivatives, LPS derivatives, synthetic peptide matrixes or GMDP, Interleukin 1, Interleukin 2, Montanide ISA-51 and QS-21. Various saponin extracts have also been suggested to be useful as adjuvants in immunogenic compositions. Granulocyte-macrophage colony stimulating factor (GM-CSF) may also be used as an adjuvant.

Preferred adjuvants to be used with the invention include oil/surfactant-based adjuvants such as Montanide adjuvants (available from Seppic, Belgium), preferably Montanide ISA-51. Other preferred adjuvants are bacterial DNA based adjuvants, such as adjuvants including CpG oligonucleotide sequences. Yet other preferred adjuvants are viral dsRNA based adjuvants, such as poly I:C. GM-CSF and imidazoquinolines are also examples of preferred adjuvants.

The adjuvant is most preferably a Montanide ISA adjuvant. The Montanide ISA adjuvant is preferably Montanide ISA 51 or Montanide ISA 720.

In Goding, Monoclonal Antibodies: Principles & Practice (2nd edition, 1986) at pages 61-63 it is also noted that, when an antigen of interest is of low molecular weight, or is poorly immunogenic, coupling to an immunogenic carrier is recommended. A polypeptide of the invention may therefore be coupled to a carrier. A carrier may be present independently of an adjuvant. The function of a carrier can be, for example, to increase the molecular weight of a polypeptide fragment in order to increase activity or immunogenicity, to confer stability, to increase the biological activity, or to increase serum half-life. Furthermore, a carrier may aid in presenting the polypeptide or fragment thereof to T-cells. Thus, in the composition, the polypeptide may be associated with a carrier such as those set out below.

The carrier may be any suitable carrier known to a person skilled in the art, for example a protein or an antigen presenting cell, such as a dendritic cell (DC). Carrier proteins include keyhole limpet hemocyanin, serum proteins such as transferrin, bovine serum albumin, human serum albumin, thyroglobulin or ovalbumin, immunoglobulins, or hormones, such as insulin or palmitic acid. Alternatively, the carrier protein may be tetanus toxoid or diphtheria toxoid. Alternatively, the carrier may be a dextran such as sepharose. The carrier must be physiologically acceptable to humans and safe.

If the composition comprises an excipient, it must be 'pharmaceutically acceptable' in the sense of being compatible with the other ingredients of the composition and not deleterious to the recipient thereof. Auxiliary substances, such as wetting or emulsifying agents, pH buffering substances and the like, may be present in the excipient. These excipients and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Formulation of a suitable composition can be carried out using standard pharmaceutical formulation chemistries and methodologies all of which are readily available to the reasonably skilled artisan. Such compositions may be prepared, packaged, or sold in a form suitable for bolus administration or for continuous administration. Injectable compositions may be prepared, packaged, or sold in unit dosage form, such as in ampoules or in multi-dose containers optionally containing a preservative. Compositions include, but are not limited to, suspensions, solutions, emulsions in oily or aqueous vehicles, pastes, and implantable sustained-release or biodegradable formulations. In one embodiment of a composition, the active ingredient is provided in dry (for e.g., a powder or granules) form for reconstitution with a suitable vehicle (e. g., sterile pyrogenfree water) prior to administration of the reconstituted composition. The composition may be prepared, packaged, or sold in the form of a sterile injectable aqueous or oily suspension or solution. This suspension or solution may be formulated according to the known art, and may comprise, in addition to the active ingredient, additional ingredients such as the adjuvants, excipients and auxiliary substances described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or diglycerides. Other compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. Alternatively, the active ingredients of the composition may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyornithine, spermine, spermidine, as well as conjugates of these molecules.

The composition of the invention may be a polynucleotide vaccine, preferably wherein the polynucleotide is DNA or mRNA, and most preferably wherein polynucleotide is mRNA and composition comprises an mRNA (cancer) vaccine.

mRNA cancer vaccines may be formulated using one or more excipients to: (1) increase stability; (2) increase cell transfection; (3) permit the sustained or delayed release (e.g., from a depot formulation); (4) alter the biodistribution (e.g., target to specific tissues or cell types); (5) increase the translation of encoded protein in vivo; and/or (6) alter the release profile of encoded protein (antigen) in vivo. In addition to traditional excipients such as any and all solvents, dispersion media, diluents, or other liquid vehicles, dispersion or suspension aids, surface active agents, isotonic agents, thickening or emulsifying agents, preservatives, excipients of the present invention can include, without limitation, lipidoids, liposomes, lipid nanoparticles, polymers, lipoplexes, core-shell nanoparticles, peptides, proteins, cells transfected with mRNA cancer vaccines (e.g., for transplantation into a subject), hyaluronidase, nanoparticle mimics and combinations thereof.

The mRNA and/or compositions disclosed herein may include stabilising elements. Naturally-occurring eukaryotic mRNA molecules have been found to contain stabilizing elements, including, but not limited to the 5' and 3' UTRs, the 5' cap and the 3' tail discussed elsewhere in this document. Other stabilizing elements that may be included in mRNA as disclosed herein may include for instance a histone stem-loop. In some embodiments, the histone stem-loop is generally derived from histone genes, and includes an intramolecular base pairing of two neighbored partially or entirely reverse complementary sequences separated by a spacer, consisting of a short sequence, which forms the loop of the structure. The mRNA may have one or more AU-rich sequences removed. Such sequences may be destabilising. The RNA vaccine may or may not contain a enhancer and/or promoter sequence, which may be modified or unmodified or which may be activated or inactivated.

In preferred embodiments, the mRNA cancer vaccine may be formulated in a lipid-polycation complex. The formation of the lipid-polycation complex may be accomplished by methods known in the art and/or as described in U.S. Pub. No. 20120178702, herein incorporated by reference in its entirety. As a non-limiting example, the polycation may include a cationic peptide or a polypeptide such as, but not limited to, polylysine, poly ornithine and/or polyarginine and the cationic peptides described in International Pub. No. WO2012013326 or US Patent Pub. No. US20130142818; each of which is herein incorporated by reference in its entirety. In another embodiment, the mRNA cancer vaccines may be formulated in a lipid-polycation complex which may further include a non-cationic lipid such as, but not limited to, cholesterol or dioleoyl-phosphatidylethanolamine (DOPE). The liposome formulation may be influenced by, but not limited to, the selection of the cationic lipid component, the degree of cationic lipid saturation, the nature of the PEGylation, ratio of all components and biophysical parameters such as size. In one example by Semple et al. (Semple et al. Nature Biotech. 2010 28: 172-176; herein incorporated by reference in its entirety), the liposome formulation was composed of 57.1 % cationic lipid, 7.1% dipalmitoylphosphatidylcholine, 34.3 % cholesterol, and 1.4% PEG-c-DMA. As another example, changing the composition of the cationic lipid could more effectively deliver siRNA to various antigen presenting cells (Basha et al. Mol Ther. 2011 19:2186-2200; herein incorporated by reference in its entirety).

Liposome formulations may comprise from about 35 to about 45% cationic lipid, from about 40% to about 50% cationic lipid, from about 50% to about 60% cationic lipid and/or from about 55% to about 65% cationic lipid. In some embodiments, the ratio of lipid to mRNA in liposomes may be from about 5: 1 to about 20: 1, from about 10: 1 to about 25: 1, from about 15: 1 to about 30: 1 and/or at least 30: 1. The ratio of PEG in the lipid nanoparticle (LNP) formulations may be increased or decreased and/or the carbon chain length of the PEG lipid may be modified from C 14 to C18 to alter the pharmacokinetics and/or biodistribution of the LNP formulations. As a non-limiting example, LNP formulations may contain from about 0.5% to about 3.0%, from about 1.0% to about 3.5%, from about 1.5% to about 4.0%, from about 2.0% to about 4.5%, from about 2.5% to about 5.0% and/or from about 3.0% to about 6.0% of the lipid molar ratio of PEG-c-DOMG (R-3-[(co-methoxy-poly(ethyleneglycol)2000)carbamoyl)]-l,2-dimyristyloxypropyl-3-amine) (also referred to herein as PEG-DOMG) as compared to the cationic lipid, DSPC and cholesterol. In another embodiment the PEG-c-DOMG may be replaced with a PEG lipid such as, but not limited to, PEG- DSG (1,2-Distearoyl-sn-glycerol, methoxypolyethylene glycol), PEG-DMG (1,2-Dimyristoyl-sn-glycerol) and/or PEG-DPG (1,2-Dipalmitoyl-sn-glycerol, methoxypolyethylene glycol). The cationic lipid may be selected from any lipid known in the art such as, but not limited to, DLin-MC3-DMA, D Lin-DMA, C 12-200 and DLin-KC2-DMA.

The mRNA cancer vaccine formulation comprising the polynucleotide may be a nanoparticle which may comprise at least one lipid. The lipid may be selected from, but is not limited to, DLin-DMA, DLin-K-DMA, 98N12-5, C12-200, DLin-MC3-DMA, DLin-KC2-DMA, DODMA, PLGA, PEG, PEG-DMG, PEGylated lipids and amino alcohol lipids. In another aspect, the lipid may be a cationic lipid such as, but not limited to, DLin-DMA, DLin-D-DMA, DLin-MC3-DMA, DLin-KC2-DMA, DODMA and amino alcohol lipids. The amino alcohol cationic lipid may be the lipids described in and/or made by the methods described in US Patent Publication No. US20130150625, herein incorporated by reference in its entirety. As a non-limiting example, the cationic lipid may be 2-amino-3-[(9Z,12Z)-octadeca-9,12-dien-l-yloxy]-2-{[(9Z,2Z)-octadeca-9,12-dien-l-yloxy]methyl}propan-l-ol (Compound 1 in US20130150625); 2-amino-3-[(9Z)-octadec-9-en-l-yloxy]-2-{[(9Z)-octadec-9-en-l-yloxy]methyl}propan-l-ol (Compound 2 in US20130150625); 2-amino-3-[(9Z,12Z)-octadeca-9,12-dien-l-yloxy]-2-[(octyloxy)methyl]propan-l-ol (Compound 3 in US20130150625); and 2-(dimethylamino)-3-[(9Z,12Z)-octadeca-9,12-dien-l-yloxy]-2-{[(9Z,12Z)-octadeca-9,12-dien-l-yloxy]methyl}propan-l-ol (Compound 4 in US20130150625); or any pharmaceutically acceptable salt or stereoisomer thereof.

Lipid nanoparticle formulations typically comprise a lipid, in particular, an ionizable cationic lipid, for example, 2,2-dilinoleyl-4-dimethylaminoethyl-[l,3]-dioxolane (DLin-KC2-DMA), dilinoleyl-methyl-4-dimethylaminobutyrate (DLin-MC3-DMA), or di((Z)-non-2-en-1-yl) 9-((4-(dimethylamino)butanoyl)oxy)heptadecanedioate (L319), and further comprise a neutral lipid, a sterol and a molecule capable of reducing particle aggregation, for example a PEG or PEG-modified lipid. The lipid nanoparticle formulation may consist essentially of (i) at least one lipid selected from the group consisting of 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319); (ii) a neutral lipid selected from DSPC, DPPC, POPC, DOPE and SM; (iii) a sterol, e.g., cholesterol; and (iv) a PEG-lipid, e.g., PEG-DMG or PEG-cDMA, in a molar ratio of about 20-60% cationic lipid: 5-25% neutral lipid: 25-55% sterol; 0.5-15% PEG-lipid. The formulation may include from about 25% to about 75% on a molar basis of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), e.g., from about 35 to about 65%, from about 45 to about 65%, about 60%, about 57.5%, about 50% or about 40% on a molar basis.

The formulation may include from about 0.5% to about 15% on a molar basis of the neutral lipid e.g., from about 3 to about 12%, from about 5 to about 10% or about 15%, about 10%, or about 7.5% on a molar basis. Exemplary neutral lipids include, but are not limited to, DSPC, POPC, DPPC, DOPE and SM. The formulation may include from about 5% to about 50% on a molar basis of the sterol (e.g., about 15 to about 45%, about 20 to about 40%, about 40%, about 38.5%, about 35%, or about 31% on a molar basis. An exemplary sterol is cholesterol. The formulation may include from about 0.5% to about 20% on a molar basis of the PEG or PEG-modified lipid (e.g., about 0.5 to about 10%, about 0.5 to about 5%, about 1.5%, about 0.5%, about 1.5%, about 3.5%, or about 5% on a molar basis. The PEG or PEG modified lipid may comprise a PEG molecule of an average molecular weight of 2,000 Da, or an average molecular weight of less than 2,000, for example around 1,500 Da, around 1,000 Da, or around 500 Da. Exemplary PEG-modified lipids include, but are not limited to, PEG-distearoyl glycerol (PEG-DMG) (also referred herein as PEG-C14 or C14-PEG), PEG-cDMA (further discussed in Reyes et al. J. Controlled Release, 107, 276-287 (2005) the contents of which are herein incorporated by reference in its entirety)

The formulations herein may include 25-75% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), 0.5-15% of the neutral lipid, 5-50% of the sterol, and 0.5-20% of the PEG or PEG-modified lipid on a molar basis.

In one embodiment, the formulations of the inventions include 35-65% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), 3-12% of the neutral lipid, 15-45% of the sterol, and 0.5-10% of the PEG or PEG-modified lipid on a molar basis.

The formulations herein may include 45-65% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), 5-10% of the neutral lipid, 25-40% of the sterol, and 0.5-10% of the PEG or PEG-modified lipid on a molar basis.

The formulations herein may include about 60% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 7.5% of the neutral lipid, about 31 % of the sterol, and about 1.5% of the PEG or PEG-modified lipid on a molar basis.

The formulations herein may include about 50% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 10% of the neutral lipid, about 38.5 % of the sterol, and about 1.5% of the PEG or PEG-modified lipid on a molar basis.

The formulations herein may include include about 50% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 10% of the neutral lipid, about 35 % of the sterol, about 4.5% or about 5% of the PEG or PEG-modified lipid, and about 0.5% of the targeting lipid on a molar basis.

The formulations herein may include about 40% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 15% of the neutral lipid, about 40% of the sterol, and about 5% of the PEG or PEG-modified lipid on a molar basis.

In one embodiment, the formulations include about 57.2% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 7.1% of the neutral lipid, about 34.3% of the sterol, and about 1.4% of the PEG or PEG-modified lipid on a molar basis.

The formulations herein may include about 57.5% of a cationic lipid selected from the PEG lipid is PEG-cDMA (PEG-cDMA is further discussed in Reyes et al. (J. Controlled Release, 107, 276-287 (2005), the contents of which are herein incorporated by reference in its entirety), about 7.5% of the neutral lipid, about 31.5 % of the sterol, and about 3.5% of the PEG or PEG-modified lipid on a molar basis.

In preferred embodiments, lipid nanoparticle formulation consists essentially of a lipid mixture in molar ratios of about 20-70% cationic lipid: 5-45% neutral lipid: 20-55% cholesterol: 0.5-15% PEG-modified lipid; more preferably in a molar ratio of about 20-60% cationic lipid: 5-25% neutral lipid: 25-55% cholesterol: 0.5-15% PEG-modified lipid.

In particular embodiments, the molar lipid ratio is approximately 50/10/38.5/1.5 (mol% cationic lipid/neutral lipid, e.g., DSPC/Chol/PEG-modified lipid, e.g., PEG-DMG, PEG-DSG or PEG-DPG), 57.2/7.1134.3/1.4 (mol% cationic lipid/ neutral lipid, e.g.,
DPPC/Chol/ PEG-modified lipid, e.g., PEG-cDMA), 40/15/40/5 (mol% cationic lipid/ neutral lipid, e.g., DSPC/Chol/ PEG-modified lipid, e.g., PEG-DMG), 50/10/35/4.5/0.5 (mol% cationic lipid/ neutral lipid, e.g., DSPC/Chol/ PEG-modified lipid, e.g., PEG-DSG),
50/10/35/5 (cationic lipid/ neutral lipid, e.g., DSPC/Chol/ PEG-modified lipid, e.g., PEG-DMG), 40/10/40/10 (mol% cationic lipid/ neutral lipid, e.g., DSPC/Chol/ PEG-modified lipid, e.g., PEG-DMG or PEG-cDMA), 35/15/40/10 (mol% cationic lipid/ neutral lipid, e.g., DSPC/Chol/ PEG-modified lipid, e.g., PEG-DMG or PEG-cDMA) or 52/13/30/5 (mol% cationic lipid/ neutral lipid, e.g., DSPC/Chol/ PEG-modified lipid, e.g., PEG-DMG or PEG-cDMA).

Exemplary lipid nanoparticle compositions and methods of making same are described, for example, in Semple et al. (2010) Nat. Biotechnol. 28: 172-176; Jayarama et al. (2012), Angew. Chem. Int. Ed., 51: 8529-8533; and Maier et al. (2013) Molecular Therapy 21, 1570-1578 (the contents of each of which are incorporated herein by reference in their entirety).

The lipid nanoparticle formulations described herein may comprise a cationic lipid, a PEG lipid and a structural lipid and optionally comprise a non-cationic lipid. As a non-limiting example, the lipid nanoparticle may comprise about 40-60% of cationic lipid, about 5-15% of a non-cationic lipid, about 1-2% of a PEG lipid and about 30-50% of a structural lipid. As another non-limiting example, the lipid nanoparticle may comprise about 50% cationic lipid, about 10% non-cationic lipid, about 1.5% PEG lipid and about 38.5% structural lipid. As yet another non-limiting example, the lipid nanoparticle may comprise about 55% cationic lipid, about 10% non-cationic lipid, about 2.5% PEG lipid and about 32.5% structural lipid. In one embodiment, the cationic lipid may be any cationic lipid described herein such as, but not limited to, DLin-KC2-DMA, DLin-MC3-DMA and L319.

The lipid nanoparticle formulations described herein may be 4 component lipid nanoparticles. The lipid nanoparticle may comprise a cationic lipid, a non-cationic lipid, a PEG lipid and a structural lipid. As a non-limiting example, the lipid nanoparticle may comprise about 40-60% of cationic lipid, about 5-15% of a non-cationic lipid, about 1-2% of a PEG lipid and about 30-50% of a structural lipid. As another non-limiting example, the lipid nanoparticle may comprise about 50% cationic lipid, about 10% non-cationic lipid, about 1.5% PEG lipid and about 38.5% structural lipid. As yet another non-limiting example, the lipid nanoparticle may comprise about 55% cationic lipid, about 10% non-cationic lipid, about 2.5% PEG lipid and about 32.5% structural lipid. In one embodiment, the cationic lipid may be any cationic lipid described herein such as, but not limited to, DLin-KC2-DMA, DLin-MC3-DMA and L319.

The lipid nanoparticle formulations described herein may comprise a cationic lipid, a non-cationic lipid, a PEG lipid and a structural lipid. As a non-limiting example, the lipid nanoparticle comprise about 50% of the cationic lipid DLin-KC2-DMA, about 10% of the non-cationic lipid DSPC, about 1.5% of the PEG lipid PEG-DOMG and about 38.5% of the structural lipid cholesterol. As a non-limiting example, the lipid nanoparticle comprise about 50% of the cationic lipid DLin-MC3-DMA, about 10% of the non-cationic lipid DSPC, about 1.5% of the PEG lipid PEG-DOMG and about 38.5% of the structural lipid cholesterol. As a non-limiting example, the lipid nanoparticle comprise about 50% of the cationic lipid DLin-MC3-DMA, about 10% of the non-cationic lipid DSPC, about 1.5% of the PEG lipid PEG-DMG and about 38.5% of the structural lipid cholesterol. As yet another non-limiting example, the lipid nanoparticle comprise about 55% of the cationic lipid L319, about 10% of the non-cationic lipid DSPC, about 2.5% of the PEG lipid PEG-DMG and about 32.5% of the structural lipid cholesterol.

Relative amounts of the active ingredient, the pharmaceutically acceptable excipient, and/or any additional ingredients in a pharmaceutical composition in accordance with the present disclosure may vary, depending upon the identity, size, and/or condition of the subject being treated and further depending upon the route by which the composition is to be administered. For example, the composition may comprise between 0.1% and 99% (w/w) of the active ingredient. By way of example, the composition may comprise between 0.1% and 100%, e.g., between .5 and 50%, between 1-30%, between 5-80%, at least 80% (w/w) active ingredient.

The mRNA cancer vaccine composition may comprise the polynucleotide described herein, formulated in a lipid nanoparticle comprising MC3,
Cholesterol, DSPC and PEG2000-DMG, the buffer trisodium citrate, sucrose and water for injection. As a non-limiting example, the composition comprises: 2.0 mg/mL of drug substance (e.g., polynucleotides encoding H10N8 influenza virus), 21.8 mg/mL of MC3, 10.1 mg/mL of cholesterol, 5.4 mg/mL of DSPC, 2.7 mg/mL of PEG2000-DMG, 5.16 mg/mL of trisodium citrate, 71 mg/mL of sucrose and about 1.0 mL of water for injection.

RNA vaccines can be formulated using one or more liposomes, lipoplexes, or lipid nanoparticles. Pharmaceutical compositions of RNA vaccines may include liposomes. Liposomes are artificially-prepared vesicles which may primarily be composed of a lipid bilayer and may be used as a delivery vehicle for the administration of nutrients and pharmaceutical formulations. Liposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50 nm in diameter, and a large unilamellar vesicle (LUV) which may be between 50 and 500 nm in diameter. Liposome design may include, but is not limited to, opsonins or ligands in order to improve the attachment of liposomes to unhealthy tissue or to activate events such as, but not limited to, endocytosis. Liposomes may contain a low or a high pH in order to improve the delivery of the pharmaceutical formulations. The formation of liposomes may depend on the physicochemical characteristics such as, but not limited to, the pharmaceutical formulation entrapped and the liposomal ingredients , the nature of the medium in which the lipid vesicles are dispersed, the effective
concentration of the entrapped substance and its potential toxicity, any additional processes involved during the application and/or delivery of the vesicles, the optimization size, polydispersity and the shelf-life of the vesicles for the intended application, and the batch-to-batch reproducibility and possibility of large-scale production of safe and efficient liposomal products.

As a non-limiting example, liposomes such as synthetic membrane vesicles may be prepared by the methods, apparatus and devices described in US Patent Publication No. US20130177638, US20130177637, US20130177636, US20130177635, US20130177634, US20130177633, US20130183375, US20130183373 and US20130183372, the contents of each of which are herein incorporated by reference in its entirety. Pharmaceutical compositions described herein may include, without limitation, liposomes such as those formed from l,2-dioleyloxy-N,N-dimethylaminopropane (DODMA) liposomes, DiLa2 liposomes from Marina Biotech (Bothell, WA), l,2-dilinoleyloxy-3-dimethylaminopropane (DLin-DMA), 2,2-dilinoleyl-4-(2-dimethylaminoethyl)-[l,3]-dioxolane (DLin-KC2-DMA), and MC3 (US 20100324120; herein incorporated by reference in its entirety) and liposomes which may deliver small molecule drugs such as, but not limited to, DOXIL^{®} from Janssen Biotech, Inc. (Horsham, PA).

Pharmaceutical compositions described herein may include, without limitation, liposomes such as those formed from the synthesis of stabilized plasmid-lipid particles (SPLP) or stabilized nucleic acid lipid particle (SNALP) that have been previously described and shown to be suitable for oligonucleotide delivery in vitro and in vivo (see Wheeler et al. Gene Therapy. 1999 6:271-281 ; Zhang et al. Gene Therapy. 1999 6: 1438-1447; Jeffs et al. Pharm Res. 2005 22:362-372; Morrissey et al., Nat Biotechnol. 2005 2: 1002-1007; Zimmermann et al., Nature. 2006 441: 111-114; Heyes et al. J Contr Rel. 2005 107:276-287; Semple et al. Nature Biotech. 2010 28: 172-176; Judge et al. J Clin Invest. 2009 119:661-673; deFougerolles Hum Gene Ther. 2008 19: 125-132; U.S. Patent Publication No US20130122104; all of which are incorporated herein in their entireties). The original manufacture method by Wheeler et al. was a detergent dialysis method, which was later improved by Jeffs et al. and is referred to as the spontaneous vesicle formation method. The liposome formulations are composed of 3 to 4 lipid components in addition to the polynucleotide.

As an example a liposome can contain, but is not limited to, 55% cholesterol, 20% disteroylphosphatidyl choline (DSPC), 10% PEG-S-DSG, and 15% 1,2-dioleyloxy-N,N-dimethylaminopropane (DODMA), as described by Jeffs et al. As another example, certain liposome formulations may contain, but are not limited to, 48% cholesterol, 20% DSPC, 2% PEG-c-DMA, and 30% cationic lipid, where the cationic lipid can be 1,2-distearloxy-N,N-dimethylaminopropane (DSDMA), DODMA, DLin-DMA, or 1,2-dilinolenyloxy-3-dimethylaminopropane (DLenDMA), as described by Heyes et al.

Liposome formulations may comprise from about about 25.0% cholesterol to about 40.0% cholesterol, from about 30.0% cholesterol to about 45.0% cholesterol, from about 35.0% cholesterol to about 50.0% cholesterol and/or from about 48.5% cholesterol to about 60% cholesterol. In a preferred embodiment, formulations may comprise a percentage of cholesterol selected from the group consisting of 28.5%, 31.5%, 33.5%, 36.5%, 37.0%, 38.5%, 39.0% and 43.5%. In some embodiments, formulations may comprise from about 5.0% to about 10.0% DSPC and/or from about 7.0% to about 15.0% DSPC.

Pharmaceutical compositions may include liposomes which may be formed to deliver polynucleotides which may encode at least one immunogen (antigen) or any other polypeptide of interest. The RNA vaccine may be encapsulated by the liposome and/or it may be contained in an aqueous core which may then be encapsulated by the liposome (see International Pub. Nos. WO2012031046, WO2012031043, WO2012030901 and WO2012006378 and US Patent Publication No. US20130189351, US20130195969 and US20130202684; the contents of each of which are herein incorporated by reference in their entirety).

Liposomes may be formulated for targeted delivery. As a non-limiting example, the liposome may be formulated for targeted delivery to the liver. The liposome used for targeted delivery may include, but is not limited to, the liposomes described in and methods of making liposomes described in US Patent Publication No. US20130195967, the contents of which are herein incorporated by reference in its entirety.

In another embodiment, the polynucleotide which may encode an immunogen (antigen) may be formulated in a cationic oil-in-water emulsion where the emulsion particle comprises an oil core and a cationic lipid which can interact with the polynucleotide anchoring the molecule to the emulsion particle (see International Pub. No. WO2012006380; herein incorporated by reference in its entirety).

RNA vaccines may also be formulated in a water-in-oil emulsion comprising a continuous hydrophobic phase in which the hydrophilic phase is dispersed. As a non-limiting example, the emulsion may be made by the methods described in International Publication No. WO201087791, the contents of which are herein incorporated by reference in its entirety. The lipid formulation may include at least cationic lipid, a lipid which may enhance transfection and a least one lipid which contains a hydrophilic head group linked to a lipid moiety (International Pub. No. WO2011076807 and U.S. Pub. No. 20110200582; the contents of each of which is herein incorporated by reference in their entirety). In another embodiment, the polynucleotides encoding an immunogen may be formulated in a lipid vesicle which may have crosslinks between functionalized lipid bilayers (see U.S. Pub. No. 20120177724, the contents of which is herein incorporated by reference in its entirety).

Polynucleotides may be formulated in a lipsome as described in International Patent Publication No. WO2013086526, the contents of which is herein incorporated by reference in its entirety. The RNA vaccines may be encapsulated in a liposome using reverse pH gradients and/or optimized internal buffer compositions as described in International Patent Publication No. WO2013086526, the contents of which is herein incorporated by reference in its entirety.

RNA vaccine pharmaceutical compositions may be formulated in liposomes such as, but not limited to, DiLa2 liposomes (Marina Biotech, Bothell, WA), SMARTICLES^{®} (Marina Biotech, Bothell, WA), neutral DOPC (1,2-dioleoyl-sn-glycero-3-phosphocholine) based liposomes (e.g., siRNA delivery for ovarian cancer (Landen et al. Cancer Biology & Therapy 2006 5(12)1708-1713); herein incorporated by reference in its entirety) and hyaluronan-coated liposomes (Quiet Therapeutics, Israel).

The cationic lipid may be a low molecular weight cationic lipid such as those described in US Patent Application No. 20130090372, the contents of which are herein incorporated by reference in its entirety.

The RNA vaccines may be formulated in a lipid vesicle which may have crosslinks between functionalized lipid bilayers.

The RNA vaccines may be formulated in a liposome comprising a cationic lipid. The liposome may have a molar ratio of nitrogen atoms in the cationic lipid to the phophates in the RNA (N:P ratio) of between 1: 1 and 20: 1 as described in International Publication No. WO2013006825, herein incorporated by reference in its entirety. In another embodiment, the liposome may have a N:P ratio of greater than 20: 1 or less than 1:1.

The RNA vaccines may be formulated in a lipid-polycation complex. The formation of the lipid-polycation complex may be accomplished by methods known in the art and/or as described in U.S. Pub. No. 20120178702, herein incorporated by reference in its entirety. As a non-limiting example, the polycation may include a cationic peptide or a polypeptide such as, but not limited to, polylysine, polyornithine and/or polyarginine and the cationic peptides described in International Pub. No. WO2012013326 or US Patent Pub. No. US20130142818; each of which is herein incorporated by reference in its entirety. In another embodiment, the RNA vaccines may be formulated in a lipid-polycation complex which may further include a non-cationic lipid such as, but not limited to, cholesterol or dioleoyl phosphatidylethanolamine (DOPE).

The RNA vaccines may be formulated in an aminoalcohol lipidoid. Aminoalcohol lipidoids which may be used in the present invention may be prepared by the methods described in U.S. Patent No. 8,450,298, herein incorporated by reference in its entirety.

The liposome formulation may be influenced by, but not limited to, the selection of the cationic lipid component, the degree of cationic lipid saturation, the nature of the PEGylation, ratio of all components and biophysical parameters such as size. In one example by Semple et al. (Semple et al. Nature Biotech. 2010 28: 172-176; herein incorporated by reference in its entirety), the liposome formulation was composed of 57.1 % cationic lipid, 7.1% dipalmitoylphosphatidylcholine, 34.3 % cholesterol, and 1.4% PEG-c-DMA. As another example, changing the composition of the cationic lipid could more effectively deliver siRNA to various antigen presenting cells (Basha et al. Mol Ther. 2011 19:2186-2200; herein incorporated by reference in its entirety). In some embodiments, liposome formulations may comprise from about 35 to about 45% cationic lipid, from about 40% to about 50% cationic lipid, from about 50% to about 60% cationic lipid and/or from about 55% to about 65% cationic lipid. In some embodiments, the ratio of lipid to mRNA in liposomes may be from about about 5: 1 to about 20: 1, from about 10: 1 to about 25: 1, from about 15: 1 to about 30: 1 and/or at least 30: 1.

In some embodiments, the ratio of PEG in the lipid nanoparticle (LNP) formulations may be increased or decreased and/or the carbon chain length of the PEG lipid may be modified from C 14 to C18 to alter the pharmacokinetics and/or biodistribution of the LNP formulations. As a non-limiting example, LNP formulations may contain from about 0.5% to about 3.0%, from about 1.0% to about 3.5%, from about 1.5% to about 4.0%, from about 2.0% to about 4.5%, from about 2.5% to about 5.0% and/or from about 3.0% to about 6.0% of the lipid molar ratio of PEG-c-DOMG (R-3-[(co-methoxy-poly(ethyleneglycol)2000)carbamoyl)]-l,2-dimyristyloxypropyl-3-amine) (also referred to herein as PEG-DOMG) as compared to the cationic lipid, DSPC and cholesterol. In another embodiment the PEG-c-DOMG may be replaced with a PEG lipid such as, but not limited to, PEG- DSG (1,2-Distearoyl-sn-glycerol, methoxypolyethylene glycol), PEG-DMG (1,2-Dimyristoyl-sn-glycerol) and/or PEG-DPG (1,2-Dipalmitoyl-sn-glycerol, methoxypolyethylene glycol). The cationic lipid may be selected from any lipid known in the art such as, but not limited to, DLin-MC3-DMA, D Lin-DMA, C 12-200 and DLin-KC2-DMA.

The RNA vaccines may be formulated in a lipid nanoparticle such as those described in International Publication No. WO2012170930, the contents of which is herein incorporated by reference in its entirety.

The RNA vaccine formulation comprising the polynucleotide may be a nanoparticle which may comprise at least one lipid. The lipid may be selected from, but is not limited to, DLin-DMA, DLin-K-DMA, 98N12-5, C 12-200, DLin-MC3-DMA, DLin- KC2-DMA, DODMA, PLGA, PEG, PEG-DMG, PEGylated lipids and amino alcohol lipids. In another aspect, the lipid may be a cationic lipid such as, but not limited to, DLin-DMA, DLin-D-DMA, DLin-MC3-DMA, DLin-KC2-DMA, DODMA and amino alcohol lipids. The amino alcohol cationic lipid may be the lipids described in and/or made by the methods described in US Patent Publication No. US20130150625, herein incorporated by reference in its entirety. As a non-limiting example, the cationic lipid may be 2-amino-3-[(9Z,12Z)-octadeca-9, 12-dien- 1 -yloxy] - 2-{[(9Z,2Z)-octadeca-9, 12-dien- 1 -yloxy]methyl jpropan- 1 -ol (Compound 1 in US20130150625); 2-amino-3-[(9Z)-octadec-9-en-1-yloxy]-2-{ [(9Z)-octadec-9-en-l-yloxy]methyl}propan-l-ol (Compound 2 in US20130150625); 2-amino-3-[(9Z,12Z)-octadeca-9,12-dien-l-yloxy]-2-[(octyloxy)methyl]propan-l-ol (Compound 3 in US20130150625); and 2-(dimethylamino)-3-[(9Z,12Z)-octadeca-9,12-dien-l-yloxy]-2-{ [(9Z,12Z)-octadeca-9,12-dien-l-yloxy]methyl}propan-l-ol (Compound 4 in US20130150625); or any pharmaceutically acceptable salt or stereoisomer thereof.

Lipid nanoparticle formulations typically comprise a lipid, in particular, an ionizable cationic lipid, for example, 2,2-dilinoleyl-4-dimethylaminoethyl-[l,3]-dioxolane (DLin-KC2-DMA), dilinoleyl-methyl-4-dimethylaminobutyrate (DLin-MC3-DMA), or di((Z)-non-2-en-1-yl) 9-((4-(dimethylamino)butanoyl)oxy)heptadecanedioate (L319), and further comprise a neutral lipid, a sterol and a molecule capable of reducing particle aggregation, for example a PEG or PEG-modified lipid.

The lipid nanoparticle formulation may consist essentially of (i) at least one lipid selected from the group consisting of 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319); (ii) a neutral lipid selected from DSPC, DPPC, POPC, DOPE and SM; (iii) a sterol, e.g., cholesterol; and (iv) a PEG-lipid, e.g., PEG-DMG or PEG-cDMA, in a molar ratio of about 20-60% cationic lipid: 5-25% neutral lipid: 25-55% sterol; 0.5-15% PEG-lipid.

In one embodiment, the formulation includes from about 25% to about 75% on a molar basis of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), e.g., from about 35 to about 65%, from about 45 to about 65%, about 60%, about 57.5%, about 50% or about 40% on a molar basis.

In one embodiment, the formulation includes from about 0.5% to about 15% on a molar basis of the neutral lipid e.g., from about 3 to about 12%, from about 5 to about 10% or about 15%, about 10%, or about 7.5% on a molar basis. Exemplary neutral lipids include, but are not limited to, DSPC, POPC, DPPC, DOPE and SM. In one embodiment, the formulation includes from about 5% to about 50% on a molar basis of the sterol (e.g., about 15 to about 45%, about 20 to about 40%, about 40%, about 38.5%, about 35%, or about 31% on a molar basis. An exemplary sterol is cholesterol. In one embodiment, the formulation includes from about 0.5% to about 20% on a molar basis of the PEG or PEG-modified lipid (e.g., about 0.5 to about 10%, about 0.5 to about 5%, about 1.5%, about 0.5%, about 1.5%, about 3.5%, or about 5% on a molar basis. In one embodiment, the PEG or PEG modified lipid comprises a PEG molecule of an average molecular weight of 2,000 Da. In other embodiments, the PEG or PEG modified lipid comprises a PEG molecule of an average molecular weight of less than 2,000, for example around 1,500 Da, around 1,000 Da, or around 500 Da. Exemplary PEG-modified lipids include, but are not limited to, PEG-distearoyl glycerol (PEG-DMG) (also referred herein as PEG-C14 or C14-PEG), PEG-cDMA (further discussed in Reyes et al. J. Controlled Release, 107, 276-287 (2005) the contents of which are herein incorporated by reference in its entirety)

In one embodiment, the formulations include 25-75% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), 0.5-15% of the neutral lipid, 5-50% of the sterol, and 0.5-20% of the PEG or PEG-modified lipid on a molar basis.

In one embodiment, the formulations include 35-65% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-l-yl) 9-((4-(dimethylamino)butanoyl)oxy)heptadecanedioate (L319), 3-12% of the neutral lipid, 15-45% of the sterol, and 0.5-10% of the PEG or PEG-modified lipid on a molar basis.

In one embodiment, the formulations include 45-65% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3 -DMA) , and di((Z)-non-2-en-l-yl) 9-((4-(dimethylamino)butanoyl)oxy)heptadecanedioate (L319), 5-10% of the neutral lipid, 25-40% of the sterol, and 0.5-10% of the PEG or PEG-modified lipid on a molar basis.

In one embodiment, the formulations include about 60% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 7.5% of the neutral lipid, about 31 % of the sterol, and about 1.5% of the PEG or PEG-modified lipid on a molar basis.

In one embodiment, the formulations include about 50% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 10% of the neutral lipid, about 38.5 % of the sterol, and about 1.5% of the PEG or PEG-modified lipid on a molar basis.

In one embodiment, the formulations include about 50% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 10% of the neutral lipid, about 35 % of the sterol, about 4.5% or about 5% of the PEG or PEG-modified lipid, and about 0.5% of the targeting lipid on a molar basis.

In one embodiment, the formulations of include about 40% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 15% of the neutral lipid, about 40% of the sterol, and about 5% of the PEG or PEG-modified lipid on a molar basis.

In one embodiment, the formulations include about 57.2% of a cationic lipid selected from 2,2-dilinoleyl-4-dimethylaminoethyl-[l,3]-dioxolane (DLin-KC2-DMA), dilinoleyl-methyl-4-dimethylaminobutyrate (DLin-MC3-DMA), and di((Z)-non-2-en-1-yl) 9-((4-(dimethylamino)butanoyl)oxy)heptadecanedioate (L319), about 7.1% of the neutral lipid, about 34.3% of the sterol, and about 1.4% of the PEG or PEG-modified lipid on a molar basis.

In one embodiment, the formulations include about 57.5% of a cationic lipid selected from the PEG lipid is PEG-cDMA (PEG-cDMA is further discussed in Reyes et al. (J. Controlled Release, 107, 276-287 (2005), the contents of which are herein incorporated by reference in its entirety), about 7.5% of the neutral lipid, about 31.5 % of the sterol, and about 3.5% of the PEG or PEG-modified lipid on a molar basis.

In preferred embodiments, lipid nanoparticle formulation consists essentially of a lipid mixture in molar ratios of about 20-70% cationic lipid: 5-45% neutral lipid: 20-55% cholesterol: 0.5-15% PEG-modified lipid; more preferably in a molar ratio of about 20-60% cationic lipid: 5-25% neutral lipid: 25-55% cholesterol: 0.5-15% PEG-modified lipid.

In particular embodiments, the molar lipid ratio is approximately 50/10/38.5/1.5 (mol% cationic lipid/neutral lipid, e.g., DSPC/Chol/PEG-modified lipid, e.g., PEG-DMG, PEG-DSG or PEG-DPG), 57.2/7.1134.3/1.4 (mol% cationic lipid/ neutral lipid, e.g.,
DPPC/Chol/ PEG-modified lipid, e.g., PEG-cDMA), 40/15/40/5 (mol% cationic lipid/ neutral lipid, e.g., DSPC/Chol/ PEG-modified lipid, e.g., PEG-DMG), 50/10/35/4.5/0.5 (mol% cationic lipid/ neutral lipid, e.g., DSPC/Chol/ PEG-modified lipid, e.g., PEG-DSG),
50/10/35/5 (cationic lipid/ neutral lipid, e.g., DSPC/Chol/ PEG-modified lipid, e.g., PEG-DMG), 40/10/40/10 (mol% cationic lipid/ neutral lipid, e.g., DSPC/Chol/ PEG-modified lipid, e.g., PEG-DMG or PEG-cDMA), 35/15/40/10 (mol% cationic lipid/ neutral lipid, e.g., DSPC/Chol/ PEG-modified lipid, e.g., PEG-DMG or PEG-cDMA) or 52/13/30/5 (mol% cationic lipid/ neutral lipid, e.g., DSPC/Chol/ PEG-modified lipid, e.g., PEG-DMG or PEG-cDMA).

Exemplary lipid nanoparticle compositions and methods of making same are described, for example, in Semple et al. (2010) Nat. Biotechnol. 28: 172-176; Jayarama et al. (2012), Angew. Chem. Int. Ed., 51: 8529-8533; and Maier et al. (2013) Molecular Therapy 21, 1570-1578 (the contents of each of which are incorporated herein by reference in their entirety).

In one embodiment, the lipid nanoparticle formulations described herein may comprise a cationic lipid, a PEG lipid and a structural lipid and optionally comprise a non-cationic lipid. As a non-limiting example, the lipid nanoparticle may comprise about 40-60% of cationic lipid, about 5-15% of a non-cationic lipid, about 1-2% of a PEG lipid and about 30-50% of a structural lipid. As another non-limiting example, the lipid nanoparticle may comprise about 50% cationic lipid, about 10% non-cationic lipid, about 1.5% PEG lipid and about 38.5% structural lipid. As yet another non-limiting example, the lipid nanoparticle may comprise about 55% cationic lipid, about 10% non-cationic lipid, about 2.5% PEG lipid and about 32.5% structural lipid. In one embodiment, the cationic lipid may be any cationic lipid described herein such as, but not limited to, DLin-KC2-DMA, DLin-MC3-DMA and L319.

In one embodiment, the lipid nanoparticle formulations described herein may be 4 component lipid nanoparticles. The lipid nanoparticle may comprise a cationic lipid, a non-cationic lipid, a PEG lipid and a structural lipid. As a non-limiting example, the lipid nanoparticle may comprise about 40-60% of cationic lipid, about 5-15% of a non-cationic lipid, about 1-2% of a PEG lipid and about 30-50% of a structural lipid. As another non-limiting example, the lipid nanoparticle may comprise about 50% cationic lipid, about 10% non-cationic lipid, about 1.5% PEG lipid and about 38.5% structural lipid. As yet another non-limiting example, the lipid nanoparticle may comprise about 55% cationic lipid, about 10% non-cationic lipid, about 2.5% PEG lipid and about 32.5% structural lipid. In one embodiment, the cationic lipid may be any cationic lipid described herein such as, but not limited to, DLin-KC2-DMA, DLin-MC3-DMA and L319.

In one embodiment, the lipid nanoparticle formulations described herein may comprise a cationic lipid, a non-cationic lipid, a PEG lipid and a structural lipid. As a non-limiting example, the lipid nanoparticle comprise about 50% of the cationic lipid DLin-KC2-DMA, about 10% of the non-cationic lipid DSPC, about 1.5% of the PEG lipid PEG-DOMG and about 38.5% of the structural lipid cholesterol. As a non-limiting example, the lipid nanoparticle comprise about 50% of the cationic lipid DLin-MC3-DMA, about 10% of the non-cationic lipid DSPC, about 1.5% of the PEG lipid PEG-DOMG and about 38.5% of the structural lipid cholesterol. As a non-limiting example, the lipid nanoparticle comprise about 50% of the cationic lipid DLin-MC3 -DM A, about 10% of the non-cationic lipid DSPC, about 1.5% of the PEG lipid PEG-DMG and about 38.5% of the structural lipid cholesterol. As yet another non-limiting example, the lipid nanoparticle comprise about 55% of the cationic lipid L319, about 10% of the non-cationic lipid DSPC, about 2.5% of the PEG lipid PEG-DMG and about 32.5% of the structural lipid cholesterol.

In one embodiment, the cationic lipid may be selected from, but not limited to, a cationic lipid described in International Publication Nos. WO2012040184, WO2011153120, WO2011149733, WO2011090965, WO2011043913, WO2011022460, WO2012061259, WO2012054365, WO2012044638, WO2010080724, WO201021865, WO2008103276, WO2013086373 and WO2013086354, US Patent Nos. 7,893,302, 7,404,969, 8,283,333, and 8,466,122 and US Patent Publication No. US20100036115, US20120202871, US20130064894, US20130129785, US20130150625, US20130178541 and US20130225836; the contents of each of which are herein incorporated by reference in their entirety. In another embodiment, the cationic lipid may be selected from, but not limited to, formula A described in International Publication Nos. WO2012040184, WO2011153120, WO2011149733, WO2011090965, WO2011043913, WO2011022460, WO2012061259, WO2012054365, WO2012044638 and WO2013116126 or US Patent Publication No. US20130178541 and US20130225836; the contents of each of which is herein incorporated by reference in their entirety. In yet another embodiment, the cationic lipid may be selected from, but not limited to, formula CLI-CLXXIX of International Publication No. WO2008103276, formula CLI-CLXXIX of US Patent No. 7,893,302, formula CLI-CLXXXXII of US Patent No. 7,404,969 and formula I- VI of US Patent Publication No. US20100036115, formula I of US Patent Publication No US20130123338; each of which is herein incorporated by reference in their entirety. As a non-limiting example, the cationic lipid may be selected from (20Z,23Z)-N,N-dimethylnonacosa-20,23-dien-10-amine, (17Z,20Z)-N,N-dimemylhexacosa-17,20-dien-9-amine, (lZ,19Z)-N5N-dimethylpentacosa-l 6, 19-dien-8-amine, (13Z,16Z)-N,N-dimethyldocosa-13,16-dien-5-amine, (12Z,15Z)-N,N-dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimeihyloctacosa-19,22-dien-9-amine, (18Z,21 Z)-N,N-dimethylheptacosa- 18 ,21 -dien-8 -amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21 Z ,24Z)-N,N-dimethyltriaconta-21,24-dien-9-amine, (18Z)-N,N-dimetylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, l-[(HZ,14Z)-l-nonylicosa-l l,14-dien-l-yl] pyrrolidine, (20Z)-N,N-dimethylheptacos-20-en-l 0-amine, (15Z)-N,N-dimethyl eptacos-15-en-l 0-amine, (14Z)-N,N-dimethylnonacos- 14-en-10-amine, (17Z)-N,N-dimethylnonacos- 17-en-10-amine, (24Z)-N,N-dimethyltritriacont-24-en-10-amine, (20Z)-N,N-dimethylnonacos-20-en-l 0-amine, (22Z)-N,N-dimethylhentriacont-22-en-10-amine, (16Z)-N,N-dimethylpentacos-16-en-8-amine, ( 12Z, 15Z)-N,N-dimethyl-2-nonylhenicosa- 12, 15-dien- 1-amine, ( 13Z, 16Z)-N,N-dimethyl-3-nonyldocosa-13,16-dien-l-amine, N,N-dimethyl-l-[(lS,2R)-2-octylcyclopropyl] eptadecan-8-amine, 1 - [( 1 S ,2R)-2-hexylcyclopropyl] -N,N-dimethylnonadecan- 10-amine, N,N-dimethyl- 1 -[(IS ,2R)-2-octylcyclopropyl] nonadecan- 10-amine, N,N-dimethyl-21 - [(IS ,2R)-2-octylcyclopropyl]henicosan-10-amine,N,N-dimethyl- 1-[( 1 S ,2S)-2- { [(lR,2R)-2-pentylcyclopropyl] methyl} cyclopropyl] nonadecan- 10-amine ,N,N-dimethyl- 1 - [( 1 S ,2R)-2-octylcyclopropyl]hexadecan-8-amine, N,N-dimethyl-[(lR,2S)-2-undecylcyclopropyl]tetradecan-5-amine, N,N-dimethyl-3-{7-[(lS,2R)-2-octylcyclopropyl]heptyl} dodecan- 1-amine, l-[(lR,2S)-2-hepty lcyclopropyl]-N,N-dimethyloctadecan-9-amine, 1 - [( 1 S ,2R)-2-decylcyclopropyl] -N,N-dimethylpentadecan-6-amine, N,N-dimethyl-l-[(lS,2R)-2-octylcyclopropyl]pentadecan-8-amine, R-N,N-dimethyl- 1-[(9Z,12Z)-octadeca-9,12-dien-l-yloxy]-3-(octyloxy)propan-2-amine, S-N,N-dimethyl-l-[(9Z,12Z)-octadeca-9,12-dien-l-yloxy]-3-(octyloxy)propan-2-amine, l-{2-[(9Z,12Z)-octadeca-9,12-dien-l-yloxy]-l-[(octyloxy)methyl]ethyl}pyrrolidine, (2S)-N,N-dimethyl-l-[(9Z,12Z)-octadeca-9,12-dien-l-yloxy]-3-[(5Z)-oct-5-en-l-yloxy]propan-2-amine, l-{2-[(9Z, 12Z)-octadeca-9, 12-dien- 1 -yloxy] - 1 - [(octyloxy)methyl] ethyl } azetidine, (2S )- 1 -(hexyloxy)-N,N-dimethyl-3-[(9Z,12Z)-octadeca-9,12-dien-l-yloxy]propan-2-amine, (2S)-1-(heptyloxy)-N,N-dimethyl-3-[(9Z,12Z)-octadeca-9,12-dien-l-yloxy]propan-2-amine, N,N-dimethyl-l-(nonyloxy)-3-[(9Z,12Z)-octadeca-9,12-dien-l-yloxy]propan-2-amine, N,N-dimethyl-l-[(9Z)-octadec-9-en-l-yloxy]-3-(octyloxy)propan-2-amine; (2S)-N,N-dimethyl-l-[(6Z,9Z,12Z)-octadeca-6,9,12-trien-l-yloxy]-3-(octyloxy)propan-2-amine, (2S)-1-[(1 lZ,14Z)-icosa-ll,14-dien-l-yloxy]-N,N-dimethyl-3-(pentyloxy)propan-2-amine, (2S)-1-(hexyloxy)-3-[(llZ,14Z)-icosa-1ll,14-dien-l-yloxy]-N,N-dimethylpropan-2-amine, 1-[(1 lZ,14Z)-icosa-ll,14-dien-l-yloxy]-N,N-dimethyl-3-(octyloxy)propan-2-amine, 1-[( 13Z, 16Z)-docosa-13 , 16-dien-l-yloxy] -N,N-dimethyl-3-(octyloxy)propan-2-amine, (2S)- 1 -[( 13Z, 16Z)-docosa- 13 , 16-dien- 1 -yloxy] -3-(hexyloxy)-N,N-dimethylpropan-2-amine, (2S)- 1 -[(13Z)-docos-13-en-l-yloxy]-3-(hexyloxy)-N,N-dimethylpropan-2-amine, l-[(13Z)-docos-13-en- 1 -yloxy] -N,N-dimethyl-3 -(octyloxy)propan-2-amine, 1 - [(9Z)-hexadec-9-en- 1 -yloxy] -N,N-dimethyl-3-(octyloxy)propan-2-amine, (2R)-N,N-dimethyl-H(l-metoylo ctyl)oxy]-3-[(9Z,12Z)-octadeca-9,12-dien-l-yloxy]propan-2-amine, (2R)-1-[(3,7-dimethyloctyl)oxy]-N,N-dimethyl-3-[(9Z,12Z)-octadeca-9,12-dien-l-yloxy]propan-2-amine, N,N-dimethyl-l-(octyloxy)-3-({ 8-[(lS,2S)-2-{ [(lR,2R)-2-pentylcyclopropyl]methyl}cyclopropyl]octyl} oxy)propan-2-amine, N,N-dimethyl- 1-{ [8-(2-oc 1 ylcyclopropyl)octyl] oxy } -3 -(octyloxy)propan-2-amine and (11E,20Z,23Z)-N,N-dimethylnonacosa-ll,20,2-trien-10-amine or a pharmaceutically acceptable salt or stereoisomer thereof.

In one embodiment, the lipid may be a cleavable lipid such as those described in International Publication No. WO2012170889, herein incorporated by reference in its entirety. In another embodiment, the lipid may be a cationic lipid such as, but not limited to, Formula (I) of U.S. Patent Application No. US20130064894, the contents of which are herein incorporated by reference in its entirety.

The cationic lipid may be synthesized by methods known in the art and/or as described in International Publication Nos. WO2012040184, WO2011153120, WO2011149733, WO2011090965, WO2011043913, WO2011022460, WO2012061259, WO2012054365, WO2012044638, WO2010080724, WO201021865, WO2013086373 and WO2013086354; the contents of each of which are herein incorporated by reference in their entirety.

The cationic lipid may be a trialkyl cationic lipid. Non-limiting examples of trialkyl cationic lipids and methods of making and using the trialkyl cationic lipids are described in International Patent Publication No. WO2013126803, the contents of which are herein incorporated by reference in its entirety.

LNP formulations of the RNA vaccines may contain PEG-c-DOMG at 3% lipid molar ratio. In another embodiment, the LNP formulations of the RNA vaccines may contain PEG-c-DOMG at 1.5% lipid molar ratio. Pharmaceutical compositions of the RNA vaccines may include at least one of the PEGylated lipids described in International Publication No. WO2012099755, the contents of which is herein incorporated by reference in its entirety.

The LNP formulation may contain PEG-DMG 2000 (1,2-dimyristoyl-sn-glycero-3-phophoethanolamine-N-[methoxy(polyethylene glycol)-2000). In one embodiment, the LNP formulation may contain PEG-DMG 2000, a cationic lipid known in the art and at least one other component. In another embodiment, the LNP formulation may contain PEG-DMG 2000, a cationic lipid known in the art, DSPC and cholesterol. As a non-limiting example, the LNP formulation may contain PEG-DMG 2000, DLin-DMA, DSPC and cholesterol. As another non-limiting example the LNP formulation may contain PEG-DMG 2000, DLin-DMA, DSPC and cholesterol in a molar ratio of 2:40: 10:48 (see e.g., Geall et al., Nonviral delivery of selfamplifying RNA vaccines, PNAS 2012; PMID:
22908294; herein incorporated by reference in its entirety).

An LNP formulation may be formulated by the methods described in International Publication Nos. WO2011127255 or WO2008103276, the contents of each of which is herein incorporated by reference in their entirety. As a non-limiting example, the RNA vaccines described herein may be encapsulated in LNP formulations as described in WO2011127255 and/or WO2008103276; each of which is herein incorporated by reference in their entirety.

The RNA vaccines described herein may be formulated in a nanoparticle to be delivered by a parenteral route as described in U.S. Pub. No. US20120207845; the contents of which are herein incorporated by reference in its entirety.

In one embodiment, the RNA vaccines may be formulated in a lipid nanoparticle made by the methods described in US Patent Publication No US20130156845 or

International Publication No WO2013093648 or WO2012024526, each of which is herein incorporated by reference in its entirety. The lipid nanoparticles described herein may be made in a sterile environment by the system and/or methods described in US Patent Publication No. US20130164400, herein incorporated by reference in its entirety.

An LNP formulation may be formulated in a nanoparticle such as a nucleic acid-lipid particle described in US Patent No. 8,492,359, the contents of which are herein incorporated by reference in its entirety. As a non-limiting example, the lipid particle may comprise one or more active agents or therapeutic agents; one or more cationic lipids comprising from about 50 mol % to about 85 mol % of the total lipid present in the particle; one or more non-cationic lipids comprising from about 13 mol % to about 49.5 mol % of the total lipid present in the particle; and one or more conjugated lipids that inhibit aggregation of particles comprising from about 0.5 mol % to about 2 mol % of the total lipid present in the particle. The nucleic acid in the nanoparticle may be the polynucleotides described herein and/or are known in the art.

An LNP formulation may be formulated by the methods described in International Publication Nos. WO2011127255 or WO2008103276, the contents of each of which are herein incorporated by reference in their entirety. As a non-limiting example, modified RNA described herein may be encapsulated in LNP formulations as described in WO2011127255 and/or WO2008103276; the contents of each of which are herein incorporated by reference in their entirety. LNP formulations described herein may comprise a polycationic composition. As a non-limiting example, the polycationic composition may be selected from formula 1-60 of US Patent Publication No. US20050222064; the content of which is herein incorporated by reference in its entirety. LNP formulations comprising a polycationic composition may be used for the delivery of the modified RNA described herein in vivo and/or in vitro.

In one embodiment, the LNP formulations described herein may additionally comprise a permeability enhancer molecule. Non-limiting permeability enhancer molecules are described in US Patent Publication No. US20050222064; the content of which is herein incorporated by reference in its entirety.

The RNA vaccine pharmaceutical compositions may be formulated in liposomes such as, but not limited to, DiLa2 liposomes (Marina Biotech, Bothell, WA), SMARTICLES^{®} (Marina Biotech, Bothell, WA), neutral DOPC (1,2-dioleoyl-sn-glycero-3-phosphocholine) based liposomes (e.g., siRNA delivery for ovarian cancer (Landen et al. Cancer Biology & Therapy 2006 5(12)1708-1713); herein incorporated by reference in its entirety) and hyaluronan-coated liposomes (Quiet Therapeutics, Israel).

In one embodiment, the RNA vaccines may be formulated in a lyophilized gel-phase liposomal composition as described in US Publication No. US2012060293, herein incorporated by reference in its entirety.

The RNA vaccine may be associated with a cationic or polycationic compounds, including protamine, nucleoline, spermine or spermidine, or other cationic peptides or proteins, such as poly-L-lysine (PLL), polyarginine, basic polypeptides, cell penetrating peptides (CPPs), including HIV-binding peptides, HIV-1 Tat (HIV), Tat-derived peptides, Penetratin, VP22 derived or analog peptides, Pestivirus Erns, HSV, VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapediaderived peptides (particularly from Drosophila antennapedia), pAntp, plsl, FGF, Lactoferrin, Transportan, Buforin-2, Bac715-24, SynB, SynB(l), pVEC, hCT-derived peptides, SAP, histones, cationic polysaccharides, for example chitosan, polybrene, cationic polymers, e.g. polyethyleneimine (PEI), cationic lipids, e.g. DOTMA: [l-(2,3-sioleyloxy)propyl)]-N,N,N-trimethylammonium chloride, DMRIE, di-C14-amidine, DOTIM, SAINT, DC-Choi, BGTC, CTAP, DOPC, DODAP, DOPE: Dioleylphosphatidylethanol-amine, DOSPA, DODAB, DOIC, DMEPC, DOGS: Dioctadecylamidoglicylspermin, DIMRI: Dimyristooxypropyl dimethyl hydroxyethyl ammonium bromide, DOTAP: dioleoyloxy-3-(trimethylammonio)propane, DC-6-14: 0,0-ditetradecanoyl-N-.alpha.-trimethylammonioacetyl)diethanolamine chloride, CLIP 1: rac-[(2,3-dioctadecyloxypropyl)(2-hydroxyethyl)]-dimethylammonium chloride, CLIP6: rac-[2(2,3-dihexadecyloxypropyloxymethyloxy)ethyl]-trimethylammonium, CLIP9: rac-[2(2,3-dihexadecyloxypropyloxysuccinyloxy)ethyl]-trimethylammo- nium, oligofectamine, or cationic or polycationic polymers, e.g. modified polyaminoacids, such as beta-aminoacid-polymers or reversed polyamides, etc., modified polyethylenes, such as PVP (poly(N-ethyl-4-vinylpyridinium bromide)), etc., modified acrylates, such as pDMAEMA(poly(dimethylaminoethyl methylacrylate)), etc., modified amidoamines such as pAMAM (poly(amidoamine)), etc., modified polybetaminoester (PBAE), such as diamine end modified 1,4 butanediol diacrylate-co-5 -amino- 1-pentanol polymers, etc., dendrimers, such as polypropylamine dendrimers or pAMAM based dendrimers, etc., polyimine(s), such as PEL poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

Alternatively the RNA vaccine is not associated with a cationic or polycationic compounds.

The nanoparticle formulations may comprise a phosphate conjugate. The phosphate conjugate may increase in vivo circulation times and/or increase the targeted delivery of the nanoparticle. Phosphate conjugates for use with the present invention may be made by the methods described in International Application No. WO2013033438 or US Patent Publication No. US20130196948, the contents of each of which are herein incorporated by reference in its entirety. As a non-limiting example, the phosphate conjugates may include a compound of any one of the formulas described in International Application No. WO2013033438, herein incorporated by reference in its entirety.

The nanoparticle formulation may comprise a polymer conjugate. The polymer conjugate may be a water soluble conjugate. The polymer conjugate may have a structure as described in U.S. Patent Application No. 20130059360, the contents of which are herein incorporated by reference in its entirety. In one aspect, polymer conjugates with the
polynucleotides of the present invention may be made using the methods and/or segmented polymeric reagents described in U.S. Patent Application No. 20130072709, herein incorporated by reference in its entirety. In another aspect, the polymer conjugate may have pendant side groups comprising ring moieties such as, but not limited to, the polymer conjugates described in US Patent Publication No. US20130196948, the contents of which is herein incorporated by reference in its entirety.

The nanoparticle formulations may comprise a conjugate to enhance the delivery of nanoparticles of the present invention in a subject. Further, the conjugate may inhibit phagocytic clearance of the nanoparticles in a subject. In one aspect, the conjugate may be a "self peptide designed from the human membrane protein CD47 (e.g., the "self particles described by Rodriguez et al (Science 2013 339, 971-975), herein incorporated by reference in its entirety). As shown by Rodriguez et al. the self peptides delayed macrophage-mediated clearance of nanoparticles which enhanced delivery of the nanoparticles. In another aspect, the conjugate may be the membrane protein CD47 (e.g., see Rodriguez et al. Science 2013 339, 971-975, herein incorporated by reference in its entirety). Rodriguez et al. showed that, similarly to "self peptides, CD47 can increase the circulating particle ratio in a subject as compared to scrambled peptides and PEG coated nanoparticles.

RNA vaccines may be formulated in nanoparticles which comprise a conjugate to enhance the delivery of the nanoparticles of the present invention in a subject. The conjugate may be the CD47 membrane or the conjugate may be derived from the CD47 membrane protein, such as the "self peptide described previously. In another aspect the nanoparticle may comprise PEG and a conjugate of CD47 or a derivative thereof. In yet another aspect, the nanoparticle may comprise both the "self peptide described above and the membrane protein CD47. A "self peptide and/or CD47 protein may be conjugated to a viruslike particle or pseudovirion, as described herein for delivery of the RNA vaccines.

The formulations herein may include RNA vaccine pharmaceutical compositions comprising the polynucleotides of the present invention and a conjugate which may have a degradable linkage. Non-limiting examples of conjugates include an aromatic moiety comprising an ionizable hydrogen atom, a spacer moiety, and a water-soluble polymer. As a non-limiting example, pharmaceutical compositions comprising a conjugate with a degradable linkage and methods for delivering such pharmaceutical compositions are described in US Patent Publication No. US20130184443, the contents of which are herein incorporated by reference in its entirety.

The nanoparticle formulations may be a carbohydrate nanoparticle comprising a carbohydrate carrier and a RNA vaccine. As a non-limiting example, the carbohydrate carrier may include, but is not limited to, an anhydride-modified phytoglycogen or glycogen-type material, phtoglycogen octenyl succinate, phytoglycogen beta-dextrin, anhydride-modified phytoglycogen beta-dextrin. (See e.g., International Publication No. WO2012109121; the contents of which are herein incorporated by reference in its entirety).

Nanoparticle formulations may be coated with a surfactant or polymer in order to improve the delivery of the particle. In one embodiment, the nanoparticle may be coated with a hydrophilic coating such as, but not limited to, PEG coatings and/or coatings that have a neutral surface charge. The hydrophilic coatings may help to deliver nanoparticles with larger payloads such as, but not limited to, RNA vaccines within the central nervous system. As a non-limiting example nanoparticles comprising a hydrophilic coating and methods of making such nanoparticles are described in US Patent Publication No. US20130183244, the contents of which are herein incorporated by reference in its entirety.

In one embodiment, the lipid nanoparticles of the present invention may be hydrophilic polymer particles. Non-limiting examples of hydrophilic polymer particles and methods of making hydrophilic polymer particles are described in US Patent Publication No. US20130210991, the contents of which are herein incorporated by reference in its entirety.

In another embodiment, the lipid nanoparticles of the present invention may be hydrophobic polymer particles.

Lipid nanoparticle formulations may be improved by replacing the cationic lipid with a biodegradable cationic lipid which is known as a rapidly eliminated lipid nanoparticle (reLNP). Ionizable cationic lipids, such as, but not limited to, DLinDMA, DLin-KC2-DMA, and DLin-MC3-DMA, have been shown to accumulate in plasma and tissues over time and may be a potential source of toxicity. The rapid metabolism of the rapidly eliminated lipids can improve the tolerability and therapeutic index of the lipid nanoparticles by an order of magnitude from a 1 mg/kg dose to a 10 mg/kg dose in rat. Inclusion of an enzymatically degraded ester linkage can improve the degradation and metabolism profile of the cationic component, while still maintaining the activity of the reLNP formulation. The ester linkage can be internally located within the lipid chain or it may be terminally located at the terminal end of the lipid chain. The internal ester linkage may replace any carbon in the lipid chain. In one embodiment, the internal ester linkage may be located on either side of the saturated carbon.

In one embodiment, an immune response may be elicited by delivering a lipid nanoparticle which may include a nanospecies, a polymer and an immunogen. (U.S. Publication No. 20120189700 and International Publication No. WO2012099805; each of which is herein incorporated by reference in their entirety). The polymer may encapsulate the nanospecies or partially encapsulate the nanospecies. The immunogen may be a recombinant protein, a modified RNA and/or a polynucleotide described herein. In one embodiment, the lipid nanoparticle may be formulated for use in a vaccine such as, but not limited to, against a pathogen. Lipid nanoparticles may be engineered to alter the surface properties of particles so the lipid nanoparticles may penetrate the mucosal barrier. Mucus is located on mucosal tissue such as, but not limted to, oral (e.g., the buccal and esophageal membranes and tonsil tissue), ophthalmic, gastrointestinal (e.g., stomach, small intestine, large intestine, colon, rectum), nasal, respiratory (e.g., nasal, pharyngeal, tracheal and bronchial membranes), genital (e.g., vaginal, cervical and urethral membranes). Nanoparticles larger than 10-200 nm which are preferred for higher drug encapsulation efficiency and the ability to provide the sustained delivery of a wide array of drugs have been thought to be too large to rapidly diffuse through mucosal barriers. Mucus is continuously secreted, shed, discarded or digested and recycled so most of the trapped particles may be removed from the mucosal tissue within seconds or within a few hours. Large polymeric nanoparticles (200nm -500nm in diameter) which have been coated densely with a low molecular weight polyethylene glycol (PEG) diffused through mucus only 4 to 6-fold lower than the same particles diffusing in water (Lai et al. PNAS 2007 104(5): 1482-487; Lai et al. Adv Drug Deliv Rev. 2009 61(2): 158-171; each of which is herein incorporated by reference in their entirety). The transport of nanoparticles may be determined using rates of permeation and/or fluorescent microscopy techniques including, but not limited to, fluorescence recovery after photobleaching (FRAP) and high resolution multiple particle tracking (MPT). As a non-limiting example, compositions which can penetrate a mucosal barrier may be made as described in U.S. Pat. No. 8,241,670 or International Patent Publication No. WO2013110028, the contents of each of which are herein incorporated by reference in its entirety.

The lipid nanoparticle engineered to penetrate mucus may comprise a polymeric material (i.e. a polymeric core) and/or a polymer-vitamin conjugate and/or a tri-block copolymer. The polymeric material may include, but is not limited to, polyamines, polyethers, polyamides, polyesters, polycarbamates, polyureas, polycarbonates, poly(styrenes), polyimides, polysulfones, polyurethanes, polyacetylenes, polyethylenes, polyethyeneimines, polyisocyanates, polyacrylates, polymethacrylates, polyacrylonitriles, and polyarylates. The polymeric material may be biodegradable and/or biocompatible. Non-limiting examples of biocompatible polymers are described in International Patent Publication No. WO2013116804, the contents of which are herein incorporated by reference in its entirety. The polymeric material may additionally be irradiated. As a non-limiting example, the polymeric material may be gamma irradiated (See e.g., International App. No.WO201282165, herein incorporated by reference in its entirety). Non-limiting examples of specific polymers include poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L-lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) and copolymers and mixtures thereof, polydioxanone and its copolymers, polyhydroxyalkanoates, polypropylene fumarate, polyoxymethylene, poloxamers, poly(ortho)esters, poly(butyric acid), poly(valeric acid), poly(lactide-co-caprolactone), PEG-PLGA-PEG and trimethylene carbonate, polyvinylpyrrolidone. The lipid nanoparticle may be coated or associated with a co-polymer such as, but not limited to, a block co-polymer (such as a branched polyether-polyamide block copolymer described in
International Publication No. WO2013012476, herein incorporated by reference in its entirety), and (poly(ethylene glycol))-(poly(propylene oxide))-(poly(ethylene glycol)) triblock copolymer (see e.g., US Publication 20120121718 and US Publication 20100003337 and U.S. Pat. No. 8,263,665; each of which is herein incorporated by reference in their entirety). The co-polymer may be a polymer that is generally regarded as safe (GRAS) and the formation of the lipid nanoparticle may be in such a way that no new chemical entities are created. For example, the lipid nanoparticle may comprise poloxamers coating PLGA nanoparticles without forming new chemical entities which are still able to rapidly penetrate human mucus (Yang et al. Angew. Chem. Int. Ed. 2011 50:2597-2600; the contents of which are herein incorporated by reference in its entirety).

A non-limiting scalable method to produce nanoparticles which can penetrate human mucus is described by Xu et al. (See e.g., J Control Release 2013, 170(2):279-86; the contents of which are herein incorporated by reference in its entirety).

The vitamin of the polymer-vitamin conjugate may be vitamin E. The vitamin portion of the conjugate may be substituted with other suitable components such as, but not limited to, vitamin A, vitamin E, other vitamins, cholesterol, a hydrophobic moiety, or a hydrophobic component of other surfactants (e.g., sterol chains, fatty acids, hydrocarbon chains and alkylene oxide chains).

The lipid nanoparticle engineered to penetrate mucus may include surface altering agents such as, but not limited to, polynucleotides, anionic proteins (e.g., bovine serum albumin), surfactants (e.g., cationic surfactants such as for example dimethyldioctadecyl-ammonium bromide), sugars or sugar derivatives (e.g., cyclodextrin), nucleic acids, polymers (e.g., heparin, polyethylene glycol and poloxamer), mucolytic agents (e.g., N-acetylcysteine, mugwort, bromelain, papain, clerodendrum, acetylcysteine, bromhexine, carbocisteine, eprazinone, mesna, ambroxol, sobrerol, domiodol, letosteine, stepronin, tiopronin, gelsolin, thymosin β4 dornase alfa, neltenexine, erdosteine) and various DNases including rhDNase. The surface altering agent may be embedded or enmeshed in the particle's surface or disposed (e.g., by coating, adsorption, covalent linkage, or other process) on the surface of the lipid nanoparticle. (see e.g., US Publication 20100215580 and US Publication 20080166414 and US20130164343; the contents of each of which is herein incorporated by reference in their entirety).

The mucus penetrating lipid nanoparticles may comprise at least one polynucleotide described herein. The polynucleotide may be encapsulated in the lipid nanoparticle and/or disposed on the surface of the particle. The polynucleotide may be
covalently coupled to the lipid nanoparticle. Formulations of mucus penetrating lipid nanoparticles may comprise a plurality of nanoparticles. Further, the formulations may contain particles which may interact with the mucus and alter the structural and/or adhesive properties of the surrounding mucus to decrease mucoadhesion which may increase the delivery of the mucus penetrating lipid nanoparticles to the mucosal tissue.

The mucus penetrating lipid nanoparticles may be a hypotonic formulation comprising a mucosal penetration enhancing coating. The formulation may be hypotonic for the epithelium to which it is being delivered. Non-limiting examples of hypotonic formulations may be found in International Patent Publication No. WO2013110028, the contents of which are herein incorporated by reference in its entirety. In order to enhance the delivery through the mucosal barrier the RNA vaccine formulation may comprise or be a hypotonic solution. Hypotonic solutions were found to increase the rate at which mucoinert particles such as, but not limited to, mucus-penetrating particles, were able to reach the vaginal epithelial surface (See e.g., Ensign et al. Biomaterials 2013 34(28):6922-9; the contents of which is herein incorporated by reference in its entirety).

In one embodiment, the RNA vaccine is formulated as a lipoplex, such as, without limitation, the ATUPLEXTM system, the DACC system, the DBTC system and other siRNA-lipoplex technology from Silence Therapeutics (London, United Kingdom), STEMFECTTM from STEMGENT^{®} (Cambridge, MA), and polyethylenimine (PEI) or protamine-based targeted and non-targeted delivery of nucleic acids acids (Aleku et al. Cancer Res. 2008 68:9788-9798; Strumberg et al. Int J Clin Pharmacol Ther 2012 50:76-78; Santel et al., Gene Ther 2006 13: 1222-1234; Santel et al., Gene Ther 2006 13: 1360-1370; Gutbier et al., Pulm Pharmacol. Ther. 2010 23:334-344; Kaufmann et al. Microvasc Res 2010 80:286-293 Weide et al. J Immunother. 2009 32:498-507; Weide et al. J Immunother. 2008 31: 180-188; Pascolo Expert Opin. Biol. Ther. 4: 1285-1294; Fotin-Mleczek et al., 2011 J. Immunother. 34: 1-15; Song et al., Nature Biotechnol. 2005, 23:709-717; Peer et al., Proc Natl Acad Sci USA. 2007 6; 104:4095-4100; deFougerolles Hum Gene Ther. 2008 19: 125-132; all of which are incorporated herein by reference in its entirety).

Such formulations may also be constructed or compositions altered such that they passively or actively are directed to different cell types in vivo, including but not limited to hepatocytes, immune cells, tumor cells, endothelial cells, antigen presenting cells, and leukocytes (Akinc et al. Mol Ther. 2010 18: 1357-1364; Song et al., Nat Biotechnol. 2005 23:709-717; Judge et al., J Clin Invest. 2009 119:661-673; Kaufmann et al., Microvasc Res 2010 80:286-293; Santel et al., Gene Ther 2006 13: 1222-1234; Santel et al., Gene Ther 2006 13: 1360-1370; Gutbier et al., Pulm Pharmacol. Ther. 2010 23:334-344; Basha et al., Mol. Ther. 2011 19:2186-2200; Fenske and Cullis, Expert Opin Drug Deliv. 2008 5:25-44; Peer et al., Science. 2008 319:627-630; Peer and Lieberman, Gene Ther. 2011 18: 1127-1133; all of which are incorporated herein by reference in its entirety). One example of passive targeting of formulations to liver cells includes the DLin-DMA, DLin-KC2-DMA and DLin-MC3 -DMA-based lipid nanoparticle formulations which have been shown to bind to apolipoprotein E and promote binding and uptake of these formulations into hepatocytes in vivo (Akinc et al. Mol Ther. 2010 18: 1357-1364; herein incorporated by reference in its entirety). Formulations can also be selectively targeted through expression of different ligands on their surface as exemplified by, but not limited by, folate, transferrin, N-acetylgalactosamine (GalNAc), and antibody targeted approaches (Kolhatkar et al., Curr Drug Discov Technol. 2011 8: 197-206; Musacchio and Torchilin, Front Biosci. 2011 16: 1388-1412; Yu et al., Mol Membr Biol. 2010 27:286-298; Patil et al., Crit Rev Ther Drug Carrier Syst. 2008 25: 1-61; Benoit et al., Biomacromolecules. 2011 12:2708-2714; Zhao et al., Expert Opin Drug Deliv. 2008 5:309-319; Akinc et al., Mol Ther. 2010 18: 1357-1364; Srinivasan et al., Methods Mol Biol. 2012 820: 105-116; Ben-Arie et al., Methods Mol Biol. 2012 757:497-507; Peer 2010 J Control Release. 20:63-68; Peer et al., Proc Natl Acad Sci USA. 2007 104:4095-4100; Kim et al., Methods Mol Biol. 2011 721:339-353; Subramanya et al., Mol Ther. 2010 18:2028-2037; Song et al., Nat Biotechnol. 2005 23:709-717; Peer et al., Science. 2008 319:627-630; Peer and Lieberman, Gene Ther. 2011 18: 1127-1133; all of which are incorporated herein by reference in its entirety).

The RNA vaccine may be formulated as a solid lipid nanoparticle. A solid lipid nanoparticle (SLN) may be spherical with an average diameter between 10 to 1000 nm. SLN possess a solid lipid core matrix that can solubilize lipophilic molecules and may be stabilized with surfactants and/or emulsifiers. In a further embodiment, the lipid nanoparticle may be a self-assembly lipid-polymer nanoparticle (see Zhang et al., ACS Nano, 2008, 2 (8), pp 1696-1702; the contents of which are herein incorporated by reference in its entirety). As a non-limiting example, the SLN may be the SLN described in International Patent Publication No. WO2013105101, the contents of which are herein incorporated by reference in its entirety. As another non-limiting example, the SLN may be made by the methods or processes described in International Patent Publication No. WO2013105101, the contents of which are herein incorporated by reference in its entirety.

### Ill

Liposomes, lipoplexes, or lipid nanoparticles may be used to improve the efficacy of polynucleotides directed protein production as these formulations may be able to increase cell transfection by the RNA vaccine; and/or increase the translation of encoded protein. One such example involves the use of lipid encapsulation to enable the effective systemic delivery of polyplex plasmid DNA (Heyes et al., Mol Ther. 2007 15:713-720; herein incorporated by reference in its entirety). The liposomes, lipoplexes, or lipid nanoparticles may also be used to increase the stability of the polynucleotide.

RNA vaccines of the present invention can also be formulated for controlled release and/or targeted delivery. As used herein, "controlled release" refers to a pharmaceutical composition or compound release profile that conforms to a particular pattern of release to effect a therapeutic outcome. In one embodiment, the RRNA vaccines may be encapsulated into a delivery agent described herein and/or known in the art for controlled release and/or targeted delivery. As used herein, the term "encapsulate" means to enclose, surround or encase. As it relates to the formulation of the compounds of the invention, encapsulation may be substantial, complete or partial. The term "substantially encapsulated" means that at least greater than 50, 60, 70, 80, 85, 90, 95, 96, 97, 98, 99, 99.9, 99.9 or greater than 99.999% of the pharmaceutical composition or compound of the invention may be enclosed, surrounded or encased within the delivery agent. "Partially encapsulated" means that less than 10, 10, 20, 30, 40 50 or less of the pharmaceutical composition or compound of the invention may be enclosed, surrounded or encased within the delivery agent.

Advantageously, encapsulation may be determined by measuring the escape or the activity of the pharmaceutical composition or compound of the invention using fluorescence and/or electron micrograph. For example, at least 1, 5, 10, 20, 30, 40, 50, 60, 70, 80, 85, 90, 95, 96, 97, 98, 99, 99.9, 99.99 or greater than 99.99% of the pharmaceutical composition or compound of the invention are encapsulated in the delivery agent. A controlled release formulation may include, but is not limited to, tri-block co-polymers. As a non-limiting example, the formulation may include two different types of tri-block co-polymers (International Pub. No. WO2012131104 and WO2012131106; the contents of each of which is herein incorporated by reference in its entirety).

RNA vaccines may be encapsulated into a lipid nanoparticle or a rapidly eliminated lipid nanoparticle and the lipid nanoparticles or a rapidly eliminated lipid nanoparticle may then be encapsulated into a polymer, hydrogel and/or surgical sealant described herein and/or known in the art. As a non-limiting example, the polymer, hydrogel or surgical sealant may be PLGA, ethylene vinyl acetate (EVAc), poloxamer, GELSITE^{®} (Nanotherapeutics, Inc. Alachua, FL), HYLENEX^{®} (Halozyme Therapeutics, San Diego CA), surgical sealants such as fibrinogen polymers (Ethicon Inc. Cornelia, GA), TISSELL^{®} (Baxter International, Inc Deerfield, IL), PEG-based sealants, and COSEAL^{®} (Baxter International, Inc Deerfield, IL).

The lipid nanoparticle may be encapsulated into any polymer known in the art which may form a gel when injected into a subject. As another non-limiting example, the lipid nanoparticle may be encapsulated into a polymer matrix which may be biodegradable.

The RNA vaccine formulation for controlled release and/or targeted delivery may also include at least one controlled release coating. Controlled release coatings include, but are not limited to, OPADRY^{®}, polyvinylpyrrolidone/vinyl acetate copolymer, polyvinylpyrrolidone, hydroxypropyl methylcellulose, hydroxypropyl cellulose, hydroxyethyl cellulose, EUDRAGIT RL^{®}, EUDRAGIT RS^{®} and cellulose derivatives such as ethylcellulose aqueous dispersions (AQUACOAT^{®} and SURELEASE^{®}).

In one embodiment, the RNA vaccine controlled release and/or targeted delivery formulation may comprise at least one degradable polyester which may contain polycationic side chains. Degradeable polyesters include, but are not limited to, poly(serine ester), poly(L-lactide-co-L-lysine), poly(4-hydroxy-L-proline ester), and combinations thereof. In another embodiment, the degradable polyesters may include a PEG conjugation to form a PEGylated polymer. The RNA vaccine controlled release and/or targeted delivery formulation comprising at least one polynucleotide may comprise at least one PEG and/or PEG related polymer derivatives as described in US Patent No. 8,404,222, herein incorporated by reference in its entirety.

The RNA vaccine controlled release delivery formulation comprising at least one polynucleotide may be the controlled release polymer system described in US20130130348, herein incorporated by reference in its entirety. RNA vaccines of the present invention may be encapsulated in a therapeutic nanoparticle, referred to herein as "therapeutic nanoparticle RRNA vaccines." Therapeutic nanoparticles may be formulated by methods described herein and known in the art such as, but not limited to, International Pub Nos. WO2010005740, WO2010030763, WO2010005721, WO2010005723, WO2012054923, US Pub. Nos. US20110262491, US20100104645, US20100087337, US20100068285, US20110274759, US20100068286, US20120288541, US20130123351 and US20130230567 and US Pat No. 8,206,747, 8,293,276, 8,318,208 and 8,318,211; the contents of each of which are herein incorporated by reference in their entirety. In another embodiment, therapeutic polymer nanoparticles may be identified by the methods described in US Pub No. US20120140790, the contents of which is herein incorporated by reference in its entirety.

A therapeutic nanoparticle RNA vaccine may be formulated for sustained release. As used herein, "sustained release" refers to a pharmaceutical composition or compound that conforms to a release rate over a specific period of time. The period of time may include, but is not limited to, hours, days, weeks, months and years. As a non-limiting example, the sustained release nanoparticle may comprise a polymer and a therapeutic agent such as, but not limited to, the polynucleotides of the present invention (see International Pub No. 2010075072 and US Pub No. US20100216804, US20110217377 and US20120201859, each of which is herein incorporated by reference in their entirety). In another non-limiting example, the sustained release formulation may comprise agents which permit persistent bioavailability such as, but not limited to, crystals, macromolecular gels and/or particulate suspensions (see US Patent Publication No US20130150295, the contents of which is herein incorporated by reference in its entirety).

In one embodiment, the therapeutic nanoparticle RNA vaccines may be formulated to be target specific. As a non-limiting example, the therapeutic nanoparticles may include a corticosteroid (see International Pub. No. WO2011084518; herein incorporated by reference in its entirety). As a non-limiting example, the therapeutic nanoparticles may be formulated in nanoparticles described in International Pub No. WO2008121949, WO2010005726, WO2010005725, WO2011084521 and US Pub No. US20100069426, US20120004293 and US20100104655, each of which is herein incorporated by reference in their entirety.

The nanoparticles of the present invention may comprise a polymeric matrix. As a non-limiting example, the nanoparticle may comprise two or more polymers such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polylysine, poly(ethylene imine), poly(serine ester), poly(L-lactide-co-L-lysine), poly(4-hydroxy-L-proline ester) or combinations thereof.

In one embodiment, the therapeutic nanoparticle comprises a diblock copolymer. In one embodiment, the diblock copolymer may include PEG in combination with a polymer such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polylysine, poly(ethylene imine), poly(serine ester), poly(L-lactide-co-L-lysine), poly(4-hydroxy-L-proline ester) or combinations thereof. In another embodiment, the diblock copolymer may comprise the diblock copolymers described in European Patent Publication No. the contents of which are herein incorporated by reference in its entirety. In yet another embodiment, the diblock copolymer may be a high-X diblock copolymer such as those described in International Patent Publication No. WO2013120052, the contents of which are herein incorporated by reference in its entirety.

As a non-limiting example the therapeutic nanoparticle comprises a PLGA-PEG block copolymer (see US Pub. No. US20120004293 and US Pat No. 8,236,330, each of which is herein incorporated by reference in their entirety). In another non-limiting example, the therapeutic nanoparticle is a stealth nanoparticle comprising a diblock copolymer of PEG and PLA or PEG and PLGA (see US Pat No 8,246,968 and International Publication No. WO2012166923, the contents of each of which are herein incorporated by reference in its entirety). In yet another non-limiting example, the therapeutic nanoparticle is a stealth nanoparticle or a target- specific stealth nanoparticle as described in US Patent Publication No. US20130172406, the contents of which are herein incorporated by reference in its entirety.

The therapeutic nanoparticle may comprise a multiblock copolymer (See e.g., U.S. Pat. No. 8,263,665 and 8,287,910 and US Patent Pub. No. US20130195987; the contents of each of which are herein incorporated by reference in its entirety). In yet another non-limiting example, the lipid nanoparticle comprises the block copolymer PEG-PLGA-PEG (see e.g., the thermosensitive hydrogel (PEG-PLGA-PEG) was used as a TGF-betal gene delivery vehicle in Lee et al. Thermosensitive Hydrogel as a Tgf-βḯ Gene Delivery Vehicle Enhances Diabetic Wound Healing. Pharmaceutical Research, 2003 20(12): 1995-2000; as a controlled gene delivery system in Li et al. Controlled Gene Delivery System Based on Thermosensitive Biodegradable Hydrogel. Pharmaceutical Research 2003 20(6):884-888; and Chang et al., Non-ionic amphiphilic biodegradable PEG-PLGA-PEG copolymer enhances gene delivery efficiency in rat skeletal muscle. J Controlled Release. 2007 118:245-253; each of which is herein incorporated by reference in its entirety).

The RNA vaccines of the present invention may be formulated in lipid nanoparticles comprising the PEG-PLGA-PEG block copolymer. The therapeutic nanoparticle may comprise a multiblock copolymer (See e.g., U.S. Pat. No. 8,263,665 and 8,287,910 and US Patent Pub. No. US20130195987; the contents of each of which are herein incorporated by reference in its entirety). The block copolymers described herein may be included in a polyion complex comprising a non-polymeric micelle and the block copolymer. (See e.g., U.S. Pub. No. 20120076836; herein incorporated by reference in its entirety).

The therapeutic nanoparticle may comprise at least one acrylic polymer. Acrylic polymers include but are not limited to, acrylic acid, methacrylic acid, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), polycyanoacrylates and combinations thereof.

The therapeutic nanoparticles may comprise at least one poly(vinyl ester) polymer. The poly(vinyl ester) polymer may be a copolymer such as a random copolymer. As a non-limiting example, the random copolymer may have a structure such as those described in International Application No. WO2013032829 or US Patent Publication No US20130121954, the contents of which are herein incorporated by reference in its entirety. In one aspect, the poly(vinyl ester) polymers may be conjugated to the polynucleotides described herein. In another aspect, the poly(vinyl ester) polymer which may be used in the present invention may be those described in, herein incorporated by reference in its entirety.

The therapeutic nanoparticle may comprise at least one diblock copolymer. The diblock copolymer may be, but it not limited to, a poly(lactic) acid-poly(ethylene)glycol copolymer (see e.g., International Patent Publication No. WO2013044219; herein incorporated by reference in its entirety). As a non-limiting example, the therapeutic nanoparticle may be used to treat cancer (see International publication No. WO2013044219; herein incorporated by reference in its entirety).

In one embodiment, the therapeutic nanoparticles may comprise at least one cationic polymer described herein and/or known in the art.

The therapeutic nanoparticles may comprise at least one amine-containing polymer such as, but not limited to polylysine, polyethylene imine,
poly(amidoamine) dendrimers, poly (beta- amino esters) (See e.g., U.S. Pat. No. 8,287,849; herein incorporated by reference in its entirety) and combinations thereof.

In another embodiment, the nanoparticles described herein may comprise an amine cationic lipid such as those described in International Patent Application No.

WO2013059496, the contents of which are herein incorporated by reference in its entirety. In one aspect the cationic lipids may have an amino-amine or an amino-amide moiety.

The therapeutic nanoparticles may comprise at least one degradable polyester which may contain polycationic side chains. Degradeable polyesters include, but are not limited to, poly(serine ester), poly(L-lactide-co-L-lysine), poly(4-hydroxy-L-proline ester), and combinations thereof. In another embodiment, the degradable polyesters may include a PEG conjugation to form a PEGylated polymer.

The therapeutic nanoparticle may include a conjugation of at least one targeting ligand. The targeting ligand may be any ligand known in the art such as, but not limited to, a monoclonal antibody. (Kirpotin et al, Cancer Res. 2006 66:6732-6740; herein incorporated by reference in its entirety).

The therapeutic nanoparticle may be formulated in an aqueous solution which may be used to target cancer (see International Pub No. WO2011084513 and US Pub No. US20110294717, each of which is herein incorporated by reference in their entirety).

The therapeutic nanoparticle RNA vaccines, e.g., therapeutic nanoparticles comprising at least one RNA vaccine may be formulated using the methods described by Podobinski et al in US Patent No. 8,404,799, the contents of which are herein incorporated by reference in its entirety.

The RNA vaccines may be encapsulated in, linked to and/or associated with synthetic nanocarriers. Synthetic nanocarriers include, but are not limited to, those described in International Pub. Nos. WO2010005740, WO2010030763, WO201213501, WO2012149252, WO2012149255, WO2012149259, WO2012149265, WO2012149268, WO2012149282, WO2012149301, WO2012149393, WO2012149405, WO2012149411, WO2012149454 and WO2013019669, and US Pub. Nos. US20110262491, US20100104645, US20100087337 and US20120244222, each of which is herein incorporated by reference in their entirety. The synthetic nanocarriers may be formulated using methods known in the art and/or described herein. As a non-limiting example, the synthetic nanocarriers may be formulated by the methods described in International Pub Nos. WO2010005740, WO2010030763 and WO201213501and US Pub. Nos. US20110262491, US20100104645, US20100087337 and US2012024422, each of which is herein incorporated by reference in their entirety. In another embodiment, the synthetic nanocarrier formulations may be lyophilized by methods described in International Pub. No. WO2011072218 and US Pat No. 8,211,473; the content of each of which is herein incorporated by reference in their entirety. In yet another embodiment, formulations of the present invention, including, but not limited to, synthetic nanocarriers, may be lyophilized or reconstituted by the methods described in US Patent Publication No. US20130230568, the contents of which are herein incorporated by reference in its entirety.

In one embodiment, the synthetic nanocarriers may contain reactive groups to release the polynucleotides described herein (see International Pub. No. WO20120952552 and US Pub No. US20120171229, each of which is herein incorporated by reference in their entirety).

The synthetic nanocarriers may contain an immuno stimulatory agent to enhance the immune response from delivery of the synthetic nanocarrier. As a non-limiting example, the synthetic nanocarrier may comprise a Thl immuno stimulatory agent which may enhance a Thl-based response of the immune system (see International Pub No. WO2010123569 and US Pub. No. US20110223201, each of which is herein incorporated by reference in its entirety). The synthetic nanocarriers may be formulated for targeted release. In one embodiment, the synthetic nanocarrier is formulated to release the polynucleotides at a specified pH and/or after a desired time interval. As a non-limiting example, the synthetic nanoparticle may be formulated to release the RNA vaccines after 24 hours and/or at a pH of 4.5 (see International Pub. Nos. WO2010138193 and WO2010138194 and US Pub Nos. US20110020388 and US20110027217, each of which is herein incorporated by reference in their entireties).

The synthetic nanocarriers may be formulated for controlled and/or sustained release of the polynucleotides described herein. As a non-limiting example, the synthetic nanocarriers for sustained release may be formulated by methods known in the art, described herein and/or as described in International Pub No. WO2010138192 and US Pub No. 20100303850, each of which is herein incorporated by reference in their entirety.

In one embodiment, the RNA vaccine may be formulated for controlled and/or sustained release wherein the formulation comprises at least one polymer that is a crystalline side chain (CYSC) polymer. CYSC polymers are described in U.S. Patent No. 8,399,007, herein incorporated by reference in its entirety.

The synthetic nanocarrier may be formulated for use as a vaccine. In one embodiment, the synthetic nanocarrier may encapsulate at least one polynucleotide which encode at least one antigen. As a non-limiting example, the synthetic nanocarrier may include at least one antigen and an excipient for a vaccine dosage form (see International Pub No. WO2011150264 and US Pub No. US20110293723, each of which is herein incorporated by reference in their entirety). As another non-limiting example, a vaccine dosage form may include at least two synthetic nanocarriers with the same or different antigens and an excipient (see International Pub No. WO2011150249 and US Pub No. US20110293701, each of which is herein incorporated by reference in their entirety). The vaccine dosage form may be selected by methods described herein, known in the art and/or described in International Pub No. WO2011150258 and US Pub No. US20120027806, each of which is herein incorporated by reference in their entirety).

The synthetic nanocarrier may comprise at least one polynucleotide which encodes at least one adjuvant. As non-limiting example, the adjuvant may comprise dimethyldioctadecylammonium-bromide, dimethyldioctadecylammonium-chloride, dimethyldioctadecylammonium-phosphate or dimethyldioctadecylammonium-acetate (DDA) and an apolar fraction or part of said apolar fraction of a total lipid extract of a mycobacterium (See e.g, U.S. Pat. No. 8,241,610; herein incorporated by reference in its entirety). In another embodiment, the synthetic nanocarrier may comprise at least one polynucleotide and an adjuvant. As a non-limiting example, the synthetic nanocarrier comprising and adjuvant may be formulated by the methods described in International Pub No. WO2011150240 and US Pub No. US20110293700, each of which is herein incorporated by reference in its entirety. The synthetic nanocarrier may encapsulate at least one polynucleotide which encodes a peptide, fragment or region from a virus. As a non-limiting example, the synthetic nanocarrier may include, but is not limited to, the nanocarriers described in International Pub No. WO2012024621, WO201202629, WO2012024632 and US Pub No. US20120064110, US20120058153 and US20120058154, each of which is herein incorporated by reference in their entirety.

T synthetic nanocarrier may be coupled to a polynucleotide which may be able to trigger a humoral and/or cytotoxic T lymphocyte (CTL) response (See e.g., International Publication No. WO2013019669, herein incorporated by reference in its entirety).

In one embodiment, the RNA vaccine may be encapsulated in, linked to and/or associated with zwitterionic lipids. Non-limiting examples of zwitterionic lipids and methods of using zwitterionic lipids are described in US Patent Publication No. US20130216607, the contents of which are herein incorporated by reference in its entirety. In one aspect, the zwitterionic lipids may be used in the liposomes and lipid nanoparticles described herein.

In one embodiment, the RNA vaccine may be formulated in colloid nanocarriers as described in US Patent Publication No. US20130197100, the contents of which are herein incorporated by reference in its entirety.

The nanoparticle may be optimized for oral administration. The nanoparticle may comprise at least one cationic biopolymer such as, but not limited to, chitosan or a derivative thereof. As a non-limiting example, the nanoparticle may be formulated by the methods described in U.S. Pub. No. 20120282343; herein incorporated by reference in its entirety. In some embodiments, LNPs comprise the lipid KL52 (an amino-lipid disclosed in U.S. Application Publication No. 2012/0295832 expressly incorporated herein by reference in its entirety). Activity and/or safety (as measured by examining one or more of ALT/AST, white blood cell count and cytokine induction) of LNP administration may be improved by incorporation of such lipids. LNPs comprising KL52 may be administered intravenously and/or in one or more doses. In some embodiments, administration of LNPs comprising KL52 results in equal or improved mRNA and/or protein expression as compared to LNPs comprising MC3.

In some embodiments, RNA vaccine may be delivered using smaller LNPs. Such particles may comprise a diameter from below 0.1 um up to 100 nm such as, but not limited to, less than 0.1 um, less than 1.0 um, less than 5 um, less than 10 um, less than 15 um, less than 20 um, less than 25 um, less than 30 um, less than 35 um, less than 40 um, less than 50 um, less than 55 um, less than 60 um, less than 65 um, less than 70 um, less than 75 um, less than 80 um, less than 85 um, less than 90 um, less than 95 um, less than 100 um, less than 125 um, less than 150 um, less than 175 um, less than 200 um, less than 225 um, less than 250 um, less than 275 um, less than 300 um, less than 325 um, less than 350 um, less than 375 um, less than 400 um, less than 425 um, less than 450 um, less than 475 um, less than 500 um, less than 525 um, less than 550 um, less than 575 um, less than 600 um, less than 625 um, less than 650 um, less than 675 um, less than 700 um, less than 725 um, less than 750 um, less than 775 um, less than 800 um, less than 825 um, less than 850 um, less than 875 um, less than 900 um, less than 925 um, less than 950 um, less than 975 um.

RNA vaccines may be delivered using smaller LNPs which may comprise a diameter from about 1 nm to about 100 nm, from about 1 nm to about 10 nm, about 1 nm to about 20 nm, from about 1 nm to about 30 nm, from about 1 nm to about 40 nm, from about 1 nm to about 50 nm, from about 1 nm to about 60 nm, from about 1 nm to about 70 nm, from about 1 nm to about 80 nm, from about 1 nm to about 90 nm, from about 5 nm to about from 100 nm, from about 5 nm to about 10 nm, about 5 nm to about 20 nm, from about 5 nm to about 30 nm, from about 5 nm to about 40 nm, from about 5 nm to about 50 nm, from about 5 nm to about 60 nm, from about 5 nm to about 70 nm, from about 5 nm to about 80 nm, from about 5 nm to about 90 nm, about 10 to about 50 nM, from about 20 to about 50 nm, from about 30 to about 50 nm, from about 40 to about 50 nm, from about 20 to about 60 nm, from about 30 to about 60 nm, from about 40 to about 60 nm, from about 20 to about 70 nm, from about 30 to about 70 nm, from about 40 to about 70 nm, from about 50 to about 70 nm, from about 60 to about 70 nm, from about 20 to about 80 nm, from about 30 to about 80 nm, from about 40 to about 80 nm, from about 50 to about 80 nm, from about 60 to about 80 nm, from about 20 to about 90 nm, from about 30 to about 90 nm, from about 40 to about 90 nm, from about 50 to about 90 nm, from about 60 to about 90 nm and/or from about 70 to about 90 nm.

Such LNPs may be synthesized using methods comprising microfluidic mixers. Exemplary microfluidic mixers may include, but are not limited to a slit interdigitial micromixer including, but not limited to those manufactured by Microinnova (Allerheiligen bei Wildon, Austria) and/or a staggered herringbone micromixer (SHM) (Zhigaltsev, I.V. et al., Bottom-up design and synthesis of limit size lipid nanoparticle systems with aqueous and triglyceride cores using millisecond microfluidic mixing have been published (Langmuir. 2012. 28:3633-40; Belliveau, N.M. et al., Microfluidic synthesis of highly potent limit-size lipid nanoparticles for in vivo delivery of siRNA. Molecular Therapy-Nucleic Acids. 2012. I:e37; Chen, D. et al., Rapid discovery of potent siRNA-containing lipid nanoparticles enabled by controlled microfluidic formulation. J Am Chem Soc. 2012. 134(16):6948-51; each of which is herein incorporated by reference in its entirety). In some embodiments, methods of LNP generation comprising SHM, further comprise the mixing of at least two input streams wherein mixing occurs by microstructure-induced chaotic advection (MICA). According to this method, fluid streams flow through channels present in a herringbone pattern causing rotational flow and folding the fluids around each other. This method may also comprise a surface for fluid mixing wherein the surface changes orientations during fluid cycling. Methods of generating LNPs using SHM include those disclosed in U.S. Application Publication Nos. 2004/0262223 and 2012/0276209, each of which is expressly incorporated herein by reference in their entirety.

The RNA vaccine of the present invention may be formulated in lipid nanoparticles created using a micromixer such as, but not limited to, a Slit Interdigital Micro structured Mixer (SIMM-V2) or a Standard Slit Interdigital Micro Mixer (SSIMM) or Caterpillar (CPMM) or Impinging-jet (IJMM)from the Institut fiir Mikrotechnik Mainz CmzbH, Mainz Germany). The RNA vaccines of the present invention may be formulated in lipid nanoparticles created using microfluidic technology (see Whitesides, George M. The Origins and the Future of Microfluidic s. Nature, 2006 442: 368-373; and Abraham et al. Chaotic Mixer for Microchannels. Science, 2002 295: 647-651; each of which is herein incorporated by reference in its entirety). As a non-limiting example, controlled microfluidic formulation includes a passive method for mixing streams of steady pressure-driven flows in micro channels at a low Reynolds number (See e.g., Abraham et al. Chaotic Mixer for Microchannels. Science, 2002 295: 647-651; which is herein incorporated by reference in its entirety). The RNA vaccines of the present invention may be formulated in lipid nanoparticles created using a micromixer chip such as, but not limited to, those from Harvard Apparatus (Holliston, MA) or Dolomite Microfluidics (Royston, UK). A micromixer chip can be used for rapid mixing of two or more fluid streams with a split and recombine mechanism.

The RNA vaccines of the invention may be formulated for delivery using the drug encapsulating microspheres described in International Patent Publication No. WO2013063468 or U.S. Patent No. 8,440,614, each of which is herein incorporated by reference in its entirety. The microspheres may comprise a compound of the formula (I), (II), (III), (IV), (V) or (VI) as described in International Patent Publication No. WO2013063468, the contents of which are herein incorporated by reference in its entirety. In another aspect, the amino acid, peptide, polypeptide, lipids (APPL) are useful in delivering the RNA vaccines of the invention to cells (see International Patent Publication No.WO2013063468, the contents of which is herein incorporated by reference in its entirety).

The RNA vaccines of the invention may be formulated in lipid nanoparticles having a diameter from about 10 to about 200 nm such as, but not limited to, about 10 to about 20 nm, about 10 to about 30 nm, about 10 to about 40 nm, about 10 to about 50 nm, about 10 to about 60 nm, about 10 to about 70 nm, about 10 to about 80 nm,
about 10 to about 90 nm, about 20 to about 30 nm, about 20 to about 40 nm, about 20 to about 50 nm, about 20 to about 60 nm, about 20 to about 70 nm, about 20 to about 80 nm, about 20 to about 90 nm, about 20 to about 100 nm, about 30 to about 40 nm, about 30 to about 50 nm, about 30 to about 60 nm, about 30 to about 70 nm, about 30 to about 80 nm, about 30 to about 90 nm, about 30 to about 100 nm, about 40 to about 50 nm, about 40 to about 60 nm, about 40 to about 70 nm, about 40 to about 80 nm, about 40 to about 90 nm, about 40 to about 100 nm, about 50 to about 60 nm, about 50 to about 70 nm about 50 to about 80 nm, about 50 to about 90 nm, about 50 to about 100 nm, about 50 to about 150 nm, about 50 to about 200 nm, about 60 to about 70 nm, about 60 to about 80 nm, about 60 to about 90 nm, about 60 to about 100 nm, about 60 to about 150 nm, about 60 to about 200 nm, about 70 to about 80 nm, about 70 to about 90 nm, about 70 to about 100 nm, about 70 to about 150 nm, about 70 to about 200 nm, about 80 to about 90 nm, about 80 to about 100 nm, about 80 to about 150 nm, about 80 to about 200 nm, about 90 to about 100 nm, about 90 to about 150 nm, and/or about 90 to about 200 nm.

The lipid nanoparticles may have a diameter from about 10 to 500 nm.

In one embodiment, the lipid nanoparticle may have a diameter greater than 100 nm, greater than 150 nm, greater than 200 nm, greater than 250 nm, greater than 300 nm, greater than 350 nm, greater than 400 nm, greater than 450 nm, greater than 500 nm, greater than 550 nm, greater than 600 nm, greater than 650 nm, greater than 700 nm, greater than 750 nm, greater than 800 nm, greater than 850 nm, greater than 900 nm, greater than 950 nm or greater than 1000 nm.

The lipid nanoparticle may be a limit size lipid nanoparticle as described in International Patent Publication No. WO2013059922, the contents of which are herein incorporated by reference in its entirety. The limit size lipid nanoparticle may comprise a lipid bilayer surrounding an aqueous core or a hydrophobic core; where the lipid bilayer may comprise a phospholipid such as, but not limited to, diacylphosphatidylcholine, a diacylphosphatidylethanolamine, a ceramide, a sphingomyelin, a dihydrosphingomyelin, a cephalin, a cerebroside, a C8-C20 fatty acid diacylphophatidylcholine, and 1-palmitoyl-2-oleoyl phosphatidylcholine (POPC). In another aspect the limit size lipid nanoparticle may comprise a polyethylene glycol-lipid such as, but not limited to, DLPE-PEG, DMPE-PEG, DPPC-PEG and DSPE-PEG.

The RNA vaccines may be delivered, localized and/or concentrated in a specific location using the delivery methods described in International Patent Publication No. WO2013063530, the contents of which are herein incorporated by reference in its entirety. As a non-limiting example, a subject may be administered an empty polymeric particle prior to, simultaneously with or after delivering the RNA vaccines to the subject. The empty polymeric particle undergoes a change in volume once in contact with the subject and becomes lodged, embedded, immobilized or entrapped at a specific location in the subject.

The RNA vaccines may be formulated in an active substance release system (See e.g., US Patent Publication No. US20130102545, the contents of which is herein incorporated by reference in its entirety). The active substance release system may comprise 1) at least one nanoparticle bonded to an oligonucleotide inhibitor strand which is hybridized with a catalytically active nucleic acid and 2) a compound bonded to at least one substrate molecule bonded to a therapeutically active substance (e.g., polynucleotides described herein), where the therapeutically active substance is released by the cleavage of the substrate molecule by the catalytically active nucleic acid.

The RNA vaccines may be formulated in a nanoparticle comprising an inner core comprising a non-cellular material and an outer surface comprising a cellular membrane. The cellular membrane may be derived from a cell or a membrane derived from a virus. As a non-limiting example, the nanoparticle may be made by the methods described in International Patent Publication No. WO2013052167, herein incorporated by reference in its entirety. As another non-limiting example, the nanoparticle described in International Patent Publication No. WO2013052167, herein incorporated by reference in its entirety, may be used to deliver the RNA vaccines described herein.

The RNA vaccines may be formulated in porous nanoparticle-supported lipid bilayers (protocells). Protocells are described in International Patent Publication No. WO2013056132, the contents of which are herein incorporated by reference in its entirety.

The RNA vaccines described herein may be formulated in polymeric nanoparticles as described in or made by the methods described in US Patent No. 8,420,123 and 8,518,963 and European Patent No. EP2073848B1, the contents of each of which are herein incorporated by reference in their entirety. As a non-limiting example, the polymeric nanoparticle may have a high glass transition temperature such as the nanoparticles described in or nanoparticles made by the methods described in US Patent No. 8,518,963, the contents of which are herein incorporated by reference in its entirety. As another non-limiting example, the polymer nanoparticle for oral and parenteral formulations may be made by the methods described in European Patent No. EP2073848B1, the contents of which are herein incorporated by reference in its entirety.

The RNA vaccines described herein may be formulated in nanoparticles used in imaging. The nanoparticles may be liposome nanoparticles such as those described in US Patent Publication No US20130129636, herein incorporated by reference in its entirety. As a non-limiting example, the liposome may comprise gadolinium(III)2-{4,7-bis-carboxymethyl-10-[(N,N-distearylamidomethyl-N'-amido-methyl]-1,4,7, 10-tetra-azacyclododec-1-yl}-acetic acid and a neutral, fully saturated phospholipid component (see e.g., US Patent Publication No US20130129636, the contents of which is herein incorporated by reference in its entirety).

The nanoparticles which may be used in the present invention may be formed by the methods described in U.S. Patent Application No. US20130130348, the contents of which is herein incorporated by reference in its entirety. The nanoparticles of the present invention may further include nutrients such as, but not limited to, those which deficiencies can lead to health hazards from anemia to neural tube defects (see e.g, the nanoparticles described in International Patent Publication No WO2013072929, the contents of which is herein incorporated by reference in its entirety). As a non-limiting example, the nutrient may be iron in the form of ferrous, ferric salts or elemental iron, iodine, folic acid, vitamins or micronutrients.

RNA vaccines of the present invention may be formulated in a swellable nanoparticle. The swellable nanoparticle may be, but is not limited to, those described in U.S. Patent No. 8,440,231, the contents of which is herein incorporated by reference in its entirety. As a non-limiting embodiment, the swellable nanoparticle may be used for delivery of the RNA vaccines of the present invention to the pulmonary system (see e.g., U.S. Patent No. 8,440,231, the contents of which is herein incorporated by reference in its entirety).

RNA vaccines of the present invention may be formulated in polyanhydride nanoparticles such as, but not limited to, those described in U.S. Patent No. 8,449,916, the contents of which is herein incorporated by reference in its entirety.

The nanoparticles and microparticles of the present invention may be geometrically engineered to modulate macrophage and/or the immune response. In one aspect, the geometrically engineered particles may have varied shapes, sizes and/or surface charges in order to incorporated the polynucleotides of the present invention for targeted delivery such as, but not limited to, pulmonary delivery (see e.g., International Publication No WO2013082111, the contents of which is herein incorporated by reference in its entirety). Other physical features the geometrically engineering particles may have include, but are not limited to, fenestrations, angled arms, asymmetry and surface roughness, charge which can alter the interactions with cells and tissues. As a non-limiting example, nanoparticles of the present invention may be made by the methods described in International Publication No WO2013082111, the contents of which is herein incorporated by reference in its entirety.

Nanoparticles of the present invention may be water soluble nanoparticles such as, but not limited to, those described in International Publication No. WO2013090601, the contents of which is herein incorporated by reference in its entirety. The nanoparticles may be inorganic nanoparticles which have a compact and zwitterionic ligand in order to exhibit good water solubility. The nanoparticles may also have small hydrodynamic diameters (HD), stability with respect to time, pH, and salinity and a low level of non-specific protein binding. In one embodiment the nanoparticles of the present invention may be developed by the methods described in US Patent Publication No. US20130172406, the contents of which are herein incorporated by reference in its entirety.

Nanoparticles of the present invention may be stealth nanoparticles or target- specific stealth nanoparticles such as, but not limited to, those described in US Patent Publication No. US20130172406; the contents of which is herein incorporated by reference in its entirety. The nanoparticles of the present invention may be made by the methods described in US Patent Publication No. US20130172406, the contents of which are herein incorporated by reference in its entirety.

The stealth or target- specific stealth nanoparticles may comprise a polymeric matrix. The polymeric matrix may comprise two or more polymers such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polyesters, polyanhydrides, polyethers, polyurethanes, polymethacrylates, polyacrylates, polycyanoacrylates or combinations thereof. The nanoparticle may be a nanoparticle-nucleic acid hybrid structure having a high density nucleic acid layer. As a non-limiting example, the nanoparticle-nucleic acid hybrid structure may made by the methods described in US Patent Publication No. US20130171646, the contents of which are herein incorporated by reference in its entirety. The nanoparticle may comprise a nucleic acid such as, but not limited to, polynucleotides described herein and/or known in the art.

Nanoparticles may be embedded in in the core a nanostructure or coated with a low density porous 3-D structure or coating which is capable of carrying or associating with at least one pay load within or on the surface of the nanostructure. Non-limiting examples of the nanostructures comprising at least one nanoparticle are described in International Patent Publication No. WO2013123523, the contents of which are herein incorporated by reference in its entirety.

Other formulations include those which are described in US20170136121 A1, US9221891 B2, EP2971033 B1 and US20160331828 A1 which are herein incorporated by reference in their entirety.

Also provided is a method of producing a an mRNA encoding at least one immunogenic peptide fragment, the method comprising:
(a) binding a first polynucleotide comprising an open reading frame encoding the immunogenic peptide fragment and a second polynucleotide comprising a 5'-UTR to a polynucleotide conjugated to a solid support;
(b) ligating the 3 '-terminus of the second polynucleotide to the 5 '-terminus of the first polynucleotide under suitable conditions, wherein the suitable conditions comprise a DNA Ligase, thereby producing a first ligation product;
(c) ligating the 5' terminus of a third polynucleotide comprising a 3'-UTR to the 3'-terminus of the first ligation product under suitable conditions, wherein the suitable conditions comprise an RNA Ligase, thereby producing a second ligation product; and
(d) releasing the second ligation product from the solid support,
thereby producing an mRNA encoding the immunogenic peptide fragment.

Also provided is a kit for preparing an mRNA cancer vaccine. The kit may have one or more containers housing one or more polynucleotides comprising a 5'-ORF, one or more polynucleotides comprising a 3'-ORF, one or more polynucleotides comprising a poly(A) tail, a ligase enzyme, and instructions for ligating one or more polynucleotides comprising an ORF encoding a patient specific epitope to the one or more polynucleotides comprising the a 5'-ORF, 3'-ORF, and poly(A) tail.

Exemplary preferred compositions according to the invention include: but are not limited to, the following:
- 2 or more different polypeptides according to the invention, and more preferably wherein the composition comprises 2 to about 20 different polypeptides, and optionally wherein the different polypeptides are comprised in a continuous polypeptide sequence
- A polynucleotide according to the invention, preferably wherein the polynucleotide is mRNA, and more preferably wherein the mRNA encodes 2 to about 20 different polypeptides, and optionally wherein the polynucleotide is a single mRNA molecule encoding 2 to about 20 different polypeptides
- A plurality of polypeptides of the invention, preferably wherein the plurality comprises 2-20 different polypeptides of the invention, and preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- A polypeptide comprising preferably 2-20 different polypeptides of the invention, preferably wherein each polypeptide of the invention is an 8-11mer (e.g., a plurality of camyotopes as defined herein according to SEQ ID NOs 98 to 611), and optionally at least one adjuvant, pharmaceutically acceptable carrier, preservative and/or excipient
- An mRNA molecule comprising a sequence encoding a plurality of polypeptides of the invention, preferably wherein the mRNA molecule encodes 2-20 different polypeptides of the invention, and preferably wherein the polypeptides are 8-11mers (e.g., a plurality of camyotopes as defined herein according to SEQ ID NOs 98 to 611), and optionally wherein the mRNA molecules encodes the plurality of polypeptides in-frame to form a continuous polypeptide, and optionally at least one adjuvant, pharmaceutically acceptable carrier, preservative and/or excipient
- A plurality of mRNA molecules encoding a plurality of polypeptides of the invention, preferably wherein the plurality of polypeptides comprises 2-20 different polypeptides of the invention, and preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- A DNA molecule comprising a sequence encoding a plurality of polypeptides of the invention, preferably wherein the DNA molecule encodes 2-20 different polypeptides of the invention, and preferably wherein the polypeptides are 8-1 1mers (e.g., a plurality of camyotopes as defined herein according to SEQ ID NOs 98 to 611), and optionally wherein the DNA molecules encodes the plurality of polypeptides in-frame to form a continuous polypeptide, and optionally at least one adjuvant, pharmaceutically acceptable carrier, preservative and/or excipient
- A plurality of DNA molecules encoding a plurality of polypeptides of the invention, preferably wherein the plurality of polypeptides comprises 2-20 different polypeptides of the invention, and preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- A viral vector comprising a sequence encoding a plurality of polypeptides of the invention, preferably wherein the viral vector encodes 2-20 different polypeptides of the invention, and preferably wherein the polypeptides are 8-11mers (e.g., a plurality of camyotopes as defined herein according to SEQ ID NOs 98 to 611), and optionally wherein the viral vector encodes the plurality of polypeptides in-frame to form a continuous polypeptide, and optionally at least one adjuvant, pharmaceutically acceptable carrier, preservative and/or excipient
- A plurality of viral vectors encoding a plurality of polypeptides of the invention, preferably wherein the plurality of polypeptides comprises 2-20 different polypeptides of the invention, and preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- A plurality of polypeptides of the invention, preferably wherein the plurality comprises 2-20 different polypeptides of the invention (e.g., a plurality of camyotopes as defined herein according to SEQ ID NOs 98 to 611), and preferably wherein the polypeptides are 8-100mers, and optionally at least one adjuvant, pharmaceutically acceptable carrier, preservative and/or excipient
- An mRNA molecule comprising a sequence encoding a plurality of polypeptides of the invention, preferably wherein the DNA molecule encodes 2-20 different polypeptides of the invention (e.g., a plurality of camyotopes as defined herein according to SEQ ID NOs 98 to 611), and preferably wherein the polypeptides are 8-100mers, and optionally wherein the DNA molecules encodes the plurality of polypeptides in-frame to form a continuous polypeptide, and optionally at least one adjuvant, pharmaceutically acceptable carrier, preservative and/or excipient
- A plurality of mRNA molecules encoding a plurality of polypeptides of the invention, preferably wherein the plurality of polypeptides comprises 2-20 different polypeptides of the invention (e.g., a plurality of camyotopes as defined herein according to SEQ ID NOs 98 to 611), and preferably wherein the polypeptides are 8-100mers, and optionally at least one adjuvant, pharmaceutically acceptable carrier, preservative and/or excipient
- A DNA molecule comprising a sequence encoding a plurality of polypeptides of the invention, preferably wherein the DNA molecule encodes 2-20 different polypeptides of the invention (e.g., a plurality of camyotopes as defined herein according to SEQ ID NOs 98 to 611), and preferably wherein the polypeptides are 8-100mers, and optionally wherein the DNA molecules encodes the plurality of polypeptides in-frame to form a continuous polypeptide, and optionally at least one adjuvant, pharmaceutically acceptable carrier, preservative and/or excipient
- A plurality of DNA molecules encoding a plurality of polypeptides of the invention, preferably wherein the plurality of polypeptides comprises 2-20 different polypeptides of the invention (e.g., a plurality of camyotopes as defined herein according to SEQ ID NOs 98 to 611), and preferably wherein the polypeptides are 8-100mers, and optionally at least one adjuvant, pharmaceutically acceptable carrier, preservative and/or excipient
- A viral vector comprising a sequence encoding a plurality of polypeptides of the invention, preferably wherein the viral vector encodes 2-20 different polypeptides of the invention (e.g., a plurality of camyotopes as defined herein according to SEQ ID NOs 98 to 611), and preferably wherein the polypeptides are 8-100mers, and optionally wherein the viral vector encodes the plurality of polypeptides in-frame to form a continuous polypeptide, and optionally at least one adjuvant, pharmaceutically acceptable carrier, preservative and/or excipient
- A plurality of viral vectors encoding a plurality of polypeptides of the invention, preferably wherein the plurality of polypeptides comprises 2-20 different polypeptides of the invention (e.g., a plurality of camyotopes as defined herein according to SEQ ID NOs 98 to 611), and preferably wherein the polypeptides are 8-100mers, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention, preferably wherein said polypeptides are 8-11mers (e.g., a plurality of camyotopes as defined herein according to SEQ ID NOs 98 to 611), wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention, preferably wherein said polypeptides are 8-11mers (e.g., a plurality of camyotopes as defined herein according to SEQ ID NOs 98 to 611), preferably wherein the maturation is by transfection with an mRNA carrying the coding sequences of said plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention, preferably wherein said polypeptides are 8-11mers (e.g., a plurality of camyotopes as defined herein according to SEQ ID NOs 98 to 611), preferably wherein the maturation is by transfection with a plurality of mRNA molecules wherein each mRNA molecule carries the coding sequence of one of the plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention (e.g., a plurality of camyotopes as defined herein according to SEQ ID NOs 98 to 611), preferably wherein said polypeptides are 8-100mers, preferably wherein the maturation is by transfection with an mRNA carrying the coding sequences of said plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention (e.g., a plurality of camyotopes as defined herein according to SEQ ID NOs 98 to 611), preferably wherein said polypeptides are 8-100mers, preferably wherein the maturation is by transfection with a plurality of mRNA molecules wherein each mRNA molecule carries the coding sequence of one of the plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention, preferably wherein said polypeptides are 8-11mers (e.g., a plurality of camyotopes as defined herein according to SEQ ID NOs 98 to 611), preferably wherein the maturation is by infection with a viral vector carrying the coding sequences of said plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention, preferably wherein said polypeptides are 8-11mers (e.g., a plurality of camyotopes as defined herein according to SEQ ID NOs 98 to 611), preferably wherein the maturation is by infection with a plurality of viral vectors wherein each viral vector molecule carries the coding sequence of one of the plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention (e.g., a plurality of camyotopes as defined herein according to SEQ ID NOs 98 to 611), preferably wherein said polypeptides are 8-100mers, preferably wherein the maturation is by infection with a viral vector carrying the coding sequences of said plurality of polypeptides, wherein said syngeneic DCs are suitable for reintroduction to the patient, and optionally at least one adjuvant, pharmaceutically acceptable carrier, preservative and/or excipient
- Syngeneic (from the patient) dendritic cells (DCs) matured ex vivo by incubation with a plurality of polypeptides of the invention, preferably wherein the plurality of polypeptides comprises 2-20 different polypeptides of the invention (e.g., a plurality of camyotopes as defined herein according to SEQ ID NOs 98 to 611), preferably wherein said polypeptides are 8-100mers, preferably wherein the maturation is by infection with a plurality of viral vectors wherein each viral vector molecule carries the coding sequence of one of the plurality of polypeptides, wherein said syngeneic DCs are suitable for reintroduction to the patient, and optionally at least one adjuvant, pharmaceutically acceptable carrier, preservative and/or excipient
- CAR-T cells carrying receptors raised against one or more polypeptides of the invention, preferably up to 20 different polypeptides, preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- CAR-T cells carrying receptors raised against one or more polypeptides of the invention, preferably up to 20 different polypeptides (e.g., a plurality of camyotopes as defined herein according to SEQ ID NOs 98 to 611), preferably wherein the polypeptides are 8-100mers, and optionally at least one adjuvant, pharmaceutically acceptable carrier, preservative and/or excipient
- A plurality of antibodies raised against one or more polypeptides of the invention, preferably up to 20 different polypeptides of the invention, and preferably wherein the polypeptides are 8-11mers (e.g., a plurality of camyotopes as defined herein according to SEQ ID NOs 98 to 611), and optionally at least one adjuvant, pharmaceutically acceptable carrier, preservative and/or excipient
- A plurality of antibodies raised against one or more polypeptides of the invention, preferably up to 20 different polypeptides of the invention (e.g., a plurality of camyotopes as defined herein according to SEQ ID NOs 98 to 611), and preferably wherein the polypeptides are 8-100mers, and optionally at least one adjuvant, pharmaceutically acceptable carrier, preservative and/or excipient
- Any of the described vaccine compositions in combination with other tumour antigens as a combination vaccine, and optionally at least one adjuvant, pharmaceutically acceptable carrier, preservative and/or excipient
- Any of the described vaccine compositions, and optionally at least one adjuvant, pharmaceutically acceptable carrier, preservative and/or excipient, preferably wherein the composition is suitable for any kind of administration route (e.g., intravenous, intramuscular, intrathecal)
- Any of the described vaccine compositions, and optionally at least one adjuvant, pharmaceutically acceptable carrier, preservative and/or excipient, in any kind of combination with other therapy modalities

### Methods of treatment, methods of diagnosis and other methods of use

The polypeptide, polynucleotide, vector, cell or composition of the invention may be used in a method of treating or preventing a disease or condition in a subject. The polypeptide, polynucleotide, vector, cell or composition of the invention may be used in the manufacture of a medicament for use in a method of treating or preventing a disease or condition in a subject.

The terms "treatment," "treating," or "ameliorating" refers to either a therapeutic treatment or prophylactic/preventative treatment. A treatment is therapeutic if at least one symptom of disease in an individual receiving treatment improves or a treatment can delay worsening of a progressive disease in an individual or prevent onset of additional associated diseases.

The terms "prevent," "prophylaxis," and "prophylactically" refer to the administration of a compound prior to the onset of disease (e.g., prior to the onset of certain symptoms of a disease). Preventing disease may include reducing the likelihood that the disease will occur, delaying onset of the disease, ameliorating long term symptoms, or delaying eventual progression of the disease.

The method comprises administering to the said subject the said polypeptide, the said polynucleotide, the said vector, the said cell or the said composition. Administration may be of a therapeutically or prophylactically effective quantity of the said polypeptide, the said polynucleotide, the said vector, the said cell or the said composition, to a subject in need thereof. The administration may be by any suitable route, such as intranasally, intravenously (IV), intradermally, intramuscularly (IM), intrathecally (IT), or intraperitoneally. In some embodiments, the administration is a single administration. IV, IM and IT are preferred. In some embodiments, the subject is administered said polypeptide, polynucleotide, vector, cell or composition more than once.

In methods of the invention whereby a cell of the invention is to be administered to a subject, it is preferable for the cell to be syngeneic with respect to the subject.

The disease or condition may typically be colorectal cancer. The method of treatment or prevention may comprise eliciting an immune response against colorectal cancer in an individual.

The method may comprise simultaneous or sequential administration with an additional cancer therapy. The additional cancer therapy may be selected from a cytokine therapy, a T-cell therapy, an NK therapy, an immune system checkpoint inhibitor, chemotherapy, radiotherapy, immunostimulating substances, gene therapy, or an antibody. The additional cancer therapy may preferably be any known colorectal cancer therapy.

Immune system checkpoint inhibitors are preferred as an additional cancer therapy. Examples of immune system checkpoints include:
a) The interaction between Indoleamine 2,3-dioxygenase (IDOl) and its substrate;
b) The interaction between PD1 and PDL1 and/or PD1 and PDL2;
c) The interaction between CTLA4 and CD86 and/or CTLA4 and CD80;
d) The interaction between B7-H3 and/or B7-H4 and their respective ligands;
e) The interaction between HVEM and BTLA;
f) The interaction between GAL9 and TIM3;
g) The interaction between MHC class I or II and LAG3; and
h) The interaction between MHC class I or II and KIR.

A checkpoint inhibitor may be any immunomodulatory agent (such as an antibody) which blocks or inhibits an immune system checkpoint, or it may be an immunotherapeutic composition comprising a component of an immune system checkpoint, or an immunogenic fragment of said component, which stimulates targeting of the checkpoint by the immune system.

The additional cancer therapy may be an antibody.

The antibody may be Abagovomab, Abciximab, Actoxumab, Adalimumab, Adecatumumab, Afelimomab, Afutuzumab, Alacizumab pegol, ALD518, Alemtuzumab, Alirocumab, Altumomab pentetate, Amatuximab, Anatumomab mafenatox, Anrukinzumab, Apolizumab, Arcitumomab, Aselizumab, Atinumab, Atlizumab (= tocilizumab), Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumab mertansine, Blinatumomab, Blosozumab, Brentuximab vedotin, Briakinumab, Brodalumab, Canakinumab, Cantuzumab mertansine, Cantuzumab ravtansine, Caplacizumab, Capromab pendetide, Carlumab, Catumaxomab, CC49, Cedelizumab, Certolizumab pegol, Cetuximab, Ch.14.18, Citatuzumab bogatox, Cixutumumab, Clazakizumab, Clenoliximab, Clivatuzumab tetraxetan, Conatumumab, Concizumab, Crenezumab, CR6261, Dacetuzumab, Daclizumab, Dalotuzumab, Daratumumab, Demcizumab, Denosumab, Detumomab, Dorlimomab aritox, Drozitumab, Duligotumab, Dupilumab, Dusigitumab, Ecromeximab, Eculizumab, Edobacomab, Edrecolomab, Efalizumab, Efungumab, Elotuzumab Elsilimomab, Enavatuzumab, Enlimomab pegol, Enokizumab, Enoticumab, Ensituximab, Epitumomab cituxetan, Epratuzumab, Erlizumab, Ertumaxomab, Etaracizumab, Etrolizumab, Evolocumab, Exbivirumab, Fanolesomab, Faralimomab Farletuzumab, Fasinumab, FBTA05, Felvizumab, Fezakinumab, Ficlatuzumab, Figitumumab, Flanvotumab, Fontolizumab, Foralumab, Foravirumab, Fresolimumab, Fulranumab, Futuximab, Galiximab,Ganitumab, Gantenerumab, Gavilimomab, Gemtuzumab ozogamicin, Gevokizumab, Girentuximab,Glembatumumab vedotin, Golimumab, Gomiliximab,GS6624, Ibalizumab, Ibritumomab tiuxetan, Icrucumab, Igovomab, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lampalizumab, Lebrikizumab, Lemalesomab, Lerdelimumab, Lexatumumab, Libivirumab, Ligelizumab, Lintuzumab, Lirilumab, Lodelcizumab, Lorvotuzumab mertansine, Lucatumumab, Lumiliximab, Mapatumumab, Maslimomab, Mavrilimumab, Matuzumab, Mepolizumab, Metelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Narnatumab, Natalizumab, Nebacumab, Necitumumab, Nerelimomab, Nesvacumab, Nimotuzumab, Nivolumab, Nofetumomab merpentan, Obinutuzumab, Ocaratuzumab, Ocrelizumab, Odulimomab, Ofatumumab, Olaratumab, Olokizumab, Omalizumab, Onartuzumab, Oportuzumab monatox, Oregovomab, Orticumab, Otelixizumab, Oxelumab, Ozanezumab, Ozoralizumab, Pagibaximab, Palivizumab, Panitumumab, Panobacumab, Parsatuzumab, Pascolizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Polatuzumab vedotin, Ponezumab, Priliximab, Pritoxaximab, Pritumumab, PRO 140, Quilizumab, Racotumomab, Radretumab, Rafivirumab, Ramucirumab, Ranibizumab,Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Seribantumab, Setoxaximab, Sevirumab, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tefibazumab, Telimomab aritox, Tenatumomab, Teneliximab, Teplizumab, Teprotumumab, TGN1412, Ticilimumab (= tremelimumab), Tildrakizumab, Tigatuzumab, TNX-650, Tocilizumab (= atlizumab), Toralizumab, Tositumomab, Tralokinumab, Trastuzumab, TRBS07, Tregalizumab, Tremelimumab Tucotuzumab celmoleukin, Tuvirumab, Ublituximab, Urelumab, Urtoxazumab, Ustekinumab, Vapaliximab, Vatelizumab, Vedolizumab, Veltuzumab,Vepalimomab Vesencumab, Visilizumab, Volociximab, Vorsetuzumab mafodotin, Votumumab, Zalutumumab, Zanolimumab, Zatuximab, Ziralimumab or Zolimomab aritox.

Preferred antibodies include Natalizumab, Vedolizumab, Belimumab, Atacicept, Alefacept, Otelixizumab, Teplizumab, Rituximab, Ofatumumab, Ocrelizumab, Epratuzumab, Alemtuzumab, Abatacept, Eculizumab, Omalizumab, Canakinumab, Meplizumab, Reslizumab, Tocilizumab, Ustekinumab, Briakinumab, Etanercept, Inlfliximab, Adalimumab, Certolizumab pegol, Golimumab, Trastuzumab, Gemtuzumab, Ozogamicin, Ibritumomab, Tiuxetan, Tostitumomab, Cetuximab, Bevacizumab, Panitumumab, Denosumab, Ipilimumab, Brentuximab and Vedotin.

Anti-PD1 antibodies such as nivolumab and pembrolizumab are also preferred.

The additional cancer therapy may be selected from the group consisting of Actimide, Azacitidine, Azathioprine, Bleomycin, Carboplatin, Capecitabine, Cisplatin, Chlorambucil, Cyclophosphamide, Cytarabine, Dauno-rubicin, Docetaxel, Doxifluridine, Doxorubicin, Epirubicin, Etoposide, Fludarabine, Fluor-ouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine and Vinorelbine.

The invention also provides a method of diagnosing a disease or condition in a subject. The method comprises analyzing a sample taken from an individual for the presence of one or more of the camyoRNA, camyopeptide or camyotope sequences disclosed herein, wherein the presence of said sequence, optionally at a level that is elevated relative to the corresponding level in a sample taken from a healthy subject, is indicative that the individual has colorectal cancer, or is at increased risk thereof. The sample may be any suitable sample, such as a tumour biopsy or other tissue or fluid sample. The sequence may be detected in the sample by any suitable method, including methods described herein. For example, the method may include measuring an immune response elicited against the sample.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

**Table 3**

| **CamyoRNA** | **Expression Coverage** | **baseMean** | **log2FoldChang e** | **Normal mean TPM** | **Tumor mean TPM** | **SEQ ID NO** |
|---|---|---|---|---|---|---|
| Inc-TRPCS-3:1 | 97.5 | 318.5721773 | 6.008808492 | 0.103389388 | 5.163076308 | 1 |
| Inc-HOXB8-1:5 | 77.92 | 557.1827017 | 6.245253412 | 0.071819909 | 4.408348056 | 2 |
| Inc-SLC39A10-2:1 | 75.83 | 116.3258911 | 7.160784732 | 0.046219876 | 5.080354424 | 3 |
| Inc-TH-1:1 | 72.92 | 1882.813689 | 8.274754045 | 0.057297395 | 14.15389676 | 4 |
| Inc-SLC39A10-7:1 | 71.25 | 155.4054043 | 7.363136044 | 0.021145659 | 2.798184874 | 5 |
| CYP1B1-AS1:12 | 62.92 | 60.54029527 | 7.48002405 | 0.125151118 | 18.04120882 | 6 |
| Inc-LRRTM4-3:3 | 59.17 | 45.85682261 | 7.478925532 | 0.011667005 | 1.35285096 | 7 |
| LINC02418:9 | 55.42 | 183.5313217 | 11.18174359 | 0.000600914 | 1.354811113 | 8 |
| LINC02563:3 | 50 | 5.785799437 | 7.718261333 | 0.010117382 | 1.635045411 | 9 |
| Inc-OR52J3-1:1 | 45.83 | 48.42286448 | 6.44292717 | 0.061776652 | 4.465071022 | 10 |
| Inc-FANCM-10:1 | 43.33 | 60.0286497 | 7.382898449 | 0.06247368 | 8.570731129 | 11 |
| Inc-HSFS-2:1 | 42.08 | 30.58961936 | 7.43878821 | 0.014749631 | 2.58211221 | 12 |
| Inc-TACR3-1:1 | 25 | 30.6932299 | 7.315185116 | 0.030091188 | 3.825574581 | 13 |
| Inc-CAMK1D-2:1 | 12.92 | 20.38474189 | 5.370875612 | 0.078014653 | 3.159821959 | 14 |
| Inc-PLA2G1B-2:3 | 8.33 | 378.3920214 | 5.184820977 | 0.133099558 | 2.976924064 | 15 |
| Inc-CTR9-1:2 | 7.92 | 37.56762553 | 6.313637203 | 0.358941273 | 14.36570473 | 16 |
| LINC01715:13 | 7.5 | 17.40558898 | 6.27086754 | 0.014818106 | 1.078654387 | 17 |
| Inc-CHM-1:1 | 6.25 | 1.278129006 | 7.766517095 | 0.010291955 | 2.432198743 | 18 |

**Table 4**

| **Camyo - peptide** | **SEQ ID NO** | **Peptide Sequence** | **Translation Coverage (MS)** | **Camyo RNA** | **Coding Probability** | **Peptide Length** | **BMSt_ max_ sample** | **BMST_ median _sample** |
|---|---|---|---|---|---|---|---|---|
| IncTRPC531 16031801 | 19 | MNVPKGQTGNSSRGPGDGGNKDH WKESDRKDGKKDQDSRSAPEPKKP EENPASKFSSASNYAALS* | 81.05 | Inc-TRPCS-3:1 | 0.92672619 | 66 | 0.00042 0875 | 0 |
| IncTRPC531 701830 | 20 | * | 47.37 | Inc-TRPCS-3:1 | 0.94672619 | 43 | 0 | 0 |
| IncTRPC531 668830 | 21 | * | 47.37 | Inc-TRPCS-3:1 | 0.94857142 9 | 54 | 0 | 0 |
| IncTRPC531 8691010 | 22 | MMTTEEAGTAMKTDMTDGMIGRG APEMITLGMIIGVMIDVPPKDPN* | 45.26 | Inc-TRPCS-3:1 | 0.98369047 6 | 47 | 0 | 0 |
| IncTRPC531 722830 | 23 | MGIGMAHAGIWMDMVAGIAMMTK AAETMIEAMIPG* | 44.21 | Inc-TRPCS-3:1 | 0.96005952 4 | 36 | 0 | 0 |
| IncTRPC531 734830 | 24 | MAHAGIWMDMVAGIAMMTKAAET MIEAMIPG* | 44.21 | Inc-TRPCS-3:1 | 0.96065476 2 | 32 | 0 | 0 |
| IncTRPC531 755830 | 25 | MDMVAGIAMMTKAAETMIEAMIPG * | 42.11 | Inc-TRPCS-3:1 | 0.93541666 7 | 25 | 0 | 0 |
| IncTRPC531 761830 | 26 | MVAGIAMMTKAAETMIEAMIPG* | 40 | Inc-TRPCS-3:1 | 0.97273809 5 | 23 | 0 | 0 |
| IncTRPC531 8721010 | 27 | MTTEEAGTAMKTDMTDGMIGRGAP EMITLGMIIGVMIDVPPKDPN* | 33.68 | Inc-TRPCS-3:1 | 0.99077381 | 46 | 0 | 0 |
| IncTRPC531 8991010 | 28 | MKTDMTDGMIGRGAPEMITLGMIIG VMIDVPPKDPN* | 27.37 | Inc-TRPCS-3:1 | 0.99244047 6 | 37 | 0 | 0 |
| IncSLC39A1 021699888 | 29 | MYQHVHHYTVIGLGKAEEKQEPASS SYKQKSCLGVVAHACNPSTLGGQSG RITRSGDRDHPG* | 20 | Inc-SLC39A10-2:1 | 0.98184523 8 | 63 | 0 | 0 |
| IncTRPC531 9111010 | 30 | MTDGMIGRGAPEMITLGMIIGVMID VPPKDPN* | 18.95 | Inc-TRPCS-3:1 | 0.99386904 8 | 33 | 0 | 0 |
| IncTRPC531 9231010 | 31 | MIGRGAPEMITLGMIIGVMIDVPPKD PN* | 17.89 | Inc-TRPCS-3:1 | 0.98523809 5 | 29 | 0 | 0 |
| IncTRPC531 9471010 | 32 | MITLGMIIGVMIDVPPKDPN* | 16.84 | Inc-TRPCS-3:1 | 0.92511904 8 | 21 | 0 | 0 |
| IncPLA2G1B 231094711 151 | 33 | MFDTGCSNNKKPPMQMGLKYGWW SGNTSYTRTHGQALTLNVMVVIIRG GAGREQNLSSFAQWQYRSQ* | 16.84 | Inc-PLA2G1B-2:3 | 0.90059523 8 | 68 | 0.00040 8497 | 0 |
| LINC024189 23092465 | 34 | MAAEPLVGAECRVCSVNMPGKGIM HVRDRVEKDCARLHHTTQNGTQFR TYK* | 14.74 | LINC02418:9 | 0.98642857 1 | 52 | 0.00026 7094 | 0 |
| IncTRPC531 160328 | 35 | MTWKEMFRQLGTVTMTMCIGCLQL TVPSFPLLHGLLGNPISTGAVFPNRH PTLLF* | 12.63 | Inc-TRPCS-3:1 | 0.84208333 3 | 56 | 0 | 0 |
| IncTRPC531 28328 | 36 | * | 12.63 | Inc-TRPCS-3:1 | 0.99416666 7 | 100 | 0 | 0 |
| LINC024189 23602465 | 37 | MPGKGIMHVRDRVEKDCARLHHTT QNGTQFRTYK* | 12.63 | LINC02418:9 | 0.95797619 | 35 | 0 | 0 |
| IncHSF5214 81574 | 38 | MLVSKDGAGKPSTTDCKHLNFRRPF SDYYF* | 12.63 | Inc-HSF5-2:1 | 0.97113095 2 | 31 | 0 | 0 |
| IncSLC39A1 021811913 | 39 | MPVILALWEAKAGGSRDQEIETILA NTVKPRLY* | 11.58 | Inc-SLC39A10-2:1 | 0.99279761 9 | 34 | 0 | 0 |
| IncPLA2G1B 231098611 151 | 40 | * | 11.58 | Inc-PLA2G1B-2:3 | 0.86601190 5 | 55 | 0 | 0 |
| IncPLA2G1B 231099211 151 | 41 | MGLKYGWWSGNTSYTRTHGQALTL NVMVVIIRGGAGREQNLSSFAQWQY RSQ* | 11.58 | Inc-PLA2G1B-2:3 | 0.77011904 8 | 53 | 0 | 0 |
| IncTRPC531 779830 | 42 | MMTKAAETMIEAMIPG* | 10.53 | Inc-TRPCS-3:1 | 0.94839285 7 | 17 | 0 | 0 |
| LINC024189 23782465 | 43 | MHVRDRVEKDCARLHHTTQNGTQF RTYK* | 10.53 | LINC02418:9 | 0.96172619 | 29 | 0 | 0 |
| IncTRPC531 782830 | 44 | MTKAAETMIEAMIPG* | 9.47 | Inc-TRPCS-3:1 | 0.92982142 9 | 16 | 0 | 0 |
| LINC024189 31663253 | 45 | MSYNIYMHIERGCEYSCRRASKPKT HRE* | 9.47 | LINC02418:9 | 0.73767857 1 | 29 | 0 | 0 |
| IncSLC39A1 071155617 69 | 46 | MKRRNLKTDTPKENAMWRHREKL AIYKPTRIAWNKFFHHTPQKESTLT APPLQSCSLQNCETIHFCCLRR* | 9.47 | Inc-SLC39A10-7:1 | 0.83404761 9 | 71 | 0 | 0 |
| IncSLC39A1 071154117 69 | 47 | MVDVLMKRRNLKTDTPKENAMWR HREKLAIYKPTRIAWNKFFHHTPQK ESTLTAPPLQSCSLQNCETIHFCCLR R* | 9.47 | Inc-SLC39A10-7:1 | 0.96023809 5 | 76 | 0.00036 5497 | 0 |
| IncTRPC531 9621010 | 48 | MIIGVMIDVPPKDPN* | 8.42 | Inc-TRPCS-3:1 | 0.74732142 9 | 16 | 0 | 0 |
| IncFANCM1 0125163 | 49 | MARLKTVRSSCLDRKATGCSPGSCV RILTALTMPRKGRSCCFIRD* | 8.42 | Inc-FANCM-10:1 | 0.73607142 9 | 46 | 0 | 0 |
| IncFANCM1 0122163 | 50 | MMARLKTVRSSCLDRKATGCSPGSC VRILTALTMPRKGRSCCFIRD* | 8.42 | Inc-FANCM-10:1 | 0.70726190 5 | 47 | 0.00029 5508 | 0 |
| IncPLA2G1B 238302839 5 | 51 | MVRSCLYEKYRKYPAEVGGGSLEAW KQRLK* | 7.37 | Inc-PLA2G1B-2:3 | 0.82886904 8 | 31 | 0 | 0 |
| IncCAMKID 211343143 9 | 52 | MILSLNVEIRGRGKENPMELVAGEA LVSLRQ* | 6.32 | Inc-CAMK1D-2:1 | 0.86863095 2 | 32 | 0.00043 4028 | 0 |
| IncTH11352 83804 | 53 | MCSQWPWQNLTKSAPATRPASKDA LSRRPLQLSSDCPRDTLSEHCPAKTR QLQNCERRWFHDFLAVVTAVGQLW VTLKGHSDKGHLPACVT* | 5.26 | Inc-TH-1:1 | 0.80690476 2 | 92 | 0 | 0 |
| IncTH11352 23804 | 54 | * | 5.26 | Inc-TH-1:1 | 0.84428571 4 | 94 | 0 | 0 |
| IncCTR9122 15350 | 55 | MRPLETHWRAGQTKDNFTDFRRLA LISHNLRSQDAGHSKKEKEV* | 5.26 | Inc-CTR9-1:2 | 0.97946428 6 | 45 | 0 | 0 |
| IncHOXB815 19402135 | 56 | * | 5.26 | Inc-HOXB8-1:5 | 0.89386904 8 | 65 | 0.00010 6838 | 0 |
| IncPLA2G1B 239255934 2 | 57 | MGTESQFPPLGMKDKVLEVSSDDGY TTM* | 3.16 | Inc-PLA2G1B-2:3 | 0.92601190 5 | 29 | 0 | 0 |
| LINC024189 33743500 | 58 | * | 3.16 | LINC02418:9 | 0.95273809 5 | 42 | 0 | 0 |
| LINC024189 29103054 | 59 | MTGALCLRSAKPRSEVLTRLHQEQG TRLPRGSWSTEGRDAHKHTMSK* | 3.16 | LINC02418:9 | 0.77065476 2 | 48 | 0 | 0 |
| LINC024189 28443054 | 60 | MELRIISTTRAGVRQDAQSIHPMTG ALCLRSAKPRSEVLTRLHQEQGTRL PRGSWSTEGRDAHKHTMSK* | 3.16 | LINC02418:9 | 0.88232142 9 | 70 | 0 | 0 |
| IncTACR311 255462 | 61 | MKRAWPPCHSGERQSKTKKTESCS ATQAGVEGSGVISAHFNLRLLGSSDS PASASRVAGTTAASLQVS* | 3.16 | Inc-TACR3-1:1 | 0.83869047 6 | 69 | 0 | 0 |
| LINC017151 320532128 | 62 | MRSEGQDQPGQYGETLSLLKIQKI* | 3.16 | LINC01715:13 | 0.71226190 5 | 25 | 0 | 0 |
| IncPLA2G1B 233009306 9 | 63 | MPGLLISTWQHRFMKPLTL* | 2.11 | Inc-PLA2G1B-2:3 | 0.81863095 2 | 20 | 0 | 0 |
| IncPLA2G1B 236574662 5 | 64 | MSFLTILANTVKPHLY* | 2.11 | Inc-PLA2G1B-2:3 | 0.81607142 9 | 17 | 0 | 0 |
| IncPLA2G1B 239761984 5 | 65 | MSRGRNKLALRSGSIVANEVYPRRD YC* | 2.11 | Inc-PLA2G1B-2:3 | 0.82434523 8 | 28 | 0 | 0 |
| LINC024189 25582597 | 66 | MSATLMPSRPEE* | 2.11 | LINC02418:9 | 0.88488095 2 | 13 | 0 | 0 |
| IncHSF5214 97638 | 67 | * | 2.11 | Inc-HSF5-2:1 | 0.88666666 7 | 47 | 0 | 0 |
| IncTH11332 23427 | 68 | MVWTRRCGASPTSYNWGRLTTTVE GTPWNSQTTS* | 2.11 | Inc-TH-1:1 | 0.76315476 2 | 35 | 0 | 0 |
| IncHOXB815 14691532 | 69 | MQILEYECWPEIRYFLPSSC* | 2.11 | Inc-HOXB8-1:5 | 0.74285714 3 | 21 | 0 | 0 |
| IncHOXB815 11121196 | 70 | MYAKHMGVQVHHPKAMASSVFPKS LRL* | 2.11 | Inc-HOXB8-1:5 | 0.81333333 3 | 28 | 0 | 0 |
| LINC025633 122206 | 71 | MQWKLSVLCPAVPSSIQESTPVDER RN* | 2.11 | LINC02563:3 | 0.98005952 4 | 28 | 0 | 0 |
| IncTRPC531 98164 | 72 | MGVLVEEAPMFPNQSAGLMKR* | 1.05 | Inc-TRPCS-3:1 | 0.94101190 5 | 22 | 0 | 0 |
| IncSLC39A1 021686782 | 73 | MGLGHVPTCSSLYSDWTWKGRREA RASILIL* | 1.05 | Inc-SLC39A10-2:1 | 0.92333333 3 | 32 | 0 | 0 |
| IncSLC39A1 021271373 | 74 | MKSVEVRPLDALAWKSHILLAKAN HKATTNSRR* | 1.05 | Inc-SLC39A10-2:1 | 0.85857142 9 | 34 | 0 | 0 |
| IncSLC39A1 021250373 | 75 | MTARFKRMKSVEVRPLDALAWKSH ILLAKANHKATTNSRR* | 1.05 | Inc-SLC39A10-2:1 | 0.87922619 | 41 | 0 | 0 |
| IncPLA2G1B 236136628 0 | 76 | MIKVKESKFTTLLVFSFYPGCELNKK SAAVAHACNPSPWGGRGGRIT* | 1.05 | Inc-PLA2G1B-2:3 | 0.81559523 8 | 48 | 0 | 0 |
| IncPLA2G1B 231404148 8 | 77 | MLARLVSNSRPQVIRQPRPPKMLGL QA* | 1.05 | Inc-PLA2G1B-2:3 | 0.82273809 5 | 28 | 0 | 0 |
| IncPLA2G1B 239288934 2 | 78 | MKDKVLEVSSDDGYTTM* | 1.05 | Inc-PLA2G1B-2:3 | 0.84059523 8 | 18 | 0 | 0 |
| LINC024189 857923 | 79 | MKARAVAKGTSPSGEASSAQL* | 1.05 | LINC02418:9 | 0.81178571 4 | 22 | 0 | 0 |
| LINC024189 745778 | 80 | MDGFLEEEAR* | 1.05 | LINC02418:9 | 0.91916666 7 | 11 | 0 | 0 |
| LINC024189 706778 | 81 | MREHRGEAPTLGFMDGFLEEEAR* | 1.05 | LINC02418:9 | 0.935 | 24 | 0 | 0 |
| LINC024189 42034404 | 82 | MSLLIRECLSSLYLNFSKRENAFRTS NSDGSRVFLAALFLNEDSPGGSLCCR DTQISPCSCSFQSF* | 1.05 | LINC02418:9 | 0.82738095 2 | 67 | 0 | 0 |
| LINC024189 38063863 | 83 | MMKPRYTAYLAFRQGKPA* | 1.05 | LINC02418:9 | 0.85059523 8 | 19 | 0 | 0 |
| IncSLC39A1 071160117 69 | 84 | MWRHREKLAIYKPTRIAWNKFFHH TPQKESTLTAPPLQSCSLQNCETIHF CCLRR* | 1.05 | Inc-SLC39A10-7:1 | 0.88946428 6 | 56 | 0 | 0 |
| IncTH11213 42221 | 85 | MPDQDWAQPPALQVDRLRSAASEA AWDG* | 1.05 | Inc-TH-1:1 | 0.84398809 5 | 29 | 0 | 0 |
| IncTH11398 04010 | 86 | MMRKEMRPA* | 1.05 | Inc-TH-1:1 | 0.78119047 6 | 10 | 0 | 0 |
| IncCAMK1D 211394143 9 | 87 | MELVAGEALVSLRQ* | 1.05 | Inc-CAMK1D-2:1 | 0.76119047 6 | 15 | 0 | 0 |
| IncCAMKID 211465152 2 | 88 | MEKRSQAKTAVGQSFGET* | 1.05 | Inc-CAMK1D-2:1 | 0.76785714 3 | 19 | 0 | 0 |
| IncHOXB815 12971396 | 89 | MGNNTLEWSGNCWPLSPAVLPEGN PNRRSDSQ* | 1.05 | Inc-HOXB8-1:5 | 0.88904761 9 | 33 | 0 | 0 |
| IncHOXB815 37173807 | 90 | * | 1.05 | Inc-HOXB8-1:5 | 0.83178571 4 | 30 | 0 | 0 |
| IncHOXB815 28042888 | 91 | MQKMEATGRGWESLGFRKEAPGSP VLL* | 1.05 | Inc-HOXB8-1:5 | 0.79678571 4 | 28 | 0 | 0 |
| IncTACR311 275302 | 92 | MSFWRETE* | 1.05 | Inc-TACR3-1:1 | 0.85648809 5 | 9 | 0 | 0 |
| LINC017151 3855891 | 93 | MRKLFGNCVTV* | 1.05 | LINC01715:13 | 0.70976190 5 | 12 | 0 | 0 |
| IncCHM111 11138 | 94 | MAPSAGNP* | 1.05 | Inc-CHM-1:1 | 0.71839285 7 | 9 | 0 | 0 |
| IncOR52J31 128127 | 95 | MSDRREEEGNVEWVASILIKKGTDL PSTVRIT* | 1.05 | Inc-OR52J3-1:1 | 0.96279761 9 | 33 | 0 | 0 |
| CYP1B1AS1 1257141 | 96 | MGAVCEALRQYSPGGFQGSRAASVF SR* | 1.05 | CYP1B1-AS1:12 | 0.94404761 9 | 28 | 0 | 0 |
| IncLRRTM4 33474639 | 97 | MDSAPEEENLETLRLSETYSQLPKL KMFVAGSGTSGFRAGTFIAPVMKKR MKCG* | 1.05 | Inc-LRRTM4-3:3 | 0.90595238 1 | 55 | 0 | 0 |

**Table 5**

| **Camyo -tope** | **SEQ ID NO** | **Camyo -tope Size** | **Camyo Peptide ID** | **Camyopeptide sequence** | **Camyo RNA** | **neoIM_ score** | **nr_ presented_ hlas_ neoMS** | **presented_ hlas_ neoMS** |
|---|---|---|---|---|---|---|---|---|
| MYQHVHH YTVI | 98 | 11 | lncSLC3 9A1021 699888 | MYQHVHHYTVIGLGKAEEK QEPASSSYKQKSCLGVVAH ACNPSTLGGQSGRITRSGDR DHPG* | IncSLC39A 10-2:1 | 0.77835 | 1 | ['A*24:02'] |
| KLFGNCVT V | 99 | 9 | LINC01 715138 55891 | MRKLFGNCVTV* | LINC01 715:13 | 0.7602 | 5 | ['A^{∗}03:01', 'A^{∗}24:02', 'B^{∗}07:02', 'B^{∗}08:01', 'B^{∗}44:02'] |
| LNFRRPFS DYY | 100 | 11 | lncHSF5 214815 74 | MLVSKDGAGKPSTTDCKHL NFRRPFSDYYF* | IncHSF5-2:1 | 0.74035 | 1 | ['A^{∗}01:01'] |
| SFLTILANT VK | 101 | 11 | lncPLA2 G1B236 574662 5 | MSFLTILANTVKPHLY* | IncPLA2G1 B-2:3 | 0.73795 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| LISEDPFLI TI | 102 | 11 | lncHSF5 214976 38 | * | IncHSF5-2:1 | 0.73625 | 1 | ['A^{∗}02:01'] |
| YTAYLAFR QGK | 103 | 11 | LINC02 418938 063863 | MMKPRYTAYLAFRQGKPA* | LINC02 418:9 | 0.736 | 3 | ['A*01:01', 'A^{∗}03:01', 'A^{∗}11:01'] |
| LISTWQHR FMK | 104 | 11 | lncPLA2 G1B233 009306 9 | MPGLLISTWQHRFMKPLTL * | IncPLA2G1 B-2:3 | 0.7339 | 2 | ['A*03:01', 'A^{∗}11:01'] |
| QILEYECW PEI | 105 | 11 | lncHOX B81514 691532 | MQILEYECWPEIRYFLPSSC * | IncHOXB8-1:5 | 0.72685 | 1 | ['A^{∗}02:01'] |
| MAHAGIW MDMV | 106 | 11 | lncTRPC 531701 830 | * | IncTRPC5-3:1 | 0.72285 | 1 | ['C*06:02'] |
| MAHAGIW MDMV | 107 | 11 | IncTRPC 531668 830 | * | IncTRPC5-3:1 | 0.72285 | 1 | ['C*06:02'] |
| MAHAGIW MDMV | 108 | 11 | IncTRPC 531722 830 | MGIGMAHAGIWMDMVAGI AMMTKAAETMIEAMIPG* | IncTRPC5-3:1 | 0.72285 | 1 | ['C*06:02'] |
| MAHAGIW MDMV | 109 | 11 | IncTRPC 531734 830 | MAHAGIWMDMVAGIAMM TKAAETMIEAMIPG* | IncTRPC5-3:1 | 0.72285 | 1 | ['C^{∗}06:02'] |
| ILISEDPFL | 110 | 9 | lncHSF5 214976 38 | * | IncHSF5-2:1 | 0.7204 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| STWQHRF MKPL | 111 | 11 | lncPLA2 G1B233 009306 9 | MPGLLISTWQHRFMKPLTL * | IncPLA2G1 B-2:3 | 0.72015 | 1 | ['A*03:01'] |
| STYVSKPV SWA | 112 | 11 | IncTRPC 531283 28 | * | IncTRPC5-3:1 | 0.717 | 6 | ['A*02:01', 'A^{∗}03:01', 'A^{∗}11:01', 'B^{∗}07:02', 'B^{∗}08:01', 'C^{∗}07:01'] |
| KTVRSSCL DRK | 113 | 11 | IncFANC M10125 163 | MARLKTVRSSCLDRKATGC SPGSCVRILTALTMPRKGRS CCFIRD* | Inc-FANCM-10:1 | 0.71275 | 2 | ['A*03:01', 'A^{∗}11:01'] |
| KTVRSSCL DRK | 114 | 11 | IncFANC M10122 163 | MMARLKTVRSSCLDRKATG CSPGSCVRILTALTMPRKGR SCCFIRD* | Inc-FANCM-10:1 | 0.71275 | 2 | ['A*03:01', 'A^{∗}11:01'] |
| IIGVMIDVP PK | 115 | 11 | IncTRPC 531869 1010 | MMTTEEAGTAMKTDMTD GMIGRGAPEMITLGMIIGV MIDVPPKDPN* | IncTRPC5-3:1 | 0.7119 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 116 | 11 | IncTRPC 531872 1010 | MTTEEAGTAMKTDMTDG MIGRGAPEMITLGMIIGVMI DVPPKDPN* | IncTRPC5-3:1 | 0.7119 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 117 | 11 | IncTRPC 531899 1010 | MKTDMTDGMIGRGAPEMI TLGMIIGVMIDVPPKDPN* | IncTRPC5-3:1 | 0.7119 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 118 | 11 | IncTRPC 531911 1010 | MTDGMIGRGAPEMITLGMI IGVMIDVPPKDPN* | IncTRPC5-3:1 | 0.7119 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 119 | 11 | IncTRPC 531923 1010 | MIGRGAPEMITLGMIIGVMI DVPPKDPN* | IncTRPC5-3:1 | 0.7119 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 120 | 11 | IncTRPC 531947 1010 | MITLGMIIGVMIDVPPKDPN * | IncTRPC5-3:1 | 0.7119 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| IIGVMIDVP PK | 121 | 11 | IncTRPC 531962 1010 | MIIGVMIDVPPKDPN* | IncTRPC5-3:1 | 0.7119 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| SIHPMTGA LCL | 122 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.71105 | 2 | ['A*02:01', 'C^{∗}03:04'] |
| MIIGVMID V | 123 | 9 | IncTRPC 531869 1010 | MMTTEEAGTAMKTDMTD GMIGRGAPEMITLGMIIGV MIDVPPKDPN* | IncTRPC5-3:1 | 0.7108 | 16 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 124 | 9 | IncTRPC 531872 1010 | MTTEEAGTAMKTDMTDG MIGRGAPEMITLGMIIGVMI DVPPKDPN* | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 125 | 9 | IncTRPC 531899 1010 | MKTDMTDGMIGRGAPEMI TLGMIIGVMIDVPPKDPN* | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 126 | 9 | IncTRPC 531911 1010 | MTDGMIGRGAPEMITLGMI IGVMIDVPPKDPN* | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 127 | 9 | IncTRPC 531923 1010 | MIGRGAPEMITLGMIIGVMI DVPPKDPN* | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 128 | 9 | IncTRPC 531947 1010 | MITLGMIIGVMIDVPPKDPN * | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| MIIGVMID V | 129 | 9 | IncTRPC 531962 1010 | MIIGVMIDVPPKDPN* | IncTRPC5-3:1 | 0.7108 | 16 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| APTLGFMD GFL | 130 | 11 | LINC02 418970 6778 | MREHRGEAPTLGFMDGFLE EEAR* | LINC02 418:9 | 0.70625 | 4 | ['B^{∗}07:02', 'B^{∗}08:01', 'B^{∗}35:01', 'B^{∗}51:01'] |
| GQALTLNV MVV | 131 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.70575 | 1 | ['A^{∗}02:01'] |
| GQALTLNV MVV | 132 | 11 | IncPLA2 G1B231 098611 151 | * | IncPLA2G1 B-2:3 | 0.70575 | 1 | ['A^{∗}02:01'] |
| GQALTLNV MVV | 133 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.70575 | 1 | ['A^{∗}02:01'] |
| SYNWGRL TTTV | 134 | 11 | IncTH11 332234 27 | MVWTRRCGASPTSYNWGR LTTTVEGTPWNSQTTS* | Inc-TH-1:1 | 0.7051 | 3 | ['A^{∗}24:02', 'C^{∗}05:01', 'C^{∗}04:01'] |
| ITISKDVHI VL | 135 | 11 | lncHSF5 214976 38 | * | IncHSF5-2:1 | 0.69995 | 8 | ['A*02:01', 'A^{∗}03:01', 'A^{∗}11:01', 'B^{∗}08:01', 'C^{∗}07:01', 'C^{∗}07:02', 'C^{∗}06:02', 'C^{∗}03:04'] |
| MVQENQV QLIV | 136 | 11 | lncHSF5 214976 38 | * | IncHSF5-2:1 | 0.6994 | 1 | ['A^{∗}02:01'] |
| GFHQVLFF QKK | 137 | 11 | IncHOX B81519 402135 | * | IncHOXB8-1:5 | 0.69875 | 2 | ['A^{∗}03:01', 'A^{∗}11:01'] |
| FLTILANT V | 138 | 9 | IncPLA2 G1B236 574662 5 | MSFLTILANTVKPHLY* | IncPLA2G1 B-2:3 | 0.6943 | 13 | ['A^{∗}01:01', 'A^{∗}03:01', 'A^{∗}24:02', 'A^{∗}11:01', 'B^{∗}07:02', 'B^{∗}08:01', 'B^{∗}35:01', 'C^{∗}07:01', 'C^{∗}07:02', 'C^{∗}05:01', 'C^{∗}04:01', 'C^{∗}06:02', 'C^{∗}03:04'] |
| RRWFHDF LAW | 139 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.6941 | 1 | ['C^{∗}06:02'] |
| RRWFHDF LAW | 140 | 11 | IncTH11 352238 04 | * | Inc-TH-1:1 | 0.6941 | 1 | ['C*06:02'] |
| TSYTRTHG QAL | 141 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.6937 | 3 | ['B*08:01', 'C^{∗}07:01', 'C^{∗}03:04'] |
| TSYTRTHG QAL | 142 | 11 | IncPLA2 G1B231 098611 151 | * | IncPLA2G1 B-2:3 | 0.6937 | 3 | ['B*08:01', 'C^{∗}07:01', 'C^{∗}03:04'] |
| TSYTRTHG QAL | 143 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.6937 | 3 | ['B*08:01', 'C^{∗}07:01', 'C^{∗}03:04'] |
| WMDMVA GIAMM | 144 | 11 | IncTRPC 531701 830 | * | IncTRPC5-3:1 | 0.6927 | 3 | ['A*01:01', 'C^{∗}05:01', 'C^{∗}04:01'] |
| WMDMVA GIAMM | 145 | 11 | IncTRPC 531668 830 | * | IncTRPC5-3:1 | 0.6927 | 3 | ['A*01:01', 'C^{∗}05:01', 'C^{∗}04:01'] |
| WMDMVA GIAMM | 146 | 11 | IncTRPC 531722 830 | MGIGMAHAGIWMDMVAGI AMMTKAAETMIEAMIPG* | IncTRPC5-3:1 | 0.6927 | 3 | ['A*01:01', 'C*05:01', 'C*04:01'] |
| WMDMVA GIAMM | 147 | 11 | IncTRPC 531734 830 | MAHAGIWMDMVAGIAMM TKAAETMIEAMIPG* | IncTRPC5-3:1 | 0.6927 | 3 | ['A*01:01', 'C*05:01', 'C*04:01'] |
| IYKPTRIA WNK | 148 | 11 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.69235 | 2 | ['A*03:01', 'A^{∗}11:01'] |
| IYKPTRIA WNK | 149 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.69235 | 2 | ['A*03:01', 'A^{∗}11:01'] |
| IYKPTRIA WNK | 150 | 11 | IncSLC3 9A1071 160117 69 | MWRHREKLAIYKPTRIAW NKFFHHTPQKESTLTAPPL QSCSLQNCETIHFCCLRR* | IncSLC39A 10-7:1 | 0.69235 | 2 | ['A*03:01', 'A^{∗}11:01'] |
| KFTTLLVF SFY | 151 | 11 | IncPLA2 G1B236 136628 0 | MIKVKESKFTTLLVFSFYPG CELNKKSAAVAHACNPSPW GGRGGRIT* | IncPLA2G1 B-2:3 | 0.6914 | 2 | ['A*01:01', 'A^{∗}24:02'] |
| CLYEKYRK YPA | 152 | 11 | IncPLA2 G1B238 302839 5 | MVRSCLYEKYRKYPAEVGG GSLEAWKQRLK* | IncPLA2G1 B-2:3 | 0.68965 | 1 | ['A^{∗}02:01'] |
| ALTLNVMV V | 153 | 9 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.68945 | 4 | ['A*03:01', 'A^{∗}11:01', 'B^{∗}08:01', 'C^{∗}07:01'] |
| ALTLNVMV V | 154 | 9 | IncPLA2 G1B231 098611 151 | * | IncPLA2G1 B-2:3 | 0.68945 | 4 | ['A*03:01', 'A^{∗}11:01', 'B^{∗}08:01', 'C^{∗}07:01'] |
| ALTLNVMV V | 155 | 9 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.68945 | 4 | ['A*03:01', 'A^{∗}11:01', 'B^{∗}08:01', 'C^{∗}07:01'] |
| FSFYPGCEL NK | 156 | 11 | IncPLA2 G1B236 136628 0 | MIKVKESKFTTLLVFSFYPG CELNKKSAAVAHACNPSPW GGRGGRIT* | IncPLA2G1 B-2:3 | 0.68445 | 1 | ['A^{∗}11:01'] |
| AVVTAVGQ LWV | 157 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.68175 | 1 | ['A^{∗}02:01'] |
| AVVTAVGQ LWV | 158 | 11 | IncTH11 352238 04 | * | Inc-TH-1:1 | 0.68175 | 1 | ['A^{∗}02:01'] |
| AMMTKAA ETMI | 159 | 11 | IncTRPC 531701 830 | * | IncTRPC5-3:1 | 0.68125 | 1 | ['A^{∗}02:01'] |
| AMMTKAA ETMI | 160 | 11 | IncTRPC 531668 830 | * | IncTRPC5-3:1 | 0.68125 | 1 | ['A^{∗}02:01'] |
| AMMTKAA ETMI | 161 | 11 | IncTRPC 531722 830 | MGIGMAHAGIWMDMVAGI AMMTKAAETMIEAMIPG* | IncTRPC5-3:1 | 0.68125 | 1 | ['A^{∗}02:01'] |
| AMMTKAA ETMI | 162 | 11 | IncTRPC 531734 830 | MAHAGIWMDMVAGIAMM TKAAETMIEAMIPG* | IncTRPC5-3:1 | 0.68125 | 1 | ['A^{∗}02:01'] |
| AMMTKAA ETMI | 163 | 11 | IncTRPC 531755 830 | MDMVAGIAMMTKAAETMI EAMIPG* | IncTRPC5-3:1 | 0.68125 | 1 | ['A^{∗}02:01'] |
| AMMTKAA ETMI | 164 | 11 | IncTRPC 531761 830 | MVAGIAMMTKAAETMIEA MIPG* | IncTRPC5-3:1 | 0.68125 | 1 | ['A^{∗}02:01'] |
| DPFLITISK DV | 165 | 11 | IncHSF5 214976 38 | * | IncHSF5-2:1 | 0.68005 | 4 | ['B*07:02', 'B^{∗}08:01', 'B^{∗}35:01', 'B^{∗}51:01'] |
| ETLSLLHIQ KI | 166 | 11 | LINC01 715132 053212 8 | MRSEGQDQPGQYGETLSLL KIQKI* | LINC01 715:13 | 0.6795 | 1 | ['B*44:02'] |
| NFTDFRRL AL | 167 | 10 | IncCTR9 122153 50 | MRPLETHWRAGQTKDNFT DFRRLALISHNLRSQDAGHS KKEKEV* | IncCTR9-1:2 | 0.6789 | 1 | ['B*44:02'] |
| FTDFRRLA LIS | 168 | 11 | IncCTR9 122153 50 | MRPLETHWRAGQTKDNFT DFRRLALISHNLRSQDAGHS KKEKEV* | IncCTR9-1:2 | 0.6789 | 1 | ['A^{∗}01:01'] |
| KLAIYKPT RIA | 169 | 11 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.6768 | 1 | ['A^{∗}02:01'] |
| KLAIYKPT RIA | 170 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.6768 | 1 | ['A^{∗}02:01'] |
| KLAIYKPT RIA | 171 | 11 | IncSLC3 9A1071 160117 69 | MWRHREKLAIYKPTRIAW NKFFHHTPQKESTLTAPPL QSCSLQNCETIHFCCLRR* | IncSLC39A 10-7:1 | 0.6768 | 1 | ['A^{∗}02:01'] |
| VMIQTGIG M | 172 | 9 | IncTRPC 531668 830 | * | IncTRPC5-3:1 | 0.6766 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| LISEDPFLI | 173 | 9 | lncHSF5 214976 38 | * | IncHSF5-2:1 | 0.6766 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', |
| | | | | | | | | 'B^{∗}51:01', 'C^{∗}07:01', 'C^{∗}07:02', 'C^{∗}05:01', 'C^{∗}04:01', 'C^{∗}06:02', 'C^{∗}03:04'] |
| NFTDFRRL ALI | 174 | 11 | IncCTR9 122153 50 | MRPLETHWRAGQTKDNFT DFRRLALISHNLRSQDAGHS KKEKEV* | IncCTR9-1:2 | 0.67485 | 1 | ['A*24:02'] |
| ALETSIGIV MI | 175 | 11 | IncTRPC 531668 830 | * | IncTRPC5-3:1 | 0.67305 | 3 | ['A*02:01', 'A^{∗}01:01', 'C^{∗}05:01'] |
| SYTRTHGQ AL | 176 | 10 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.6727 | 2 | ['B*44:02', 'B^{∗}44:03'] |
| SYTRTHGQ AL | 177 | 10 | IncPLA2 G1B231 098611 151 | * | IncPLA2G1 B-2:3 | 0.6727 | 2 | ['B*44:02', 'B^{∗}44:03'] |
| SYTRTHGQ AL | 178 | 10 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.6727 | 2 | ['B*44:02', 'B^{∗}44:03'] |
| LTLNVMW IIR | 179 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.6724 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| LTLNVMW IIR | 180 | 11 | IncPLA2 G1B231 098611 151 | * | IncPLA2G1 B-2:3 | 0.6724 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| LTLNVMW IIR | 181 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.6724 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| KFSSASNY AAL | 182 | 11 | IncTRPC 531160 31801 | MNVPKGQTGNSSRGPGDG GNKDHWKESDRKDGKKDQ DSRSAPEPKKPEENPASKFS SASNYAALS* | IncTRPC5-3:1 | 0.672 | 5 | ['A^{∗}24:02', 'C^{∗}07:01', 'C^{∗}07:02', 'C^{∗}05:01', 'C^{∗}*04:01'] |
| VNMPGKGI MHV | 183 | 11 | LINC02 418923 092465 | MAAEPLVGAECRVCSVNMP GKGIMHVRDRVEKDCARLH HTTQNGTQFRTYK* | LINC02 418:9 | 0.6717 | 3 | ['A*02:01', 'C^{∗}07:01', 'C^{∗}06:02'] |
| RILTALTM PRK | 184 | 11 | IncFANC M10125 163 | MARLKTVRSSCLDRKATGC SPGSCVRILTALTMPRKGRS CCFIRD* | Inc-FANCM-10:1 | 0.671 | 2 | ['A*03:01', 'A^{∗}11:01'] |
| RILTALTM PRK | 185 | 11 | IncFANC M10122 163 | MMARLKTVRSSCLDRKATG CSPGSCVRILTALTMPRKGR SCCFIRD* | Inc-FANCM-10:1 | 0.671 | 2 | ['A*03:01', 'A^{∗}11:01'] |
| TFIAPVMK KRM | 186 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.6709 | 1 | ['A*24:02'] |
| ANEVYPRR DYC | 187 | 11 | IncPLA2 G1B239 761984 5 | MSRGRNKLALRSGSIVANE VYPRRDYC* | IncPLA2G1 B-2:3 | 0.6706 | 1 | ['A^{∗}01:01'] |
| VMIQTGIG MGI | 188 | 11 | IncTRPC 531668 830 | * | IncTRPC5-3:1 | 0.66965 | 1 | ['A^{∗}02:01'] |
| EMFRQLGT VTM | 189 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.66965 | 5 | ['A^{∗}02:01', 'B^{∗}44:02', 'B^{∗}44:03', 'B^{∗}35:01', 'B^{∗}51:01'] |
| EMFRQLGT VTM | 190 | 11 | IncTRPC 531283 28 | * | IncTRPC5-3:1 | 0.66965 | 5 | ['A*02:01', 'B^{∗}44:02', 'B^{∗}44:03', 'B^{∗}35:01', 'B^{∗}51:01'] |
| GIMHVRDR VEK | 191 | 11 | LINC02 418923 092465 | MAAEPLVGAECRVCSVNMP GKGIMHVRDRVEKDCARLH HTTQNGTQFRTYK* | LINC02 418:9 | 0.6692 | 2 | ['A*03:01', 'A^{∗}11:01'] |
| IMHVRDRV EK | 192 | 11 | LINC02 418923 602465 | MPGKGIMHVRDRVEKDCA RLHHTTQNGTQFRTYK* | LINC02 418:9 | 0.6692 | 2 | ['A*03:01', 'A^{∗}11:01'] |
| VHHYTVIG LGK | 193 | 11 | IncSLC3 9A1021 699888 | MYQHVHHYTVIGLGKAEEK QEPASSSYKQKSCLGVVAH ACNPSTLGGQSGRITRSGDR DHPG* | IncSLC39A 10-2:1 | 0.6687 | 3 | ['A*03:01', 'A^{∗}11:01', 'C^{∗}03:04'] |
| TTVEGTP WNSQ | 194 | 11 | IncTH11 332234 27 | MVWTRRCGASPTSYNWGR LTTTVEGTPWNSQTTS* | Inc-TH-1:1 | 0.66825 | 1 | ['A^{∗}01:01'] |
| APATRPAS KDA | 195 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.66705 | 4 | ['B*07:02', 'B^{∗}08:01', 'B^{∗}35:01', 'B^{∗}51:01'] |
| APATRPAS KDA | 196 | 11 | IncTH11 352238 04 | * | Inc-TH-1:1 | 0.66705 | 4 | ['B*07:02', 'B^{∗}08:01', 'B^{∗}35:01', 'B^{∗}51:01'] |
| KYGWWSG NTSY | 197 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.6649 | 4 | ['A*01:01', 'A^{∗}24:02', 'B^{∗}44:03', 'C^{∗}07:01'] |
| KYGWWSG NTSY | 198 | 11 | IncPLA2 G1B231 098611 151 | * | IncPLA2G1 B-2:3 | 0.6649 | 4 | ['A*01:01', 'A^{∗}24:02', 'B^{∗}44:03', 'C^{∗}07:01'] |
| KYGWWSG NTSY | 199 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.6649 | 4 | ['A*01:01', 'A^{∗}24:02', 'B^{∗}44:03', 'C^{∗}07:01'] |
| TYSQLPKL KMF | 200 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.66325 | 2 | ['A*01:01', 'A^{∗}24:02'] |
| GLLGNPIST GA | 201 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.66255 | 1 | ['A^{∗}02:01'] |
| GLLGNPIST GA | 202 | 11 | IncTRPC 531283 28 | * | IncTRPC5-3:1 | 0.66255 | 1 | ['A^{∗}02:01'] |
| QLWELNQ ETQI | 203 | 11 | IncHOX B81537 173807 | * | IncHOXB8-1:5 | 0.66075 | 1 | ['A^{∗}02:01'] |
| GFRAGTFI APV | 204 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.66 | 1 | ['A*24:02'] |
| TQNGTQFR TYK | 205 | 11 | LINC02 418923 092465 | MAAEPLVGAECRVCSVNMP GKGIMHVRDRVEKDCARLH HTTQNGTQFRTYK* | LINC02 418:9 | 0.65855 | 5 | ['A*01:01', 'A^{∗}03:01', 'A^{∗}11:01', 'C^{∗}06:02', 'C^{∗}03:04'] |
| TQNGTQFR TYK | 206 | 11 | LINC02 418923 602465 | MPGKGIMHVRDRVEKDCA RLHHTTQNGTQFRTYK* | LINC02 418:9 | 0.65855 | 5 | ['A*01:01', 'A^{∗}03:01', 'A^{∗}11:01', 'C^{∗}06:02', 'C^{∗}03:04'] |
| TQNGTQFR TYK | 207 | 11 | LINC02 418923 782465 | MHVRDRVEKDCARLHHTT QNGTQFRTYK* | LINC02 418:9 | 0.65855 | 5 | ['A*01:01', 'A^{∗}03:01', 'A^{∗}11:01', 'C^{∗}06:02', 'C^{∗}03:04'] |
| LTILANTV KPH | 208 | 11 | IncPLA2 G1B236 574662 5 | MSFLTILANTVKPHLY* | IncPLA2G1 B-2:3 | 0.6585 | 1 | ['A*03:01'] |
| AVFPNRHP TLL | 209 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.65805 | 14 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}35:01', 'B^{∗}51:01', 'C^{∗}07:01', 'C^{∗}07:02', 'C^{∗}05:01', 'C^{∗}06:02', 'C^{∗}03:04'] |
| AVFPNRHP TLL | 210 | 11 | IncTRPC 531283 28 | * | IncTRPC5-3:1 | 0.65805 | 14 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}35:01', 'B^{∗}51:01', 'C^{∗}07:01', 'C^{∗}07:02', 'C^{∗}05:01', 'C^{∗}06:02', 'C^{∗}03:04'] |
| SASNYAAL | 211 | 8 | IncTRPC 531160 31801 | MNVPKGQTGNSSRGPGDG GNKDHWKESDRKDGKKDQ DSRSAPEPKKPEENPASKFS SASNYAALS* | IncTRPC5-3:1 | 0.65785 | 1 | ['A^{∗}02:01'] |
| KPTRIAWN KFF | 212 | 11 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.65725 | 4 | ['B*07:02', 'B^{∗}08:01', 'B^{∗}35:01', 'B^{∗}51:01'] |
| KPTRIAWN KFF | 213 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.65725 | 4 | ['B*07:02', 'B^{∗}08:01', 'B^{∗}35:01', 'B^{∗}51:01'] |
| KPTRIAWN KFF | 214 | 11 | IncSLC3 9A1071 160117 69 | MWRHREKLAIYKPTRIAW NKFFHHTPQKESTLTAPPL QSCSLQNCETIHFCCLRR* | IncSLC39A 10-7:1 | 0.65725 | 4 | ['B*07:02', 'B^{∗}08:01', 'B^{∗}35:01', 'B^{∗}51:01'] |
| ASSVFPKSL RL | 215 | 11 | IncHOX B81511 121196 | MYAKHMGVQVHHPKAMA SSVFPKSLRL* | IncHOXB8-1:5 | 0.6566 | 2 | ['A*03:01', 'C^{∗}03:04'] |
| MTTEEAGT AMK | 216 | 11 | IncTRPC 531869 1010 | MMTTEEAGTAMKTDMTD GMIGRGAPEMITLGMIIGV MIDVPPKDPN* | IncTRPC5-3:1 | 0.65645 | 15 | ['A*02:01', 'A^{∗}01:01', 'A^{∗}03:01', 'A^{∗}24:02', 'A^{∗}11:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| MTTEEAGT AMK | 217 | 11 | IncTRPC 531872 1010 | MTTEEAGTAMKTDMTDG MIGRGAPEMITLGMIIGVMI DVPPKDPN* | IncTRPC5-3:1 | 0.65645 | 15 | ['A*02:01', 'A^{∗}01:01', 'A^{∗}03:01', 'A^{∗}24:02', 'A^{∗}11:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| FHDFLAW | 218 | 8 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.65565 | 3 | ['B^{∗}44:02', 'B^{∗}44:03', 'B^{∗}51:01'] |
| FHDFLAW | 219 | 8 | IncTH11 352238 04 | * | Inc-TH-1:1 | 0.65565 | 3 | ['B^{∗}44:02', 'B^{∗}44:03', 'B^{∗}51:01'] |
| MIALETSIG IV | 220 | 11 | IncTRPC 531668 830 | * | IncTRPC5-3:1 | 0.6551 | 1 | ['A^{∗}02:01'] |
| NFRRPFSD YYF | 221 | 11 | IncHSF5 214815 74 | MLVSKDGAGKPSTTDCKHL NFRRPFSDYYF* | IncHSF5-2:1 | 0.65255 | 1 | ['A^{∗}24:02'] |
| EMITLGMII GV | 222 | 11 | IncTRPC 531869 1010 | MMTTEEAGTAMKTDMTD GMIGRGAPEMITLGMIIGV MIDVPPKDPN* | IncTRPC5-3:1 | 0.6517 | 1 | ['A^{∗}02:01'] |
| EMITLGMII GV | 223 | 11 | IncTRPC 531872 1010 | MTTEEAGTAMKTDMTDG MIGRGAPEMITLGMIIGVMI DVPPKDPN* | IncTRPC5-3:1 | 0.6517 | 1 | ['A^{∗}02:01'] |
| EMITLGMII GV | 224 | 11 | IncTRPC 531899 1010 | MKTDMTDGMIGRGAPEMI TLGMIIGVMIDVPPKDPN* | IncTRPC5-3:1 | 0.6517 | 1 | ['A^{∗}02:01'] |
| EMITLGMII GV | 225 | 11 | IncTRPC 531911 1010 | MTDGMIGRGAPEMITLGMI IGVMIDVPPKDPN* | IncTRPC5-3:1 | 0.6517 | 1 | ['A^{∗}02:01'] |
| EMITLGMII GV | 226 | 11 | IncTRPC 531923 1010 | MIGRGAPEMITLGMIIGVMI DVPPKDPN* | IncTRPC5-3:1 | 0.6517 | 1 | ['A^{∗}02:01'] |
| FRQLGTVT MTM | 227 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.65125 | 2 | ['C*07:02', 'C^{∗}06:02'] |
| FRQLGTVT MTM | 228 | 11 | IncTRPC 531283 28 | * | IncTRPC5-3:1 | 0.65125 | 2 | ['C*07:02', 'C^{∗}06:02'] |
| GAPEMITL GMI | 229 | 11 | IncTRPC 531869 1010 | MMTTEEAGTAMKTDMTD GMIGRGAPEMITLGMIIGV MIDVPPKDPN* | IncTRPC5-3:1 | 0.651 | 2 | ['A*24:02', 'C^{∗}06:02'] |
| GAPEMITL GMI | 230 | 11 | IncTRPC 531872 1010 | MTTEEAGTAMKTDMTDG MIGRGAPEMITLGMIIGVMI DVPPKDPN* | IncTRPC5-3:1 | 0.651 | 2 | ['A*24:02', 'C^{∗}06:02'] |
| GAPEMITL GMI | 231 | 11 | IncTRPC 531899 1010 | MKTDMTDGMIGRGAPEMI TLGMIIGVMIDVPPKDPN* | IncTRPC5-3:1 | 0.651 | 2 | ['A*24:02', 'C^{∗}06:02'] |
| GAPEMITL GMI | 232 | 11 | IncTRPC 531911 1010 | MTDGMIGRGAPEMITLGMI IGVMIDVPPKDPN* | IncTRPC5-3:1 | 0.651 | 2 | ['A*24:02', 'C^{∗}06:02'] |
| GAPEMITL GMI | 233 | 11 | IncTRPC 531923 1010 | MIGRGAPEMITLGMIIGVMI DVPPKDPN* | IncTRPC5-3:1 | 0.651 | 2 | ['A*24:02', 'C^{∗}06:02'] |
| FSFVLVRR PSR | 234 | 11 | IncHOX B81519 402135 | * | IncHOXB8-1:5 | 0.6509 | 1 | ['A*03:01'] |
| EIETILANT VK | 235 | 11 | IncSLC3 9A1021 811913 | MPVILALWEAKAGGSRDQE IETILANTVKPRLY* | IncSLC39A 10-2:1 | 0.65 | 16 | ['A^{∗}02:01', 'A^{∗}01:01', 'A^{∗}03:01', 'A^{∗}24:02', 'A^{∗}11:01', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| FIAPVMKK RMK | 236 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.64945 | 2 | ['A^{∗}03:01', 'A^{∗}11:01'] |
| SQFPPLGM KDK | 237 | 11 | IncPLA2 G1B239 255934 2 | MGTESQFPPLGMKDKVLEV SSDDGYTTM* | IncPLA2G1 B-2:3 | 0.64935 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| FGFHQVLF FQK | 238 | 11 | IncHOX B81519 402135 | * | IncHOXB8-1:5 | 0.64935 | 6 | ['A^{∗}01:01', 'A^{∗}03:01', 'A^{∗}24:02', 'A^{∗}11:01', 'C^{∗}06:02', 'C^{∗}03:04'] |
| MSYNIYMH IER | 239 | 11 | LINC02 418931 663253 | MSYNIYMHIERGCEYSCRR ASKPKTHRE* | LINC02 418:9 | 0.6493 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| ISKDVHIVL RL | 240 | 11 | lncHSF5 214976 38 | * | IncHSF5-2:1 | 0.6491 | 6 | ['A*03:01', 'B^{∗}08:01', 'C*07:01', 'C^{∗}07:02', 'C^{∗}06:02', 'C^{∗}03:04'] |
| EYECWPEI RYF | 241 | 11 | IncHOX B81514 691532 | MQILEYECWPEIRYFLPSSC * | IncHOXB8-1:5 | 0.649 | 1 | ['A*24:02'] |
| ISTTRAGV RQD | 242 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.64875 | 6 | ['A*01:01', 'A^{∗}03:01', 'A^{∗}24:02', 'A^{∗}11:01', 'C^{∗}07:01', 'C^{∗}06:02'] |
| GTDLPSTV RIT | 243 | 11 | lncOR52 J311281 27 | MSDRREEEGNVEWVASILI KKGTDLPSTVRIT* | Inc-OR52J3-1:1 | 0.64865 | 3 | ['A*01:01', 'A^{∗}03:01', 'A^{∗}11:01'] |
| TAVGQLW VTLK | 244 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.6483 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| TAVGQLW VTLK | 245 | 11 | IncTH11 352238 04 | * | Inc-TH-1:1 | 0.6483 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| GMAHAGI WMDM | 246 | 11 | IncTRPC 531701 830 | * | IncTRPC5-3:1 | 0.64715 | 1 | ['A^{∗}02:01'] |
| GMAHAGI WMDM | 247 | 11 | IncTRPC 531668 830 | * | IncTRPC5-3:1 | 0.64715 | 1 | ['A^{∗}02:01'] |
| GMAHAGI WMDM | 248 | 11 | IncTRPC 531722 830 | MGIGMAHAGIWMDMVAGI AMMTKAAETMIEAMIPG* | IncTRPC5-3:1 | 0.64715 | 1 | ['A^{∗}02:01'] |
| GTFIAPVM KKR | 249 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.6464 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| IWMDMVA GIAM | 250 | 11 | IncTRPC 531701 830 | * | IncTRPC5-3:1 | 0.64635 | 2 | ['A*24:02', 'C^{∗}05:01'] |
| IWMDMVA GIAM | 251 | 11 | IncTRPC 531668 830 | * | IncTRPC5-3:1 | 0.64635 | 2 | ['A*24:02', 'C^{∗}05:01'] |
| IWMDMVA GIAM | 252 | 11 | IncTRPC 531722 830 | MGIGMAHAGIWMDMVAGI AMMTKAAETMIEAMIPG* | IncTRPC5-3:1 | 0.64635 | 2 | ['A*24:02', 'C^{∗}05:01'] |
| IWMDMVA GIAM | 253 | 11 | IncTRPC 531734 830 | MAHAGIWMDMVAGIAMM TKAAETMIEAMIPG* | IncTRPC5-3:1 | 0.64635 | 2 | ['A*24:02', 'C^{∗}05:01'] |
| YEKYRKYP AEV | 254 | 11 | IncPLA2 G1B238 302839 5 | MVRSCLYEKYRKYPAEVGG GSLEAWKQRLK* | IncPLA2G1 B-2:3 | 0.64595 | 1 | ['B*44:02'] |
| LISEDPFL | 255 | 8 | lncHSF5 214976 38 | * | IncHSF5-2:1 | 0.64575 | 9 | ['A*02:01', 'A^{∗}01:01', 'A^{∗}03:01', 'A^{∗}11:01', 'C^{∗}07:02', 'C^{∗}05:01', 'C^{∗}04:01', 'C^{∗}06:02', 'C^{∗}03:04'] |
| KLALRSGSI VA | 256 | 11 | IncPLA2 G1B239 761984 5 | MSRGRNKLALRSGSIVANE VYPRRDYC* | IncPLA2G1 B-2:3 | 0.6454 | 1 | ['A^{∗}02:01'] |
| APEMITLG MII | 257 | 11 | IncTRPC 531869 1010 | MMTTEEAGTAMKTDMTD GMIGRGAPEMITLGMIIGV MIDVPPKDPN* | IncTRPC5-3:1 | 0.6428 | 4 | ['B^{∗}07:02', 'B^{∗}08:01', 'B^{∗}35:01', 'B^{∗}51:01'] |
| APEMITLG MII | 258 | 11 | IncTRPC 531872 1010 | MTTEEAGTAMKTDMTDG MIGRGAPEMITLGMIIGVMI DVPPKDPN* | IncTRPC5-3:1 | 0.6428 | 4 | ['B^{∗}07:02', 'B^{∗}08:01', 'B^{∗}35:01', 'B^{∗}51:01'] |
| APEMITLG MII | 259 | 11 | IncTRPC 531899 1010 | MKTDMTDGMIGRGAPEMI TLGMIIGVMIDVPPKDPN* | IncTRPC5-3:1 | 0.6428 | 4 | ['B*07:02', 'B^{∗}08:01', 'B^{∗}35:01', 'B^{∗}51:01'] |
| APEMITLG MII | 260 | 11 | IncTRPC 531911 1010 | MTDGMIGRGAPEMITLGMI IGVMIDVPPKDPN* | IncTRPC5-3:1 | 0.6428 | 4 | ['B*07:02', 'B^{∗}08:01', 'B^{∗}35:01', 'B^{∗}51:01'] |
| APEMITLG MII | 261 | 11 | IncTRPC 531923 1010 | MIGRGAPEMITLGMIIGVMI DVPPKDPN* | IncTRPC5-3:1 | 0.6428 | 4 | ['B*07:02', 'B^{∗}08:01', 'B^{∗}35:01', 'B^{∗}51:01'] |
| FLAVVTAV | 262 | 8 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.64205 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| FLAVVTAV | 263 | 8 | IncTH11 352238 04 | * | Inc-TH-1:1 | 0.64205 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| AMKTDMT DGMI | 264 | 11 | IncTRPC 531869 1010 | MMTTEEAGTAMKTDMTD GMIGRGAPEMITLGMIIGV MIDVPPKDPN* | IncTRPC5-3:1 | 0.64 | 1 | ['A^{∗}02:01'] |
| AMKTDMT DGMI | 265 | 11 | IncTRPC 531872 1010 | MTTEEAGTAMKTDMTDG MIGRGAPEMITLGMIIGVMI DVPPKDPN* | IncTRPC5-3:1 | 0.64 | 1 | ['A^{∗}02:01'] |
| PMFPNQSA GLM | 266 | 11 | IncTRPC 531981 64 | MGVLVEEAPMFPNQSAGL MKR* | IncTRPC5-3:1 | 0.63975 | 3 | ['B*07:02', 'B^{∗}35:01', 'B^{∗}51:01'] |
| GVISAHFN LRL | 267 | 11 | IncTACR 311255 462 | MKRAWPPCHSGERQSKTK KTESCSATQAGVEGSGVISA HFNLRLLGSSDSPASASRVA GTTAASLQVS* | IncTACR3-1:1 | 0.63965 | 3 | ['A*02:01', 'A^{∗}03:01', 'A^{∗}11:01'] |
| KHMGVQV HHPK | 268 | 11 | IncHOX B81511 121196 | MYAKHMGVQVHHPKAMA SSVFPKSLRL* | IncHOXB8-1:5 | 0.6391 | 1 | ['A*03:01'] |
| DMVAGIA MMTK | 269 | 11 | IncTRPC 531701 830 | * | IncTRPC5-3:1 | 0.639 | 13 | ['A*02:01', 'A^{∗}01:01', 'A^{∗}03:01', 'A^{∗}24:02', 'A^{∗}11:01', 'B^{∗}08:01', 'B^{∗}44:02', 'C^{∗}07:01', 'C^{∗}07:02', 'C^{∗}05:01', 'C^{∗}04:01', 'C^{∗}06:02', 'C^{∗}03:04'] |
| DMVAGIA MMTK | 270 | 11 | IncTRPC 531668 830 | * | IncTRPC5-3:1 | 0.639 | 13 | ['A*02:01', 'A^{∗}01:01', 'A^{∗}03:01', 'A^{∗}24:02', 'A^{∗}11:01', 'B^{∗}08:01', 'B^{∗}44:02', 'C^{∗}07:01', 'C^{∗}07:02', 'C^{∗}05:01', 'C^{∗}04:01', 'C^{∗}06:02', 'C^{∗}03:04'] |
| DMVAGIA MMTK | 271 | 11 | IncTRPC 531722 830 | MGIGMAHAGIWMDMVAGI AMMTKAAETMIEAMIPG* | IncTRPC5-3:1 | 0.639 | 13 | ['A*02:01', 'A^{∗}01:01', 'A^{∗}03:01', 'A^{∗}24:02', 'A^{∗}11:01', 'B^{∗}08:01', 'B^{∗}44:02', 'C^{∗}07:01', 'C^{∗}07:02', 'C^{∗}05:01', 'C^{∗}04:01', 'C^{∗}06:02', 'C^{∗}03:04'] |
| DMVAGIA MMTK | 272 | 11 | IncTRPC 531734 830 | MAHAGIWMDMVAGIAMM TKAAETMIEAMIPG* | IncTRPC5-3:1 | 0.639 | 13 | ['A^{∗}02:01', 'A^{∗}1:01', 'A^{∗}03:01', 'A^{∗}24:02', 'A^{∗}11:01', 'B^{∗}08:01', 'B^{∗}44:02', 'C^{∗}07:01', 'C^{∗}07:02', 'C^{∗}05:01', 'C^{∗}04:01', 'C^{∗}06:02', 'C^{∗}03:04'] |
| DMVAGIA MMTK | 273 | 11 | IncTRPC 531755 830 | MDMVAGIAMMTKAAETMI EAMIPG* | IncTRPC5-3:1 | 0.639 | 13 | ['A*02:01', 'A^{∗}01:01', 'A^{∗}03:01', 'A^{∗}24:02', 'A^{∗}11:01', 'B^{∗}08:01', 'B^{∗}44:02', 'C^{∗}07:01', 'C^{∗}07:02', 'C^{∗}05:01', 'C^{∗}04:01', 'C^{∗}06:02', 'C^{∗}03:04'] |
| SSASNYAA L | 274 | 9 | IncTRPC 531160 31801 | MNVPKGQTGNSSRGPGDG GNKDHWKESDRKDGKKDQ DSRSAPEPKKPEENPASKFS SASNYAALS* | IncTRPC5-3:1 | 0.6388 | 2 | ['A^{∗}03:01', 'A^{∗}11:01'] |
| TTQNGTQF RTY | 275 | 11 | LINC02 418923 092465 | MAAEPLVGAECRVCSVNMP GKGIMHVRDRVEKDCARLH HTTQNGTQFRTYK* | LINC02 418:9 | 0.63875 | 7 | ['A^{∗}01:01', 'A^{∗}03:01', 'A^{∗}11:01', 'B^{∗}35:01', 'C^{∗}07:01', 'C^{∗}07:02', 'C^{∗}06:02'] |
| TTQNGTQF RTY | 276 | 11 | LINC02 418923 602465 | MPGKGIMHVRDRVEKDCA RLHHTTQNGTQFRTYK* | LINC02 418:9 | 0.63875 | 7 | ['A^{∗}01:01', 'A^{∗}03:01', 'A^{∗}11:01', 'B^{∗}35:01', 'C^{∗}07:01', 'C^{∗}07:02', 'C^{∗}06:02'] |
| TTQNGTQF RTY | 277 | 11 | LINC02 418923 782465 | MHVRDRVEKDCARLHHTT QNGTQFRTYK* | LINC02 418:9 | 0.63875 | 7 | ['A^{∗}01:01', 'A^{∗}03:01', 'A^{∗}11:01', 'B^{∗}35:01', 'C^{∗}07:01', 'C^{∗}07:02', 'C^{∗}06:02'] |
| GQTKDNFT DFR | 278 | 11 | IncCTR9 122153 50 | MRPLETHWRAGQTKDNFT DFRRLALISHNLRSQDAGHS KKEKEV* | IncCTR9-1:2 | 0.63865 | 3 | 'A^{∗}03:01', 'A^{∗}11:01', 'C^{∗}03:04'] |
| ASPTSYNW GRL | 279 | 11 | IncTH11 332234 27 | MVWTRRCGASPTSYNWGR LTTTVEGTPWNSQTTS* | Inc-TH-1:1 | 0.6386 | 4 | ['A*02:01', 'A^{∗}01:01', 'A^{∗}24:02', 'C^{∗}06:02'] |
| AQSIHPMT GAL | 280 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.6372 | 1 | ['C*03:04'] |
| DFLAWTA V | 281 | 9 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.63705 | 16 | ['A^{∗}02:01', 'A^{∗}03:01', 'A*24:02', 'A^{∗}11:01', 'B*07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| DFLAWTA V | 282 | 9 | IncTH11 352238 04 | * | Inc-TH-1:1 | 0.63705 | 16 | ['A^{∗}02:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| VPSFPLLH GLL | 283 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.63465 | 4 | ['B^{∗}07:02', 'B^{∗}08:01', 'B^{∗}35:01', 'B^{∗}51:01'] |
| VPSFPLLH GLL | 284 | 11 | IncTRPC 531283 28 | * | IncTRPC5-3:1 | 0.63465 | 4 | ['B*07:02', 'B^{∗}08:01', 'B^{∗}35:01', 'B^{∗}51:01'] |
| VDVLMKR RNLK | 285 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.6335 | 2 | ['A^{∗}03:01', 'A^{∗}11:01'] |
| VISAHFNL | 286 | 8 | IncTACR 311255 462 | MKRAWPPCHSGERQSKTK KTESCSATQAGVEGSGVISA HFNLRLLGSSDSPASASRVA GTTAASLQVS* | IncTACR3-1:1 | 0.63315 | 15 | ['A^{∗}02:01', 'A^{∗}01:01', 'A^{∗}03:01', 'A^{∗}11:01', 'B^{∗}0702', 'B^{∗}08:01', 'B^{∗}44:02', 'B^{∗}44:03', 'B^{∗}35:01', 'C^{∗}07:01', 'C^{∗}07:02', 'C^{∗}05:01', 'C^{∗}04:01', 'C^{∗}06:02', 'C^{∗}03:04'] |
| PQVIRQPR PPK | 287 | 11 | IncPLA2 G1B231 404148 8 | MLARLVSNSRPQVIRQPRP PKMLGLQA* | IncPLA2G1 B-2:3 | 0.6331 | 1 | ['A^{∗}11:01'] |
| PPMQMGL KYGW | 288 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.63295 | 1 | ['B^{∗}51:01'] |
| LLIRECLSS LY | 289 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.63265 | 1 | ['A^{∗}01:01'] |
| ISEDPFLI | 290 | 8 | IncHSF5 214976 38 | * | IncHSF5-2:1 | 0.6303 | 2 | ['B*07:02', 'B^{∗}35:01'] |
| LWVTLKG HSDK | 291 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.62915 | 2 | ['A*03:01', 'A^{∗}11:01'] |
| LWVTLKG HSDK | 292 | 11 | IncTH11 352238 04 | * | Inc-TH-1:1 | 0.62915 | 2 | ['A*03:01', 'A^{∗}11:01'] |
| GTESQFPP LGM | 293 | 11 | IncPLA2 G1B239 255934 2 | MGTESQFPPLGMKDKVLEV SSDDGYTTM* | IncPLA2G1 B-2:3 | 0.6287 | 1 | ['A^{∗}01:01'] |
| MPVILALW EAK | 294 | 11 | IncSLC3 9A1021 811913 | MPVILALWEAKAGGSRDQE IETILANTVKPRLY* | IncSLC39A 10-2:1 | 0.6286 | 2 | ['A*03:01', 'A^{∗}11:01'] |
| SHPEGHLT RPL | 295 | 11 | IncHOX B81519 402135 | * | IncHOXB8-1:5 | 0.6282 | 6 | ['C*07:01', 'C^{∗}07:02', 'C^{∗}05:01', 'C^{∗}04:01', 'C^{∗}06:02', 'C^{∗}03:04'] |
| LRSGSIVAN EV | 296 | 11 | IncPLA2 G1B239 761984 5 | MSRGRNKLALRSGSIVANE VYPRRDYC* | IncPLA2G1 B-2:3 | 0.628 | 1 | ['C*06:02'] |
| VPSSIQEST PV | 297 | 11 | LINC02 563312 2206 | MQWKLSVLCPAVPSSIQES TPVDERRN* | LINC02 563:3 | 0.62795 | 4 | ['B*07:02', 'B^{∗}08:01', 'B^{∗}35:01', 'B^{∗}51:01'] |
| MFVAGSGT SGF | 298 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.62765 | 7 | ['A*01:01', 'A^{∗}24:02', 'B^{∗}44:03', 'B^{∗}35:01', 'B^{∗}51:01', 'C^{∗}05:01', 'C^{∗}04:01'] |
| YMHIERGC EY | 299 | 10 | LINC02 418931 663253 | MSYNIYMHIERGCEYSCRR ASKPKTHRE* | LINC02 418:9 | 0.6276 | 6 | ['A*02:01', 'A^{∗}01:01', 'A^{∗}24:02', 'A^{∗}11:01', 'B^{∗}44:02', 'B^{∗}44:03'] |
| QYGETLSL LKI | 300 | 11 | LINC01 715132 053212 8 | MRSEGQDQPGQYGETLSLL KIQKI* | LINC01 715:13 | 0.62745 | 2 | ['A*01:01', 'A^{∗}24:02'] |
| AMASSVFP KSL | 301 | 11 | IncHOX B81511 121196 | MYAKHMGVQVHHPKAMA SSVFPKSLRL* | IncHOXB8-1:5 | 0.6274 | 3 | ['A*02:01', 'A^{∗}03:01', 'C^{∗}03:04'] |
| PISTGAVFP NR | 302 | 11 | IncTRPC 531160 328 | MTWKEMFRQLGTVTMTM CIGCLQLTVPSFPLLHGLLG NPISTGAVFPNRHPTLLF* | IncTRPC5-3:1 | 0.62715 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| PISTGAVFP NR | 303 | 11 | IncTRPC 531283 28 | * | IncTRPC5-3:1 | 0.62715 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| STLGGQSG RIT | 304 | 11 | IncSLC3 9A1021 699888 | MYQHVHHYTVIGLGKAEEK QEPASSSYKQKSCLGVVAH ACNPSTLGGQSGRITRSGDR DHPG* | IncSLC39A 10-2:1 | 0.62575 | 2 | ['A*03:01', 'A^{∗}11:01'] |
| KVLEVSSD DGY | 305 | 11 | IncPLA2 G1B239 255934 2 | MGTESQFPPLGMKDKVLEV SSDDGYTTM* | IncPLA2G1 B-2:3 | 0.6255 | 10 | ['A*02:01', 'A^{∗}01:01', 'A^{∗}03:01', 'A^{∗}11:01', 'B^{∗}44:03', 'B^{∗}35:01', 'C^{∗}07:01', 'C^{∗}07:02', 'C^{∗}06:02', 'C^{∗}03:04'] |
| KVLEVSSD DGY | 306 | 11 | IncPLA2 G1B239 288934 2 | MKDKVLEVSSDDGYTTM* | IncPLA2G1 B-2:3 | 0.6255 | 10 | ['A^{∗}02:01', 'A^{∗}01:01', 'A^{∗}03:01', 'A^{∗}11:01', 'B^{∗}44:03', 'B^{∗}35:01', 'C^{∗}07:01', 'C^{∗}07:02', 'C^{∗}06:02', 'C^{∗}03:04'] |
| KAAETMIE AMI | 307 | 11 | IncTRPC 531701 830 | * | IncTRPC5-3:1 | 0.6252 | 6 | ['A^{∗}02:01', 'A^{∗}03:01', 'C^{∗}07:01', 'C^{∗}07:02', 'C^{∗}06:02', 'C^{∗}03:04'] |
| KAAETMIE AMI | 308 | 11 | IncTRPC 531668 830 | * | IncTRPC5-3:1 | 0.6252 | 6 | ['A^{∗}02:01', 'A^{∗}03:01', 'C^{∗}07:01', 'C^{∗}07:02', 'C^{∗}06:02', 'C^{∗}03:04'] |
| KAAETMIE AMI | 309 | 11 | IncTRPC 531722 830 | MGIGMAHAGIWMDMVAGI AMMTKAAETMIEAMIPG* | IncTRPC5-3:1 | 0.6252 | 6 | ['A^{∗}02:01', 'A^{∗}03:01', 'C^{∗}07:01', 'C^{∗}07:02', 'C^{∗}06:02', 'C^{∗}03:04'] |
| KAAETMIE AMI | 310 | 11 | IncTRPC 531734 830 | MAHAGIWMDMVAGIAMM TKAAETMIEAMIPG* | IncTRPC5-3:1 | 0.6252 | 6 | ['A^{∗}02:01', 'A^{∗}03:01', 'C^{∗}07:01', 'C^{∗}07:02', 'C^{∗}06:02', 'C^{∗}03:04'] |
| ^{∗} AMI | 311 | 11 | IncTRPC 531755 830 | MDMVAGIAMMTKAAETMI EAMIPG* | IncTRPC5-3:1 | 0.6252 | 6 | ['A*02:01', 'A^{∗}03:01', 'C^{∗}07:01', 'C^{∗}07:02', 'C^{∗}06:02', 'C^{∗}03:04'] |
| KAAETMIE AMI | 312 | 11 | IncTRPC 531761 830 | MVAGIAMMTKAAETMIEA MIPG* | IncTRPC5-3:1 | 0.6252 | 6 | ['A^{∗}02:01', 'A^{∗}03:01', 'C^{∗}07:01', 'C^{∗}07:02', 'C^{∗}06:02', 'C^{∗}03:04'] |
| KAAETMIE AMI | 313 | 11 | IncTRPC 531779 830 | MMTKAAETMIEAMIPG* | IncTRPC5-3:1 | 0.6252 | 6 | ['A*02:01', 'A^{∗}03:01', 'C^{∗}07:01', 'C^{∗}07:02', 'C^{∗}06:02', 'C^{∗}03:04'] |
| KAAETMIE AMI | 314 | 11 | IncTRPC 531782 830 | MTKAAETMIEAMIPG* | IncTRPC5-3:1 | 0.6252 | 6 | ['A*02:01', 'A^{∗}03:01', 'C^{∗}07:01', 'C^{∗}07:02', 'C^{∗}06:02', 'C^{∗}03:04'] |
| RWFHDFL AVVT | 315 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.6245 | 1 | ['A*24:02'] |
| RWFHDFL AVVT | 316 | 11 | IncTH11 352238 04 | * | Inc-TH-1:1 | 0.6245 | 1 | ['A*24:02'] |
| QSGRITRS GDR | 317 | 11 | IncSLC3 9A1021 699888 | MYQHVHHYTVIGLGKAEEK QEPASSSYKQKSCLGVVAH ACNPSTLGGQSGRITRSGDR DHPG* | IncSLC39A 10-2:1 | 0.62325 | 1 | ['A*03:01'] |
| MFPNQSAG LM | 318 | 10 | IncTRPC 531981 64 | MGVLVEEAPMFPNQSAGL MKR* | IncTRPC5-3:1 | 0.62285 | 14 | ['A*02:01', 'A^{∗}01:01', 'A^{∗}03:01', 'A^{∗}24:02', 'A^{∗}11:01', 'B^{∗}44:02', 'B^{∗}44:03', 'B^{∗}51:01', 'C^{∗}07:01', 'C^{∗}07:02', 'C^{∗}05:01', 'C^{∗}04:01', 'C^{∗}06:02', 'C^{∗}03:04'] |
| AMWRHRE KLAI | 319 | 11 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.62285 | 1 | ['A^{∗}02:01'] |
| AMWRHRE KLAI | 320 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.62285 | 1 | ['A^{∗}02:01'] |
| ALAWKSHI LLA | 321 | 11 | IncSLC3 9A1021 271373 | MKSVEVRPLDALAWKSHIL LAKANHKATTNSRR* | IncSLC39A 10-2:1 | 0.622 | 1 | ['A^{∗}02:01'] |
| ALAWKSHI LLA | 322 | 11 | IncSLC3 9A1021 250373 | MTARFKRMKSVEVRPLDAL AWKSHILLAKANHKATTNS RR* | IncSLC39A 10-2:1 | 0.622 | 1 | ['A^{∗}02:01'] |
| FLITISKDV HI | 323 | 11 | IncHSF5 214976 38 | * | IncHSF5-2:1 | 0.62195 | 1 | ['A^{∗}02:01'] |
| SHILLAKA NHK | 324 | 11 | IncSLC3 9A1021 271373 | MKSVEVRPLDALAWKSHIL LAKANHKATTNSRR* | IncSLC39A 10-2:1 | 0.62175 | 2 | ['A*03:01', 'A^{∗}11:01'] |
| SHILLAKA NHK | 325 | 11 | IncSLC3 9A1021 250373 | MTARFKRMKSVEVRPLDAL AWKSHILLAKANHKATTNS RR* | IncSLC39A 10-2:1 | 0.62175 | 2 | ['A*03:01', 'A^{∗}11:01'] |
| FRAGTFIA PVM | 326 | 11 | IncLRRT M43347 4639 | MDSAPEEENLETLRLSETYS QLPKLKMFVAGSGTSGFRA GTFIAPVMKKRMKCG* | IncLRRTM4 -3:3 | 0.62125 | 3 | ['C*07:01', 'C^{∗}07:02', 'C^{∗}06:02'] |
| LYSDWTW KGRR | 327 | 11 | IncSLC3 9A1021 686782 | MGLGHVPTCSSLYSDWTW KGRREARASILIL* | IncSLC39A 10-2:1 | 0.6207 | 2 | ['A*03:01', 'A^{∗}11:01'] |
| SIVANEVY PRR | 328 | 11 | IncPLA2 G1B239 761984 5 | MSRGRNKLALRSGSIVANE VYPRRDYC* | IncPLA2G1 B-2:3 | 0.62005 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| EEGNVEW VASI | 329 | 11 | IncOR52 J311281 27 | MSDRREEEGNVEWVASILI KKGTDLPSTVRIT* | Inc-OR52J3-1:1 | 0.61965 | 2 | ['B*44:02', 'B^{∗}44:03'] |
| GTGGGSTY VSK | 330 | 11 | IncTRPC 531283 28 | * | IncTRPC5-3:1 | 0.61865 | 10 | ['A*01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| NVEWVASI LIK | 331 | 11 | IncOR52 J311281 27 | MSDRREEEGNVEWVASILI KKGTDLPSTVRIT* | Inc-OR52J3-1:1 | 0.61815 | 8 | ['A^{∗}01:01', 'A^{∗}03:01', 'A^{∗}24:02', 'A^{∗}11:01', 'C^{∗}07:01', 'C^{∗}07:02', 'C^{∗}06:02', 'C^{∗}03:04'] |
| GSIVANEV YPR | 332 | 11 | IncPLA2 G1B239 761984 5 | MSRGRNKLALRSGSIVANE VYPRRDYC* | IncPLA2G1 B-2:3 | 0.6179 | 13 | ['A^{∗}02:01', 'A^{∗}01:01', 'A^{∗}03:01', 'A^{∗}24:02', 'A^{∗}11:01', 'B^{∗}44:02', 'B^{∗}51:01', 'C^{∗}07:01', 'C^{∗}07:02', 'C^{∗}05:01', 'C^{∗}04:01', 'C^{∗}06:02', 'C^{∗}03:04'] |
| VSWADET MTWK | 333 | 11 | IncTRPC 531283 28 | * | IncTRPC5-3:1 | 0.617 | 13 | ['A*02:01', 'A^{∗}01:01', 'A^{∗}03:01', 'A^{∗}24:02', 'A^{∗}11:01', 'B^{∗}44:03', 'B^{∗}35:01', 'B^{∗}51:01', 'C^{∗}07:01', 'C^{∗}07:02', 'C^{∗}04:01', 'C^{∗}06:02', 'C^{∗}03:04'] |
| QNLSSFAQ WQY | 334 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.61675 | 1 | ['A^{∗}01:01'] |
| QNLSSFAQ WQY | 335 | 11 | IncPLA2 G1B231 098611 151 | * | IncPLA2G1 B-2:3 | 0.61675 | 1 | ['A^{∗}01:01'] |
| QNLSSFAQ WQY | 336 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.61675 | 1 | ['A^{∗}01:01'] |
| EEGNVEW V | 337 | 8 | lncOR52 J311281 27 | MSDRREEEGNVEWVASILI KKGTDLPSTVRIT* | Inc-OR52J3-1:1 | 0.61565 | 2 | ['A*03:01', 'A^{∗}11:01'] |
| RTSNSDGS RVF | 338 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.6156 | 5 | ['A*01:01', 'A^{∗}03:01', 'A^{∗}24:02', 'B^{∗}44:03', 'C^{∗}07:01'] |
| GHLTRPLE QTK | 339 | 11 | IncHOX B81519 402135 | * | IncHOXB8-1:5 | 0.6156 | 2 | ['A*03:01', 'A^{∗}11:01'] |
| SWADETM TWK | 340 | 10 | IncTRPC 531283 28 | * | IncTRPC5-3:1 | 0.61555 | 12 | ['A^{∗}02:01', 'A^{∗}01:01', 'A^{∗}24:02', 'B^{∗}44:02', 'B^{∗}44:03', 'B^{∗}51:01', 'C^{∗}07:01', 'C^{∗}07:02', 'C^{∗}05:01', 'C^{∗}04:01', 'C^{∗}06:02', 'C^{∗}03:04'] |
| IALETSIGIV M | 341 | 11 | IncTRPC 531668 830 | * | IncTRPC5-3:1 | 0.61525 | 8 | ['B^{∗}07:02', 'B^{∗}08:01', 'B^{∗}35:01','B^{∗}51:01', 'C^{∗}07:01', 'C^{∗}07:02', 'C^{∗}06:02', 'C^{∗}03:04'] |
| STTDCKHL NFR | 342 | 11 | lncHSF5 214815 74 | MLVSKDGAGKPSTTDCKHL NFRRPFSDYYF* | IncHSF5-2:1 | 0.6152 | 1 | ['A^{∗}03:01'] |
| RLVSNSRP QVI | 343 | 11 | IncPLA2 G1B231 404148 8 | MLARLVSNSRPQVIRQPRP PKMLGLQA* | IncPLA2G1 B-2:3 | 0.6149 | 1 | ['A^{∗}02:01'] |
| KPVSWAD ETMT | 344 | 11 | IncTRPC 531283 28 | * | IncTRPC5-3:1 | 0.6142 | 1 | ['B*07:02'] |
| GVISAHFN L | 345 | 9 | IncTACR 311255 462 | MKRAWPPCHSGERQSKTK KTESCSATQAGVEGSGVISA HFNLRLLGSSDSPASASRVA GTTAASLQVS* | IncTACR3-1:1 | 0.614 | 17 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| RTHGQALT LNV | 346 | 11 | IncPLA2 G1B231 094711 151 | MFDTGCSNNKKPPMQMGL KYGWWSGNTSYTRTHGQA LTLNVMVVIIRGGAGREQN LSSFAQWQYRSQ* | IncPLA2G1 B-2:3 | 0.61395 | 1 | ['A^{∗}03:01'] |
| RTHGQALT LNV | 347 | 11 | IncPLA2 G1B231 098611 151 | * | IncPLA2G1 B-2:3 | 0.61395 | 1 | ['A^{∗}03:01'] |
| RTHGQALT LNV | 348 | 11 | IncPLA2 G1B231 099211 151 | MGLKYGWWSGNTSYTRTH GQALTLNVMVVIIRGGAGR EQNLSSFAQWQYRSQ* | IncPLA2G1 B-2:3 | 0.61395 | 1 | ['A^{∗}03:01'] |
| TAMKTDM TDGM | 349 | 11 | IncTRPC 531869 1010 | MMTTEEAGTAMKTDMTD GMIGRGAPEMITLGMIIGV MIDVPPKDPN* | IncTRPC5-3:1 | 0.6127 | 3 | ['B^{∗}08:01', 'B^{∗}35:01', 'B^{∗}51:01'] |
| TAMKTDM TDGM | 350 | 11 | IncTRPC 531872 1010 | MTTEEAGTAMKTDMTDG MIGRGAPEMITLGMIIGVMI DVPPKDPN* | IncTRPC5-3:1 | 0.6127 | 3 | ['B^{∗}08:01', 'B^{∗}35:01', 'B^{∗}51:01'] |
| SEDPFLITI S | 351 | 10 | lncHSF5 214976 38 | * | IncHSF5-2:1 | 0.61205 | 1 | ['B^{∗}51:01'] |
| LLGNPIST | 352 | 8 | IncTRPC 531160 328 | MTWKEMFRQLGTVTMTM CIGCLQLTVPSFPLLHGLLG NPISTGAVFPNRHPTLLF* | IncTRPC5-3:1 | 0.61075 | 11 | ['A^{∗}02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| LLGNPIST | 353 | 8 | IncTRPC 531283 28 | * | IncTRPC5-3:1 | 0.61075 | 11 | ['A*02:01', 'A^{∗}01:01', 'A^{∗}03:01', 'A^{∗}24:02', 'A^{∗}11:01', 'C^{∗}07:01', 'C^{∗}07:02', 'C^{∗}05:01', 'C^{∗}04:01', 'C^{∗}06:02', 'C^{∗}03:04'] |
| QENQVQLI VSI | 354 | 11 | lncHSF5 214976 38 | * | IncHSF5-2:1 | 0.6104 | 2 | ['B*44:02', 'B^{∗}44:03'] |
| NSDGSRVF LAA | 355 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.6098 | 1 | ['A^{∗}01:01'] |
| TSNSDGSR VFL | 356 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.60965 | 3 | ['C*07:02', 'C^{∗}06:02', 'C^{∗}03:04'] |
| AIYKPTRI | 357 | 8 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.6096 | 13 | ['A*01:01', 'A^{∗}03:01', 'A^{∗}24:02', 'A^{∗}11:01', 'B^{∗}07:02', 'B^{∗}44:02', 'B^{∗}44:03', 'C^{∗}07:01', 'C^{∗}07:02', 'C^{∗}05:01', 'C^{∗}04:01', 'C^{∗}06:02', 'C^{∗}03:04'] |
| AIYKPTRI | 358 | 8 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.6096 | 13 | ['A*01:01', 'A^{∗}03:01', 'A^{∗}24:02', 'A^{∗}11:01', 'B^{∗}07:02', 'B^{∗}44:02', 'B^{∗}44:03', 'C^{∗}07:01', 'C^{∗}07:02', 'C^{∗}05:01', 'C^{∗}04:01', 'C^{∗}06:02', 'C^{∗}03:04'] |
| AIYKPTRI | 359 | 8 | IncSLC3 9A1071 160117 69 | MWRHREKLAIYKPTRIAW NKFFHHTPQKESTLTAPPL QSCSLQNCETIHFCCLRR* | IncSLC39A 10-7:1 | 0.6096 | 13 | ['A*01:01', 'A^{∗}03:01', 'A^{∗}24:02', 'A^{∗}11:01', 'B^{∗}07:02', 'B^{∗}44:02', 'B^{∗}44:03', 'C^{∗}07:01', 'C^{∗}07:02', 'C^{∗}05:01', 'C^{∗}04:01', 'C^{∗}06:02', 'C^{∗}03:04'] |
| SFYPGCEL NKK | 360 | 11 | IncPLA2 G1B236 136628 0 | MIKVKESKFTTLLVFSFYPG CELNKKSAAVAHACNPSPW GGRGGRIT* | IncPLA2G1 B-2:3 | 0.6095 | 1 | ['A^{∗}11:01'] |
| FLAVVTAV GQL | 361 | 11 | IncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | Inc-TH-1:1 | 0.6086 | 1 | ['A^{∗}02:01'] |
| FLAVVTAV GQL | 362 | 11 | IncTH11 352238 04 | * | Inc-TH-1:1 | 0.6086 | 1 | ['A^{∗}02:01'] |
| EMITLGMII | 363 | 9 | IncTRPC 531869 1010 | MMTTEEAGTAMKTDMTD GMIGRGAPEMITLGMIIGV MIDVPPKDPN* | IncTRPC5-3:1 | 0.60725 | 3 | ['A^{∗}03:01', 'A^{∗}24:02', 'A^{∗}11:01'] |
| EMITLGMII | 364 | 9 | IncTRPC 531872 1010 | MTTEEAGTAMKTDMTDG MIGRGAPEMITLGMIIGVMI DVPPKDPN* | IncTRPC5-3:1 | 0.60725 | 3 | ['A*03:01', 'A^{∗}24:02', 'A^{∗}11:01'] |
| EMITLGMII | 365 | 9 | IncTRPC 531899 1010 | MKTDMTDGMIGRGAPEMI TLGMIIGVMIDVPPKDPN* | IncTRPC5-3:1 | 0.60725 | 3 | ['A*03:01', 'A^{∗}24:02', 'A^{∗}11:01'] |
| EMITLGMII | 366 | 9 | IncTRPC 531911 1010 | MTDGMIGRGAPEMITLGMI IGVMIDVPPKDPN* | IncTRPC5-3:1 | 0.60725 | 3 | ['A*03:01', 'A^{∗}24:02', 'A^{∗}11:01'] |
| EMITLGMII | 367 | 9 | IncTRPC 531923 1010 | MIGRGAPEMITLGMIIGVMI DVPPKDPN* | IncTRPC5-3:1 | 0.60725 | 3 | ['A*03:01', 'A^{∗}24:02', 'A^{∗}11:01'] |
| VLVEEAPM F | 368 | 9 | IncTRPC 531981 64 | MGVLVEEAPMFPNQSAGL MKR* | IncTRPC5-3:1 | 0.60695 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| LTALTMPR KGR | 369 | 11 | IncFANC M10125 163 | MARLKTVRSSCLDRKATGC SPGSCVRILTALTMPRKGRS CCFIRD* | Inc-FANCM-10:1 | 0.60695 | 1 | ['A^{∗}03:01'] |
| LTALTMPR KGR | 370 | 11 | IncFANC M10122 163 | MMARLKTVRSSCLDRKATG CSPGSCVRILTALTMPRKGR SCCFIRD* | Inc-FANCM-10:1 | 0.60695 | 1 | ['A^{∗}03:01'] |
| LTAPPLQS CSL | 371 | 11 | IncSLC3 9A1071 155617 69 | MKRRNLKTDTPKENAMW RHREKLAIYKPTRIAWNKF FHHTPQKESTLTAPPLQSCS LQNCETIHFCCLRR* | IncSLC39A 10-7:1 | 0.60685 | 1 | ['C^{∗}03:04'] |
| LTAPPLQS CSL | 372 | 11 | IncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | IncSLC39A 10-7:1 | 0.60685 | 1 | ['C^{∗}03:04'] |
| LTAPPLQS CSL | 373 | 11 | IncSLC3 9A1071 160117 69 | MWRHREKLAIYKPTRIAW NKFFHHTPQKESTLTAPPL QSCSLQNCETIHFCCLRR* | IncSLC39A 10-7:1 | 0.60685 | 1 | ['C^{∗}03:04'] |
| GSLEAWK QRLK | 374 | 11 | IncPLA2 G1B238 302839 5 | MVRSCLYEKYRKYPAEVGG GSLEAWKQRLK* | IncPLA2G1 B-2:3 | 0.60655 | 2 | ['A^{∗}03:01', 'A^{∗}11:01'] |
| REFGFHQV LFF | 375 | 11 | IncHOX B81519 402135 | * | IncHOXB8-1:5 | 0.60655 | 3 | ['B*44:02', 'B^{∗}44:03', 'C^{∗}07:02'] |
| ALAWKSHI | 376 | 8 | IncSLC3 9A1021 271373 | MKSVEVRPLDALAWKSHIL LAKANHKATTNSRR* | IncSLC39A 10-2:1 | 0.60625 | 1 | ['A*24:02'] |
| ALAWKSHI | 377 | 8 | IncSLC3 9A1021 250373 | MTARFKRMKSVEVRPLDAL AWKSHILLAKANHKATTNS RR* | IncSLC39A 10-2:1 | 0.60625 | 1 | ['A*24:02'] |
| MMTTEEA GTAM | 378 | 11 | IncTRPC 531869 1010 | MMTTEEAGTAMKTDMTD GMIGRGAPEMITLGMIIGV MIDVPPKDPN* | IncTRPC5-3:1 | 0.606 | 1 | ['A^{∗}02:01'] |
| GMIGRGAP EMI | 379 | 11 | IncTRPC 531869 1010 | MMTTEEAGTAMKTDMTD GMIGRGAPEMITLGMIIGV MIDVPPKDPN* | IncTRPC5-3:1 | 0.60595 | 1 | ['A^{∗}02:01'] |
| GMIGRGAP EMI | 380 | 11 | IncTRPC 531872 1010 | MTTEEAGTAMKTDMTDG MIGRGAPEMITLGMIIGVMI DVPPKDPN* | IncTRPC5-3:1 | 0.60595 | 1 | ['A^{∗}02:01'] |
| GMIGRGAP EMI | 381 | 11 | IncTRPC 531899 1010 | MKTDMTDGMIGRGAPEMI TLGMIIGVMIDVPPKDPN* | IncTRPC5-3:1 | 0.60595 | 1 | ['A^{∗}02:01'] |
| GMIGRGAP EMI | 382 | 11 | lncTRPC 531911 1010 | MTDGMIGRGAPEMITLGMI IGVMIDVPPKDPN* | lncTRPC5-3:1 | 0.60595 | 1 | ['A^{∗}02:01'] |
| IWMDMVA GI | 383 | 9 | lncTRPC 531701 830 | * | lncTRPC5-3:1 | 0.6059 | 12 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01'] |
| IWMDMVA GI | 384 | 9 | lncTRPC 531668 830 | * | lncTRPC5-3:1 | 0.6059 | 12 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01'] |
| IWMDMVA GI | 385 | 9 | lncTRPC 531722 830 | MGIGMAHAGIWMDMVAGI AMMTKAAETMIEAMIPG* | lncTRPC5-3:1 | 0.6059 | 12 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01'] |
| IWMDMVA GI | 386 | 9 | lncTRPC 531734 830 | MAHAGIWMDMVAGIAMM TKAAETMIEAMIPG* | lncTRPC5-3:1 | 0.6059 | 12 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01'] |
| FLITISKDV | 387 | 9 | lncHSF5 214976 38 | * | lncHSF5-2:1 | 0.6058 | 17 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| QTKDNFTD FRR | 388 | 11 | lncCTR9 122153 50 | MRPLETHWRAGQTKDNFT DFRRLALISHNLRSQDAGHS KKEKEV* | lncCTR9-1:2 | 0.6058 | 3 | ['A^{∗}01:01', 'A^{∗}03:01', 'A^{∗}11:01'] |
| SLNVEIRG RGK | 389 | 11 | lncCAM K1D211 343143 9 | MILSLNVEIRGRGKENPME LVAGEALVSLRQ* | lnc-CAMK1 D-2:1 | 0.60575 | 3 | ['A^{∗}01:01', 'A^{∗}03:01', 'A^{∗}11:01'] |
| DALAWKS HILL | 390 | 11 | lncSLC3 9A1021 271373 | MKSVEVRPLDALAWKSHIL LAKANHKATTNSRR* | lncSLC39A 10-2:1 | 0.60555 | 3 | ['B^{∗}08:01', 'B^{∗}35:01', 'B^{∗}51:01'] |
| DALAWKS HILL | 391 | 11 | lncSLC3 9A1021 250373 | MTARFKRMKSVEVRPLDAL AWKSHILLAKANHKATTNS RR* | lncSLC39A 10-2:1 | 0.60555 | 3 | ['B^{∗}08:01', 'B^{∗}35:01', 'B^{∗}51:01'] |
| SSDSPASAS RV | 392 | 11 | lncTACR 311255 462 | MKRAWPPCHSGERQSKTK KTESCSATQAGVEGSGVISA HFNLRLLGSSDSPASASRVA GTTAASLQVS* | lncTACR3-1:1 | 0.60545 | 3 | ['A*01:01', 'C^{∗}05:01', 'C^{∗}04:01'] |
| MVAGIAM MTKA | 393 | 11 | lncTRPC 531701 830 | * | lncTRPC5-3:1 | 0.60505 | 2 | ['A*02:01', 'A^{∗}03:01'] |
| MVAGIAM MTKA | 394 | 11 | lncTRPC 531668 830 | * | lncTRPC5-3:1 | 0.60505 | 2 | ['A*02:01', 'A^{∗}03:01'] |
| MVAGIAM MTKA | 395 | 11 | lncTRPC 531722 830 | MGIGMAHAGIWMDMVAGI AMMTKAAETMIEAMIPG* | lncTRPC5-3:1 | 0.60505 | 2 | ['A*02:01', 'A^{∗}03:01'] |
| MVAGIAM MTKA | 396 | 11 | lncTRPC 531734 830 | MAHAGIWMDMVAGIAMM TKAAETMIEAMIPG* | lncTRPC5-3:1 | 0.60505 | 2 | ['A*02:01', 'A^{∗}03:01'] |
| MVAGIAM MTKA | 397 | 11 | lncTRPC 531755 830 | MDMVAGIAMMTKAAETMI EAMIPG* | lncTRPC5-3:1 | 0.60505 | 2 | ['A*02:01', 'A^{∗}03:01'] |
| MVAGIAM MTKA | 398 | 11 | lncTRPC 531761 830 | MVAGIAMMTKAAETMIEA MIPG* | lncTRPC5-3:1 | 0.60505 | 2 | ['A*02:01', 'A^{∗}03:01'] |
| MPDQDWA QPPA | 399 | 11 | lncTH11 213422 21 | MPDQDWAQPPALQVDRLR SAASEAAWDG* | lnc-TH-1:1 | 0.6039 | 3 | ['B*07:02', 'B^{∗}08:01', 'B^{∗}35:01'] |
| GGFQGSRA ASV | 400 | 11 | CYP1B1 AS1125 7141 | MGAVCEALRQYSPGGFQGS RAASVFSR* | CYP1B1 -AS1:12 | 0.6022 | 1 | ['A^{∗}02:01'] |
| LAIYKPTRI AW | 401 | 11 | lncSLC3 9A1071 155617 69 | MKRRNLKTDTPKENAMW RHREKLAIYKPTRIAWNKF FHHTPQKESTLTAPPLQSCS LQNCETIHFCCLRR* | lncSLC39A 10-7:1 | 0.6018 | 3 | ['B*08:01', 'B^{∗}44:03', 'B^{∗}51:01'] |
| LAIYKPTRI AW | 402 | 11 | lncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | lncSLC39A 10-7:1 | 0.6018 | 3 | ['B*08:01', 'B^{∗}44:03', 'B^{∗}51:01'] |
| LAIYKPTRI AW | 403 | 11 | lncSLC3 9A1071 160117 69 | MWRHREKLAIYKPTRIAW NKFFHHTPQKESTLTAPPL QSCSLQNCETIHFCCLRR* | lncSLC39A 10-7:1 | 0.6018 | 3 | ['B*08:01', 'B^{∗}44:03', 'B^{∗}51:01'] |
| MTDGMIG RGAP | 404 | 11 | lncTRPC 531869 1010 | MMTTEEAGTAMKTDMTD GMIGRGAPEMITLGMIIGV MIDVPPKDPN* | lncTRPC5-3:1 | 0.60155 | 1 | ['A^{∗}01:01'] |
| MTDGMIG RGAP | 405 | 11 | lncTRPC 531872 1010 | MTTEEAGTAMKTDMTDG MIGRGAPEMITLGMIIGVMI DVPPKDPN* | lncTRPC5-3:1 | 0.60155 | 1 | ['A^{∗}01:01'] |
| MTDGMIG RGAP | 406 | 11 | lncTRPC 531899 1010 | MKTDMTDGMIGRGAPEMI TLGMIIGVMIDVPPKDPN* | lncTRPC5-3:1 | 0.60155 | 1 | ['A^{∗}01:01'] |
| MTDGMIG RGAP | 407 | 11 | lncTRPC 531911 1010 | MTDGMIGRGAPEMITLGMI IGVMIDVPPKDPN* | lncTRPC5-3:1 | 0.60155 | 1 | ['A^{∗}01:01'] |
| HVHHYTVI | 408 | 8 | lncSLC3 9A1021 699888 | MYQHVHHYTVIGLGKAEEK QEPASSSYKQKSCLGVVAH ACNPSTLGGQSGRITRSGDR DHPG* | lncSLC39A 10-2:1 | 0.60075 | 3 | ['A*01:01', 'A^{∗}03:01', 'A^{∗}11:01'] |
| GLLGNPIST | 409 | 9 | lncTRPC 531160 328 | MTWKEMFRQLGTVTMTM CIGCLQLTVPSFPLLHGLLG NPISTGAVFPNRHPTLLF* | lncTRPC5-3:1 | 0.5998 | 8 | ['B^{∗}07:02', 'B^{∗}51:01', 'C^{∗}07:01', 'C^{∗}07:02', 'C^{∗}05:01', 'C^{∗}04:01', 'C^{∗}06:02', 'C^{∗}03:04'] |
| GLLGNPIST | 410 | 9 | lncTRPC 531283 28 | * | lncTRPC5-3:1 | 0.5998 | 8 | ['B^{∗}07:02', 'B^{∗}51:01', 'C^{∗}07:01', 'C^{∗}07:02', 'C^{∗}05:01', 'C^{∗}04:01', 'C^{∗}06:02', 'C^{∗}03:04'] |
| MYAKHMG V | 411 | 8 | lncHOX B81511 121196 | MYAKHMGVQVHHPKAMA SSVFPKSLRL* | IncHOXB8-1:5 | 0.5994 | 3 | ['A*01:01', 'A^{∗}24:02', 'B^{∗}51:01'] |
| RAGTFIAP VMK | 412 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.59935 | 3 | ['A*03:01', 'A^{∗}11:01', 'C^{∗}03:04'] |
| TKDNFTDF | 413 | 8 | lncCTR9 122153 50 | MRPLETHWRAGQTKDNFT DFRRLALISHNLRSQDAGHS KKEKEV* | lncCTR9-1:2 | 0.59875 | 5 | ['B*08:01', 'B^{∗}44:02', 'B^{∗}44:03', 'B^{∗}35:01', 'B^{∗}51:01'] |
| LSSLYLNFS KR | 414 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.59855 | 3 | ['A*03:01', 'C^{∗}06:02', 'C^{∗}03:04'] |
| PSREFGFH QVL | 415 | 11 | lncHOX B81519 402135 | * | lncHOXB8-1:5 | 0.59845 | 4 | ['B*07:02', 'B^{∗}08:01', 'B^{∗}35:01', 'B^{∗}51:01'] |
| GASPTSYN WGR | 416 | 11 | lncTH11 332234 27 | MVWTRRCGASPTSYNWGR LTTTVEGTPWNSQTTS* | lnc-TH-1:1 | 0.59795 | 8 | ['A*02:01', 'A^{∗}01:01', 'A^{∗}03:01', 'A^{∗}24:02', 'A^{∗}11:01', 'C^{∗}07:02', 'C^{∗}06:02', 'C^{∗}03:04'] |
| DVHIVLRL LLL | 417 | 11 | lncHSF5 214976 38 | * | lncHSF5-2:1 | 0.5976 | 1 | ['B^{∗}08:01'] |
| ENGFSFVL VRR | 418 | 11 | lncHOX B81519 402135 | * | IncHOXB8-1:5 | 0.59595 | 6 | ['A*01:01', 'A^{∗}03:01', 'A^{∗}11:01', 'C^{∗}07:02', 'C^{∗}06:02', 'C^{∗}03:04'] |
| AQEENGFS FVL | 419 | 11 | lncHOX B81519 402135 | * | lncHOXB8-1:5 | 0.59565 | 4 | ['A*02:01', 'A^{∗}01:01', 'C^{∗}05:01', 'C^{∗}04:01'] |
| KTDMTDG MIGR | 420 | 11 | lncTRPC 531869 1010 | MMTTEEAGTAMKTDMTD GMIGRGAPEMITLGMIIGV MIDVPPKDPN* | lncTRPC5-3:1 | 0.59555 | 3 | ['A*01:01', 'A^{∗}03:01', 'A^{∗}11:01'] |
| KTDMTDG MIGR | 421 | 11 | lncTRPC 531872 1010 | MTTEEAGTAMKTDMTDG MIGRGAPEMITLGMIIGVMI DVPPKDPN* | lncTRPC5-3:1 | 0.59555 | 3 | ['A*01:01', 'A^{∗}03:01', 'A^{∗}11:01'] |
| KTDMTDG MIGR | 422 | 11 | lncTRPC 531899 1010 | MKTDMTDGMIGRGAPEMI TLGMIIGVMIDVPPKDPN* | lncTRPC5-3:1 | 0.59555 | 3 | ['A*01:01', 'A^{∗}03:01', 'A^{∗}11:01'] |
| EVSSDDGY TTM | 423 | 11 | lncPLA2 G1B239 255934 2 | MGTESQFPPLGMKDKVLEV SSDDGYTTM* | lncPLA2G1 B-2:3 | 0.59545 | 1 | ['B^{∗}51:01'] |
| EVSSDDGY TTM | 424 | 11 | lncPLA2 G1B239 288934 2 | MKDKVLEVSSDDGYTTM* | lncPLA2G1 B-2:3 | 0.59545 | 1 | ['B^{∗}51:01'] |
| MVAGIAM M | 425 | 8 | lncTRPC 531701 830 | * | lncTRPC5-3:1 | 0.5951 | 10 | ['A*02:01', 'A^{∗}01:01', 'A^{∗}03:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| MVAGIAM M | 426 | 8 | lncTRPC 531668 830 | * | lncTRPC5-3:1 | 0.5951 | 10 | ['A^{∗}02:01', 'A^{∗}01:01', 'A^{∗}3:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| MVAGIAM M | 427 | 8 | lncTRPC 531722 830 | MGIGMAHAGIWMDMVAGI AMMTKAAETMIEAMIPG* | lncTRPC5-3:1 | 0.5951 | 10 | ['A^{∗}02:01', 'A^{∗}01:01', 'A^{∗}03:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| MVAGIAM M | 428 | 8 | lncTRPC 531734 830 | MAHAGIWMDMVAGIAMM TKAAETMIEAMIPG* | lncTRPC5-3:1 | 0.5951 | 10 | ['A*02:01', 'A^{∗}01:01', 'A^{∗}03:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| MVAGIAM M | 429 | 8 | lncTRPC 531755 830 | MDMVAGIAMMTKAAETMI EAMIPG* | lncTRPC5-3:1 | 0.5951 | 10 | ['A*02:01', 'A^{∗}01:01', 'A^{∗}03:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| MVAGIAM M | 430 | 8 | lncTRPC 531761 830 | MVAGIAMMTKAAETMIEA MIPG* | lncTRPC5-3:1 | 0.5951 | 10 | ['A*02:01', 'A^{∗}01:01', 'A^{∗}03:01', 'A^{∗}11:01', 'C^{∗}07:01', 'C^{∗}07:02', 'C^{∗}05:01', 'C^{∗}04:01', 'C^{∗}06:02', 'C^{∗}03:04'] |
| ALQVDRLR SAA | 431 | 11 | lncTH11 213422 21 | MPDQDWAQPPALQVDRLR SAASEAAWDG* | lnc-TH-1:1 | 0.5949 | 1 | ['A^{∗}02:01'] |
| LGNPISTG AVF | 432 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.5945 | 6 | ['^{∗}A01:01', 'A^{∗}24:02', 'B^{∗}51:01', 'C^{∗}07:01', 'C^{∗}07:02', 'C^{∗}03:04'] |
| LGNPISTG AVF | 433 | 11 | lncTRPC 531283 28 | * | lncTRPC5-3:1 | 0.5945 | 6 | ['A^{∗}01:01', 'A^{∗}**2**4:02', 'B^{∗}51:01', 'C^{∗}07:01', 'C^{∗}07:02', 'C^{∗}03:04'] |
| AGTFIAPV MKK | 434 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.5944 | 7 | ['A^{∗}01:01', 'A^{∗}03:01', 'A^{∗}24:02', 'A^{∗}11:01', 'C^{∗}07:02', 'C^{∗}06:02', 'C^{∗}03:04'] |
| GMKDKVL EV | 435 | 9 | lncPLA2 G1B239 255934 2 | MGTESQFPPLGMKDKVLEV SSDDGYTTM* | lncPLA2G1 B-2:3 | 0.59395 | 17 | ['A*02:01', 'A^{∗}01:01', 'A^{∗}03:01', 'A^{∗}24:02', 'A^{∗}**1**1:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| GFQGSRAA SV | 436 | 10 | CYP1B1 AS1125 7141 | MGAVCEALRQYSPGGFQGS RAASVFSR* | CYP1B1 -AS1:12 | 0.59395 | 2 | ['B*44:02', 'B^{∗}44:03'] |
| SSRGPGDG GNK | 437 | 11 | lncTRPC 531160 31801 | MNVPKGQTGNSSRGPGDG GNKDHWKESDRKDGKKDQ DSRSAPEPKKPEENPASKFS SASNYAALS* | lncTRPC5-3:1 | 0.59375 | 2 | ['A*03:01', 'A^{∗}11:01'] |
| RGAPEMIT LGM | 438 | 11 | lncTRPC 531869 1010 | MMTTEEAGTAMKTDMTD GMIGRGAPEMITLGMIIGV MIDVPPKDPN* | lncTRPC5-3:1 | 0.5933 | 2 | ['C*07:01', 'C^{∗}07:02'] |
| RGAPEMIT LGM | 439 | 11 | lncTRPC 531872 1010 | MTTEEAGTAMKTDMTDG MIGRGAPEMITLGMIIGVMI DVPPKDPN* | lncTRPC5-3:1 | 0.5933 | 2 | ['C*07:01', 'C*07:02'] |
| RGAPEMIT LGM | 440 | 11 | lncTRPC 531899 1010 | MKTDMTDGMIGRGAPEMI TLGMIIGVMIDVPPKDPN* | lncTRPC5-3:1 | 0.5933 | 2 | ['C*07:01', 'C^{∗}07:02'] |
| RGAPEMIT LGM | 441 | 11 | lncTRPC 531911 1010 | MTDGMIGRGAPEMITLGMI IGVMIDVPPKDPN* | lncTRPC5-3:1 | 0.5933 | 2 | ['C*07:01', 'C^{∗}07:02'] |
| RGAPEMIT LGM | 442 | 11 | lncTRPC 531923 1010 | MIGRGAPEMITLGMIIGVMI DVPPKDPN* | lncTRPC5-3:1 | 0.5933 | 2 | ['C*07:01', 'C^{∗}07:02'] |
| GWWSGNT SYTR | 443 | 11 | lncPLA2 G1B231 094711 151 | MFDTGCSNNKKPPMQMGL KYGWWSGNTSYTRTHGQA LTLNVMVVIIRGGAGREQN LSSFAQWQYRSQ* | nc-PLA2G1 B-2:3 | 0.59255 | 8 | ['A*02:01', 'A^{∗}01:01', 'A^{∗}03:01', 'A^{∗}24:02', 'A^{∗}11:01', 'C^{∗}07:02', 'C^{∗}06:02', 'C^{∗}03:04'] |
| GWWSGNT SYTR | 444 | 11 | lncPLA2 G1B231 098611 151 | * | lncPLA2G1 B-2:3 | 0.59255 | 8 | ['A^{∗}02:01', 'A^{∗}01:01', 'A^{∗}03:01', 'A^{∗}24:02', 'A^{∗}11:01', 'C^{∗}07:02', 'C^{∗}06:02', 'C^{∗}03:04'] |
| GWWSGNT SYTR | 445 | 11 | lncPLA2 G1B231 099211 151 | MGLKYGWWSGNTSYTRTH GQALTLNVMVVIIRGGAGR EQNLSSFAQWQYRSQ* | lncPLA2G1 B-2:3 | 0.59255 | 8 | ['A^{∗}02:01', 'A^{∗}01:01', 'A^{∗}03:01', 'A^{∗}24:02', 'A^{∗}11:01', 'C^{∗}07:02', 'C^{∗}06:02', 'C^{∗}03:04'] |
| EAQEENGF SFV | 446 | 11 | lncHOX B81519 402135 | * | lnc-HOXB8-1:5 | 0.592 | 1 | ['B^{∗}51:01'] |
| VEWVASILI | 447 | 9 | lncOR52 J311281 27 | MSDRREEEGNVEWVASILI KKGTDLPSTVRIT* | lncOR52J3-1:1 | 0.5919 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| DGMIGRGA PEM | 448 | 11 | lncTRPC 531869 1010 | MMTTEEAGTAMKTDMTD GMIGRGAPEMITLGMIIGV MIDVPPKDPN* | lncTRPC5-3:1 | 0.5911 | 1 | ['B^{∗}08:01'] |
| DGMIGRGA PEM | 449 | 11 | lncTRPC 531872 1010 | MTTEEAGTAMKTDMTDG MIGRGAPEMITLGMIIGVMI DVPPKDPN* | lncTRPC5-3:1 | 0.5911 | 1 | ['B^{∗}08:01'] |
| DGMIGRGA PEM | 450 | 11 | lncTRPC 531899 1010 | MKTDMTDGMIGRGAPEMI TLGMIIGVMIDVPPKDPN* | lncTRPC5-3:1 | 0.5911 | 1 | ['B^{∗}08:01'] |
| DGMIGRGA PEM | 451 | 11 | lncTRPC 531911 1010 | MTDGMIGRGAPEMITLGMI IGVMIDVPPKDPN* | lncTRPC5-3:1 | 0.5911 | 1 | ['B^{∗}08:01'] |
| VHIVLRLLL LK | 452 | 11 | lncHSF5 214976 38 | * | lncHSF5-2:1 | 0.59105 | 2 | ['A*03:01', 'A*11:01'] |
| DMVAGIA MM | 453 | 9 | lncTRPC 531701 830 | * | lncTRPC5-3:1 | 0.59095 | 17 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C**0**^{∗}7:02', 'C^{∗}05:01', 'C^{∗}04:01', 'C^{∗}06:02', 'C^{∗}03:04'] |
| DMVAGIA MM | 454 | 9 | lncTRPC 531668 830 | * | lncTRPC5-3:1 | 0.59095 | 17 | ['A^{∗}02:01', 'A^{∗}01:01', 'A^{∗}**0**3:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| DMVAGIA MM | 455 | 9 | lncTRPC 531722 830 | MGIGMAHAGIWMDMVAGI AMMTKAAETMIEAMIPG* | lncTRPC5-3:1 | 0.59095 | 17 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| DMVAGIA MM | 456 | 9 | lncTRPC 531734 830 | MAHAGIWMDMVAGIAMM TKAAETMIEAMIPG* | lncTRPC5-3:1 | 0.59095 | 17 | ['A^{∗}02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| DMVAGIA MM | 457 | 9 | lncTRPC 531755 830 | MDMVAGIAMMTKAAETMI EAMIPG* | lncTRPC5-3:1 | 0.59095 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| APMFPNQS AGL | 458 | 11 | lncTRPC 531981 64 | MGVLVEEAPMFPNQSAGL MKR* | lncTRPC5-3:1 | 0.5905 | 4 | ['B^{∗}07:02', 'B^{∗}08:01', 'B^{∗}35:01', 'B^{∗}51:01'] |
| GSRDQEIE TIL | 459 | 11 | lncSLC3 9A1021 811913 | MPVILALWEAKAGGSRDQE IETILANTVKPRLY* | lnc-SLC39A 10-2:1 | 0.59005 | 2 | ['C*07:01', 'C^{∗}06:02'] |
| RSGSIVAN EVY | 460 | 11 | lncPLA2 G1B239 761984 5 | MSRGRNKLALRSGSIVANE VYPRRDYC* | lncPLA2G1 B-2:3 | 0.58895 | 5 | ['A*01:01', 'A^{∗}03:01', 'B^{∗}44:03', 'C^{∗}07:01', 'C^{∗}07:02'] |
| TISKDVHIV LR | 461 | 11 | lncHSF5 214976 38 | * | lncHSF5-2:1 | 0.58875 | 6 | ['A^{∗}01:01', 'A^{∗}03:01', 'A^{∗}24:02', 'A^{∗}11:01', 'C^{∗}06:02', 'C^{∗}03:04'] |
| LTDFLAED GGT | 462 | 11 | lncTRPC 531283 28 | * | lncTRPC5-3:1 | 0.58865 | 1 | ['A^{∗}01:01'] |
| RPSREFGF HQV | 463 | 11 | lncHOX B81519 402135 | * | lnc-HOXB8-1:5 | 0.5886 | 4 | ['B*07:02', 'B^{∗}08:01', 'B^{∗}35:01', 'B^{∗}51:01'] |
| LSETYSQLP KL | 464 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.58845 | 2 | ['A*01:01', 'C^{∗}05:01'] |
| ASAKKKNK KGK | 465 | 11 | lncTRPC 531283 28 | * | lncTRPC5-3:1 | 0.58785 | 2 | ['A^{∗}03:01', 'A^{∗}11:01'] |
| TQAGVEGS GVI | 466 | 11 | lncTACR 311255 462 | MKRAWPPCHSGERQSKTK KTESCSATQAGVEGSGVISA HFNLRLLGSSDSPASASRVA GTTAASLQVS* | lncTACR3-1:1 | 0.58735 | 1 | ['A^{∗}02:01'] |
| MGIGMAH AGIW | 467 | 11 | lncTRPC 531701 830 | * | lncTRPC5-3:1 | 0.5869 | 1 | ['B*44:03'] |
| MGIGMAH AGIW | 468 | 11 | lncTRPC 531668 830 | * | lncTRPC5-3:1 | 0.5869 | 1 | ['B*44:03'] |
| MGIGMAH AGIW | 469 | 11 | lncTRPC 531722 830 | MGIGMAHAGIWMDMVAGI AMMTKAAETMIEAMIPG* | lncTRPC5-3:1 | 0.5869 | 1 | ['B*44:03'] |
| GFMDGFLE EE | 470 | 10 | LINC02 418970 6778 | MREHRGEAPTLGFMDGFLE EEAR* | LINC02 418:9 | 0.5869 | 16 | ['A*02:01', 'A^{∗}01:01', 'A^{∗}03:01', 'A^{∗}24:02', 'A^{∗}11:01', 'B^{∗}07:02', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| NPISTGAV F | 471 | 9 | lncTRPC 531160 328 | MTWKEMFRQLGTVTMTM CIGCLQLTVPSFPLLHGLLG NPISTGAVFPNRHPTLLF* | lncTRPC5-3:1 | 0.5866 | 9 | ['A*02:01', 'A^{∗}01:01', 'A^{∗}03:01', 'A^{∗}24:02', 'A^{∗}11:01', 'B^{∗}07:02', 'B^{∗}44:02', 'B^{∗}44:03', 'C^{∗}06:02'] |
| NPISTGAV F | 472 | 9 | lncTRPC 531283 28 | * | lncTRPC5-3:1 | 0.5866 | 9 | ['A*02:01', 'A^{∗}01:01', 'A^{∗}03:01', 'A^{∗}24:02', 'A^{∗}11:01', 'B^{∗}07:02', 'B^{∗}44:02', 'B^{∗}44:03', 'C^{∗}06:02'] |
| SLYSDWT WKGR | 473 | 11 | lncSLC3 9A1021 686782 | MGLGHVPTCSSLYSDWTW KGRREARASILIL* | lncSLC39A 10-2:1 | 0.5861 | 8 | ['A*01:01', 'A^{∗}03:01', 'A^{∗}24:02', 'A^{∗}11:01', 'C^{∗}07:02', 'C^{∗}04:01', 'C^{∗}06:02', 'C^{∗}03:04'] |
| SGVISAHF NLR | 474 | 11 | lncTACR 311255 462 | MKRAWPPCHSGERQSKTK KTESCSATQAGVEGSGVISA HFNLRLLGSSDSPASASRVA GTTAASLQVS* | lncTACR3-1:1 | 0.58585 | 10 | ['A*02:01', 'A^{∗}01:01', 'A^{∗}03:01', 'A^{∗}**2**4:02', 'A^{∗}11:01', 'C^{∗}07:01', 'C^{∗}07:02', 'C^{∗}04:01', 'C^{∗}06:02', 'C^{∗}03:04'] |
| KVKESKFT TLL | 475 | 11 | lncPLA2 G1B236 136628 0 | MIKVKESKFTTLLVFSFYPG CELNKKSAAVAHACNPSPW GGRGGRIT* | lncPLA2G1 B-2:3 | 0.58545 | 5 | ['A*02:01', 'A^{∗}03:01', 'C^{∗}07:01', 'C^{∗}07:02', 'C^{∗}06:02'] |
| GSTYVSKP VSW | 476 | 11 | lncTRPC 531283 28 | * | lncTRPC5-3:1 | 0.585 | 1 | ['B*44:03'] |
| NMPGKGI MHVR | 477 | 11 | LINC02 418923 092465 | MAAEPLVGAECRVCSVNMP GKGIMHVRDRVEKDCARLH HTTQNGTQFRTYK* | LINC02 418:9 | 0.585 | 3 | ['A*03:01', 'A^{∗}11:01', 'C^{∗}03:04'] |
| LTVPSFPL | 478 | 8 | lncTRPC 531160 328 | MTWKEMFRQLGTVTMTM CIGCLQLTVPSFPLLHGLLG NPISTGAVFPNRHPTLLF* | lncTRPC5-3:1 | 0.58495 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| LTVPSFPL | 479 | 8 | lncTRPC 531283 28 | * | lncTRPC5-3:1 | 0.58495 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| EQGTRLPR GSW | 480 | 11 | LINC02 418929 103054 | MTGALCLRSAKPRSEVLTR LHQEQGTRLPRGSWSTEGR DAHKHTMSK* | LINC02 418:9 | 0.58445 | 2 | ['B*44:02', 'B^{∗}44:03'] |
| EQGTRLPR GSW | 481 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.58445 | 2 | ['B*44:02', 'B^{∗}44:03'] |
| ILSLNVEIR GR | 482 | 11 | lncCAM K1D211 343143 9 | MILSLNVEIRGRGKENPME LVAGEALVSLRQ* | lnc-CAMK1 D-2:1 | 0.5843 | 7 | ['A*01:01', 'A^{∗}03:01', 'A^{∗}24:02', 'A^{∗}11:01', 'C^{∗}07:02', 'C^{∗}06:02', 'C^{∗}03:04'] |
| HLNFRRPF SDY | 483 | 11 | lncHSF5 214815 74 | MLVSKDGAGKPSTTDCKHL NFRRPFSDYYF* | lncHSF5-2:1 | 0.5842 | 1 | ['A^{∗}01:01'] |
| KYPAEVGG GSL | 484 | 11 | lncPLA2 G1B238 302839 5 | MVRSCLYEKYRKYPAEVGG GSLEAWKQRLK* | lncPLA2G1 B-2:3 | 0.5837 | 11 | ['A*02:01', 'A^{∗}01:01', 'A^{∗}03:01', 'A^{∗}24:02', 'B^{∗}07:02', 'C^{∗}07:01', 'C^{∗}07:02', 'C^{∗}05:01', 'C^{∗}04:01', 'C^{∗}06:02', 'C^{∗}03:04'] |
| FMDGFLEE EA | 485 | 10 | LINC02 418970 6778 | MREHRGEAPTLGFMDGFLE EEAR* | LINC02 418:9 | 0.5832 | 4 | ['B*44:02', 'B^{∗}44:03', 'B^{∗}35:01', 'B^{∗}51:01'] |
| NLSSFAQW QYR | 486 | 11 | lncPLA2 G1B231 094711 151 | MFDTGCSNNKKPPMQMGL KYGWWSGNTSYTRTHGQA LTLNVMVVIIRGGAGREQN LSSFAQWQYRSQ* | lncPLA2G1 B-2:3 | 0.5819 | 2 | ['A*03:01', 'A^{∗}11:01'] |
| NLSSFAQW QYR | 487 | 11 | lncPLA2 G1B231 098611 151 | * | lncPLA2G1 B-2:3 | 0.5819 | 2 | ['A*03:01', 'A^{∗}11:01'] |
| NLSSFAQW QYR | 488 | 11 | lncPLA2 G1B231 099211 151 | MGLKYGWWSGNTSYTRTH GQALTLNVMVVIIRGGAGR EQNLSSFAQWQYRSQ* | lncPLA2G1 B-2:3 | 0.5819 | 2 | ['A*03:01', 'A^{∗}11:01'] |
| YTRTHGQA LTL | 489 | 11 | lncPLA2 G1B231 094711 151 | MFDTGCSNNKKPPMQMGL KYGWWSGNTSYTRTHGQA LTLNVMVVIIRGGAGREQN LSSFAQWQYRSQ* | lncPLA2G1 B-2:3 | 0.58145 | 2 | ['B*08:01', 'C^{∗}07:01'] |
| YTRTHGQA LTL | 490 | 11 | lncPLA2 G1B231 098611 151 | * | lncPLA2G1 B-2:3 | 0.58145 | 2 | ['B*08:01', 'C^{∗}07:01'] |
| YTRTHGQA LTL | 491 | 11 | lncPLA2 G1B231 099211 151 | MGLKYGWWSGNTSYTRTH GQALTLNVMVVIIRGGAGR EQNLSSFAQWQYRSQ* | lncPLA2G1 B-2:3 | 0.58145 | 2 | ['B*08:01', 'C^{∗}07:01'] |
| QTKDNFTD F | 492 | 9 | lncCTR9 122153 50 | MRPLETHWRAGQTKDNFT DFRRLALISHNLRSQDAGHS KKEKEV* | lncCTR9-1:2 | 0.58095 | 14 | ['A*02:01', 'A^{∗}01:01', 'A^{∗\}03:01', 'A^{∗}24:02', 'A^{∗}11:01', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}04:01', 'C^{∗}06:02'] |
| EENGFSFV LVR | 493 | 11 | lncHOX B81519 402135 | * | lnc-HOXB8-1:5 | 0.5808 | 11 | ['A*02:01', 'A^{∗}01:01', 'A^{∗}03:01', 'A^{∗}24:02', 'A^{∗}11:01', 'B^{∗}44:02', 'B^{∗}44:03', 'C^{∗}07:01', 'C^{∗}07:02', 'C^{∗}06:02', 'C^{∗}03:04'] |
| LLGNPISTG AV | 494 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.58025 | 1 | ['A^{∗}02:01'] |
| LLGNPISTG AV | 495 | 11 | lncTRPC 531283 28 | * | lncTRPC5-3:1 | 0.58025 | 1 | ['A^{∗}02:01'] |
| RIISTTRAG VR | 496 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.58025 | 2 | ['A*03:01', 'A^{∗}11:01'] |
| NPISTGAV FPN | 497 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.58005 | 4 | ['B*07:02', 'B^{∗}08:01', 'B^{∗}35:01', 'B^{∗}51:01'] |
| NPISTGAV FPN | 498 | 11 | lncTRPC 531283 28 | * | lncTRPC5-3:1 | 0.58005 | 4 | ['B*07:02', 'B^{∗}08:01', 'B^{∗}35:01', 'B^{∗}51:01'] |
| SIQESTPVD ER | 499 | 11 | LINC02 563312 2206 | MQWKLSVLCPAVPSSIQES TPVDERRN* | LINC02 563:3 | 0.5791 | 15 | ['A*02:01', 'A^{∗}01:01', 'A^{∗}03:01', 'A^{∗}24:02', 'A^{∗}11:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| TVPSFPLL HGL | 500 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.5787 | 14 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| TVPSFPLL HGL | 501 | 11 | lncTRPC 531283 28 | | lncTRPC5-3:1 | 0.5787 | 14 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| | | | | * | | | | |
| IAMMTKA AETM | 502 | 11 | lncTRPC 531701 830 | * | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| IAMMTKA AETM | 503 | 11 | lncTRPC 531668 830 | * | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| IAMMTKA AETM | 504 | 11 | lncTRPC 531722 830 | MGIGMAHAGIWMDMVAGI AMMTKAAETMIEAMIPG* | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| IAMMTKA AETM | 505 | 11 | lncTRPC 531734 830 | MAHAGIWMDMVAGIAMM TKAAETMIEAMIPG* | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| IAMMTKA AETM | 506 | 11 | lncTRPC 531755 830 | MDMVAGIAMMTKAAETMI EAMIPG* | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| IAMMTKA AETM | 507 | 11 | lncTRPC 531761 830 | MVAGIAMMTKAAETMIEA MIPG* | lncTRPC5-3:1 | 0.57865 | 8 | ['B*07:02', 'B^{∗}08:01', 'B^{∗}35:01', 'B^{∗}51:01', 'C^{∗}07:01', 'C^{∗}07:02', 'C^{∗}06:02', 'C^{∗}03:04'] |
| VEWVASILI KK | 508 | 11 | lncOR52 J311281 27 | MSDRREEEGNVEWVASILI KKGTDLPSTVRIT* | lncOR52J3-1:1 | 0.5782 | 8 | ['A*01:01', 'A^{∗}03:01', 'A^{∗}24:02', 'A^{∗}11:01', 'C^{∗}07:01', 'C^{∗}07:02', 'C^{∗}06:02', 'C^{∗}03:04'] |
| FLNEDSPG GS | 509 | 10 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.57745 | 4 | ['A^{∗}24:02', 'B^{∗}44:02', 'B^{∗}44:03', 'B^{∗}51:01'] |
| SPGSCVRIL TA | 510 | 11 | lncFANC M10125 163 | MARLKTVRSSCLDRKATGC SPGSCVRILTALTMPRKGRS CCFIRD* | lnc-FANCM-10:1 | 0.5773 | 3 | ['B*07:02', 'B^{∗}08:01', 'B^{∗}35:01'] |
| SPGSCVRIL TA | 511 | 11 | lncFANC M10122 163 | MMARLKTVRSSCLDRKATG CSPGSCVRILTALTMPRKGR SCCFIRD* | lnc-FANCM-10:1 | 0.5773 | 3 | ['B*07:02', 'B^{∗}08:01', 'B^{∗}35:01'] |
| GFQGSRAA SVF | 512 | 11 | CYP1B1 AS1125 7141 | MGAVCEALRQYSPGGFQGS RAASVFSR* | CYP1B1 -AS1:12 | 0.5768 | 3 | ['A*24:02', 'B^{∗}51:01', 'C^{∗}05:01'] |
| EALRQYSP GGF | 513 | 11 | CYP1B1 AS1125 7141 | MGAVCEALRQYSPGGFQGS RAASVFSR* | CYP1B1 -AS1:12 | 0.57655 | 2 | ['B*35:01', 'B^{∗}51:01'] |
| GPGDGGNK DHW | 514 | 11 | lncTRPC 531160 31801 | MNVPKGQTGNSSRGPGDG GNKDHWKESDRKDGKKDQ DSRSAPEPKKPEENPASKFS SASNYAALS* | lncTRPC5-3:1 | 0.5762 | 1 | ['B^{∗}51:01'] |
| AQEENGFS F | 515 | 9 | lncHOX B81519 402135 | * | lnc-HOXB8-1:5 | 0.57595 | 5 | ['B^{∗}07:02', 'B^{∗}51:01', 'C^{∗}07:01', 'C^{∗}05:01', 'C^{∗}03:04'] |
| ETILANTV KPR | 516 | 11 | lncSLC3 9A1021 811913 | MPVILALWEAKAGGSRDQE IETILANTVKPRLY* | lncSLC39A 10-2:1 | 0.57555 | 11 | ['A*01:01', 'A^{∗}03:01', 'A^{∗}24:02', 'A^{∗}11:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| AFRTSNSD GSR | 517 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.575 | 2 | ['A*03:01', 'A^{∗}11:01'] |
| TTQNGTQF | 518 | 8 | LINC02 418923 092465 | MAAEPLVGAECRVCSVNMP GKGIMHVRDRVEKDCARLH HTTQNGTQFRTYK* | LINC02 418:9 | 0.5748 | 13 | ['A*02:01', 'A^{∗}03:01', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}04:01', 'C^{∗}06:02', 'C^{∗}03:04'] |
| TTQNGTQF | 519 | 8 | LINC02 418923 602465 | MPGKGIMHVRDRVEKDCA RLHHTTQNGTQFRTYK* | LINC02 418:9 | 0.5748 | 13 | ['A*02:01', 'A^{∗}03:01', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}04:01', 'C^{∗}06:02', 'C^{∗}03:04'] |
| TTQNGTQF | 520 | 8 | LINC02 418923 782465 | MHVRDRVEKDCARLHHTT QNGTQFRTYK* | LINC02 418:9 | 0.5748 | 13 | ['A*02:01', 'A^{∗}03:01', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}04:01', 'C^{∗}06:02', 'C^{∗}03:04'] |
| LVAGEALV | 521 | 8 | lncCAM K1D211 343143 9 | MILSLNVEIRGRGKENPME LVAGEALVSLRQ* | lnc-CAMK1 D-2:1 | 0.5745 | 5 | ['A*01:01', 'B^{∗}07:02', 'B^{∗}44:02', 'B^{∗}44:03', 'B^{∗}35:01'] |
| LVAGEALV | 522 | 8 | lncCAM K1D211 394143 9 | MELVAGEALVSLRQ* | lnc-CAMK1 D-2:1 | 0.5745 | 5 | ['A*01:01', 'B^{∗}07:02', 'B^{∗}44:02', 'B^{∗}44:03', 'B^{∗}35:01'] |
| QEIETILAN TV | 523 | 11 | lncSLC3 9A1021 811913 | MPVILALWEAKAGGSRDQE IETILANTVKPRLY* | lncSLC39A 10-2:1 | 0.5741 | 2 | ['B*44:02', 'B*44:03'] |
| FPNRHPTL L | 524 | 9 | lncTRPC 531160 328 | MTWKEMFRQLGTVTMTM CIGCLQLTVPSFPLLHGLLG NPISTGAVFPNRHPTLLF* | lncTRPC5-3:1 | 0.5735 | 4 | ['A*02:01', 'A^{∗}03:01', 'A^{∗}24:02', 'A^{∗}11:01'] |
| FPNRHPTL L | 525 | 9 | lncTRPC 531283 28 | * | lncTRPC5-3:1 | 0.5735 | 4 | ['A*02:01', 'A^{∗}03:01', 'A^{∗}24:02', 'A^{∗}11:01'] |
| ILISEDPFLI T | 526 | 11 | lncHSF5 214976 38 | * | lncHSF5-2:1 | 0.5733 | 1 | ['A^{∗}02:01'] |
| GAVFPNRH PTL | 527 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.57155 | 4 | ['B*08:01', 'C^{∗}07:01', 'C^{∗}07:02', 'C^{∗}03:04'] |
| GAVFPNRH PTL | 528 | 11 | lncTRPC 531283 28 | * | lncTRPC5-3:1 | 0.57155 | 4 | ['B*08:01', 'C^{∗}07:01', 'C^{∗}07:02', 'C^{∗}03:04'] |
| SREFGFHQ VLF | 529 | 11 | lncHOX B81519 402135 | * | lnc-HOXB8-1:5 | 0.57155 | 3 | ['C*07:02', 'C^{∗}04:01', 'C^{∗}06:02'] |
| AVFPNRHP TL | 530 | 10 | lncTRPC 531160 328 | MTWKEMFRQLGTVTMTM CIGCLQLTVPSFPLLHGLLG NPISTGAVFPNRHPTLLF* | lncTRPC5-3:1 | 0.57105 | 1 | ['B^{∗}51:01'] |
| AVFPNRHP TL | 531 | 10 | lncTRPC 531283 28 | * | lncTRPC5-3:1 | 0.57105 | 1 | ['B^{∗}51:01'] |
| EAPTLGFM DGF | 532 | 11 | LINC02 418970 6778 | MREHRGEAPTLGFMDGFLE EEAR* | LINC02 418:9 | 0.57045 | 4 | ['A*01:01', 'A^{∗}24:02', 'B^{∗}35:01', 'B^{∗}51:01'] |
| KTRQLQNC ERR | 533 | 11 | lncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | lnc-TH-1:1 | 0.57035 | 1 | ['A*03:01'] |
| KTRQLQNC ERR | 534 | 11 | lncTH11 352238 04 | * | lnc-TH-1:1 | 0.57035 | 1 | ['A*03:01'] |
| YRKYPAEV | 535 | 8 | lncPLA2 G1B238 302839 5 | MVRSCLYEKYRKYPAEVGG GSLEAWKQRLK* | lncPLA2G1 B-2:3 | 0.57 | 3 | ['A*01:01', 'A^{∗}03:01', 'A^{∗}11:01'] |
| LAWKSHIL LAK | 536 | 11 | lncSLC3 9A1021 271373 | MKSVEVRPLDALAWKSHIL LAKANHKATTNSRR* | lncSLC39A 10-2:1 | 0.569 | 2 | ['A*03:01', 'A^{∗}11:01'] |
| LAWKSHIL LAK | 537 | 11 | lncSLC3 9A1021 250373 | MTARFKRMKSVEVRPLDAL AWKSHILLAKANHKATTNS RR* | lncSLC39A 10-2:1 | 0.569 | 2 | ['A*03:01', 'A^{∗}11:01'] |
| SEAQEENG FSF | 538 | 11 | lncHOX B81519 402135 | * | lnc-HOXB8-1:5 | 0.56855 | 2 | ['B*44:02', 'B^{∗}44:03'] |
| NSRPQVIR QPR | 539 | 11 | lncPLA2 G1B231 404148 8 | MLARLVSNSRPQVIRQPRP PKMLGLQA* | lncPLA2G1 B-2:3 | 0.5684 | 1 | ['A*03:01'] |
| QFPPLGMK DKV | 540 | 11 | lncPLA2 G1B239 255934 2 | MGTESQFPPLGMKDKVLEV SSDDGYTTM* | lncPLA2G1 B-2:3 | 0.5679 | 1 | ['A*24:02'] |
| AETMIEAM I | 541 | 9 | lncTRPC 531701 830 | * | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 542 | 9 | lncTRPC 531668 830 | * | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 543 | 9 | lncTRPC 531722 830 | MGIGMAHAGIWMDMVAGI AMMTKAAETMIEAMIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 544 | 9 | lncTRPC 531734 830 | MAHAGIWMDMVAGIAMM TKAAETMIEAMIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 545 | 9 | lncTRPC 531755 830 | MDMVAGIAMMTKAAETMI EAMIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}7:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 546 | 9 | lncTRPC 531761 830 | MVAGIAMMTKAAETMIEA MIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 547 | 9 | lncTRPC 531779 830 | MMTKAAETMIEAMIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| AETMIEAM I | 548 | 9 | lncTRPC 531782 830 | MTKAAETMIEAMIPG* | lncTRPC5-3:1 | 0.5677 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| KSAPATRP ASK | 549 | 11 | lncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | lnc-TH-1:1 | 0.56675 | 2 | ['A*03:01', 'A^{∗}11:01'] |
| KSAPATRP ASK | 550 | 11 | lncTH11 352238 04 | * | lnc-TH-1:1 | 0.56675 | 2 | ['A*03:01', 'A^{∗}11:01'] |
| NLLLSHPE GHL | 551 | 11 | lncHOX B81519 402135 | * | lnc-HOXB8-1:5 | 0.566 | 1 | ['A^{∗}02:01'] |
| FPPLGMKD KVL | 552 | 11 | lncPLA2 G1B239 255934 2 | MGTESQFPPLGMKDKVLEV SSDDGYTTM* | lncPLA2G1 B-2:3 | 0.5654 | 11 | ['A*24:02', 'B^{∗}07:02', 'B^{∗}08:01','B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| TTEEAGTA MKT | 553 | 11 | lncTRPC 531869 1010 | MMTTEEAGTAMKTDMTD GMIGRGAPEMITLGMIIGV MIDVPPKDPN* | lncTRPC5-3:1 | 0.56535 | 1 | ['A^{∗}01:01'] |
| TTEEAGTA MKT | 554 | 11 | lncTRPC 531872 1010 | MTTEEAGTAMKTDMTDG MIGRGAPEMITLGMIIGVMI DVPPKDPN* | lncTRPC5-3:1 | 0.56535 | 1 | ['A^{∗}01:01'] |
| VEGSGVISA HF | 555 | 11 | lncTACR 311255 462 | MKRAWPPCHSGERQSKTK KTESCSATQAGVEGSGVISA HFNLRLLGSSDSPASASRVA GTTAASLQVS* | lncTACR3-1:1 | 0.56525 | 2 | ['B*44:02', 'B^{∗}44:03'] |
| ILIKKGTDL | 556 | 9 | lncOR52 J311281 27 | MSDRREEEGNVEWVASILI KKGTDLPSTVRIT* | lncOR52J3-1:1 | 0.56525 | 15 | ['A*02:01', 'A^{∗}01:01', 'A^{∗}03:01', 'A^{∗}24:02', 'A^{∗}11:01', 'B^{∗}07:02', 'B^{∗}44:02', 'B^{∗}44:03', 'B^{∗}51:01', 'C^{∗}07:01', 'C^{∗}07:02', 'C^{∗}05:01', 'C^{∗}04:01', 'C^{∗}06:02', 'C^{∗}03:04'] |
| HHTTQNG TQFR | 557 | 11 | LINC02 418923 092465 | MAAEPLVGAECRVCSVNMP GKGIMHVRDRVEKDCARLH HTTQNGTQFRTYK* | LINC02 418:9 | 0.5652 | 7 | ['A*02:01', 'A^{∗}01:01', 'A^{∗}03:01', 'A^{∗}11:01', 'C^{∗}07:02', 'C^{∗}06:02', 'C^{∗}03:04'] |
| HHTTQNG TQFR | 558 | 11 | LINC02 418923 602465 | MPGKGIMHVRDRVEKDCA RLHHTTQNGTQFRTYK* | LINC02 418:9 | 0.5652 | 7 | ['A*02:01', 'A^{∗}01:01', 'A^{∗}03:01', 'A^{∗}11:01', 'C^{∗}07:02', 'C^{∗}06:02', 'C^{∗}03:04'] |
| HHTTQNG TQFR | 559 | 11 | LINC02 418923 782465 | MHVRDRVEKDCARLHHTT QNGTQFRTYK* | LINC02 418:9 | 0.5652 | 7 | ['A*02:01', 'A^{∗}01:01', 'A^{∗}03:01', 'A^{∗}11:01', 'C^{∗}07:02', 'C^{∗}06:02', 'C^{∗}03:04'] |
| SETYSQLP KLK | 560 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.56395 | 5 | ['A*01:01', 'A^{∗}03:01', 'A^{∗}24:02', 'A^{∗}11:01', 'C^{∗}03:04'] |
| LNFSKREN AFR | 561 | 11 | LINC02 418942 034404 | MSLLIRECLSSLYLNFSKRE NAFRTSNSDGSRVFLAALFL NEDSPGGSLCCRDTQISPCS CSFQSF* | LINC02 418:9 | 0.56375 | 1 | ['A*03:01'] |
| QFSEAQEE NGF | 562 | 11 | lncHOX B81519 402135 | * | lnc-HOXB8-1:5 | 0.5637 | 3 | ['A*01:01', 'A^{∗}24:02', 'C^{∗}05:01'] |
| ANHKATT NSRR | 563 | 11 | lncSLC3 9A1021 271373 | MKSVEVRPLDALAWKSHIL LAKANHKATTNSRR* | lncSLC39A 10-2:1 | 0.56365 | 2 | ['A*03:01', 'A^{∗}11:01'] |
| ANHKATT NSRR | 564 | 11 | lncSLC3 9A1021 250373 | MTARFKRMKSVEVRPLDAL AWKSHILLAKANHKATTNS RR* | lncSLC39A 10-2:1 | 0.56365 | 2 | ['A*03:01', 'A^{∗}11:01'] |
| DQDSRSAP EPK | 565 | 11 | lncTRPC 531160 31801 | MNVPKGQTGNSSRGPGDG GNKDHWKESDRKDGKKDQ DSRSAPEPKKPEENPASKFS SASNYAALS* | lncTRPC5-3:1 | 0.5632 | 3 | ['A*03:01', 'A^{∗}11:01', 'C^{∗}03:04'] |
| VFPNRHPT LLF | 566 | 11 | lncTRPC 531160 328 | MTWKEMFRQLGTVTMTM CIGCLQLTVPSFPLLHGLLG NPISTGAVFPNRHPTLLF* | lncTRPC5-3:1 | 0.5631 | 9 | ['A*01:01', 'A^{∗}24:02', 'B^{∗}51:01', 'C^{∗}07:01', 'C^{∗}07:02', 'C^{∗}05:01', 'C^{∗}04:01', 'C^{∗}06:02', 'C^{∗}03:04'] |
| VFPNRHPT LLF | 567 | 11 | lncTRPC 531283 28 | * | lncTRPC5-3:1 | 0.5631 | 9 | ['A*01:01', 'A^{∗}24:02', 'B^{∗}51:01', 'C^{∗}07:01', 'C^{∗}07:02', 'C^{∗}05:01', 'C^{∗}04:01', 'C^{∗}06:02', 'C^{∗}03:04'] |
| GQYGETLS LL | 568 | 10 | LINC01 715132 053212 8 | MRSEGQDQPGQYGETLSLL KIQKI* | LINC01 715:13 | 0.56235 | 8 | ['A^{∗}02:01', 'A^{∗}01:01', 'B^{∗}07:02', 'B^{∗}08:01', 'B^{∗}44:02', 'B^{∗}44:03', 'B^{∗}35:01', 'B^{∗}51:01'] |
| LRSQDAGH SKK | 569 | 11 | lncCTR9 122153 50 | MRPLETHWRAGQTKDNFT DFRRLALISHNLRSQDAGHS KKEKEV* | lncCTR9-1:2 | 0.56185 | 3 | ['C*07:02', 'C^{∗}06:02', 'C^{∗}03:04'] |
| ETYSQLPK LKM | 570 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.5615 | 7 | ['A*01:01', 'A^{∗}03:01', 'A^{∗}11:01', 'B^{∗}07:02', 'B^{∗}44:02', 'B^{∗}44:03', 'B^{∗}51:01'] |
| YPAEVGGG SL | 571 | 10 | lncPLA2 G1B238 302839 5 | MVRSCLYEKYRKYPAEVGG GSLEAWKQRLK* | lncPLA2G1 B-2:3 | 0.56125 | 2 | ['B*07:02', 'B^{∗}44:02'] |
| RQYSPGGF QG | 572 | 10 | CYP1B1 AS1125 7141 | MGAVCEALRQYSPGGFQGS RAASVFSR* | CYP1B1 -AS1:12 | 0.56125 | 2 | ['B*44:02', 'B*44:03'] |
| RFKRMKSV EVR | 573 | 11 | lncSLC3 9A1021 250373 | MTARFKRMKSVEVRPLDAL AWKSHILLAKANHKATTNS RR* | lncSLC39A 10-2:1 | 0.5612 | 1 | ['A*03:01'] |
| HTTQNGT QF | 574 | 9 | LINC02 418923 092465 | MAAEPLVGAECRVCSVNMP GKGIMHVRDRVEKDCARLH HTTQNGTQFRTYK* | LINC02 418:9 | 0.5607 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B*35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| HTTQNGT QF | 575 | 9 | LINC02 418923 602465 | MPGKGIMHVRDRVEKDCA RLHHTTQNGTQFRTYK* | LINC02 418:9 | 0.5607 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| HTTQNGT QF | 576 | 9 | LINC02 418923 782465 | MHVRDRVEKDCARLHHTT QNGTQFRTYK* | LINC02 418:9 | 0.5607 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| SWSTEGRD AHK | 577 | 11 | LINC02 418929 103054 | MTGALCLRSAKPRSEVLTR LHQEQGTRLPRGSWSTEGR DAHKHTMSK* | LINC02 418:9 | 0.56005 | 2 | ['A*03:01', 'A^{∗}11:01'] |
| SWSTEGRD AHK | 578 | 11 | LINC02 418928 443054 | MELRIISTTRAGVRQDAQSI HPMTGALCLRSAKPRSEVL TRLHQEQGTRLPRGSWSTE GRDAHKHTMSK* | LINC02 418:9 | 0.56005 | 2 | ['A*03:01', 'A^{∗}11:01'] |
| LVEEAPMF | 579 | 8 | lncTRPC 531981 64 | MGVLVEEAPMFPNQSAGL MKR* | lncTRPC5-3:1 | 0.55945 | 1 | ['B*07:02'] |
| WADETMT WKEM | 580 | 11 | lncTRPC 531283 28 | * | lncTRPC5-3:1 | 0.559 | 3 | ['A*01:01', 'C^{∗}05:01', 'C^{∗}04:01'] |
| FLAEDGGT GGG | 581 | 11 | lncTRPC 531283 28 | * | lncTRPC5-3:1 | 0.55895 | 1 | ['A^{∗}02:01'] |
| HWRAGQT KDNF | 582 | 11 | lncCTR9 122153 50 | MRPLETHWRAGQTKDNFT DFRRLALISHNLRSQDAGHS KKEKEV* | lncCTR9-1:2 | 0.55865 | 2 | ['A*24:02', 'B^{∗}51:01'] |
| NGFSFVLV | 583 | 8 | lncHOX B81519 402135 | * | lnc-HOXB8-1:5 | 0.5584 | 3 | ['A*01:01', 'A^{∗}03:01', 'A^{∗}11:01'] |
| TLRLSETYS QL | 584 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.5583 | 1 | ['A^{∗}02:01'] |
| NLETLRLS ETY | 585 | 11 | lncLRRT M43347 4639 | MDSAPEEENLETLRLSETYS QLPKLKMFVAGSGTSGFRA GTFIAPVMKKRMKCG* | lnc-LRRTM4 -3:3 | 0.55735 | 3 | ['A*01:01', 'A^{∗}03:01', 'A^{∗}11:01'] |
| MQKMEAT GRGW | 586 | 11 | lncHOX B81528 042888 | MQKMEATGRGWESLGFRK EAPGSPVLL* | lnc-HOXB8-1:5 | 0.55635 | 1 | ['B*44:03'] |
| IQTGIGMGI | 587 | 9 | lncTRPC 531701 830 | * | lncTRPC5-3:1 | 0.55625 | 3 | ['A*01:01', 'B^{∗}08:01', 'B^{∗}35:01'] |
| IQTGIGMGI | 588 | 9 | lncTRPC 531668 830 | * | lncTRPC5-3:1 | 0.55625 | 3 | ['A*01:01', 'B^{∗}08:01', 'B^{∗}35:01'] |
| SPWGGRG GRIT | 589 | 11 | lncPLA2 G1B236 136628 0 | MIKVKESKFTTLLVFSFYPG CELNKKSAAVAHACNPSPW GGRGGRIT* | lncPLA2G1 B-2:3 | 0.55565 | 3 | ['B*07:02', 'B^{∗}08:01', 'B^{∗}35:01'] |
| SQAKTAVG QSF | 590 | 11 | lncCAM K1D211 465152 2 | MEKRSQAKTAVGQSFGET* | lnc-CAMK1 D-2:1 | 0.55525 | 3 | ['A*24:02', 'B^{∗}44:02', 'B^{∗}44:03'] |
| TESQFPPL GMK | 591 | 11 | lncPLA2 G1B239 255934 2 | MGTESQFPPLGMKDKVLEV SSDDGYTTM* | lncPLA2G1 B-2:3 | 0.55515 | 8 | ['A*02:01', 'A^{∗}01:01', 'A^{∗}03:01', 'A^{∗}24:02', 'A^{∗}11:01', 'C^{∗}07:02', 'C^{∗}06:02', 'C^{∗}03:04'] |
| SVNMPGKG IMH | 592 | 11 | LINC02 418923 092465 | MAAEPLVGAECRVCSVNMP GKGIMHVRDRVEKDCARLH HTTQNGTQFRTYK* | LINC02 418:9 | 0.55455 | 1 | ['A*03:01'] |
| KKPPMQM GLKY | 593 | 11 | lncPLA2 G1B231 094711 151 | MFDTGCSNNKKPPMQMGL KYGWWSGNTSYTRTHGQA LTLNVMVVIIRGGAGREQN LSSFAQWQYRSQ* | lncPLA2G1 B-2:3 | 0.55425 | 1 | ['A^{∗}01:01'] |
| HHPKAMA SSVF | 594 | 11 | lncHOX B81511 121196 | MYAKHMGVQVHHPKAMA SSVFPKSLRL* | lnc-HOXB8-1:5 | 0.55425 | 2 | ['A*24:02', 'C*07:02'] |
| TDTPKENA MWR | 595 | 11 | lncSLC3 9A1071 155617 69 | MKRRNLKTDTPKENAMW RHREKLAIYKPTRIAWNKF FHHTPQKESTLTAPPLQSCS LQNCETIHFCCLRR* | lncSLC39A 10-7:1 | 0.5542 | 4 | ['A*03:01', 'A^{∗}11:01', 'C^{∗}07:02', 'C^{∗}03:04'] |
| TDTPKENA MWR | 596 | 11 | lncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | lncSLC39A 10-7:1 | 0.5542 | 4 | ['A*03:01', 'A^{∗}11:01', 'C^{∗}07:02', 'C^{∗}03:04'] |
| QYSPGGFQ GS | 597 | 10 | CYP1B1 AS1125 7141 | MGAVCEALRQYSPGGFQGS RAASVFSR* | CYP1B1 -AS1:12 | 0.5533 | 2 | ['B*44:02', 'B^{∗}44:03'] |
| LEYECWPE IRY | 598 | 11 | lncHOX B81514 691532 | MQILEYECWPEIRYFLPSSC * | lnc-HOXB8-1:5 | 0.5532 | 2 | ['B*44:02', 'B^{∗}44:03'] |
| FHHTPQKE STL | 599 | 11 | lncSLC3 9A1071 155617 69 | MKRRNLKTDTPKENAMW RHREKLAIYKPTRIAWNKF FHHTPQKESTLTAPPLQSCS LQNCETIHFCCLRR* | lncSLC39A 10-7:1 | 0.55305 | 2 | ['C*07:01', 'C^{∗}07:02'] |
| FHHTPQKE STL | 600 | 11 | lncSLC3 9A1071 154117 69 | MVDVLMKRRNLKTDTPKE NAMWRHREKLAIYKPTRIA WNKFFHHTPQKESTLTAPP LQSCSLQNCETIHFCCLRR* | lncSLC39A 10-7:1 | 0.55305 | 2 | ['C*07:01', 'C^{∗}07:02'] |
| FHHTPQKE STL | 601 | 11 | lncSLC3 9A1071 160117 69 | MWRHREKLAIYKPTRIAW NKFFHHTPQKESTLTAPPL QSCSLQNCETIHFCCLRR* | lncSLC39A 10-7:1 | 0.55305 | 2 | ['C*07:01', 'C^{∗}07:02'] |
| PKAMASSV FPK | 602 | 11 | lncHOX B81511 121196 | MYAKHMGVQVHHPKAMA SSVFPKSLRL* | lnc-HOXB8-1:5 | 0.55285 | 4 | ['A*03:01', 'A^{∗}11:01', 'C^{∗}06:02', 'C^{∗}03:04'] |
| SEDPFLITI | 603 | 9 | lncHSF5 214976 38 | * | lncHSF5-2:1 | 0.5526 | 17 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}07:01', 'C^{∗}07:02', 'C^{∗}05:01', 'C^{∗}04:01', 'C^{∗}06:02', 'C^{∗}03:04'] |
| MFPNQSAG LMK | 604 | 11 | lncTRPC 531981 64 | MGVLVEEAPMFPNQSAGL MKR* | lncTRPC5-3:1 | 0.55215 | 6 | ['A*01:01', 'A^{∗}03:01', 'A^{∗}11:01', 'C^{∗}07:02', 'C^{∗}06:02', 'C^{∗}03:04'] |
| EVGGGSLE AWK | 605 | 11 | lncPLA2 G1B238 302839 5 | MVRSCLYEKYRKYPAEVGG GSLEAWKQRLK* | lncPLA2G1 B-2:3 | 0.5515 | 13 | ['A*02:01', 'A^{∗}01:01', 'A^{∗}03:01', 'A^{∗}24:02', 'A^{∗}11:01', 'B^{∗}44:02', 'B^{∗}44:03', 'B^{∗}51:01', 'C*07:01', 'C*07:02', 'C^{∗}04:01', 'C^{∗}06:02', 'C^{∗}03:04'] |
| EEEGNVE WV | 606 | 9 | lncOR52 J311281 27 | MSDRREEEGNVEWVASILI KKGTDLPSTVRIT* | lncOR52J3-1:1 | 0.5513 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| LETSIGIVM | 607 | 9 | lncTRPC 531668 830 | * | lncTRPC5-3:1 | 0.55125 | 13 | ['A*02:01', 'A^{∗}01:01', 'A^{∗}03:01', 'A^{∗}24:02', 'A^{∗}11:01', 'B^{∗}07:02', 'B^{∗}08:01', 'B^{∗}44:02', 'B^{∗}44:03', 'B^{∗}35:01', 'B^{∗}51:01', 'C^{∗}05:01', 'C^{∗}03:04'] |
| TLKGHSDK GHL | 608 | 11 | lncTH11 352838 04 | MCSQWPWQNLTKSAPATR PASKDALSRRPLQLSSDCPR DTLSEHCPAKTRQLQNCER RWFHDFLAVVTAVGQLWV TLKGHSDKGHLPACVT* | lnc-TH-1:1 | 0.55055 | 1 | ['A^{∗}02:01'] |
| TLKGHSDK GHL | 609 | 11 | lncTH11 352238 04 | * | lnc-TH-1:1 | 0.55055 | 1 | ['A^{∗}02:01'] |
| QEENGFSF VLV | 610 | 11 | lncHOX B81519 402135 | * | lnc-HOXB8-1:5 | 0.5505 | 2 | ['B*44:02', 'B^{∗}44:03'] |
| TVIGLGKA EEK | 611 | 11 | lncSLC3 9A1021 699888 | MYQHVHHYTVIGLGKAEEK QEPASSSYKQKSCLGVVAH ACNPSTLGGQSGRITRSGDR DHPG* | lncSLC39A 10-2:1 | 0.5504 | 4 | ['A*01:01', 'A^{∗}03:01', 'A^{∗}11:01', 'C^{∗}03:04'] |

### EXAMPLES

### Example 1- Expression and translation

RNA-seq data was obtained from colorectal cancer tissue and healthy colorectal tissue. The data was analysed to identify 18 IncRNAs (camyoRNAs; and defined according to SEQ ID NOs 1-18) as being expressed in colorectal cancer tissue, and having substantially no expression in healthy colorectal tissue or any other healthy tissue. Table 3 shows that the mean number of transcripts per million (TPM) for the 18 camyoRNAs (defined according to SEQ ID NOs 1-18) in healthy tissue is about zero, and is therefore significantly lower than the TPM observed in colorectal cancer tissue. The 18 camyoRNAs are therefore deemed to be specifically expressed in colorectal cancer tissue relative to normal tissue.

Mass spectrometry was used to derive proteomics data from the colorectal cancer tissue. The data was analysed and 79 polypeptides (camyopeptides; and defined according to SEQ ID NOs 19-97) corresponding to open reading frames (ORFs) in the 18 camyoRNAs defined according to SEQ ID NOs 1-18 were determined as being translated. Table 4 shows that the 79 camyopeptides are detected as translated in at least 1% of colorectal cancer tissue samples tested, with camyopeptides among SEQ ID NOs 19-97 being detected in up to about 81% of the tested cohort of colorectal cancer tissue samples.

### Example 2 - Presentation and immunogenicity

Next, the presentation likelihood and immunogenicity for epitopes produced by the camyopeptides defined according to SEQ ID NOs 19-97 is assessed employing methods incorporating the algorithms neoMS and neoIM, respectively, as disclosed in WO2022/013154 and WO2022/079255 (each incorporated by reference in its entirety).

neoMS is an MHC-I presentation prediction algorithm leveraging mass spectrometry-derived MHC ligandomics data to better isolate presented antigens from potentially very large sets. The neoMS model is a transformer-based, peptide-sequence-to-HLA-sequence neural network algorithm, trained on 386,647 epitopes detected in the ligandomes of 92 HLA-monoallelic datasets and 66 patient-derived HLA-multiallelic datasets. It leverages attention mechanisms in which the most relevant parts of both putative epitope and HLA alleles are isolated. Further details are provided in WO2022/013154.

neoIM is a random forest classifier specifically trained to classify short peptides of length 9-11 amino acids as immunogenic or non-immunogenic. The algorithm was trained using a positive dataset of non-self immunogenic peptide sequences, and a negative dataset consisting of MHC-I-presented peptides originating from housekeeping genes. Peptide features were constructed by performing principal component analysis on amino acid physicochemical properties and stringing together the values of the three main principal components for each amino acid in the peptide. Further details are provided in WO2022/079255.

This presentation and immunogenicity predictions are performed taking into consideration a set of alleles that are common in the populations of Europe and the USA (A*02:10, A*01:01, A*03:01, A^{∗}24:02, A^{∗}11:01, B^{∗}07:02, B*08:01, B^{∗}44:02, B*35:01, B*51:01, B^{∗}44:03, C^{∗}07:01, C^{∗}07:02, C^{∗}05:01, C^{∗}04:01, C^{∗}06:02, C*03:04). This set of HLA alleles covers 99% of the European and US populations, meaning that 99% of people in Europe and the USA are expected to express at least one of these HLA alleles. This epitope screening resulted in a total of 514 presented and immunogenic camyotopes, hence representing a set of valuable immunotherapy targets.

To conclude, a set of 79 camyopeptides was obtained showing evidence of tumour-specific expression and translation, and absence in normal tissue. From these camyopeptides, 514 camyotopes (see Table 5; and defined according to SEQ ID NOs 98-611) were computed that are predicted to be presented and immunogenic in a significant fraction of the European and US populations.

### Example 3 - Lead selection and optimisation

Parameters of the camyoRNAs, camyopeptides and camyotopes can be assessed to define optimal combinations of camyotopes to reach a wide population coverage with a set of actionable targets. This camyotope selection is highly dependent on the number of epitopes that can be integrated into the final vaccine formulation.

Figure 1 illustrates a camyotope selection of 20 camyotopes that can be used for a vaccine formulation of small peptides targeting a wide portion of the European and US populations. To increase the chances of inducing an immune response against at least one of the camyotopes, it is of interest to select a combination of camyotopes originating from different camyoRNAs that are expressed in a high percentage of the cancer samples. Hence, 10 CRC-specific camyoRNAs with the highest population coverage (on expression level) are selected ensuring that 99% of the population expresses at least one of the camyoRNAs defined according to SEQ ID NOs 1-18 (Figure 1). Moreover, 50% of the population expresses five of the 10 camyoRNAs defined according to SEQ ID NOs 1-18.

For each of the camyoRNAs, 2 non-overlapping actionable camyotopes with the highest immunogenicity score (neoIM) are selected. This results in a list of 20 camyotopes (more details can be found in Table 6). Note however that this is an illustrative selection of camyotopes. Any other strategy can be used to select a set of camyotopes that are used for the final product.

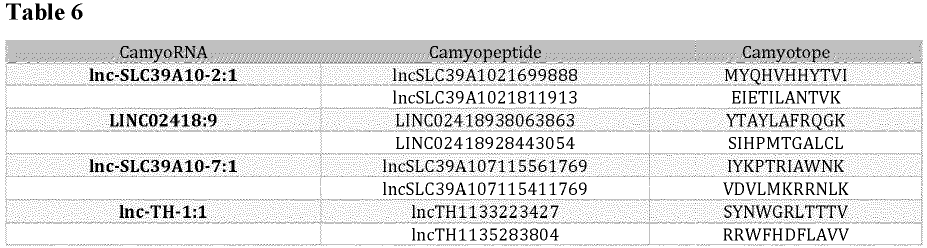

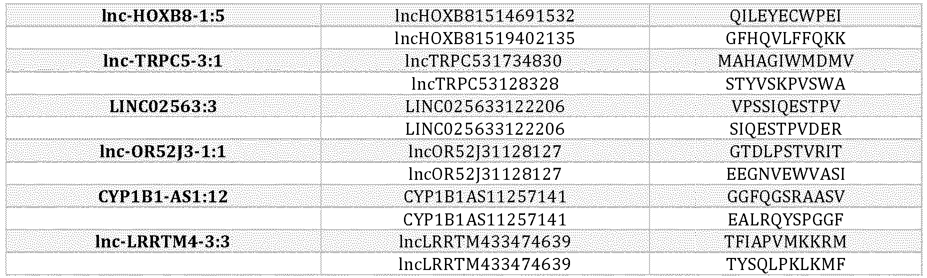

### Example 4 - product

Depending on the characteristics of the final product, camyotopes, larger windows around the camyotopes, or camyopeptides, are selected for the final product. The selection strategy can be fine-tuned based on the requirements for the final product as illustrated below:
- a set of several camyotopes (8-11mers), ideally 2 to 20 of them, provided in a vaccine formulation as individual small peptides
- a set of several camyotopes (8-1 1mers), ideally 2 to 20 of them, stringed one after the other in one long peptide
- a set of several camyotopes (8-1 1mers), ideally 2 to 20 of them, whose coding nucleotidic sequences are stringed together one after the other in one mRNA molecule
- a set of several camyotopes (8-1 1mers), ideally 2 to 20 of them, whose coding nucleotidic sequences are provided in a vaccine formulation as individual mRNA molecules
- a set of several camyotopes (8-1 1mers), ideally 2 to 20 of them, whose coding nucleotidic sequences are stringed together one after the other in one DNA molecule
- a set of several camyotopes (8-1 1mers), ideally 2 to 20 of them, whose coding nucleotidic sequences are provided in a vaccine formulation as individual DNA molecules
- a set of several camyotopes (8-1 1mers), ideally 2 to 20 of them, whose coding nucleotidic sequences are integrated within the genome (RNA or DNA) of a viral vector
- a set of several camyotopes (8-11iners), ideally 2 to 20 of them, whose coding nucleotidic sequences are integrated individually within the genome (RNA or DNA) of several viral vectors
- a set of several camyopeptides (8-100 amino-acids), ideally 2 to 20 of them, provided in a vaccine formulation as individual peptides
- a set of several camyopeptides (8-100 amino-acids), ideally 2 to 20 of them, whose coding nucleotidic sequences are stringed together one after the other in one mRNA molecule
- a set of several camyopeptides (8-100 amino-acids), ideally 2 to 20 of them, whose coding nucleotidic sequences are provided in a vaccine formulation as individual mRNA molecules
- a set of several camyopeptides (8-100 amino-acids), ideally 2 to 20 of them, whose coding nucleotidic sequences are stringed together one after the other in one DNA molecule
- a set of several camyopeptides (8-100 amino-acids), ideally 2 to 20 of them, whose coding nucleotidic sequences are provided in a vaccine formulation as individual DNA molecules
- a set of several camyopeptides (8-100 amino-acids), ideally 2 to 20 of them, whose coding nucleotidic sequences are integrated within the genome (RNA or DNA) of a viral vector
- a set of several camyopeptides (8-100 amino-acids), ideally 2 to 20 of them, whose coding nucleotidic sequences are integrated individually within the genome (RNA or DNA) of several viral vectors
- Syngeneic (from the patient) DC cells matured ex vivo by incubation with a set of several camyotopes (8-1 1mers), ideally 2 to 20 of them, and reintroduced to the patient
- Syngeneic (from the patient) DC cells matured ex vivo by transfection with a mRNA carrying the coding sequences of a set of several camyotopes (8-11mers) stringed together one after the other, ideally 2 to 20 of them, and reintroduced to the patient
- Syngeneic (from the patient) DC cells matured ex vivo by transfection with several mRNAs each carrying one coding sequence of a set of several camyotopes (8-11mers), ideally 2 to 20 of them, and reintroduced to the patient
- Syngeneic (from the patient) DC cells matured ex vivo by transfection with a mRNA carrying the coding sequences of a set of several camyopeptides (8-100 amino-acids) stringed together one after the other, ideally 2 to 20 of them, and reintroduced to the patient
- Syngeneic (from the patient) DC cells matured ex vivo by transfection with several mRNAs each carrying one coding sequence of a set of several camyopeptides (8-100 amino-acids), ideally 2 to 20 of them, and reintroduced to the patient
- Syngeneic (from the patient) DC cells matured ex vivo by infection with a viral vector carrying the coding sequences of a set of several camyotopes (8-11mers) stringed together one after the other, ideally 2 to 20 of them, and reintroduced to the patient
- Syngeneic (from the patient) DC cells matured ex vivo by infection with several viral vectors each carrying one coding sequence of a set of several camyotopes (8-11mers), ideally 2 to 20 of them, and reintroduced to the patient
- Syngeneic (from the patient) DC cells matured ex vivo by infection with a viral vector carrying the coding sequences of a set of several camyopeptides (8-100 amino-acids) stringed together one after the other, ideally 2 to 20 of them, and reintroduced to the patient
- Syngeneic (from the patient) DC cells matured ex vivo by infection with several viral vectors each carrying one coding sequence of a set of several camyopeptides (8-100 amino-acids), ideally 2 to 20 of them, and reintroduced to the patient
- T cells carrying receptors raised against one or more (ideally up to 20) camyotopes (8-11mers)
- T cells carrying receptors raised against one or more (ideally up to 20) camyopeptides (8-100 amino-acids)
- CAR-T cells carrying receptors raised against one or more (ideally up to 20) camyotopes (8-11mers)
- CAR-T cells carrying receptors raised against one or more (ideally up to 20) camyopeptides (8-100 amino-acids)
- antibodies raised against one or more (ideally up to 20) camyotopes (8-11mers)
- antibodies raised against one or more (ideally up to 20) camyotopes (8-11mers)
- any of the previously described vaccine formulations in combination with other tumour antigens as a combination vaccine
- any of the previously described vaccine formulations for any kind of administration route (IV, 1M, IT)
- any of the previously described vaccine formulations in any kind of combination with other therapy modalities.

### CamyoRNA sequences

>LINC02418:9 (SEQ ID NO: 8) 
AGTCCTGCATGCAGACCGCAGCCACTGACTGTTCCCGAGTCACCAGGCCACTCTTCTCTCCTGCCTCATCGTCTTATTCAGGGTCC
ACGTCATTCTGCCACCTATTTGAGGACACCAGGCCTTGGCTGTTTCTGCTTCTTGTACCTCCTGAGTCAGGTGGCGGTGGGGGAGG
GCAGACGCCACACACACCTGGGTCCACTGGCCGCACGGCTCCTGGAAATGCTAGTGTGACTTTCCTAAGGGTTCTTTCAAAGGACA
CTTTTAGACCTGCCTTTTGTCCCAAGGATTCTAGTTGCTTCATCTTTAGTACCACAGACTGGTGGCTAAACAACGAACATTTACTC
TGCCAGGACAACCTCGCTGCCCTCGTGGTTCCCAGCACCCGCCCGGACCTCCACACCAGGCTACTGTTTCCCCAAACGAGTGAGTG
TCCTACTCCCTTCCGGCAGCCCTGACCTTCTCCTGCCTCGGATTCTTTCATTCTCTTCTCGGCTCCTAAATTCAAGCTCCCCTTAT
AAAGCTCAGCCCAGATTCCCATGATAACGTGCTCTGCGATTATTTGCCGATTCCATCAACATTTTTTGAGTACCTGTTGTGTGCCT
GACACCTACGGCACTCAGAGCTGGAAGTGGATCCAGCTGTGAGCAGAACACAGACCCTCGGGGATCCCCCAGCCTGCTGGTAGAGA
CGACCCCCATCCAGGGAGATGCGGGAGCACCGAGGAGAAGCCCCAACCCTGGGGTTCATGGATGGCTTCCTGGAAGAGGAAGCAAG
GTGAAGTCAGAGGCTAAAAATGCACACATTTGCAGGTGTTGAGCAAGTAGTGTGGACGCGTGAGCGATGAGGCACAATAGAGCATG
AAGGCGCGGGCGGTGGCAAAGGGAACTTCGCCATCTGGGGAGGCATCCTCTGCCCAGCTTTGATCACTCCCTGGGATGTGAATAGG
AGTTTATCTTGCCAGATATATGATCTTCCAAGGTCACAGGATTTTGCGAATTTGTATCAGATTTCTCAGTTTTTAAATGTTGCCAA
CTTGTAACTTTTTAAAATAGTCAGTGGACCATACAAGACACAACTGTAGGCCAGGTGTGTTCTGCAGGCCCTTGTTGCAACATCTT
GTCTAAGTGGAATTCAGCCAGGCGCTGTCATACGCAGAGCACCTGCCGAGCCATGTGACGTGCTGCTCTCTGCCAGCCCTGATCGC
CTTCCTTTCCAGCAGGACTTGCGCTTCACACTGGACCTTGAGGCCCCATGGAGATCTGCCATTTTGGGCAGGTTCTGCTCACAGTG
CCAGTAACTTCACCTAGGAGAAGAGCGACAACTTCAAGGCCGCATGACCCATCTGATTGTCATCACACAGGTCCCCGCAGGAGAAT
TTCCATGGCGTTTCTCACTCTTTGTTTGCAAAGAATTTCTTTTTTTATCGTTTTCTTTTTCTTTTTCTATTTTCTTTTTTCTTTTT
TTTTTGAGACAGAGTCTCTCTCTGTTGCCCAGGCTGGAGTGCAGTGGCATGACCTCGGCTCACTGCAACCTCGGCCTCCTGGGTTC
AAGCAATTCTCCTGCCTCAGCCTCCCGAGTAGCTGGGATTACGGGCACACACCACAACGCTCAGCTAATTTTTGTATTTTTAGAAA
AGACAGGGTTTTACCATGTTGGCCAGGCTGGTCTCGATCTTCTGAGCTCGTGATCCACCTGCCTCGGCCTCCAAAGTGCTGGGATT
ACAGGCGTGAGCCACCACACCCAGCCTAAGAATTTCTTAGAATTAATATCTGCACTTGGCCCCACATCCTCTGAGTGATAACTTAG
AACTGTCACTTTAATTGTTGAGAGAGGATATTCAGAGAATAGTGGGTGATTTTACAAGCAAGGATATGTTTGGGGAGCATGTTCCC
GGTGCATTCTCAGGGTCTCGTGCATTCTTTTAGACATTGAAGACATCCTGATCTCCAGTGGAAGCAGAGCCCCAGTCATACACTTA
TCCACCGGGGACGAGTTCCAAGACCCCCAGGGAATGCCTGAAAGCTCGGGTAGTGCTTACCTCTATGGATGTTGTGCACAAATTTA
TTTTTCCCTCTTTACAACTTCATAGATTGAAGGTTCGTTCTTACCAAAGATCTTAGCAACCTTAGCTTATCATTCCTTTTATTTCC
TGATTAAGTGGAGAACTTTCACCTTTTCACTAAAAAAAAAAAGTGCTTTACGGCTGCCTTTGCCACGTGCACATGGTCTTTTGGGA
CCATCCTTAAGTCAAATAAGGGTGACCTGGATGCAAGCACTGTGATACAGAGAAAGTCCAGCTCACCACCAAGATGGCTGCTGAGC
CGCTGGTGGGCGCGGAGTGTCGTGTCTGCAGCGTGAACATGCCGGGCAAAGGAATAATGCATGTCAGGGATAGGGTGGAGAAGGAC
TGTGCCAGGCTTCATCACACTACTCAGAATGGCACACAGTTTAGAACTTATAAATGATTTATTTCTGGAGTGATCCACTGCATATT
TTCAGACGGTGGTTGATCGTGGGTAACTGAAAGCAAAACCTGACTGAAAGGGGCTGCTGTGTTCATGTCAGCAACGCTGATGCCCT
CGAGGCCAGAAGAGTAACCACCAATCTGGTACCTGAGCACCTGGCTGTAAGTCCACCCTCCATTTCTGGCCAGGGCCGAGGAATTC
TCTCTTTTTCTCTTTATTTTCCTCTTCATTTGAGAAGGTCAACTCTGAGCATGCTCTAAACACAAATCACCATCTCTACACTTCTT
CGGCACCAGGTGTTGCTACTGTCTTTCTGATTAGAAAAAAAGAAATATCCAGGAATTCTGGAAGACAGTGATTGATTTGCAGCTAT
GCTGCAATGGAGCTAAGAATCATTAGCACAACCCGTGCTGGTGTTCGACAAGATGCCCAATCAATTCATCCGATGACAGGTGCGCT
GTGTCTGCGAAGCGCGAAGCCACGGAGTGAGGTGCTGACCAGGCTCCACCAGGAGCAAGGAACAAGACTCCCCAGGGGCTCCTGGT
CCACAGAAGGCAGGGACGCCCACAAACATACCATGAGTAAATAGTTCCAATGTGAAGAAGACTGAAAATGCCACAGAAGTGTGAGC
CCACTGTTTTGAAATTCTTTCTGCTGAAGGGATTGAGAAGAACTCTTCCATAAAGGAGCATGACCGAGCCATGAGTTATAATATAT
ACATGCATATAGAAAGAGGATGTGAATACAGTTGCAGAAGGGCGTCAAAGCCAAAAACACACAGGGAATGACTTGAGGAAGCTGAG
GACCAAGAGCAGTGCTAGCCATGCCAGTGACTGTGGTCGCTGAGAACTGGATGATGGATGTTTGAGTGACCGTCAGGAGGATCAGC
ATGGGACCAGGAGGATCAGCATGGGACCAGGAGGATCAGCATGGGACCAGGAGGATCAGCATGGGACCAGGAAACAGCAGGCGAGT
GAATGTGAACTCCACTCTCAACAGGGAGATGACGTGGGTCTCGGAAACCTACAGATCTGAGTGCTTGCTGTCAGGTCATGGCAGGG
CTCTGACTGCACTGTGGAGGGAACATCTGTGTTTTTGTCCTCCTGGAACCTAGTGCCCCTTTCCTGGAGGCAGCATCCTATTTGAC
CTGTGTGGGTCCAACCCCACCTCTCTCCTGTAGCAGTGGCATGGGACCCGGGGCTGGGCCATTCGCAGCATTGCCACAGGCAGCAA
TGGTGATTGATCTAGGAGTTGATCATTGATCCTAACTGGGTGCTCAGGCCAAGGGGATTGATTCTTGGTATTTTTTAATCTCTTTT
TTCCACTGGAATTATAGCTAATATGATGAAGCCTAGATATACTGCGTATCTGGCTTTTAGACAGGGCAAACCTGCCTAGAAATGAA
AATGAAGTCACCACCTGTGAAAGCAGAAACAAGAGCTTGAGAGAGACTGAGTCCTGGTGAGAATTTCTGAACCCCTTGATCCAGCG
ATGCCTGAAGCCAGAACTACTTGTAAACTTTTCAGTCATGCAATCCTGTCACTTCTGTTTGTTCTTAAGTGCATTTTGTATGGTTT
CTGCCGCTGACCACGGAAACGTCCTTCACTAACAGGGAAGCAGAGTTACCCCATTTGAGAGCCTCAGAAGGAAGATGGCATTCATG
CAAAGGAAGCATGCGTTTGCTCTCACTTCCTGAGAGAAAGCCTGAAAATGAAGCCAATCTCATTTTCTTTCAGAGATGAGTCTCTT
GATCAGAGAATGCCTCAGTTCCCTGTATCTGAATTTTAGCAAGCGAGAGAATGCATTTAGGACAAGCAACAGCGATGGCTCCCGTG
TGTTCTTGGCTGCTCTGTTTCTAAATGAAGATTCACCTGGAGGCAGCCTTTGCTGCAGGGACACCCAGATCTCACCTTGTTCTTGT
TCTTTTCAATCATTTTAGCAAAGGCTCAGCCTACTGATCCGATTTAGAGATGAGCCATGAAAGCAGATATTTAGATAATATTTTCC
CAACTGCCTGGAGCATTAGGTTAGAACTAGTGAGATAAAAATATAATGAAAATAATAGAAAGCTTTGACCCTCAGATTT
>lnc-TACR3-1:1 (SEQ ID NO: 13) 
CAGCCCGCCTGCACCCAGGTGAAATAGACAGCCATGTTGCTCACACAAAGCCTGTTTGCTGGTCTCTTCACACTGACTCGAGTGAA
ATTTGGTGCCGTGACTAGGATCGGGGGACCTCCCTTGGGAGATCAATCCCCCGTCCTCCTACACTTTGCTCTGTGAGAAAGATCCA
CCTACAACCTCAGGTCCTCAGACCAACCAGCCCAAGAAACATCTCACCAATTTCAAATCCGTGATAGATCACAACAAGAGATCATG
AAGAGGGCATGGCCGCCATGTCATTCTGGAGAGAGACAGAGTAAAACAAAGAAGACAGAGTCTTGTTCTGCAACCCAGGCTGGAGT
GCAGGGCAGTGGTGTGATCTCGGCTCACTTCAACCTCCGCCTCTTAGCTTCAAGTGATTCTCCTGCCTCAGCCTCCCGAGTAGCTG
GGACTACAGCAGCCTCTCTACAAGTCTCCTAACTTAACCTGATGGGAAAACACTTCCTACCACTAACAGCATCTACTTGATTACTG
ATACTTTGATCATGGAGTTTGGGCATGCCACTTGATAGAAATTTGAAGAGCAATTATATTTTTCAAAAAGAGTTTTGAATAATGTT
AAGATAGATTGCAACATGACTATCAATTCTTCCCTTCCCATCAAAGGAGAGAGTCCGTTTATCCAGCCTTTGAATCTTGATTATTC
ATGTGACTTGCTTCACCCAATGTAACATTAATAAGCACAATACAAGCAGAGGCTTGCCAAGAACTTGGTTTGTTTCTAATGCTTAG
AAGAAGAATGGTGTATGCCATATTTCTGCATTTAGAACTCACGTGGAGACATGTGT
>lnc-SLC39A10-2:1 (SEQ ID NO: 3) 
CCACCATGTGAAGAAGGTCCTTGCTTCCTCTTCATCATCTATCATGATTGCAAGTTTAGGCCTCCTCAGCCACGTGCAACTTTTAT
TTCACCCTGAAGCCAAAGAGAACTTTCTGAAAGACAAATTTGCTCATGCTATAATAGTTACTGACAAATATTTAAAGGACTAAGTT
GTGTAAGGAATCTACACTGTCAGGCAGAACCAGGATTCCTGGAAGTAGAACTGGGCTGCTTCATAAGTCTGACTTCATATGACAGC
TAGATTCAAAAGGATGAAATCAGTAGAGGTGAGACCTCTTGATGCCTTGGCTTGGAAGTCACATATTCTATTGGCCAAAGCAAATC
ACAAGGCCACCACAAATTCAAGGAGATGAAGAAATAGACTCTACCTCTCTTGATTGGCTTATAATATGGTCAGTTCTTCAGAGGAA
GAGGAAAATTTCATCTGGCCTCAAATCTCAGTGATCGCATTTGTGGGAACATAATGTCTGAAGTAAAGACTAAGTAGAAGTCTGAC
AAGCAAAAAAAGAAAAAAAGACAAAGACACTCCAAGCAAGAGAAACAACCTGAGCAGAAGCACCCAGGTATGACAGAAAACGTAGA
GAGGAAAGGCTTCCAAAAGCAACAGAGATTCACAGGAAGCTCCACAGAAAAACGGGTGGTGCAAAAAATTAGGCAAGAGGGAAAAT
GGGGTTAGGCCATGTACCAACATGTTCATCATTATACAGTGATTGGACTTGGAAAGGCAGAAGAGAAGCAAGAGCCAGCATCCTCA
TCTTATAAACAGAAAAGCTGTCTGGGCGTGGTGGCTCATGCCTGTAATCCTAGCACTTTGGGAGGCCAAAGCGGGCGGATCACGAG
ATCAGGAGATCGAGACCATCCTGGCTAACACAGTGAAACCCCGTCTCTACTAAAAATACAAAAAATATTAGCCGGGCATAGTGGCG
GGCGCCCGTAGTCCCAGCTACTCGGGAGGCTGAGGCAGGAGAA
>lnc-CHM-1:1 (SEQ ID NO: 18) 
GCCATCTTTAGCCGAACTAAGGAAAAGTCCTGCAACATTTTTAGCGCCCAACGTGGAGGTCGAGAAACGGCCGTCAAACTCCAAAT
GGTGATGCAACTGGAACCTCTGATGATGGCCCCTTCTGCCGGGAACCCTTAAATAGGCCTCTGATGGAGCCCTGACTGCCATTTCC
CCCAAACAGCACCACCTATCAGCAGGAATCAGTTAAGATCAGTCCTCGT
>LINC02563:3 (SEQ ID NO: 9 
AACCAGGATAAGTTGATCACCCTCCCTGAGAGTTTGGGATTCTATGAGGACTCCTGTCTAGAGTGCAGCGCCACTGCGAGGGTGGT
CCTTTATTGTCAGAAACCTCATTCCCAGAAGGAGACATGCAGTGGAAACTGAGCGTCCTCTGCCCTGCGGTGCCCAGCTCCATTCA
AGAAAGCACACCTGTGGATGAGAGGCGCAATTAACGTTATCAGTCGTGAAGGATCAGAAGGAAAACCCAGTGGCCGTTGGGAATTT
AGAGATGGTGTTTCGCCAT
>lnc-CTR9-1:2 (SEQ ID NO: 16) 
GAGTGTTTTCCAGGACATGCTAGTTCCAAAAGATACTCCTTAAAAAAGAGTTCGATAGTCAAAACGTTGCTTCCAGAAGAAGACTG
TCTCATTACTGTTACATCTCTGGCCCTGGAGTAGCCCCTGGCAAATATGTACTCAGTACATGTTTGCGAGACGCATGGACTTGTAG
AACAGTTCCACAGCTGTCCATCACTGATGTTCCATACCTAGCGATGCGGCCCCTCGAAACGCACTGGAGAGCTGGGCAGACCAAGG
ATAA[?]TTCACAGACTTTAGGAGACTTGCCCTCATATCTCATAATCTTCGCTCACAGGACGCTGGACATTCAAAGAAAGAAAAAGAA
GTATAGAAAAGAGAGAAAAACGTCAAGAACTCCAGCATAAAGTCCTCTTAATAACGCGTCTTTTTATAATCATCGGGCAATAAATT
TCTGAATGGAC
>lnc-HOXB8-1:5 (SEQ ID NO: 2) 
CCGGTGGGGTGCAGCCCTGCTGAAGCCCCAGAGCTCAGCCCAGTGCTGGGCACATAGTAGGCGCTCAGGAAATGATTGCTGAATGT
GGAATGAAAGTCCCCAGCAGGCAAAAGGCTGTGACTTCTGTTAGTGGGGTGGTGGTCCTCTGGGGCGAGTGTATGGAGAACTTCAA
GGCCTCCTCCTGGCTGCCCAGGAAGTAGAGGGCGCCAAAGGCGACGTGCCGGCCGCCAGCTCCAGGCCGAGCCCCGAGCGCCTGCA
GGAACACGCCCCTTCACCCGGCGCGGGACGCAGAGCTGCGAGAGAATCTTGTTCAGCGCGGACTCAACGCCAGGGCGCCGCCTAGA
GGTTGGTCTCTGTCTCGGCCTCACCCGCCGGGAGACCACAGAGCTGCTTCCCCAGCCGCCCGCCGCCAGAAATTGGAAAAAAAAA
AAAAAAAAAAAAAAAAATCCAGCTGGGGTCTAGGAACTCGGCTTCTGGCACCTCTGAATTCTCCGAGACTGTCTCCTCCCTCCCCGC
CTGTAATGAACCCTGTGAAGGGAGACAGGCCAGGAAGTCCCAGAAATATTTATTCTTGTGACTCTCACAAAATGGAAAAGGGTCTC
AATTTTTGTTTCTTTAAGGAACTTGTGTTCTGCGTCTGTGTCTACACTGCCTCCTCTCACCAACCAAATTGTCTAGCCCCCCTCCA
GTTACGCTAGAACTCTGCTTTATCTTCAAGGAAGAAAGGGAGTGGGGAGAAGTTACCTCTAAACCCTCCAGCATGGCCATCAATTT
TCTGAATAATTTGGAGGTCAACATGCTTTTGGAAAAGTGTTTGGAAAACGTTTGGGGGTGAAAAGAAATGTTGCCTAACTGGCCTT
TACTCACGGAGTGACCATCATCTCAGCTAGTTAGTGTATGAGCGGCACCAGCACCCCGGCCATGGGTGTCTGTGCTGAGCCTCTGA
AAGTTGACTTATCCTTTGGGGAGCCCTCGGAAAGACACAGCTGGTATCTATGGGAATGGCTTTGGGGTGTCCTTTGGCACCATACA
GATAATTTTGTTTTTTTAATTGGAATCCATGGCCTGGGTTCTTGGGATGGGGGTAGGGGGAAGCCACAAAGCCCCTGGAAATGTAT
GCAAAACATATGGGTGTGCAGGTGCATCATCCTAAGGCTATGGCCTCTTCAGTTTTTCCAAAGAGTCTAAGACTATAAAAGGTTTC
CATCTCCTTTTCTCAGCCAGGGAGCCACAGAGACCCAGGCCAGCAGTAGGCTGGGAGCCAGCACACCGGCCCCCACCAGGTGCCCG
GTAGGGGATGGGTAATAACACTCTTGAATGGTCTGGAAACTGCTGGCCCCTCAGTCCTGCCGTCCTCCCAGAGGGAAATCCTAATA
GAAGAAGTGACAGCCAGTAGCTGCCATCTCAGTATTTTGAATGAAAATATAACATGATAAAGAGCAAATGAGGGAGGCTGGAGGGG
 
CCTCCCCATGCAAATACTGGAATATGAATGTTGGCCAGAAATAAGATACTTCCTGCCCTCCAGCTGTTAAAGAGCTCCCCAATTCC
TCCCTTTTGGTGCTAGGAAGGGGCCAGAGGGCACTGCCCCCAGACCACCACCCACCTCTGGAGAGCTGGGGCCGAGTGGGACTGCC
TGCAGCAGAGTTAACCCTTCAGAAAATTCAAAACCTATACTGCCTAGTATTTTCCCTGCCCGTTTATTTTTTAATCAGATTTTTAA
AATATCCAGCAGCACTTTGGTGCCTTTTCTTGGGGTTGGGGAGTGGGGTTATCTATGTGTGTATCTATGGGAAGGTGGGTGCCTTC
AGTGCACTAAACCCCCTCTTGGGCCTGCAGCTGGACCTCACTCCTGTACCTGAACACTCTGCCTGAGCCTGAGCTAATCTTAGCCA
TCCATTGTGTCTCAAAACTGTTTTGGAGGTGAATAACTGTGAGGCAGGATGGAGAACCTTTTGCTCTCCCATCCAGAAGGGCACCT
AACCAGGCCCCTGGAGCAGACAAAAGGAGCAGGAAGTCAATCACTTCGATCCCAGTTCTCTGAAGCCCAAGAAGAAAACGGATTTT
CCTTCGTTTTGGTTCGGAGGCCTAGTAGAGAATTTGGATTCCACCAAGTTCTCTTTTTTCAAAAAAAGTAAACGGTCCAGAGCAGA
CAAAAACTGTGGAAACTTGAGGCCTGGGTAGTAGTGGTTTTGTTTGATTTTGAGGCTTTAAAGAGATAAGGAGACGGTGGTGGAGC
TCCGCCACGCCGCGTGGCTCTCACTTCCACGCCGAGGAAGACCGACCGGCCCCTAGGTTTATCCTCTGGGAGCCCCCGGCCCAATC
AGCCCCCTTCAGGCCTTGGGAGTCACCCCGAGTCTTATCTTCTGTCCCCTTCCAGCAAGTACCCGGCAATAAACGGGCAGGGCTGG
GCAAAGGTCTGGCCGCTAGAAACGGAAAAGGAAAGAGAAAAGTAAAGTTAAACAAAAAACAAAAACAAAAAACCCTTGGGTCGGGC
GCGGTGGCTTACGCCTGTAATCCCAGCACTTTGGGAGGCCGAGGAGGGTGGATCACGAGGTCAGGAGTTCAAGATCAGCTTGGCCA
ATATTGTGAAACCCCGTCTCTACTAAAAATACAAAAATTAGCCGGGCGTGGTGGCGGGCGCCTGTAGTTCCAGCTACTCGGGAGGC
TGAGGCAGGAGAATCGCTTGAACCGGGAGGCGGAGGTTGCAGTGAGCCGAGATCGCGCCACTGCACTCCAGCCTGGGTGACACAGC
AGGACTCTGTCAAAAAAAAAGAAAAAAGAAAAAACCCGCTTTCAGCGGAAAGATGCAAAAAATGGAGGCCACCGGGCGTGGTTGGG
AGAGCCTGGGCTTTCGCAAGGAAGCGCCAGGGAGCCCAGTTCTGCTCTAGCGAGAAGCAGGGAGCCTCATCCCAAGCCCGGCGGGC
CGAGCCGATCATAGGGTCTGCGCGGGGCTCTGTGGATTTTCCGTGTAGGCGGCGGCTTCCAGTCTCTGCCGGGCGCGGGCTGGGTC
AGCGCCGACTCCTCCGAGAGAAGAGCGCGCCTGGCGGCCGGTTCGGCGGAGGCCAAGTCCCGGGGGCTGGGGCTGCTTCTCTCCAC
GCGGACAAAGTGTGCGGGCAAAGAAAGGGGCCGAGGCGGGTGCTTCTGCCGCTCGTAAATTTGTTTCCCCTTCCTTTTAATCCGCC
CCCGGACGTCTTTCTTTTCAGGACAGGAGGGGATGTAAACCGTTTGAAGTTCATCAACACGGTGAAGTTTTATTGTATTTCCGGTC
CCCAAGATTTATACCCGGGAGGGGGAGCGGGCGGCCAGGTGGACTCCCTCGCTACCTCGGACTTCCCTGCCGGACACGTGGCCACT
CGGACCCTCGCTTCTGGGCGAGGGCGGGAGGGAGCCTGGACTGGGCCGCGGCCGCTGGCTCCGCACCTTCCGACGGCCGCGCACTG
CCGGCACCGCCGCCGCCCAGGAACTGACCGCAAACGAGGCCGCCAGACCTACACCCGCTACCAGACCCTGGAGCTGGAGAAAGAAT
TTCACTACAATCGCTACCTGACGCGGCGGCGGCGCATCGAGATCGCGCACACGCTCTGCCTCACGGAAAGACAGATCAAGATTTGG
TTTCAGAACCGGCGCATGAAGTGGAAAAAGGAGAACAAGACCGCGGGCCCGGGGACCACCGGCCAAGACAGGGCTGAAGCAGAGGA
GGAAGAGGAAGAGTGAGGGATGGAGAAAGGGCAGAGGAAGAGACATGAGAAAGGGAGAGGAAGAGAAGCCCAGCTCTGGGAACTGA
ATCAGGAAACTCAAATCGAATAGGGAAGTAAAAAAACAAAACAAAAAACAAAAAAAACAAAAAAAAAACCCTATTTAAATGAAAGG
AGTTTAAAAACATTTTTTAAGGAGGGAGAAAGGAGAAATTTTGGTTTTTCAACACTGAA
>lnc-TH-1:1 (SEQ ID NO: 4) 
CCCCCACCCCTTGGCTCCTGGGCTGCCTGGAACCTGGTTGGCTCAGGAGCTTTTTATCTACAGCTCCTCTGTCTATGTCCCCTTGG
CAGGTCCTTTTTAACATGAGTCCTCCTCCAGTGAAGGAAAGGGTCATGCCAGGGCCTGCACCTAGGGCCTCCGTTCTCCAGTGGGT
GTCTTTGCATGAAACGGCCTGGGCCCTGGCACTTGCCCCACCAGCGCTGCCTGCAACCTTGTCCCTGTACCTCGGGCCTCCACCAC
ATCCCTGGACCTGCACGAAGGAGCCCTGAGCATGGAAGTCAGGGGAAGGGAGGTCTCGACATGAGCCCAGCCATGGCCAGAGTACT
GAGCCCAGGGCCAGCAGCCTCGGCTGGCAGTGCAGGGTGATGGGTCTGTGGGCCGGGCTGCCCAGGGACCTCGGCCCTGGGGTCTG
AGGGGCACCAATGCCTTTTGCAGGCCACCATTTCCCTTTCCACTTTCCAAATGTGATTGGACACAGGTGGATGCACCTGCCTGCCC
AGCAGACAGCCCGGTTCTGACCCGTGTGGAGCTGCGTGGACTGGGCTCACCTGTGCCTTCTTGCATCAACCAGGTGTGGACCCTGA
CAAGGTGCCAGCCACTGTCCTCCAGGAGCTACCTCAGGGACACGCAGGGAGGGACTGGACAGACAGATGGAGCGCAGTCCTTGCTG
CCCGGCCAAGCCCTTGTGGGCAGGGCAGACTCCTTCAAGATCATCAAATGAAAGCAGGCAAAAACCACGTCCTTTTGTGACCTAAG
CTTGGAAGCAATGTTGTCACTTCTGCCTCATCCTATTGGTTAGAAGTGAGCCAGGAGGTCCTGTCCACATCCAAGGTGGGAGTCCC
CGCAGGACGGGGCCTGGGAGGTGGGCCCATGGGCTCCAGAGGGGCTGGACCACAGCAAGAAGAACGAGGCCAACCTCCATGGGTAG
CGCAGACCGGCTCAGGCCCTGCCCTTCCCGCCGGGGTCCCCTGTGACAGGACGCAAGGGCACAGCTGCGGAGCAGGGTGGGCTGTT
AGAGCTGGGCAGAGGCCACAGTGGCCAGAGGGCTGGTGAGAGTGGCTCTCCTGCTGCCCTAGCCACTGGCTGAAGATCTGCCTGGG
GAGGAGTGGGTTTGGGCGGCCGGGCCTAGGACCCCTTCATAGCTACCACTAATACTCATTCTCTGCTCCTGAGAAACCTCCTCCAG
GGGCCTCTGGGGTCTTGGAGATGAGACAGAGGGACCTCAGGCAGGACAGACAAGAGACCCTGGTCTCGGGTGTCCACAGAGCGGGA
GCCAGGCAGGCTGGGCGGAAGTCCTAGGCTTGGACCCTGTTACGGGACTTTCCCTGTCAGAATGGGCTCAGGAGGGAGTGAGTGCG
TGGTCCTCAGGTGTGCAAGTGCCAAGGCTGGAGCCCACCCCCTGGGAAGCAGGGCCAGGGTGGCTGGGGCGGACTTGATCCCTGGA
GAGGAGCGTGGGCAGCCAGGGCAGGGAGGAGGGCAGGCAGGAGGGAGGTCCAGGCCTGTGGGCCGACAGGGGGTGAGGAGGCAGTC
TGGGGACCTGAGAGAAGGCCGAGTGGCTGTGGGGTGGGTGACCTGGGGCCCTGACTCTGCCTTACCCTTGGCCATGGGGCCACATT
CATGCTCGGCCCCCGCCCAGCCTGGGCTGGGAAGGCATCAGCCTGGCCATGAGGCAAGGGGCCGGCCATGCCTGGGGCCAGAGGGG
GTCCCCCAGCCCTTCGCCTTCTCTCCATCTCCTGAGTAGGGCCCCTGCCCTGCTCTGACAGGGTGGTCACCTTGCCCCGCTCCGTG
CCCAGTCCTGAGCTGGCAGTGTCTCCAGCCTTTGCAGCGTGGCCCTGTCTGCCTCTGTGTGTGTCTGGGCTCCTCTCATTTGGCCG
CCCTGCCCGTCTACACTGGTGCCCCCCCACCTGCACTGTCCACCGGGCCAGGCTCTGTCTCTGCCACCTGTTGGGTGCTTGCCAGA
CTCCGCCATCCTCTCCGCTCCCAGCTTGCGGCTTCTGCCTCCACTGGCCTGGGCCTGCCCTGCCGCCGCCTCCTTGTGCCCTGGCT
GTCTGCTGCCCCCTCTGCTGCCCATTGGCCAGCCGCTGAGCTCCCTACCAGGAAGAAAAAGGGCCAGGCCATGCCGGACCAGGACT
GGGCACAGCCTCCAGCACTCCAGGTGGACAGGCTGAGAAGCGCGGCGAGCGAGGCAGCCTGGGACGGGTGATTTCTGGAACATGGG
GCCAGCCAGCCTTGGCGTGAGGTGTGGACAGCTGCCAGCTTCTTCTCAGGGCTGCTCACAGGCAGCGGGGGAGTGACCCCGTTTTC
TGCTTGGCACCTGGCTTGCCATCCCGCTCTACCCCAGGGCACTCAGACCTCAAACTCCTCAGCTGCCTGGAACACCAGGGAAGCCC
AGGCTATGTGAGCCTGTGCCCAGCAGGCCAGGGGGCACCAGGACATCCCCCTCTTCTCTGATATTCCAAACCTTCCTGCCTTTGAG
GAATGCCTCCCGGTCCTTCTGGTTTTCTAGGCCATGGGCTCACCCTGGTCTGCCAGCTCTGGAGCTCTGAGAATATTCCTGCAAAA
TGCACATCCCAGGCCTGGCCGCAGACTGTGAGTCACAGGTAGGGCCTGGGATCAGCATTTTCATGGATTCTGCAGGCACTGCTGAG
AGCAGCTGCCCGGGGTCAAGCCAGGGCGTGGGCTGTCCTCATCAGGCTCCCCTTCTCAGAGGCCGCCCTTCTGGCCTGGCCTGCAC
 
CTTTCCCCACTCACTGCCCACCAGCTGCAGACACACTCACTCCTGCCTCAGGGCCTCTGCACGGTGCAGACATTCCTCCCACAGCT
TCCCAGGGAGGCTGAAGAGCAAGGAACCTGCCTCTCCTCACGGAGCCCTCTGCTCCCGCCACACGGCTGGATTTTTCTTGGATGCC
AGCGTTTGACATCTCTATGTGAGATCTGCTTCTTCCCTGATCGTGACATTCCCTCCACCAGCAGGCAGCCGCATGAGGACCAGCGC
CTGCTCACCGCTGTGTCCCTCCTACGTGCGGGGCAGGCACCCAGCAGCAGGTGTTCGGTGCCCACTGGGAGCGTGCCTGGGCCCCT
GCTCCTGCTCCTCAGAGGGAACAAACAACTGACCTGCTGATGGAGGCTGTGAACCCACACGGGTTGTCTGCAGACGTAACGGGGCA
TCTCCTATGACGCTTCTGGAGAGGGCAAGGCTCCACGGCTGAGGGCTTTCTTTGGGTGACAACCATGTGAAATGCCATGACCTTGT
GCCACCCCGTGGCAGATGACTCTCTCAGAAGGTGGTGCGTACCCTTTCCCCATTATGGTGTGGACACGCAGATGTGGGGCCTCTCC
CACCTCTTACAACTGGGGCAGGCTGACAACCACAGTAGAAGGGACACCCTGGAACTCCCAGACCACATCATAAGAGGCCACGCAGC
TTCCTCCTGGAGCTCTCGGGCAGCTGCCCTTGGAGCCTGACTGCCATGCTGTGAGGAAGCCCAGGTGCCACCGAGGCGGCACATGC
AGATGTGCAGCCAATGGCCCTGGCAGAACCTGACAAAGTCAGCACCAGCCACCAGACCTGCCAGCAAGGATGCTCTGAGCCGACGT
CCACTCCAGCTGTCGTCTGACTGCCCACGAGACACCCTGAGTGAGCACTGCCCAGCTAAGACCCGTCAACTCCAGAACTGTGAGAG
GCGATGGTTCCACGACTTCCTGGCTGTAGTCACTGCTGTTGGGCAGCTGTGGGTGACCCTGAAGGGACACTCTGATAAGGGACATC
TGCCTGCATGTGTGACCTGAGTCCCAGCCTCAGCTTTCTCATGTGAAAAATCAGGACCACGGTAGACCCTACCTTATAAGGCAGCT
GCAAGAGTGAAATACAATGGAAAATGTACACAAGATAGGGTGGCTGTTCGACGCCCTCTTGGTTCTTCAAATCTAAGGCCCATGTC
AAGTTTTACCATCATTTTGCCATCATGATGAGAAAAGAAATGAGACCAGCCTGACCAACATGGAGAAACCCCATCTCTACTAAAAA
TACAAAATTAGCTGGGCATGGTGGCACTCGCCTGTAATCCCAGCTACTGGGGAGGCTGAGGCAGGAGAATCACTCGAACTCAGGAG
GTGGAGGTTGCAGTGAGCCGAGATCGCACCATTGCACTCTGGCCTGGGCAACAAGAATGAAACTCTGTCTCACAAAAAAAAAAAAAA
AAAAAAAAAAAGAAATGGAACTTAAACAAGGAAATAGTGTTTTCCTTGTTTTGTTTCTTGCTTATTGAAAGCACCTTGCCTGATG
GGTTGAGGGAGGGATTTTCATAAATCACTGTTGTATACACACGTAGAGAGAAAATACCAGCAAGTAAGGGTTACTGTCCCCACATT
TGAGTCCACTT
>lnc-OR52J3-1:1 (SEQ ID NO: 10) 
AGAGGTAGGAAGGCTAAACTGAGGAATTATGTCTGACAGAAGGGAAGAAGAAGGTAATGTGGAGTGGGTAGCCTCCATACTGATTA
AGAAGGGTACAGACTTACCCTCCACTGTGAGAATTACCTAAAGCTCGGTGTCTGTGATGGTCTACAGGGCTTCCGAGGCGATCAGG
CAGTGGCAGTCTTCAGCTGCAAAGCCGAGAAGATCTGGGAAGGAGTCAGTCAGGGAGCCTTGGGCCAGAGTTCCAGGGGCTCTAGG
AGTGGCTGCCAGGTGAGTTGAACAGTCCGATTTCTAGTGGGGTCCTGCACAGATGGGACACGGCTTAGGAGGAATCCTGGGCTGCG
GGCATTCCTTGGCCTGGTGGCCAGATTTCTGGCACTTGTAGCAAGCTCCTGGGGGAGGTGGTTCCGGAGGAACGCCTGGCCACTGC
GGTTTAGGCATTTGGAAGTTCTTGTGTGCTGGAGATGTGGCTGGGGTTTGTCTCACAGTGGAGGCAAGGAATTGCAACTCAAAAAT
ATGTTG
>lnc-FANCM-10:1 (SEQ ID NO: 11) 
GCAGCAGCTGCTGCACAGAGACATGATGGCTAGGCTAAAAACAGTAAGGTCAAGTTGTTTGGACAGAAAGGCTACAGGGTGCAGTC
CTGGCTCTTGTGTAAGAATTCTGACTGCACTAACCATGCCTAGGAAGGGAAGGAGTTGTTGTTTTATAAGGGATTGAGGTTTGGGA
GATTGATCGGACACGATCAGCAGGGAAAGCATGTGTGTTTGTATGAGAATTATGCCGAGATAGGTTTTAGAAGCCCATGCTGTAGC
AGGCAAGTGATAACAGGCTTTAATCCTTTAAAGCGTGCTGTGGGATGGGATATTGGCGTTGAGCAGGGTAAGGGTGATTAGGTTTT
AATCGGATGGTAATGGGCATGTGATCGGTTGCCAGGGAAGTAGAGATGTCCCAT
>lnc-LRRTM4-3:3 (SEQ ID NO: 7) 
GGGGCTAGGCGTGGCCGACCTCACACTCTCCTTGGGCAGGACCTGCTGCAGCTGCTCTCGGGGTTGGAGGGGAGGTTCCTAGGTCA
ATGGAGTTGTGTACCTAGGAGGATTATGGCTGCCTCTGCTGAGTCATGCAGATTGTCAGGAAGTGTGAGAAAGCCAGCAGTCACCG
GCCTCACCCAGCCACCCTGCCGCCATTAACAGCACTAGGCAGGCAGTGCGCAGAGCTGAGAACTTGCCCCAGGTTACCCGCCTCCC
AGCTGCTAAACAAAAGCGCAGGCTTTTCACCTGCTTCAAATTGTTACAAAGTTCAGCTACAGATTTCCTTTTACCTGCGGCCCTTTTC
CCCATACCACTGGCTGCCCTCCCGAAGAATTCCTGTGATGCCAGGCAGGAATGGCCTGGTTGGAGACCAGCTAGCTCCCAGGGCCT
TTCCCGCTGCTTCCTCTACCCCTGTATTTCGCTGGGCTCTCTAAATGGATTCAGCTCCAGAGGAGGAAAACCTTGAGACCCTGAGA
CTTTCAGAAACTTATTCACAGTTACCAAAGCTGAAAATGTTTGTGGCAGGGAGTGGGACATCCGGCTTCAGAGCAGGAACTTTCAT
TGCACCAGTGATGAAAAAGAGAATGAAATGTGGGTGACGTTGAGAAAGGCAAGAAGATTTTTGTTCAGAAGTGTGCCCAGTGCCTA
AAAGGGAGGCAAGCACAAGACTGGGCCTAACCTCCATGGTCTCTTTGGGCTGAAGACTGGTCAGGCCATTGGATTCTCTTACAAAG
ATGCCAGTAAGAACAAAGAGATCACCTGGGGAGAGGATACAGTGATGGAGCATTTGCAGAATCCCAAGAACTACATCCCTGGAACA
AAAATGATCTTTGCTGGCATTCAGAAGAAGGCAGAAAGGGCAGACTTGATAGCTTATCTCAAAAAAAGCTACTCATGAGTAATAAT
TGGCCACTGCCTTATTTATTACAAAACAGAAATGTCTCATGACTTTTCTATGTGTACCATACTTTAATAGATCTCATACACCAGAA
TTCAGATCATGAATGACTGACAGAATATTTTACTGGGCAATCCTGATTTAAAACTAAGACTGGCTTGTGGTTAAATGAATGTGTTC
AGTTTTTGAATTTTAATAGTAATTCCAATTCAGTAAATGCTATCACTGTTTACCCCTTCTA
>lnc-SLC39A10-7:1 (SEQ ID NO: 5) 
CGAGACTCTGTCTCAAAAAAGAAAAGCTAAGGACCAGGTGTTTTTCAGTGACTTACCCAAGGTCACATCCTTACTCAGGAGCAGAG
TTTAGGCAGTGTTCCAGGTCTTCTACTTCTAAATCCAACAATGTTCAGGGAGCTCTTCATTATCTGATCCCTGTCTACCTACCACT
TTCTATGGTGATACCCAGTTCCCAGCCTTGCTGTCCTTTTTCTCCTCTGGCACGTTTGTCTTCATATTTTGGGGCCTTTGCCTACA
CTTATCCCACTTAAAAGCCTTTCCCTCACTTAACCTTCTCAAATTCTGACCAAGAACTGATAACTTGCAACTCATGATTCAAGACC
TTACTGAGGAGTCTGCTCCTTTAGAGTCCAGTGTGTGTCCTTCTAATACTCCTTGATATGGTTTGGCTCTGTGTCCCCATCCAAAT
CTCATCTCGAATTGTAATCCCCACATGTCTAGGGAGAGATCCAGTGGGAGGTGATTGGATCATGGGGGCAGTTTCCCCCATGCTGT
TCTCATGATAGTGAGTGAATTCTCATGAGATCTGGTGGTTTTATAAGGTGCTCTTCCCCCTTCACCACTCATTCTTCTCTCTCCTG
CCACCTTGTGAAAAAGGACGTCTTTGCTTCCCCTTGTGCCATGAGTGTAAGTTTCCTGAGGCTTTCCCAGACACATGGAACTGAGT
CAATTAAATTCTTTTATAAATTACTGAGTCTTGAGTATTTCTTTATGGCAGTGTGAGAGCGGGCTAATACAGTCCTTATCACATTT
 
TGCTACTATTGTGTTTGGATTTGTCTCACCTTGTGAACCTGAGTGAGCTTCTTGAGGGACAGGTTCGGTGTTTTGCTCATCGTTAG
CACAATACCTAGCATGCATGCAGCAGATACTCAGTAAATAATCGTTTACATTTAAATGACCATGAAAATGGGTGAATCTCATTTAG
TAACTTTAAAAAGGGACTATTTGTTGTACAAATAACCTGTTGGCTACAAACTAAACCAATAATATAGCCAAGAAAGATGTTGGCTA
GAATTGCAGCAAGTCATACGTATCGCTAAAATAAAATCCAAAGCAGCAACCTCGGAGAAGAAAGACATCATCTTGGAACTACTGGG
CATGCAGCAGGTAAGTGGAACATCCTCTGTACCCTCCTTCCCTGCAGCTGTCACAGTGAGTCCATGTTTCCTTGACTTTTAATCAG
AGCCACTATCCAATGTTAACAACACACATACAAAAGCAGCAGCTATTAATATAAGCAGAAGACAAGAGGGTTTTCTACTTTTCTCA
CTAAGGAAACCACATTTTGATAATTCTCCAGGTGGTTACAAAGAACAAACTCAAGCAAGAATGCTGGACTAAACTTAGTCCAAAAG
GCTAATTGTGGAATGAGATGGAAAAAAAGAACAAAGGTCAATGGAAGAGAATTGTTGAAGTCCTAACCCCCAACATCTCAGAATGT
GGCTTTATTTGAAGAGAGGATTACCTTTGCAGAGGTAATCAAGTTAAAATGAGGTCAATATAGTGAGTCCTAATCCAGTATGGTTG
ATGTCCTCATGAAAAGGAGAAATTTGAAGACAGACACACCAAAGGAGAATGCCATGTGGAGACACAGGGAGAAGTTGGCAATCTAC
AAGCCAACGAGAATAGCCTGGAACAAATTCTTCCATCACACCCCTCAGAAGGAATCAACCCTGACTGCACCTCCATTGCAGAGTTG
TAGCCTCCAGAACTGTGAGACAATACATTTCTGTTGTTTAAGACGCTAAGTTTACGGAGTAAATTTCTGCTTGTTACAGCAGCCCC
AGGAAACTATTACACTCCACCTCAGCATTTTCCTCCAGGCATCCCTCAACCCTGTTCTTCCAACATTTCCCAAGAAAGAAAGAAAA
AAATATTACTTCACATATGTATATTATAAATCTTGCCATTTCATTTGCCTTCTTTTAACATCAGTGGGTAATCTTACCTCACAAAG
TTAGATGTAATATGGAAAAACCTCTACAATTTTCAAACCCATAATAATCAAGTTCTATAATCACCAAAAAAGAAAAGTACAAAAGA
AAACCAATGGTGAGAACTCTTATAAAGCAAGTACAAAGACAAAATTGGCTATGCACTATCATTAACAGAAGCTATCACGGCTCCTT
TGTAATCTTAAGCAGCTATTCCATGATCTTTTCTCTCGCAATGATGAACCGAACTTTTGATAAAATATTTGATCTCCTTTTAGCCA
AAACTCTTCTTTATAAGCCCATTTAATATTCCAGAAGGATTTTCTTTCGTTTGAAGAAATATAAGTTTGACATTCTAAAGGCATTT
GTATTTTAAAGCCTACAAAAAGATTTTTGGAGAGTACCTGGTGAAGTACCGACTTGCCCCTGTGGCTCAAAAGTTCAATTATTATA
GACATTTCACTCAGAACAGCATTTCTGTCTTTTA
>lnc-HSF5-2:1 (SEQ ID NO: 12) 
TCCTCAGTGTACTCCATATCTCCGGTTGGCTTCACAATTACTCAGTATTACTAAACCCTGGTGCAAGGCACAATGCTGGGCCCTGG
AGAGGGCCTGAAGGAGCATAAGATAACAAACATTCCTGCCCTCAAGGTGCTTACTAACGGAGCCCTTCAGATACTGTAGATATGTA
CTAGAGAACTCTACCTTGAGAATTTATAATAAGTGCTAAATAAGATATACAGTGAGTTTCTATGGGAGTTTACAATGAGAAAGAAC
AGCAGTGTGGAGAAGAGTGATCAGGGAAACTTCATTCTGTGAAAAACAAAAAGTTTCAGTCCTTTGTTTAAAATTTGCTTTTCTTT
CATGTTTCTCATCCATTTTCTTTTGCCTTTTTCATTAAATTGCTACTTTCGCTAAGCAATATTGAGTATTCTAGGCACTTAGTAAA
CGGGATCATTAAACCTCTTTTAGAATTTAGCTGAGGAGCTAGAATTTGAGTATGCTGGTATCAAAGGATGGTGCAGGAAAACCAAG
TACAACTGATTGTAAGCATCTTAATTTCAGAAGACCCTTTTCTGATTACTATTTCTAAAGATGTTCATATAGTTTTAAGGCTACTG
TTACTGAAACAGTGTAGATTAGGCCACAGGTTTTAAGTAGCAGGTGAAATAAAATCCTACACTCACTGGTATTCAAGGTGAGCATC
GAAGTTGCAGGTTTTACTCTTTACACCAGAATCCCAAGTGTAAAGCAGCTAGACTCTTTTGCTCTAGAAAACAATATCTTAACATT
TGCCTCACTTTCTTCAACTAAAGAAAAATGATAGGGCCACGTGACATTCTTCTAGAAAATTTTTTTTAAAAAAAGGATCTACCTTA
TATAACTTGACTGGGGCCCTTATAACTTATTAGTTAGAAAGCTCTGATCTCATGCCTTCTGTTTTCTTTGTTTTGTATTCCATTTT
GCTCAGCTTTCGGGGCACCATTTCATTCCCTTTACATATTCTGGATTTGGGGTTTGTCTCGTTCCCACCTTCCACATTTTCTTTGC
TGATTCTGAATGTAGCTTTGGAGGAGCATGGATAAAATAAAAGAGTGCAGAGCTTGAGGATGAGGACATGCTGCCCTGCACCCCGT
CATGTGCTCTGCTTCTGATTTACCAACTGCAGAGATAAGCAGTGTATCCTTTCTTTCATCTAAGACGAGTAATACTTTCTTCCATG
GAAAAAGCAGTAGTTCAATCTTAAAAAGAAATTAGGTCCTTTAGAAGAATTGAAGCACTAAAGCAAACTAAATCAAGTTTTGTTAC
CATTTTGTCCAGCATTGTTGTTTGTTCAATGGTACACAGGACAGTAGTTATTTGAAAGCATGTGTTAGATCATATTTATGTAGTAG
AATTGTAGAAATACAGAATTTATAAGATACAAGAAAGCTTTGAAGCTTCTGCAGATGTTTCAGATCCACTCAATTCAATGCCCTGG
TTAATACTGAAGTGATATGTAAATTTAATATTATGGGGAAATAATGGAAGAAGCCCCTGTTCTTCTACCTGAACATGGAGTCTATC
TTTTGGGAAATGACCAGTTTTGCATATTGGCCTCCCACTCTGGTGGTTTTCATTCTACATGTAGTAAGGAAACTGCCTAGAATTGT
TTTGGTCCCTGTTGATCAACGGTTAGCAAATTTTAGCATATATCAGAATCAGTTTCAGAAGGCTTGTCACTCAAACTCCAATTCCT
ATTTCAAACTACCTTTAAGATTTAAATGTTCATCTCTCATTTAGTATATCAAGACTGACTTAAATTTTCCCTACTCTAGCTTTTTT
CTAATTTCCCCATTTTGTTAATGCAACATCTTTTTTCCAATTCTCCAAGTTTAG
AACTT
>lnc-CAMK1D-2:1 (SEQ ID NO: 14) 
CACCAGTGGCCTCAGGTTACTGTGTGTACAAGGTGTTTGTGGGATATTTCTGGTCTCCCACAATTTCAGCTGATGTCCAGAGTTAA
AGGGCTCTAAGTAAGTACCCCACCTTCTATAAAGTGTTGCTAAGGAAAGCCCTCAATGCTAAGGCTTTGATACAAAATACCATTGA
TCCACCTTCCCCTCAATCTTTTTGTTTTGTTTTGTTTTGTTTTTGAGATGGAGTCTCACTCTGTCGCCAGGCTGGAATGCAGTGGC
GCGATCTTTGCTCACTGCAACCTCCGCCTCCTGGATTCAAGTGATTCTCCTGCCTCAGTCTCCTGAGTAGCTGGGATTACAGGCAT
GCCCCACCAAGCCCAGCTAATTTCTGTATATTTAGTGGAGATGGGGTTTCACCATGTTGGCCAGGCTGGTCTCAATCTCCTGACCT
CATGATCCGCCCACCTCGGTCTCCCAAAGTACTGGGATTGCAGGTGTGAGCCACCGTGCCTGGCCCCCCTCCATCTTTTTGCATCA
GAAGAGACATGAAAAGGGAGAAAATGGTAGTTGTGGTCAACTGTGTGGTCAGCAGTGTTTACAGTGGCAGACTGTCTACAGAGGTG
AAGAGGGGTGCCTTCTCTTCTCCCTTTGCAGAGGCCAAGTGGGGAAGGTTGAGGAGCAAGAAAGGGAGACCAGCATTTCCATTTCC
CAGTTGGGTTCCCATTTGGGCACCTGCCTGTCGGGCCATCTGCCCGGTAGGAAGCACTTCCTCGATAACCGTGCAGCAGTGCCTGA
CCCCAATTCATGCGGCTCAACTGTGGATTTCTCTCTTTAGTATGAGGGGTACTTAGCAGAGTTGGTTTTTTTTTTTTTTTTTTTT
TTTTTTTTTTTTCAGATTTGGCCTTTAACTGGCAAATTCTGGGTCAGGAAGAGCGCCCCTCAACATTTTTTGATGCTAATCCTTCCC
CCCCAAATCTGCCACTGGTTACAGTAACTCAGCTTTCCCACTTGAGCAGTTTGAGGGATGCAGGGTCTGACAGGAGCCCAGAGGCC
GTTCAGAGGATGGGCACCAACAGTACCCTGGCTCCAAACAGGGCAGCCAGGTGCAAGGTGGGCACAGGGCTTAGCAGGGAAGGTGG
GCCGGCAAGGCCAGGTGCATGGCAGGAGTGGCGGGGAGCCTGGTGGGCTGTGGGGAGTAGAGGTAGTTCATGGCCAGCCTGGAGCT
GAGAACATGGAGAAAGAACCCCAAACTCTTAATTTTTCTGTAGAACTTGGCTGGTGCTCAGGGCTGCTGGCAGCCTCAGCTGGAAA
TCTGTTTACCCAGAGAACGAAGCATCAGATAAGCTTTGTGGTGGAAGAGAGCCATGATTCTCAGTCTAAATGTTGAAATTAGAGGG
 
AGAGGCAAAGAGAATCCAATGGAACTTGTGGCCGGTGAAGCTCTAGTGAGCCTGAGGCAGTGACTTGGGTCTAAGGTGAAGCCTTA
TTTA[?]GGAGAAGCGATCCCAGGCGAAAACAGCTGTTGGCCAGAGTTTTGGAGAAACATGAGTTGACTCAGAATTAGGCCATGAACT
TAGG[?]TATTATTTGCCTTTTTTCC
>CYP1B1-AS1:12 (SEQ ID NO: 6) 
GAGAATTATGCTGAGATAGGTAACAGATGAGGATGAAATTTGGGCTTGACTGAAGTAATGGGGGCTGTCTGTGAAGCCTTGCGGCA
GTACAGCCCAGGGGGCTTCCAAGGCAGTCGGGCAGCATCAGTCTTCAGCCGCTAAGCCAAGAAGATCTGGGAAGCAGTCAGAGAGC
CTTGGGCCAGAGTTCCAGGGGCTCTGGGAGTGGCTGCCAGTGGTCTCAAATAGTCAAGACCCCCTGCTGGGAGGAGCTTCTATCCT
GCTCTGTGGAA
>lnc-TRPC5-3:1 (SEQ ID NO: 1) 
TTTTTGCGTTCTCTTTCCCTCTCCCAACATGGCGGCCTCAGCAAAAAAGAAGAATAAGAAGGGGAAGACTATCTCCCTAACAGACT
TTCTGGCTGAGGATGGGGGTACTGGTGGAGGAAGCACCTATGTTTCCAAACCAGTCAGCTGGGCTGATGAAACGATGACCTGGAAG
GAGATGTTTCGACAACTTGGCACAGTAACAATGACGATGTGTATAGGGTGCCTCCAATTGACCGTTCCATCCTTCCCACTGCTCCA
CGGGCTGCTCGGGAACCCAATATCGACCGGAGCCGTCTTCCCAAATCGCCACCCTACACTGCTTTTCTAGGAAACCTACCCTATGA
TGTTACAGAAGAGTCAATTAAGGAATTCTTTCGAGGATTAAATATCAGTGCAGTGCGTTTACCACGTGAACCCAGCAATCCAGAGA
GGTTGAAAGGTTTTGGTTATGCTGAATTTGAGGACCTGGATTCCCTGCTCAGTGCCCTGAGTCTCAATGAAGAGTCTCTAGGTAAC
AGGAGAATTCGAGTGGATGTTGCTGATCAAGCACAGGATAAAGACAGGGATGATCGTTCTTTTGGCCGTGATAGAAATCGGGATTC
TGACAAAACAGATACGGACTGGAGGGCTTGTCCTGCTACAGACAGCTTTGATGACTAGAAGAGGTGATGATAGCTTTGGAGACAAG
TATCGGGATCGTTATGATTCAGACCGGTATCGGGATGGGTATCGGGATGGCCCACGCCGGGATATGGATGGATATGGTGGCCGGGA
TCGCTATGATGACCAAGGCAGCAGAGACTATGATAGAGGCTATGATTCCCGGATAGGCAGTGGCAGAAGAGCATTTGGCAGCGGGT
ATCGCAGGGATGATGACTACAGAGGAGGCGGGGACCGCTATGAAGACTGATATGACAGACGGGATGATCGGTCGTGGAGCTCCAGA
GATGATTACTCTCGGGATGATTATAGGTGTGATGATAGACGTCCCCCCCAAAGACCCAAACTGAATCTAAAGCCTTGGAGTACTCT
TAAGCAAGATGATTCCTCTGCTAGTACCTCCCAGTCCACTCGAGCTGCTTCTATCTTTGGAGGGGCAAAGCCTGTTGACACAGCTG
CTAGAGAAAGAGAAGTAGAAGAACGGCTACAGAAGGAACAAGAGAAGTTGCACCGTCAGCTGACTGAGCCAAAACTAGAACGACCG
CCTCGGGAGAGACACCCAAGCTGGCGAAGTGAAGAAACTCAGGAACGGGAACGGTCAAGGACAGGAAGTGAGTCATCGCAGACTGG
GACCTCCACCACATCTGGCAGAAATGCACGAAGGAGAGAGAGTAAGAAGTCTCTAGAAAATGAAACACTCAATAAGGAGGAAGATT
GCCACTCTCCAACTTCTAAACCTCCCAAACCTGATCAGCCCCTAAAGGTAATGCCAGCCCCTCCACCAAAGGAGAATGCTTGGGTG
AAGCGAAGTTCTAACCCTCCTGCTCGATCTCAGAGCTCAGACGCAGAGCAGCAATCCCCTACAAGTGGTGGGGGAAAAGTAGCTCC
AGCTCAACCATCTGAGGAAGGACCAGCAAGGCAAGATGAAAATAAAGTAGATGGGATGAATGTCCCAAAAGGCCAAACTGGGAACT
CTAGCCGTGGTCCAGGAGACGGAGGGAACAAAGACCACTGGAAGGAGTCAGATAGGAAAGATGGCAAAAAGGATCAAGACTCCAGA
TCTGCACCTGAGCCAAAGAAACCTGAGGAAAATCCAGCTTCCAAGTTCAGTTCTGCAAGCAACTATGCTGCTCTCTCTTGATGGTG
AAGATGAAAATGAGGGAGAAGATTATGCCGAATAGACCTCTACATCCTGTGCTTTTCTCCTAGTTTCTCTCCACCCTGGAACATTC
GAGAGCAAATCAAAACCTCTATCCAGACAAGACAAAATAAAACTCACCATCTCCTGAAGATCTTTCTTACCTTTTTTTAAAAACAA
AAAATGAAACTATTTTGCATGCTGCTGCAGCCTTTAAAGTATTGAAGTAACTGGAG
>LINC01715:13 (SEQ ID NO: 17) 
ACGTGGTGGCGATATTGAGCTGTGTATACCTACAGCCGCTGAGAGCCGGTATTCCAACGCCATGAGACGCCTTCTGTCGTGAGCAG
TTGACAAGAAAGACCCAGCACTTACTGCAACCGGAAGGAGCGGTTCCTCCCTCGCTCGCGCAGCCGCTCTTCCCCGCCACTCCCTC
GGTGCCCGCCAGCACATTCCCAGCAAGCCCTGAGTATATTTGCATATCAACTCACTACATTTTTTTCTTCTAACTAAAAAATCGAA
AGGACAAATTCCAGATTCTCCTTGTGAAGTCTTCCTTTCAGTTCAGAAGAAATGGAATTCGCTCTTCAACTTCAGGAAGTTGAAAT
AAAGAGTTGCTTGGATTTTGTGTTCACCTTTACCAAAAAATGGATTTGGTAACACTGCCACCCTGCTTTGGTGACAGAGAAAGCAA
AAAGGGCTTCTGTCGTGAGTGGCACACGTAGGGCAACTCGATTGCTCTGCGTGCGGAATCGACATCAAGAGATTTCGGAAGCATAA
TTTTTTGGTATTTGGGCAGCTGGTGATCGTTGGTCCCGGCGCCCTTTCTTTACTGTTATATGTTAGGCGAAATATTACGCGTTTGG
AGTAAGTGGTGCTTTTTGTAACTGAAAAGAGATTCTGTGTGTGTTTTTTTTTTTTTTTAGAGGCTGCATAGTTATTTGTATGAACC
GCAGGTGACCTAATTAGCAAGGTAATTCAGACCTGTATCCTAGCACTTAAGAAGTGGAAGTGGGATAACCCTGCGTGTTCTCGTGT
GGGAGGCTAGCTTAAGCAACAAAGCAGGAAGGCCCCACCAACAAAAGAATTTAAAAATTATACGTGCGTGGTGGCGCGCGTATGAG
GAAATTATTTGGCAACTGTGTTACAGTATAGCAGCAAATGGAATAGACCTAGTCCCTGACTTTAGGAGACCTGCCTCAGCCCCCCG
AATGGCTGGGACTACAGGCGTGCGCCTCCGCGCCCAGCTAATTTTTGCATTTTTGGTAGAGACGGGTTCACCATGTTGGCCAGGAT
GGTCTCCATCTCTTCATCTGGTGATCCGCCCACCTCGGCCTCTAGGAGTGCTAGGATTACAGGCGTGGCCATCGCGCCCGGCCTGC
AATGTTATTTTATGAAGCAGAGGAAATTGAAATTACCTTCACCAGGCCTTAGCAAACTTTTTCTATACCTTATACCATACACAAAA
GATTAATTCAAGGCCGGGCGCAGTGGCTCACGCCTGTAATCCCAGCACTCCTAGAGGCCGAGGTGGGCGGATCACCTGAAGTCGGG
ATTTCAAGACCAGCCTGACCAACATGGAGAAACCCCTTCTCTAACTAAAAATACAAAATTAGCCAGGCGTGGTGGAGTATGCCTGT
AATCCCAGTTACTCAGGAGGCTGAGGCAGGAGAATTGCTTGAACCCAGGAGGCGGAGGTTGCAGTGAGCCGAGACCGCACCATTGC
ACTCCAGCCTGGGCAACAAGAGCAAAACTCCGTCTCAAAAAAAAAAAAAAAAAAAAGATTAAAGACCTAAATGTAAGACCTTAAAC
TATAAAACTCTTAGAGAAAACACAGGGGAAAATGACATCGAATTTGTCAGTGATTTCTTGGATATAAACAATGGTTTACAAGCAAC
AAAAAATAAATTGGTCCTCATCAAATTTTAAAATGTTAACATTAATTTTAAAATTAAAATGAAAAACCCGGCCGCGTGCGGTGGCT
CACGCCTGTAATCCCAGCACTTTGGGAGGCCGAGGTGGGCGGATCACCTGAGGTCAGGGGTTCGAGACCAGCCTAGCCAACATGGT
GAAACCCCGTCGCTCCTAAAAATACAAAAATTAGCCAGGCCTGGTGTCAGGCGCTTGTAATCCCAGCTACTTGGGAGGCTGAGGCA
GGAGAATTGCTTGCACCGGGAGGCGGTGTTGCTGTGAGCCGAGATCGTGCTACTGCACTCCAGCCTGGGCAACAGAGTGAGACTCT
GTCTCAAAAAAAAAAAAAAAAAAATAGCCGGGCATGGTGGCTCCCAGCACTTTGGGAGGCTGAGGTGGGCAGATCATGAGGTCAGA
AGGTCAGGACCAACCTGGACAATATGGTGAAACCCTGTCTCTACTAAAAATACAAAAAATTTAGCCGGGCATGGTGGTGCATGCCT
GTAATCCCAGCTACTCGGGAGGCTGAGGCAGGAGAATCGCTTGCATCCAGGAGGCAGAGGTTGCAGTGAGCCGAGATAGCGTCATT
 
GCACTCCAGCCTGGACAAGAGCGAAACTCCGTCTCAGAAAAAAAAAAAAAAGAAAGAAAGAAAACCCTTGTACATCAAAGGACACT
ATCAACAGAGTGGAAAGACAATCCACAGAATCAGAGAACATATTTGTAAATAATATATCTGATAAGGGATTAATATCCATAATACA
TAAGGAACTCATAACTCAACAACAACAACACAAACCCAGCCCAGTTCAATAATGGGCAAAAGGACCTGTATAGACATTTCTC
>lnc-PLA2G1B-2:3 (SEQ ID NO: 15)

GCTGGTAATGGTCACACTAGGTGGCGTCCGCGTGAGGTGGAGGTGTCAGCGGCTCCTGTCTGCAGCTTCCTGGGGTCCTGGCGGCG
CTCTTCTCCTGAGCCAGAATCCAGCAGGAAGGACAGAATGGGCGCTAGAGTGTGAAAAGAGGGCGAACTCACGTGGATGTGACTCT
CCGGGTCGCTCTTCCCTGTCACCCAGCTCCTCCTGCCGGTTTCCTGTGGGCATCGCCTGCATTGCTTTTATCCCGGGGACGGATTA
GGGGGGTCCAGAGGAGGACTTGCTGGCACAAATAACCCAATGGAGGCTTTCGAGCACGGCTGGCTGGGGTTTGCGATCCAACGGCC
TTTTGCTGACCTGAAAGTCAACGCAAAGCTCATCTTCATTCTAGAGAAAAAGAAGCCTCATATCTGAGATCAGGACCCCGCTTTCT
CCTTGACATTCCACAGCAAAACGTGAACAGCTGAACAAACACACAGTCCGGTTTCCCGAGACCAAAGGGTCAGGGGAAACCCCCGC
TGAAAACGTGCATCAGTGGAACAACTACTCTCTTCCAGGTCCCACCGAGGGCAAAGAGGAGAAATAGAATCCCTCACAAGAAATCC
ACAACTTCTAGAAATCCGCAGCACACACAGAACACACTTACCTCTCACAGCTCTACGGACACTTCACACCCCACAGTGGGACCGTC
TGATTGCATTTGCATTTTGGGACATGTAGTCCAGAAAGCTTAAATGATGCGAGAAAAACCACAAGTCCCACAATCCTCCGCGTCTC
GCAATTGACAGCCCCAGATCATCCAGGAACGCCCCCTGTCGGCTCTTTCTGGTCAGAGGTTCCCCCGTTAATATAAAAGCTTTTAA
TCATGGATTTTTTTCTTTGTTTTCAATTTCTGTGGGCACTTAGTAGGTGTATATATTTATAGTGTACATAAGATTTTTTTTCTTTT
TTCTTTTTTTTTTTTAAGACGGAGTTGTTGTTTTTTTTTTTTGAAAAAACTCCAGTCTGGAGTGCAGTGGCACGATCTCGGCTCAC
TGCAACCTCCCTCTCCCGGGTTCAACCGATTCTCCTGCCTCAGCCTCCCGAGTAGCTGGGATTATTTTTGTATTTTTAGTAGAGAC
GGGGGTTTCGCCATGTTGGCCAGGCTCGTCTTGAACTCCTAACCTCAGGTGATCCGCACGCCTTGACCTCCCAAAGTGCTGGGATT
ATAGGCGTGAGCCACTGCGCCCGGCCATCTTTTCTCTTATTTTTTGTCTTTTTTTCTTTTCTCTGTGCCTGTCTATGCTTTACCCA
CATGAGATGTTTTGATACAAACATGCAATGTGAAATGAGCACGTCATTAATCATGGATCTAGATTTGACTTTTTTTTAAAGTTTTT
GTATTTTTAGTAGAGACAGAGTTTCACCATGTTGGCCAGGCTGGTCTCGAACTCCCGACCTCAGGTGATCCGCCAACCTCGGCCCC
CCAAAATGCTGGGATTACAGGCCTGAGCCACTGTTCCCGGCCTAGATTTGACTCATTCTAAACATCAGAGTTTGTGATCCTTTTCA
TTCACAACGACAGTATCAAGTTATTTGCTATTGGTAAAATACCTTGAACTTGTGAAGAAATGTGTTTATACATATCTGAAAAAGTG
ATAGTTTGTTGTTGTTGTTGTTGTTGTTTGTTTGTTTGTTTTTGAGACGGAGTCTCGCTCTGTTGCCCAGGCTGGAGTGCAATGGT
GCCATTTCGGCTCACTGCAACCTCCGCCTCCCGGGTTCAAGCAATTCTCCTGCCTCAGCCTCCTGAGTAGCAGGGATTATGGGCGC
ATGCCACTACGCCCGGTTAATTTTTTTATTTTTAGTAGAGACAAGGTTTCACCATGTTAGCCAGGATGGTCTTGATCTCCTGACCT
CGTGATCTGCCCACCCTAGCCTCCCAAAGTACTGGGATTACAGGCGTAAGCCACTGTGCCCAGCCAAAAGTGATGTTTTTACAGTT
AATATGAAGCCAAATATATTATACCTGGACTAATGTACTTAACCTGCTTGGCTTCTCCAATTTTATTTATCATATTTTTTTTATTA
AGTTAATCAAACAAGGCCTGCGTCTCACTCCCAGGGATTCTGATCTACAAATTTCCTAATTGTTCCTAATGTGTAGACAACCAGAA
CTGCCGCTCTGCAGCATCACCTAAATCAATAAATGTATAAGTGTCTTTGGACATAAAGGTGGGCGGCCAGCAGGGACCTTGAAGAC
CACCTAAGGACTCCCTTCCCCCATTTTAAGATGAATTGGGCTGAGTGCAGTGGTTCCCACCTGTAATACCAACACTTTGGGAGGCC
AAGGCAGGAGGATCACTTGAGCCCAAAAGTTCAAGACCAGCTTGGGCAACATAGTGAGACCCACTCACTACAAAAAATAAACAAAA
TTAGCGGGGTGTGGTGGTGTGTGCCTGTAGTCCCAGCTACTCAAGAGGCTCAGGTGGGAGGATCACTTTGAGCCCAGGAGATCGAG
GCTGCAGTGAGCCATAATTGTGCGACTGCACTTCAGCCTGGGTGACAAAGTGAGATCCCATCTCTTCAAAAAATAATAATAATAAA
TAAAGATGAACTGAAGTGCAGAGAAGTAATGTGGAAGCTTGAGAAAGAGTTTTTAGGTTTTTGGTTCTATCATTGTATCTTTGGAG
GTAATATTGGCAAAATGACCATTTAGTAGTTTGCCCATTTCTGCTTTTTTTTTTTCTTTTTTTTGAGACAGGGTCTCGTTCTGTCA
CCCAGGCTGGAGTGCAGTTGTGACATCACAGCTCACTGCAGTTTCTACTTCCCAGGCCCAAGGGATCCTCCAACCTCATCCTCTCG
AGTAGCTGAGATCACAGGCATCCACCACCACGCCCAGCTAAATTTTGTTATTTTTTATTTTTGTCGAGACACGGTCTCACTATGTT
GCTCAGGCTGGTCTCAAACTCCTGAACTCAAGGAAAGCTCTTGCTTTGGCCTCCCAAAGTGCTGGGATTACAGGTGTGAGCCACCA
TGCCGGGCCTGCTTATTTCGACTTGGCAGCATCGTTTCATGAAGCCCCTGACTCTGTGACAACATACAGCTCTCCAGAAAGATGCT
TTGAAGACAAAACAGAATAGAGCACACAGCCCTCCATGTCTCTTCCAAGTCACTATATTCATTAAAAGATAAATGACTCTGGTCAT
TGCCTTTTCCTGCACAAGATAATGTCTGATGGGGTTAGTGATGATACCTCTGTAATCCATAACCAGATATACTCTTGCACCCAAAC
TTTGATATGATTGTGCTTTAATGTAACTTTGAAGCAAGTTTAATGTGATTTTGCAGGCGCCGTACACTCACCACTTGTATATGAGT
GGCTGAAATTCTGTGTTGGACCAGTCTGATGGAACTGCTCCCACACTATAGCCCTCGGTCTGTAATCCTCAGTAAGACTTGTAAGT
AAAACTAACTTTAATTTTTTTTTTTTTTTTTTGAGAGAGTCTAGCTCTGTTGCCCAGGCTGGAGTGCAGTGGCGCGATCTTCACTC
ACTGAAGCCTCTGCCTTCCCGGCTGAAGTGATTCTGCTGCCTCGGCCTCCGGAGTAGCTAGGATTACAGGCGCTCGCCACTGCGCC
CGGCTAATTTTTGTATTTTTAGTAGAGATGGGGTTTCGTCATGTTGGCCAGGCTGGTTTTGAACTCGTGACCTCAGGTGACCCACC
TGCCTCAGTCTCCTAAAATGCTAGGATTACAGGAGTGAGCCACTGCACCCCGCCACTTTTATTATTATTATTATTGTATTTATTTA
TTTATTTATTTATTTTTGAGACGGAGTCTCGCTCTGTCGCCCAGGCTGGAGTGTAGTGGAGAGATCTCGGCCCACTACAAGCTCCG
CCTCCCGGGTTCACGCGATTCTCCTGCCTCAGCCTCCGGACTACAGGCGCCCGCCACCACACCCGGCTAATTTTTTGTATTTTTTT
TTTAGTAGAGACGGGGTTTCACCGTGTTAGCCAGGATGGTCTCTATCTCTTGACCTCGTGATCCGCCCGCCTCAGCCTCCCAAAGT
ACTGGGATTACAGGCGTGAGCCACCGCGCCCGGCTATTTATTTATTTATTTATTTATTTATTTATTTATTTTTTGAGACGGAATAT
TGCTCTGTGCCCAGGCTGGAGTGCAGTGGCACGATCTCAGATCACTGCAACCTCCGCCTCCCGGGTTCAAGTGATTCTCCTGCCTC
AGCCTCCCGAGTAGCTGGGATTACGGGTGCCCGCCACCATGCCCGGCTAATTTTTGTATTTTTAGTAGAGACGGGGTTTCACCATG
CTTGTCAGCATGGTCTCGAACTCCTGACCTCGCGATCCGCCCGCCTCAGCCTCCCAAAGTGCTGGAATTACAGGCATGAGCCACTG
CGCCCTGCCACTTTAATTATTTAAAAGCTTATTTTTTTATTTTGGGGACATGGTCTCACTCTTTCACCCAGGCGGGAGTTCAGAGG
CACGATCACTGCGCACTACAGCCTCAACCTCTTGGACTCAAACCATCCTCCCACCTCAGCCTCCTGAGTAGCTGGGACTACAGGTG
GGCCACCAAGCCCAGCTAATTTTTGTATTTTTTTGTAGAGACGGGGTTTCACCATGTTGCCCAGGCTGGTCTCGAACTCCTGGGCT
CTAGGCTTTCCAAAGTGTTGTGATTACGGGCGTGAGCTACTGCGCCTGGCCGATTCTTTTTAACTTAGTACACAGGGAGTAGTAGC
TCCAGCAACAGCGAAAGTCAGTAGCTGTAGGATCTACTAGGCGGGGAAACTCATATGACAGCCCCCTCCTTTTTTTTTTTTTTTTT
TTTTTTTTTGAGATGGAGTTTCACTCTTGTTGACCAGGCTGGAGTGCAATGGCGCCATCTTGGCTCACCGCAACCTCCGCCTCCCG
GGTTCAAGTGATTCTCCTGCCTCAGCCTCCCCAGTATCTGGGATTACAGGTGTGAGCCACTGCGCCCGGCCACCCCCTTTTTTTTT

CAAGGCTGCAGTGAGCCATGATCCTGTGACTGCACTCCAGCCACTGACAGCCCTTTCTGCATTAGTTAACCTGACCACATCTGCTG
AAAGAAAGCACCAGAAGCTAGACTGAAATTTTCGTTGTCAAAAAAATTATAATCCCCCCCAAAATTTAAAAATTATAATCCAGCAC
ATTTTCTGCACACATTTTCTGGGTATTTGATCATAACTTTTAAAAATGTGAGAACAGTGACATTCTCCTTCCCCATTCACCAATTT
TACTATGAAAAAGTTCAAACCTAGGTTATTTTTTCAGTTCTCCAAGTGGAATGATCATCACTAATAGAGATTGGTTGGGAATTCTA
TTGTGCTTCTAGGCCCCCAAAAATCTCATGCTCTGAGGAAAAGTTAGCTGAGTTTTCAATTTTTCTCATAGTGCTGGAAAATCATC
CCTGGACGGTGAAGTCATCCAAGTCTTTTTTGTTTTGTTTTGTTTTTTCTTTCTTTCTTTCTTTCTTTTTTAATTTTTGTATGCTT
TGTTTCTATTTCTTTTTTTTTTTGTCATCCAAGTCAGCTGGTGAAACACACAATTTCACTTGATAGCTACTGAGAGAGTGTAATGG
GCCACCCATTCTAGAATTCTCAAGGGCTAGGAAGTTCCTTTGATACCCTCATGGGATTAGGATTAGGGCTTTAGTCTTTTTCTCTC
TCTCTTTTTTTTTTTTTTTTTTTTTTTTTGATTACAGGCGTGAGCCACCACACCAAGCCCCATTATTTATTTATTTTATTTTATGT
ATGTATGTATGTATGTATGTATGTATTTATTTATTTTGAGACGGAGTCTCGCTCTATCGCCCAGGCTGGAGTGCAGTGGCGCAATC
TAGGCTCACTGCAACCTCCGCCTACCGGGTTCAAGTGATTCTCCTATGTCAGCCTCCCAAGTAGCTGGGATTATAGGCGCCCGCCA
CCATGCCCAGCTAATTTTTGTATTTTTAGTAGAGACGAGGTTTCACCATCTTGGCCAGGCTGGTCTTGAACTCCTGACCTTGTGAT
CCACCCGCCTCGGCCTCCCAAAGTGCTGGGATTACAGGCGTGAGCCACCGTGCCTGGCCTAGTGTTTTTCTCACAACTCCACCCCA
TCCAGAATTAATGACTAAATCCTTGACTGGATGATCAAAGTAAAAGAATCTAAATTCACCACATTGCTAGTCTTTTCTTTTTATCC
AGGCTGTGAATTAAATAAGAAATCTGCTGCAGTGGCTCATGCCTGTAATCCCTCCCCTTGGGGAGGCCGAGGCGGGCGGATCACTT
GAGCCCAGGAGCTGGAGACCAGCATGGGCAACATGGTGAAACCCTGTATCTACAAAAAATACAGAAAAATTAGCTGGGCATGGTGG
TGTGTGCCTGTAGTCCCAGCTACCTGGGAGGCTGAGGTGGGAAGATCGCTTGAGCCCAGGAGATTTAGGCTGCACTGAGCCGTGAT
TGCACCCCTGCGCTCCAACCTAAGCGACTGAGAACATGTCTCAAAAAAAAAAAAACAAAAAAAAAAACACAAAACAAGTTAGAAAT
AATCACATAACACATCAGCATCAAACAGGCCTTTTAAAATGTCGTTTTTGACCATCCTGGCTAACACGGTGAAACCCCATCTCTAC
TAAAAATAGAAAAAATTAGCCGGCCGTGGTGGCGGGCGCCTGTAGTCCCAGCTACTCGGGAGGCTGAGGCAGGAGAATGGCGTGAA
CCCGGAAGGCGGAGCTCGCAGTGAGCCGAGATTGCGCCACTGCACTCCAGCCTGGGCGACAGAGCGAGACTCCGTCTCAAAAAAAA
AAAATGTCATTTTTGTATATGGATGATGAGTACATGAATGTTCACTGAGGAATTCTTTGAACTTTTCGTGTTTACAATTTTTTATG
ATAAAATGTTGGGGGAAATGTTACTTTCTTTTTTTTTTTTTTTTTTTTTTTTTTTGAGACGGAGTCTCGCTTTGTCACCCAGGCTG
GATTACAGTGGCATGATCTCAGCTCATGGCAACCTCTACCTCCCAGGTTCAAGCGATCCTCCTGCCTCAGCCCCCCTAGTAGCTGG
GATTACAGGCAAGCGCCACCACGCCCCGTTAATTTTTGTATTTTGTATTTTTGTAATTTTGTATTTTTGTATTTTGTATTTACAGG
GATGTCGCCATGTTACCAGGCTGATCTTGAACTCCTGACCTCAGGTGATCCACCTGCCTTGGCCTCCCAAAGCGTTGGGATTACAC
GCTCGAGCCACCACACCTAGACCAGATGTAGGTTTCTAATAGCTTTCTACATCCTGAAGGAAAACAGGCAGTGAGGGTGAAAGTTG
CTTCAAATTTTAGAAACTGTGAACATTCTGTGGAAATAAATCCTTATTATTTGAGCCTCTTTTTGAATTAAGGTGTCCAAACCCTT
TTGAATTAACATATGATCCAGCAATTTTACTTCTAGGTATGTACACCAAAGAATAGAAAACAGGGGCCAGGCGCGGTGGCTCACAC
CTGTAATCCCAGCACTTTGGGAGGCCGAGGCAGGCAGATCACTTGAGGTCAGGAGTTCAAGACCAGCCTGACCAACATGGAGAAAC
CCCGTCTCTACTAAAAAAAAAAAAAAAAAAAAAATTATCTATATATATATTTATACATATATATATATAAAATTAGCCAGGCATGG
TGGCGCATACCTGTCATCCCAGCTACTTGGGAGGCTGAGGCAGGAGAATCACCTGAACCCAGGAGGCAGAGGTTGTGGTGAGCTGA
GATTGCGCCTTTGCAGTCCAGACTGCGCAACAAGAGCGAAACTCAGTCTCAAAAAAAAAAAAAAGGAAAACAGAAACTTAAGCTGA
TATTTGCACACCAATAGCAGCATTATTCCCATAGACAAAAGGTGGAAACCAGCAATGACCATCTAAAAATAAATGGATAGGCCGGG
CGCCGTGGCTCAGCACTTTGGGAGGCTGAGGCAGGAGGATCACTTGAGGTCAGGAGCTTGAGACCAGCCTGGCCAACATGGTGAAA
CCCTGTCTCTACTAAAAATACAAAAATTAGCCAGGATTGGTGGCACGCACCTGTAATCCCAGTTAGTTACTCGGGAGGCTGAGACA
GGAGAACTGCTTGAACCCCAGAGGCGGAGGTTGCATGGGCTGAGATTGAGTCTATGTACTCCAGCTTGGGCAACAGAGCGAGACTC
CCCCTCAAAAAAAAAAAAAAAAAGGATAAACAGTTGGTCCGGGCGGCAGCTCATGCCTATAGTCCCAGCACTTTGGGAGGCCAAGA
TGGGAGGATCACTTGGGCCCAGGAGTTCGAGACCAGCCTGGGCAACATGGTGAGATCCTGTCTGTACGAAAAATACAGAAAGTATC
CAGCTGAAGTGGGAGGAGGATCACTTGAGGCCTGGAAGCAGAGGTTGAAGTGAGCTGAGATCGCGCCACTGCATTCCAGCCTGGGC
GACAGAGCCAAAAGCTGTCTCAAAAAAGAATGAATGGGACCGGGCGCGGTGGCTCACGGCTGTAATCCCAGCACTTTGGGAAACCA
AGGCGGGTGGATCACCTGAGGCCAGGAGTTCAAGACCAGCCTGACCAACATGGTGAAACCCCGTGTCTACTAAAAATAATCCCAGC
TCCTCGGGAGGCTGAGGCAGGAGAATCGCTTGAACCTGGGAGGCGGAGGTTGATCTCGCCATTGCGCTCCAGCCTGGGCAACAAAA
GCAAAACTTCGTCTCAAAAAAAAAAATAGACAAGAATAAATGGGCTGGGTGAAGTGGCTCACACCTGTAATTCCAGCACTTTGGGA
GGCCGAGGCAGGCAGATCACGTGAGGTCAGGAGTTTGAGACCAGCCTGGCCAACATGGTGAAATCCTGTCTCTACTAAAAATACAA
AGAGTGGTGGCATGCATCTGTAATCCCAGCTACTTGAGAGATGGAGGCAGGAGAATCGCTTGAATCCGGGAGGCAGTGGTTGCAAT
GAGCCGAGATCATGCCACTGCACTCCAGCCTGGGCAACAAGAGCAAAAACTCCATCTCAAAAAAAAAAAAAGATAACATCTCATAC
AGTGAAATATTATTCAGCCATAAAATTAATGAATTCCTGATATATACTACAACATGGATGAACCTTAAAAACATTATTCTGAATGA
AATAAGACTGACCTCAAGGACAAATATTGTATGATTCCATTTATATATATATAAAGTACTCACAAAGTCATAGAGATAGAAAGTAG
AAAAGAGGTTGCCACGGGGGCTGGAGGAGAAGGGAACAGGGAGTAAGGGTTTAATGGGTACAGAGTCTCAGTTTCCACCCTTGGGC
ATGAAAGATAAAGTTCTGGAGGTAAGTAGTGATGATGGTTACACAACAATGTGAACATATTCTATGCCAGTGAATTTACACTTAAG
GTTAAAATGGTAAATTTTATGTATACTTTGGCATAATTTAAAAAAAAGTTTGAAAAAGAAAAAAGACAAAACAATAATCGTCTTTG
GAGAAGTCTGTTACACTGAATCTGGAGCCAAGTGAACAGAACCCTGATTTTGATCCCTTTTCTCTCAAAAGCCCTTCGCAGTCTCT
GAATTAAGTCTATTAGCATGTTCCTCCCATAGTGCTTTGCTTCATATCAACAAAAACCTAGCTAAGTGAAATCAGCAACGATATGC
AGAAACCACCTACGCAGGTCACAAACATCTTTCTATGATTGTATAATTTTCAAGCAAGCAATAAGTGAAGATTTTTCCATAGGCCC
TAAACTCACCTTTGCGAAATAGGAAGCTGGTTTATTGGGAGTGATGAGCAGGGGGCGTAACAAATTAGCTTTGCGCAGTGGCAGTA
TCGTAGCCAATGAGGTCTATCCGAGGCGCGATTATTGCTAATTGAAAACTTTTCCCAATACCCCGCCGTGACGACTTGCAATATAG
TCGGCACTGGCAATTTTTGACAGTCTCTACGGAGACTGAATTTTCTTGCAGTTGAACAACAGAGCGCTTTTTTTGTGTGTGTGGGCG
TGCTTGGTTTTGGGAGGTTGAAGAGTACTTGTTCGCAAACTCTCTAAATGAGAGATGGGGAAGTGTCTTACCAGCTGATTCGCGAG
AGCACCTGCAAGTTTACCTGTTTTAGTGATTTGCGTTTATGAAAGTTGGAGTTGCTGATTTTGTGATCTCTACTTTTAAGGTAAAA
GCAGGGCGGTGTTGACTTTTCTTGCCAGTTGGCTGTCCTGTTTTCTTTCTCGCACTGTTTTCTTTTTGAGACGAAGTCTGTCGCTC
AGGCTGGAGTGCACTGGCGTGATCTCGGCTCGCTGCAAACTTTGCCGAACCCCTGTTTAGCGATTTCCGTTTGTGAAAGTTGGAGT
TGCTGATTTTGTGATCTCTACTTTTAAGGTAAATGCAGGGCGGTGTTGACTTTTCTTGCCAGCTGGCTGTCCTGTTTTCTTTCTCT

CGCACTGTTGTTTTCTTTTTGAGACGAAGTCTGTCGCTCAGGCTGGAGTGCACTGGCGCGATCTCGGCTCGCTGCAAACTGCCGCC
CGGGTTCAAGCGATTCTCGTGCCTCAGCCTCCCGACTAGCTGGGATTACAGGCGCCTGCCCACCGCACCCCGCTGATTTTTTTGTA
TTTTTAGTAGCGACCCCGTTTCACCATCTTGTCCAGGCTGGTCTTGAACTCCTGACCTCGTGATCCACCCGCCTCGGCCTCCCAGA
GTGCTGGGATTACAGGCGTGAGCCACCGCGCCCAGCCGTGTTCTCCATTCAGATTTTGTCACAGTGACGTCCTAAAATTATTCCCA
ACTCCCCCCACCCTGCTGTTTCCGCACCCTCGAGCACAGCTAGTCGTCCGTCTGCGCCCTCCAGGCTCGCTTTCCCACTGCTCACT
CTCCTCGCTCAAAATTCAAGTCTCAACCGAGTCATCTCTGTGACGTCACGTTGATTTGCATAAGATTCCCCAGCGTCCCAAGCGAA
TATTCTTATGGTTTTCAAAACCTGAATGTTTGACACGGGATGTTCCAACAACAAGAAACCTCCTATGCAGATGGGCCTTAAATACG
GCTGGTGGAGTGGGAACACGTCGTATACACGGACACACGGGCAGGCACTCACCCTCAATGTAATGGTAGTCATCATCCGTGGGGGA
GCGGGGCGCGAACAGAACCTTTCCAGCTTTGCGCAGTGGCAGTATCGTAGCCAATGAGGTTTATCCGAGGCGCGATTATTGCTAAT
TGAAAACTTTTCCCAATACCCCGCCATGACGACTTGAAATATAGTCGGCATTGGCAATTTTTGACAGTCTCTACGGAGACTGAATT
TTTAAAAGTCTAAAGAAAAGGGTTTTAGGAAGTTGTATTATAGCAGGTTGTGTCTTATGTTTGGTTTGATAAGTTATGAGCTGTTC
CTATGATAGTTTTTTGGTTGTTGTTCAGGAGTATTCCGTGATTGAATGTCCGGGGTTCTGTGCGGGTACTGTGAGTTGTTTTCCAA
AATGTATGACACTATGTTGGGAACTGGGTTCTAACTAAGACAGTTATCGTTAATCTTTTTTATCCAAATGCAGCCAGCGAGTGAGC
TACTCCACTTCCTTTGTCACTGAAGGTCACAGATTCCCTTAAAGGGAAGGGGAACTCTAAAACTAACACATTAATACATCCAAAGT
CTTCCTTTTTTTTTTTTTTTTTTTTTTTGAGACGGAGTTTTCGCTGTTTCCCAGGCTGGGGTACAGTGGCGCGATCTCGGCTGACT
GCAACCTCCACATTCCGGGTTCAAGCGATTCTCTGTCTCAGCCTCCGGAGTAGCTGGGATTACGGGAGCCCAACATCACGCCCGGC
TCATTTTTGTATTTTTAGTGGAGACGGGGTTTCGCCTTCTTGGCCAGGCTGGTTTCAAACTCCTGACCTCAGGTGATCCGCCTACC
TCGGCCTTCCGAAGTGCTGGGATTACAGGTGTGAACCACCGCGTTTTCTTCCTTTTAAAGCGACCAAGAGGGAAAAAGGCACAGAA
GATTGCTCAGGTCCTACTGTGGAGTCCTCGGCAGAGCTGGGGCTTATCAGGTTCTCTTGCCCCTAGTGTGGTACTTGTTCCCACTA
CACCAGCCTCTTCCAGTCTTACCTTTTTACGAAGACCTGGGCTGTCCATGGACAGAGGTCCTCAGGAGTCACAACTCTGGGCATGG
CGGATCCAGCTGGACCTGCTTAGGACACCGCCCATAAAAGACTCCTCCAGCCTGTAGCAAAAAGAGGTGGCAAAAAGGGTGGCAAC
AGTACCTACAAGGTCAAAGACGGTGCTGGGAAAAACAGCTCCAGTGGCAAATTAAACTCAGTCCCCAGTTCAAGGACCTCACAGAC
TGGAGACGTGGAGGCGAAGGTAGCAGTGAGCTGAAATTGCGCCACTGCACTCCAGCCTGGGTGACAGTGAGACTCTGTCTCAAAAA
AAAAGAAAAAAGAAAGAAGAAGAAGCCGGGAGCGGTGGCCCACGCCTGTCATCCCAGCACTTTGGGAGGCCAGGGCGGGCAGATCA
CGAGGTCAGGAGTTCAAGACCAGCCTGGCCAACATGGTGAAACCCTATCTCTACTAAAAATACAAAAAAAAAAAAAAAAAAAAAA
AATTAGCCAGCCATGGTCGTGGGCGCCTGTAATCCCAGCTACTCGGGAGGC

## Claims

1. A polypeptide encoded by an open reading frame in a long non-coding RNA gene (IncRNA), wherein the IncRNA is overexpressed in colorectal cancer cells relative to healthy cells, preferably wherein the IncRNA is defined according to any one of SEQ ID NOs 1 to 18.

2. A polypeptide comprising a 6-amino acid sequence, 7-amino acid sequence, or preferably an 8-amino acid sequence, or variant thereof, comprised within any one of the polypeptide sequences defined according to SEQ ID NOs 19 to 97.

3. The polypeptide according to claim 2, wherein the variant has at least 70%, at least 80% or at least 90% sequence identity with an 8-amino acid sequence comprised within any one of the polypeptide sequences defined according to SEQ ID NOs 19 to 97.

4. The polypeptide according to any one of the preceding claims, wherein the polypeptide comprises any one of the polypeptide sequences defined according to SEQ ID NOs 98 to 611.

5. The polypeptide according to any one of the preceding claims, wherein the polypeptide consists of a sequence of about 6 to 32 amino acids, preferably about 7 to 30 amino acids, and most preferably about 8 to 11 or about 12-28 amino acids.

6. A polynucleotide which encodes one or more polypeptides according to any one of the preceding claims, preferably wherein the polynucleotide is a DNA or mRNA molecule.

7. A vector comprising the polynucleotide according to claim 6.

8. A cell comprising the polynucleotide according to claim 6 and/or the vector according to claim 8, optionally wherein:
(i) the cell is a T cell which expresses a receptor that is specific for a polypeptide defined according to any one of claims 1 to 5, optionally wherein the T cell is engineered to express the receptor (e.g. it is a CAR-T) or wherein the T cell is raised against the said polypeptide; or
(ii) the cell is a dendritic cell (DC) matured ex vivo with a plurality of polypeptides defined according to any one of claims 1 to 5, optionally wherein:
a. the plurality of polypeptides comprises 2-20 different polypeptides; and/or
b. the one or more polypeptides are 8-11mers; and/or
c. each of the polypeptides are defined according to any 2-20 of SEQ ID NOs 98 to 611,
preferably wherein the maturation is by:
i. transfection with a plurality of mRNA molecules wherein each mRNA molecule carries the coding sequence of one of the plurality of polypeptides; or
ii. infection with a plurality of viral vectors wherein each viral vector molecule carries the coding sequence of one of the plurality of polypeptides.

9. A composition comprising the polypeptide according any one of claims 1 to 5, the polynucleotide according to claim 6, the vector according to claim 7, or the cell according to claim 8, and a pharmaceutically acceptable carrier.

10. The composition according to claim 9, wherein the composition comprises a plurality of the polypeptides according to any one of claim 1 to 5, optionally wherein:
i. the plurality comprises 2-20 different polypeptides; and/or
ii. the one or more polypeptides are 8-11mers; and/or
iii. each of the polypeptides are defined according to any 2-20 of SEQ ID NOs 98 to 611.

11. The composition according to claim 9, wherein the composition comprises the polynucleotide according to claim 6 and the polynucleotide is mRNA, optionally wherein:
i. the mRNA encodes a plurality of polypeptides according to any one of claims 1 to 5, preferably wherein the mRNA molecule encodes 2-20 different polypeptides; and/or
ii. the one or more polypeptides are 8-11mers; and/or
iii. each of the polypeptides are defined according to any 2-20 of SEQ ID NOs 98 to 611; and/or
iv. the mRNA molecule encodes a plurality of polypeptides according to any one of claims 1 to 5 in-frame to form a continuous polypeptide.

12. The composition according to claim 9, wherein the composition comprises the vector according to claim 7 and optionally wherein:
i. the vector is a viral vector; and/or
ii. the vector encodes a plurality of polypeptides according to any one of claims 1 to 5, preferably wherein the vector encodes 2-20 different polypeptides; and/or
iii. the one or more polypeptides are 8-11mers; and/or
iv. each of the polypeptides are defined according to any 2-20 of SEQ ID NOs 98 to 611; and/or
v. the vector molecule encodes a plurality of polypeptides according to any one of claims 1 to 5 in-frame to form a continuous polypeptide.

13. A method for treating or preventing colorectal cancer in an individual, the method comprising administering to the individual the polypeptide according to any one of claims 1 to 5, the polynucleotide according to claim 6, the vector according to claim 7, or the cell according to claim 8, or the composition according to any one of claims 9 to 12.

14. A method for eliciting an immune response against colorectal cancer in an individual, the method comprising administering to the individual the polypeptide according any one of claims 1 to 5, the polynucleotide according to claim 6, the vector according to claim 7, or the cell according to claim 8, or the composition according to any one of claims 9 to 12.

15. A method for diagnosing colorectal cancer in an individual, the method comprising analysing a sample taken from the individual for the presence of at least one of a IncRNA of any of SEQ ID NOs 1 to 18; a polypeptide of any of SEQ ID NOs 19 to 97; or a polypeptide of any one of SEQ ID NOs 98 to 611.
